(19) Europäisches Patentamt
European Patent Office
Office européen des brevets

(11) EP 4 541 791 A1

# (12) EUROPEAN PATENT APPLICATION

published in accordance with Art. 153(4) EPC

(43) Date of publication:
**23.04.2025 Bulletin 2025/17**

(21) Application number: **23823976.8**

(22) Date of filing: **15.06.2023**

(51) International Patent Classification (IPC):
***C07D 273/04*** (2006.01) ***A61K 31/5395*** (2006.01)
***A61K 31/55*** (2006.01) ***A61P 7/02*** (2006.01)
***A61P 35/00*** (2006.01) ***A61P 43/00*** (2006.01)
***C07D 413/04*** (2006.01) ***C07D 413/12*** (2006.01)
***C07D 413/14*** (2006.01) ***C07D 471/04*** (2006.01)
***C07D 487/04*** (2006.01) ***C07D 491/08*** (2006.01)
***C07D 491/107*** (2006.01) ***C07D 498/04*** (2006.01)
***C07D 498/10*** (2006.01)

(52) Cooperative Patent Classification (CPC):
**C07D 273/04; A61K 31/5395; A61K 31/55; A61P 7/02; A61P 35/00; A61P 43/00; C07D 401/14; C07D 413/04; C07D 413/10; C07D 413/12; C07D 413/14; C07D 471/04; C07D 487/04; C07D 491/08; C07D 491/107;**
(Cont.)

(86) International application number:
**PCT/JP2023/022253**

(87) International publication number:
**WO 2023/243686 (21.12.2023 Gazette 2023/51)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **16.06.2022 JP 2022097544**

(71) Applicant: **Japan Tobacco Inc.**
**Tokyo 105-6927 (JP)**

(72) Inventors:
- **YOKOTA, Masahiro**
  **Takatsuki-shi, Osaka 569-1125 (JP)**
- **KAYA, Tetsudo**
  **Takatsuki-shi, Osaka 569-1125 (JP)**
- **TORIZUKA, Makoto**
  **Takatsuki-shi, Osaka 569-1125 (JP)**
- **NAKAYAMA, Yasuaki**
  **Takatsuki-shi, Osaka 569-1125 (JP)**
- **IKENOGAMI, Taku**
  **Takatsuki-shi, Osaka 569-1125 (JP)**
- **MAEDA, Katsuya**
  **Takatsuki-shi, Osaka 569-1125 (JP)**
- **OTAKE, Kazuki**
  **Takatsuki-shi, Osaka 569-1125 (JP)**
- **SAKURAI, Kentaro**
  **Takatsuki-shi, Osaka 569-1125 (JP)**

(74) Representative: **Vossius & Partner Patentanwälte Rechtsanwälte mbB**
**Siebertstrasse 3**
**81675 München (DE)**

## (54) DIHYDROOXADIAZINONE COMPOUND AND PHARMACEUTICAL USE THEREOF

(57) The present invention provides a compound having a PLD inhibitory activity.

The present invention provides a compound of the following structural formula, and the like, or a pharmaceutically acceptable salt thereof.



Processed by Luminess, 75001 PARIS (FR)

(Cont. next page)

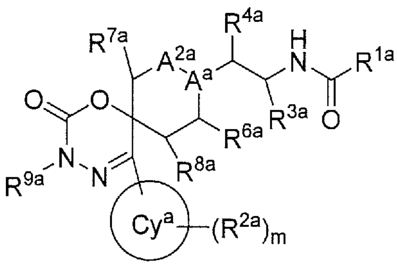

[ Ia ]

wherein each symbol is as defined in the description.

(52) Cooperative Patent Classification (CPC): (Cont.)
**C07D 498/04; C07D 498/08; C07D 498/10; C07D 519/00**

## Description

## Technical Field

The present invention relates to a dihydrooxadiazinone compound or a pharmaceutically acceptable salt thereof having a phospholipase D (hereinafter to be referred to as "PLD") inhibitory activity, a pharmaceutical composition comprising the same, and a pharmaceutical use thereof, and the like.

## Background Art

Three important factors for thrombus formation are said to be changes in blood components, changes in properties of vascular wall, and changes in blood flow. Arterial blood flow is laminar, faster in the center and slower near the vascular wall. The shear stress generated by this velocity difference exerts a strong influence on platelets flowing near the vascular wall, and enhances the platelet aggregation ability. Thrombus formation in arteries is largely triggered by arteriosclerotic plaque rupture. The reaction of the platelet membrane receptor with collagen in the subendothelial tissue exposed with arteriosclerotic plaque rupture, or von Willebrand factor (vWF) adhering to the collagen, triggers platelet activation signalling, which in turn causes platelet aggregation, thereby initiating thrombus formation. Thus, platelet aggregation initiated at the site of plaque rupture in coronary arteries and cerebral arteries may cause rapid thrombotic occlusion, resulting in the development of myocardial infarction and stroke. At the site of atherosclerotic stenosis, and the like, where these pathologies are thought to occur, it is assumed that extremely high shear stress is generated. Recent studies using an in vitro flow chamber incorporating a blood flow environment have revealed that platelet adhesion and aggregation mechanisms differ depending on the shear stress. Under high shear stress, platelet adhesion and aggregation reactions proceed by mechanisms completely different from the classical concepts established in conventional static systems and closed stirring experimental systems (Non-Patent Documents 1, 2, 3 and 4).

The primary role of thrombus formation in vivo is a hemostatic mechanism to prevent blood leakage. As the first step in hemostatic mechanism, platelets adhere to the site of damaged vascular wall (primary hemostasis). The next step following platelet adhesion is platelet aggregation reaction. Platelets firmly adhering to the damaged vascular wall capture and combine with platelets supplied through the bloodstream one after another, resulting in spatial growth of the platelet thrombus. There are many factors that activate platelets and induce platelet aggregation. Regardless of the type of triggering factor, the final reaction is cross-linking of platelets to each other by binding of fibrinogen or vWF to activated integrin $\alpha$IIb$\beta$3 (glycoprotein GPIIb/IIIa, GPIIb/IIIa) on the platelet membrane. Since all of existing anti-platelet drugs represented by aspirin and clopidogrel have an action of inhibiting signals leading to platelet activation and impairing the primary hemostatic function, the drugs carry a bleeding risk as an extension of their anti-thrombotic action (Non-Patent Document 5). As mentioned above, thrombus formation at the site of atheromatous stenosis may be mainly caused by platelet adhesion and aggregation due to high shear stress. Therefore, drugs that can inhibit only platelet aggregation reaction under high shear stress are expected to have a lower bleeding risk than existing drugs.

PLD1 and PLD2 are known as major isoforms of PLD. PLD1 is encoded by the gene of the same name, and its expression has been confirmed throughout the human body. PLD1 is a phospholipid-metabolizing enzyme that hydrolyzes a biomembrane-constituting phospholipid, phosphatidylcholine (PC) to produce phosphatidic acid (PA). Since PA produced by PLD1 acts as a signaling molecule, PLD1 is thought to regulate various cellular functions via PA. In addition, PLD2 exists as an enzyme that hydrolyzes PC into PA and choline in cells like PLD1. PLD2 is also encoded by the gene of the same name, and its expression has also been confirmed throughout the human body. PLD activity confirmed in mammalian cells and tissues is thought to be due to these PLD1 and PLD2 (Non-Patent Document 6).

As a result of phenotypic analysis using PLD1 knockout mice, it was clarified that the mice exhibit an anti-thrombotic effect without showing a bleeding risk. In addition, as a result of phenotypic analysis using PLD1/2 dual knockout mice, it was shown that, in addition to the phenotype of PLD1 knockout mice, they also have an inhibitory effect on $\alpha$-granule secretion from platelets. As a result of analysis using these mouse-derived platelets, it was shown that suppression of PLD1 and PLD2 inhibits platelet aggregation under high shear stress, but does not affect platelet aggregation induced by soluble agonists, which are shown to inhibit by existing anti-platelet drugs (Non-Patent Documents 7 and 8).

Both PLD1 and PLD2 are reported to contribute to various phenomena such as cell proliferation, survival, metabolism, migration and membrane fusion (Non-Patent Documents 9, 10 and 11). Activation of PLD1 and PLD2 is thought to contribute to multiple pathologies including cancers (Non-Patent Documents 12 and 13), and their expression and increased activity have been detected in various human cancers in the colon, breast, stomach, thyroid, brain, kidney, uterine smooth muscle and the like (Non-Patent Documents 11, 12 and 13). In addition, there are many reports that PLD1 and PLD2 enzymes are directly involved in transformation and tumorigenesis in various cancer types such as breast cancer, ovarian cancer, lung cancer, colorectal cancer, kidney cancer, pancreatic cancer, prostate cancer, and brain tumor (Non-Patent Documents 11, 12 and 13). In studies using PLD1 and PLD2 knockout mice and their respective inhibitors, it

has been confirmed that the growth of tumor tissue is suppressed (Non-Patent Documents 13 and 14).

In view of the above, PLD inhibitors are considered to be therapeutic agents (e.g., drugs for secondary prevention, drugs for relapse prevention, drugs for exacerbation prevention, etc.) or prophylactic agents for thrombosis (e.g., arterial thrombosis, acute coronary syndrome, stable angina, unstable angina, non-ST elevation myocardial infarction, ST elevation myocardial infarction, ischemic stroke, non-cardioembolic stroke, atherosclerotic stroke, cryptogenic stroke, embolic stroke of undetermined source (ESUS), lacunar stroke, transient ischemic attack, peripheral arterial disease, etc.), and thrombosis during perioperative and postoperative periods associated with revascularization (coronary artery bypass graft, percutaneous coronary intervention, carotid endarterectomy, carotid artery stenting, thrombolysis, lower extremity revascularization, etc.) or aortic valve replacement (surgical aortic valve replacement, transcatheter aortic valve replacement, etc.). Moreover, PLD inhibitors are also considered to be useful as therapeutic agents for cancers (e.g., breast cancer, ovarian cancer, lung cancer, colorectal cancer, kidney cancer, pancreatic cancer, prostate cancer, brain tumor, etc.).

## Document List

### Non-Patent Document

[Non-Patent Document 1] Cell. 1996 Jan 26; 84(2): 289-297
[Non-Patent Document 2] J Thromb Haemost. 2014; 12(3): 418-420
[Non-Patent Document 3] Nat Med. 2009 Jun; 15(6): 665-673
[Non-Patent Document 4] The Journal of Japanese College of Angiology. 2011; 51(3): 275-281
[Non-Patent Document 5] Thromb Haemost. 2010 Jun; 103(6):1128-1135
[Non-Patent Document 6] Chem Rev. 2011 Oct 12;111(10):6064-119.
[Non-Patent Document 7] Sci Signal. 2010 Jan 5; 3(103): ra1
[Non-Patent Document 8] J Thromb Haemost. 2012 Nov;10(11):2361-72
[Non-Patent Document 9] J Biol Chem. 2014 Aug 15; 289(33): 22567-22574
[Non-Patent Document 10] J Biol Chem. 2014 Aug 15;289 (33) :22557-22566.
[Non-Patent Document 11] J Biol Chem. 2014; 289(33): 22575-22582
[Non-Patent Document 12] Pharmacol Rev. 2014 Oct; 66(4): 1033-1079
[Non-Patent Document 13] Nat Rev Drug Discov. 2017 May; 16(5):351-367
[Non-Patent Document 14] Adv Biol Regul. 2018 Jan;67:134-140.

## Summary of Invention

The present invention provides a dihydrooxadiazinone compound or a pharmaceutically acceptable salt thereof having a PLD inhibitory activity, a pharmaceutical composition comprising the same, and a pharmaceutical use thereof. Accordingly, the present invention includes the embodiments exemplified below.

[Item 1] A compound represented by Formula [I] or a pharmaceutically acceptable salt thereof:

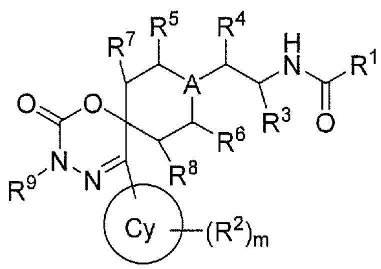

[ I ]

wherein

A is $CR^{10}$ or N;
Cy is

(1) $C_{6-10}$ aryl,
(2) 5- or 6-membered heteroaryl containing one or two nitrogen atoms as a ring constituting atom, besides carbon atom, or
(3) a 9- or 10-membered partially unsaturated fused cyclic group containing one or two oxygen atoms as a ring constituting atom, besides carbon atom;

$R^1$ is

(1) $C_{1-6}$ alkyl wherein the alkyl is optionally substituted by

(a) hydroxy,
(b) cyano,
(c) $SO_2R^{11}$ wherein $R^{11}$ is $C_{1-4}$ alkyl, or
(d) $NHCOR^{12}$ wherein $R^{12}$ is $C_{1-4}$ alkyl,

(2) $C_{2-4}$ alkenyl wherein the alkenyl is optionally substituted by $NR^{13}R^{14}$ wherein $R^{13}$ and $R^{14}$ are each independently hydrogen or $C_{1-4}$ alkyl,
(3) $C_{1-4}$ haloalkyl,
(4) $C_{1-6}$ alkoxy wherein the alkoxy is optionally substituted by phenyl,
(5) $NR^{15}R^{16}$ wherein $R^{15}$ and $R^{16}$ are each independently

(a) hydrogen,
(b) $C_{1-4}$ alkyl wherein the alkyl is optionally substituted by

(i) phenyl wherein the phenyl is optionally substituted by halogen, or
(ii) pyridyl,

(c) $C_{1-4}$ alkoxy, or
(d) $C_{3-4}$ cycloalkyl,

(6) $COR^{17}$ wherein $R^{17}$ is $C_{1-4}$ alkyl,
(7) $CONR^{18}R^{19}$ wherein $R^{18}$ and $R^{19}$ are each independently

(a) hydrogen,
(b) $C_{1-4}$ alkyl,
(c) $C_{3-4}$ cycloalkyl, or
(d) $C_{5-8}$ bridged cycloalkyl,

(8) $C_{3-4}$ cycloalkyl wherein the cycloalkyl is optionally substituted by

(a) hydroxy,
(b) halogen, or
(c) phenyl,

(9) 4 to 7-membered heterocycloalkyl containing one or two heteroatoms selected from the group consisting of nitrogen and oxygen atoms as a ring constituting atom, besides carbon atom, wherein the heterocycloalkyl is optionally substituted by

(a) hydroxy,
(b) oxo,
(c) $NR^{20}R^{21}$ wherein $R^{20}$ and $R^{21}$ are each independently hydrogen or $C_{1-4}$ alkyl, or
(d) phenyl,

(10) 6 to 11-membered spiro heterocycloalkyl containing one or two heteroatoms selected from the group consisting of nitrogen and oxygen atoms as a ring constituting atom, besides carbon atom,
(11) phenyl wherein the phenyl is optionally substituted by one or two halogens,
(12) 5 to 10-membered heteroaryl containing 1 to 3 heteroatoms selected from the group consisting of nitrogen and oxygen atoms as a ring constituting atom, besides carbon atom, wherein the heteroaryl is

optionally substituted by one or two $R^{22}$, or

(13) a 8 to 10-membered saturated or partially unsaturated fused cyclic group containing 1 to 4 heteroatoms selected from the group consisting of nitrogen and oxygen atoms as a ring constituting atom, besides carbon atom, wherein the fused cyclic group is optionally substituted by $C_{1-4}$ haloalkyl;

$R^2$ in the number of m are each independently

(1) hydroxy,
(2) cyano,
(3) halogen,
(4) $C_{1-6}$ alkyl wherein the alkyl is optionally substituted by

(a) hydroxy, or
(b) $C_{3-4}$ cycloalkyl,

(5) $C_{2-4}$ alkenyl wherein the alkenyl is optionally substituted by $C_{1-4}$ alkoxy,
(6) $C_{1-4}$ haloalkyl,
(7) $C_{1-4}$ alkoxy wherein the alkoxy is optionally substituted by 1 to 3 substituents selected from the group consisting of

(a) hydroxy, and
(b) halogen,

(8) $SR^{23}$ wherein $R^{23}$ is $C_{1-4}$ alkyl,
(9) $COR^{24}$ wherein $R^{24}$ is hydroxy or $C_{1-4}$ alkyl,
(10) $CONR^{25}R^{26}$ wherein $R^{25}$ and $R^{26}$ are each independently

(a) hydrogen,
(b) $C_{1-6}$ alkyl, or
(c) $C_{3-4}$ cycloalkyl,

(11) $SO_2R^{27}$ wherein $R^{27}$ is $C_{1-6}$ alkyl,
(12) $C_{3-4}$ cycloalkyl,
(13) 4 to 7-membered heterocycloalkyl containing one or two heteroatoms selected from the group consisting of nitrogen and oxygen atoms as a ring constituting atom, besides carbon atom, wherein the heterocycloalkyl is optionally substituted by one or two substituents selected from the group consisting of

(a) halogen,
(b) $C_{1-4}$ alkyl, and
(c) $C_{1-4}$ haloalkyl,

(14) 5 to 9-membered bridged heterocycloalkyl containing one or two heteroatoms selected from the group consisting of nitrogen and oxygen atoms as a ring constituting atom, besides carbon atom,
(15) 6 to 11-membered spiro heterocycloalkyl containing one or two heteroatoms selected from the group consisting of nitrogen and oxygen atoms as a ring constituting atom, besides carbon atom, or
(16) phenyl;

$R^3$, $R^4$, $R^5$ and $R^6$ are each independently hydrogen or $C_{1-4}$ alkyl;
$R^7$ and $R^8$ are both hydrogens, or $R^7$ and $R^8$ are bonded to each other to form a cyclopentane ring together with the carbon atoms to which they are bonded;
$R^9$ is hydrogen or $CONHR^{28}$ wherein $R^{28}$ is $C_{3-4}$ cycloalkyl;
$R^{10}$ is

(1) hydrogen,
(2) hydroxy, or
(3) halogen;

one or two $R^{22}$ are each independently

(1) halogen,
(2) $C_{1-4}$ alkyl,
(3) $C_{1-4}$ haloalkyl,
(4) $C_{1-4}$ alkoxy,
(5) $NHCOR^{29}$ wherein $R^{29}$ is $C_{1-4}$ alkyl, or
(6) $SO_2R^{30}$ wherein $R^{30}$ is $C_{1-4}$ alkyl; and

m is 0, 1, 2 or 3.

[Item 2] The compound according to Item 1 or a pharmaceutically acceptable salt thereof, wherein $R^9$ is hydrogen.
[Item 3] The compound according to Item 1 or 2, or a pharmaceutically acceptable salt thereof, wherein A is N.
[Item 4] The compound according to Item 1 or 2, or a pharmaceutically acceptable salt thereof, wherein A is $CR^{10}$ wherein $R^{10}$ is as defined in Item 1.
[Item 5] The compound according to Item 4, or a pharmaceutically acceptable salt thereof, which is represented by Formula [II]:

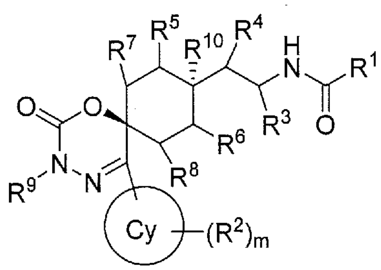

[ II ]

wherein
Cy, $R^1$, $R^2$, $R^3$, $R^4$, $R^5$, $R^6$, $R^7$, $R^8$, $R^9$, $R^{10}$ and m are as defined in Item 1.
[Item 6] The compound according to any one of Items 1 to 5, or a pharmaceutically acceptable salt thereof, wherein $R^3$ is $C_{1-4}$ alkyl.
[Item 7] A pharmaceutical composition comprising a compound as defined in any one of Items 1 to 6, or a pharmaceutically acceptable salt thereof, and a pharmaceutically acceptable carrier.
[Item 8] A PLD1 inhibitor comprising a compound as defined in any one of Items 1 to 6, or a pharmaceutically acceptable salt thereof.
[Item 9] An agent for the treatment or prophylaxis of a disease selected from the group consisting of thrombosis and cancer, comprising a compound as defined in any one of Items 1 to 6, or a pharmaceutically acceptable salt thereof.
[Item 10] A method for inhibiting PLD1 in a mammal, comprising administering a therapeutically effective amount of a compound as defined in any one of Items 1 to 6, or a pharmaceutically acceptable salt thereof to the mammal.
[Item 11] A method for treating or preventing a disease selected from the group consisting of thrombosis and cancer in a mammal, comprising administering a therapeutically effective amount of a compound as defined in any one of Items 1 to 6, or a pharmaceutically acceptable salt thereof to the mammal.
[Item 12] Use of a compound as defined in any one of Items 1 to 6, or a pharmaceutically acceptable salt thereof, for the manufacture of a PLD1 inhibitor.
[Item 13] Use of a compound as defined in any one of Items 1 to 6, or a pharmaceutically acceptable salt thereof, for the manufacture of an agent for the treatment or prophylaxis of a disease selected from the group consisting of thrombosis and cancer.
[Item 14] A compound as defined in any one of Items 1 to 6, or a pharmaceutically acceptable salt thereof, for use in the inhibition of PLD1.
[Item 15] A compound as defined in any one of Items 1 to 6, or a pharmaceutically acceptable salt thereof, for use in the treatment or prophylaxis of a disease selected from the group consisting of thrombosis and cancer.
[Item 1a] A compound represented by Formula [Ia] or a pharmaceutically acceptable salt thereof:

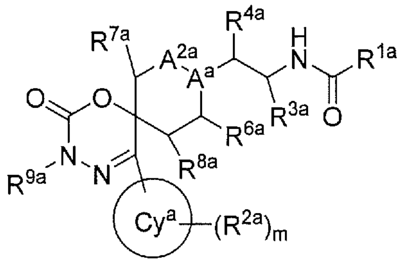

[ Ia ]

wherein

$A^a$ is $CR^{10a}$ or N;
$A^{2a}$ is $CR^{5a}$ or O;
$Cy^a$ is

(1) $C_{6-10}$ aryl,
(2) 5 to 10-membered heteroaryl containing one or two nitrogen atoms as a ring constituting atom, besides carbon atom, or
(3) a 9- or 10-membered partially unsaturated fused cyclic group containing one or two oxygen atoms as a ring constituting atom, besides carbon atom;

$R^{1a}$ is

(1) $C_{1-6}$ alkyl wherein the alkyl is optionally substituted by

(a) hydroxy,
(b) cyano,
(c) $SO_2R^{11}$ wherein $R^{11}$ is $C_{1-4}$ alkyl, or
(d) $NHCOR^{12}$ wherein $R^{12}$ is $C_{1-4}$ alkyl,

(2) $C_{2-4}$ alkenyl wherein the alkenyl is optionally substituted by 1 to 3 of

(a) $NR^{13}R^{14}$ wherein $R^{13}$ and $R^{14}$ are each independently hydrogen or $C_{1-4}$ alkyl,
(b) halogen,
(c) $COR^{35a}$ wherein $R^{35a}$ is hydroxy or $C_{1-4}$ alkoxy,
(d) $CONR^{36a}R^{37a}$ wherein $R^{36a}$ and $R^{37a}$ are each independently hydrogen or $C_{1-4}$ alkyl, or
(e) a partial structural formula:

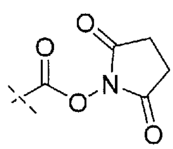

,

(3) $C_{1-4}$ haloalkyl wherein the haloalkyl is optionally substituted by hydroxy,
(4) $C_{1-6}$ alkoxy wherein the alkoxy is optionally substituted by phenyl,
(5) $NR^{15}R^{16}$ wherein $R^{15}$ and $R^{16}$ are each independently

(a) hydrogen,
(b) $C_{1-4}$ alkyl wherein the alkyl is optionally substituted by

(i) phenyl wherein the phenyl is optionally substituted by halogen, or
(ii) pyridyl,

(c) $C_{1-4}$ alkoxy, or
(d) $C_{3-4}$ cycloalkyl,

(6) $COR^{17a}$ wherein $R^{17a}$ is $C_{1-4}$ alkyl or hydroxy,
(7) $CONR^{18a}R^{19a}$ wherein $R^{18a}$ and $R^{19a}$ are each independently

(a) hydrogen,
(b) $C_{1-4}$ alkyl wherein the alkyl is optionally substituted by hydroxy,
(c) $C_{3-4}$ cycloalkyl,
(d) $C_{5-8}$ bridged cycloalkyl,
(e) $C_{1-4}$ haloalkyl, or
(f) $C_{1-4}$ alkoxy,

(8) $C_{3-4}$ cycloalkyl wherein the cycloalkyl is optionally substituted by

(a) hydroxy,
(b) halogen, or
(c) phenyl,

(9) 4 to 7-membered heterocycloalkyl containing one or two heteroatoms selected from the group consisting of nitrogen and oxygen atoms as a ring constituting atom, besides carbon atom, wherein the heterocycloalkyl is optionally substituted by

(a) hydroxy,
(b) oxo,
(c) $NR^{20}R^{21}$ wherein $R^{20}$ and $R^{21}$ are each independently hydrogen or $C_{1-4}$ alkyl, or
(d) phenyl,

(10) 6 to 11-membered spiro heterocycloalkyl containing one or two heteroatoms selected from the group consisting of nitrogen and oxygen atoms as a ring constituting atom, besides carbon atom,
(11) phenyl wherein the phenyl is optionally substituted by one or two of

(a) halogen, or
(b) $C_{1-4}$ haloalkyl,

(12) 5 to 10-membered heteroaryl containing 1 to 3 heteroatoms selected from the group consisting of nitrogen and oxygen atoms as a ring constituting atom, besides carbon atom, wherein the heteroaryl is optionally substituted by one or two $R^{22a}$,
(13) a 8 to 10-membered saturated or partially unsaturated fused cyclic group containing 1 to 4 heteroatoms selected from the group consisting of nitrogen and oxygen atoms as a ring constituting atom, besides carbon atom, wherein the fused cyclic group is optionally substituted by $C_{1-4}$ haloalkyl, or
(14) a 5 to 7-membered partially unsaturated cyclic group containing one or two nitrogen atoms as a ring constituting atom, besides carbon atom, wherein the partially unsaturated cyclic group is optionally substituted by an oxo group and $C_{1-4}$ alkyl;

$R^{2a}$ in the number of m are each independently

(1) hydroxy,
(2) cyano,
(3) halogen,
(4) $C_{1-6}$ alkyl wherein the alkyl is optionally substituted by

(a) hydroxy, or
(b) $C_{3-4}$ cycloalkyl,

(5) $C_{2-4}$ alkenyl wherein the alkenyl is optionally substituted by $C_{1-4}$ alkoxy,
(6) $C_{1-4}$ haloalkyl,
(7) $C_{1-4}$ alkoxy wherein the alkoxy is optionally substituted by 1 to 3 substituents selected from the group consisting of

(a) hydroxy, and

(b) halogen,

(8) $SR^{23a}$ wherein $R^{23a}$ is $C_{1-4}$ alkyl or $C_{1-4}$ haloalkyl,
(9) $COR^{24a}$ wherein $R^{24a}$ is

(a) hydroxy,
(b) $C_{1-4}$ alkyl, or
(c) $C_{1-4}$ alkoxy,

(10) $CONR^{25a}R^{26a}$ wherein $R^{25a}$ and $R^{26a}$ are each independently

(a) hydrogen,
(b) $C_{1-6}$ alkyl, or
(c) $C_{3-4}$ cycloalkyl, or

$R^{25a}$ and $R^{26a}$ are bonded to each other to form 4 to 7-membered heterocycloalkyl together with the nitrogen atom to which they are bonded, wherein the heterocycloalkyl contains one or two heteroatoms selected from the group consisting of nitrogen and oxygen atoms as a ring constituting atom, besides carbon atom, and is optionally substituted by one or two halogens,
(11) $SO_2R^{27}$ wherein $R^{27}$ is $C_{1-6}$ alkyl,
(12) $C_{3-4}$ cycloalkyl,
(13) 4 to 7-membered heterocycloalkyl containing one or two heteroatoms selected from the group consisting of nitrogen and oxygen atoms as a ring constituting atom, besides carbon atom, wherein the heterocycloalkyl is optionally substituted by one or two substituents selected from the group consisting of

(a) halogen,
(b) $C_{1-4}$ alkyl, and
(c) $C_{1-4}$ haloalkyl,

(14) 5 to 9-membered bridged heterocycloalkyl containing one or two heteroatoms selected from the group consisting of nitrogen and oxygen atoms as a ring constituting atom, besides carbon atom,
(15) 6 to 11-membered spiro heterocycloalkyl containing one or two heteroatoms selected from the group consisting of nitrogen and oxygen atoms as a ring constituting atom, besides carbon atom, or
(16) phenyl;

$R^{3a}$ is

(1) hydrogen,
(2) $C_{1-4}$ alkyl, or
(3) $C_{1-4}$ haloalkyl;

$R^{4a}$ is

(1) hydrogen,
(2) $C_{1-4}$ alkyl, or
(3) cyano;

$R^{5a}$ is hydrogen or $C_{1-4}$ alkyl;
the combination of $R^{6a}$, $R^{7a}$ and $R^{8a}$ is

(1) a combination where $R^{6a}$ is hydrogen or $C_{1-4}$ alkyl, and $R^{7a}$ and $R^{8a}$ are both hydrogens,
(2) a combination where $R^{6a}$ is hydrogen or $C_{1-4}$ alkyl, and $R^{7a}$ and $R^{8a}$ are bonded to each other to form a cyclopentane ring together with the spiro carbon atom and the carbon atoms to which they are bonded, or
(3) a combination where $R^{6a}$ and $R^{7a}$ are bonded to each other to form a cyclopentane ring together with the spiro carbon atom and the carbon atoms to which they are bonded, and $R^{8a}$ is hydrogen;

$R^{9a}$ is

(1) hydrogen,
(2) $CONHR^{28}$ wherein $R^{28}$ is $C_{3-4}$ cycloalkyl, or
(3) $C_{1-4}$ alkyl;

$R^{10a}$ is

(1) hydrogen,
(2) hydroxy,
(3) halogen,
(4) $C_{1-4}$ alkyl,
(5) cyano, or
(6) $C_{1-4}$ alkoxy;

one or two $R^{22a}$ are each independently

(1) halogen,
(2) $C_{1-4}$ alkyl,
(3) $C_{1-4}$ haloalkyl,
(4) $C_{1-4}$ alkoxy,
(5) $NHCOR^{29}$ wherein $R^{29}$ is $C_{1-4}$ alkyl,
(6) $SO_2R^{30}$ wherein $R^{30}$ is $C_{1-4}$ alkyl,
(7) cyano, or
(8) $C_{3-4}$ cycloalkyl; and

m is 0, 1, 2 or 3.

[Item 2a] The compound according to Item 1a or a pharmaceutically acceptable salt thereof, which is represented by Formula [IIa]:

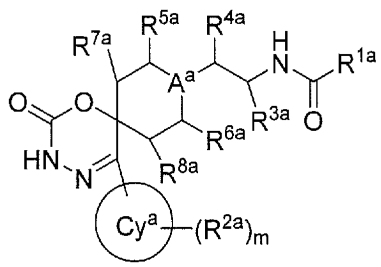

[ IIa ]

wherein
$A^a$, $Cy^a$, $R^{1a}$, $R^{2a}$, $R^{3a}$, $R^{4a}$, $R^{5a}$, $R^{6a}$, $R^{7a}$, $R^{8a}$ and m are as defined in Item 1a.
[Item 3a] The compound according to Item 1a or a pharmaceutically acceptable salt thereof, which is represented by Formula [IIIa]:

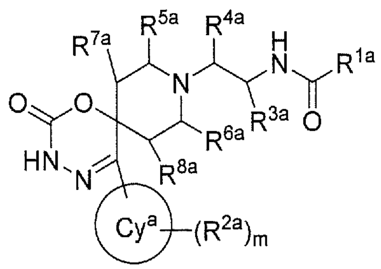

[ IIIa ]

wherein
Cya, $R^{1a}$, $R^{2a}$, $R^{3a}$, $R^{4a}$, $R^{5a}$, $R^{6a}$, $R^{7a}$, $R^{8a}$ and m are as defined in Item 1a.
[Item 4a] The compound according to Item 1a or a pharmaceutically acceptable salt thereof, which is represented by Formula [IVa]:

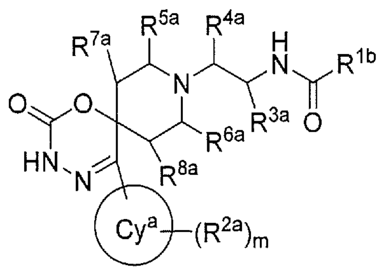

[ IVa ]

wherein

$R^{1b}$ is

(1) $C_{1-6}$ alkyl wherein the alkyl is optionally substituted by

(a) hydroxy,
(b) cyano,
(c) $SO_2R^{11}$ wherein $R^{11}$ is $C_{1-4}$ alkyl, or
(d) $NHCOR^{12}$ wherein $R^{12}$ is $C_{1-4}$ alkyl,

(2) $C_{2-4}$ alkenyl wherein the alkenyl is optionally substituted by 1 to 3 of

(a) $NR^{13}R^{14}$ wherein $R^{13}$ and $R^{14}$ are each independently hydrogen or $C_{1-4}$ alkyl,
(b) halogen,
(c) $COR^{35a}$ wherein $R^{35a}$ is hydroxy or $C_{1-4}$ alkoxy,
(d) $CONR^{36a}R^{37a}$ wherein $R^{36a}$ and $R^{37a}$ are each independently hydrogen or $C_{1-4}$ alkyl, or
(e) a partial structural formula:

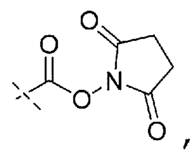

(3) $C_{1-6}$ alkoxy wherein the alkoxy is optionally substituted by phenyl,
(4) $NR^{15}R^{16}$ wherein $R^{15}$ and $R^{16}$ are each independently

(a) hydrogen,
(b) $C_{1-4}$ alkyl wherein the alkyl is optionally substituted by

(i) phenyl wherein the phenyl is optionally substituted by halogen, or
(ii) pyridyl,

(c) $C_{1-4}$ alkoxy, or
(d) $C_{3-4}$ cycloalkyl,

(5) $CONR^{18a}R^{19a}$ wherein $R^{18a}$ and $R^{19a}$ are each independently

(a) hydrogen,
(b) $C_{1-4}$ alkyl wherein the alkyl is optionally substituted by hydroxy,
(c) $C_{3-4}$ cycloalkyl,

(d) $C_{5-8}$ bridged cycloalkyl,
(e) $C_{1-4}$ haloalkyl, or
(f) $C_{1-4}$ alkoxy,

(6) 6 to 11-membered spiro heterocycloalkyl containing one or two heteroatoms selected from the group consisting of nitrogen and oxygen atoms as a ring constituting atom, besides carbon atom,
(7) 5 to 10-membered heteroaryl containing 1 to 3 heteroatoms selected from the group consisting of nitrogen and oxygen atoms as a ring constituting atom, besides carbon atom, wherein the heteroaryl is optionally substituted by one or two $R^{22a}$,
(8) a 8 to 10-membered saturated or partially unsaturated fused cyclic group containing 1 to 4 heteroatoms selected from the group consisting of nitrogen and oxygen atoms as a ring constituting atom, besides carbon atom, wherein the fused cyclic group is optionally substituted by $C_{1-4}$ haloalkyl, or
(9) a 5 to 7-membered partially unsaturated cyclic group containing one or two nitrogen atoms as a ring constituting atom, besides carbon atom, wherein the partially unsaturated cyclic group is optionally substituted by an oxo group and $C_{1-4}$ alkyl; and

$Cy^a$, $R^{2a}$, $R^{3a}$, $R^{4a}$, $R^{5a}$, $R^{6a}$, $R^{7a}$, $R^{8a}$, $R^{22a}$ and m are as defined in Item 1a.

[Item 5a] The compound according to Item 4a or a pharmaceutically acceptable salt thereof, which is represented by Formula [Va]:

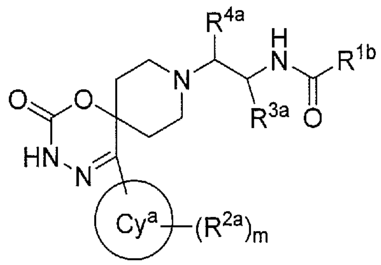

[ Va ]

wherein
$Cy^a$, $R^{2a}$, $R^{3a}$, $R^{4a}$ and m are as defined in Item 1a; and $R^{1b}$ is as defined in Item 4a.
[Item 6a] The compound according to Item 4a or a pharmaceutically acceptable salt thereof, which is represented by Formula [VIa]:

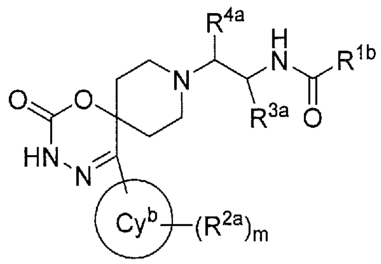

[ VIa ]

wherein

$Cy^b$ is

(1) $C_{6-10}$ aryl, or
(2) 5- or 6-membered heteroaryl containing one or two nitrogen atoms as a ring constituting atom, besides carbon atom;

$R^{1b}$ is as defined in Item 4a; and

$R^{2a}$, $R^{3a}$, $R^{4a}$ and m are as defined in Item 1a.

[Item 7a] The compound according to Item 4a or a pharmaceutically acceptable salt thereof, which is represented by Formula [VIIa]:

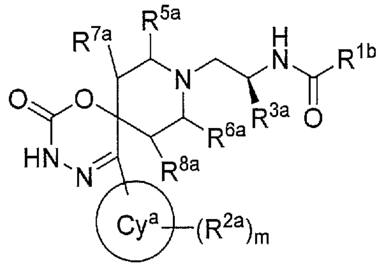

[ VIIa ]

wherein

$Cy^a$, $R^{2a}$, $R^{3a}$, $R^{5a}$, $R^{6a}$, $R^{7a}$, $R^{8a}$ and m are as defined in Item 1a; and
$R^{1b}$ is as defined in Item 4a.

[Item 8a] The compound according to Item 4a or a pharmaceutically acceptable salt thereof, which is represented by Formula [VIIIa]:

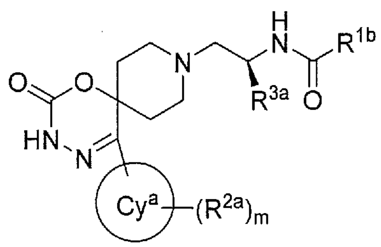

[ VIIIa ]

wherein

$Cy^a$, $R^{2a}$, $R^{3a}$ and m are as defined in Item 1a; and
$R^{1b}$ is as defined in Item 4a.

[Item 9a] The compound according to Item 6a or a pharmaceutically acceptable salt thereof, which is represented by Formula [IXa]:

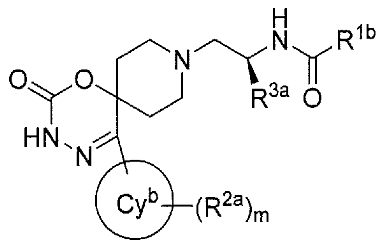

[ IXa ]

wherein

$Cy^b$ is as defined in Item 6a;
$R^{1b}$ is as defined in Item 4a; and

$R^{2a}$, $R^{3a}$ and m are as defined in Item 1a.

[Item 10a]

The compound according to Item 1a or a pharmaceutically acceptable salt thereof, which is represented by Formula [Xa]:

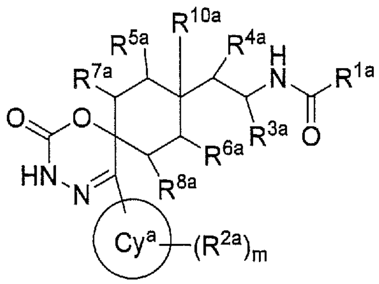

[ Xa ]

wherein

$Cy^a$, $R^{1a}$, $R^{2a}$, $R^{3a}$, $R^{4a}$, $R^{5a}$, $R^{6a}$, $R^{7a}$, $R^{8a}$, $R^{10a}$ and m are as defined in Item 1a.

[Item 11a] The compound according to Item 1a or a pharmaceutically acceptable salt thereof, which is represented by Formula [XIa]:

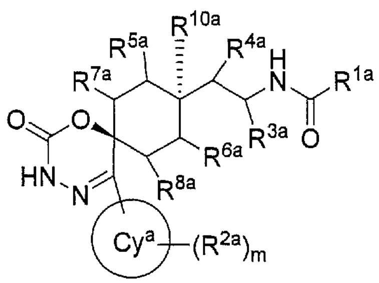

[ XIa ]

wherein

$Cy^a$, $R^{1a}$, $R^{2a}$, $R^{3a}$, $R^{4a}$, $R^{5a}$, $R^{6a}$, $R^{7a}$, $R^{8a}$, $R^{10a}$ and m are as defined in Item 1a.

[Item 12a] The compound according to Item 1a or a pharmaceutically acceptable salt thereof, which is represented by Formula [XIIa] :

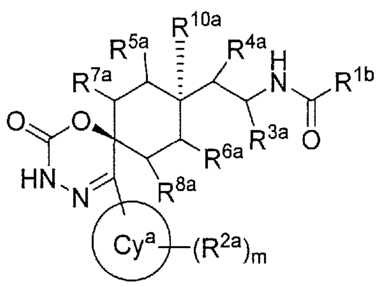

[ XIIa ]

wherein

$R^{1b}$ is

(1) $C_{1-6}$ alkyl wherein the alkyl is optionally substituted by

(a) hydroxy,

(b) cyano,
(c) $SO_2R^{11}$ wherein $R^{11}$ is $C_{1-4}$ alkyl, or
(d) $NHCOR^{12}$ wherein $R^{12}$ is $C_{1-4}$ alkyl,

(2) $C_{2-4}$ alkenyl wherein the alkenyl is optionally substituted by 1 to 3 of

(a) $NR^{13}R^{14}$ wherein $R^{13}$ and $R^{14}$ are each independently hydrogen or $C_{1-4}$ alkyl,
(b) halogen,
(c) $COR^{35a}$ wherein $R^{35a}$ is hydroxy or $C_{1-4}$ alkoxy,
(d) $CONR^{36a}R^{37a}$ wherein $R^{36a}$ and $R^{37a}$ are each independently hydrogen or $C_{1-4}$ alkyl, or
(e) a partial structural formula:

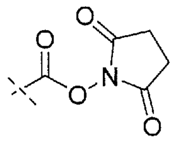
,

(3) $C_{1-6}$ alkoxy wherein the alkoxy is optionally substituted by phenyl,
(4) $NR^{15}R^{16}$ wherein $R^{15}$ and $R^{16}$ are each independently

(a) hydrogen,
(b) $C_{1-4}$ alkyl wherein the alkyl is optionally substituted by

(i) phenyl wherein the phenyl is optionally substituted by halogen, or
(ii) pyridyl,

(c) $C_{1-4}$ alkoxy, or
(d) $C_{3-4}$ cycloalkyl,

(5) $CONR^{18a}R^{19a}$ wherein $R^{18a}$ and $R^{19a}$ are each independently

(a) hydrogen,
(b) $C_{1-4}$ alkyl wherein the alkyl is optionally substituted by hydroxy,
(c) $C_{3-4}$ cycloalkyl,
(d) $C_{5-8}$ bridged cycloalkyl,
(e) $C_{1-4}$ haloalkyl, or
(f) $C_{1-4}$ alkoxy,

(6) 6 to 11-membered spiro heterocycloalkyl containing one or two heteroatoms selected from the group consisting of nitrogen and oxygen atoms as a ring constituting atom, besides carbon atom,
(7) 5 to 10-membered heteroaryl containing 1 to 3 heteroatoms selected from the group consisting of nitrogen and oxygen atoms as a ring constituting atom, besides carbon atom, wherein the heteroaryl is optionally substituted by one or two $R^{22a}$,
(8) a 8 to 10-membered saturated or partially unsaturated fused cyclic group containing 1 to 4 heteroatoms selected from the group consisting of nitrogen and oxygen atoms as a ring constituting atom, besides carbon atom, wherein the fused cyclic group is optionally substituted by $C_{1-4}$ haloalkyl, or
(9) a 5 to 7-membered partially unsaturated cyclic group containing one or two nitrogen atoms as a ring constituting atom, besides carbon atom, wherein the partially unsaturated cyclic group is optionally substituted by an oxo group and $C_{1-4}$ alkyl; and

$Cy^a$, $R^{2a}$, $R^{3a}$, $R^{4a}$, $R^{5a}$, $R^{6a}$, $R^{7a}$, $R^{8a}$, $R^{10a}$, $R^{22a}$ and m are as defined in Item 1a.

[Item 13a] The compound according to Item 12a or a pharmaceutically acceptable salt thereof, which is represented by Formula [XIIIa]:

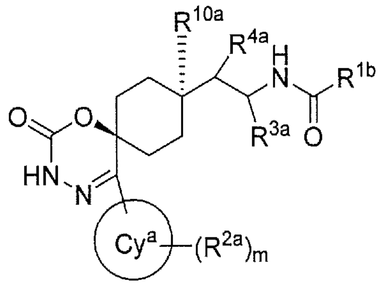

[ XIIIa ]

wherein

Cy$^a$, $R^{2a}$, $R^{3a}$, $R^{4a}$, $R^{10a}$ and m are as defined in Item 1a; and $R^{1b}$ is as defined in Item 12a.

[Item 14a] The compound according to Item 12a or a pharmaceutically acceptable salt thereof, which is represented by Formula [XIVa]:

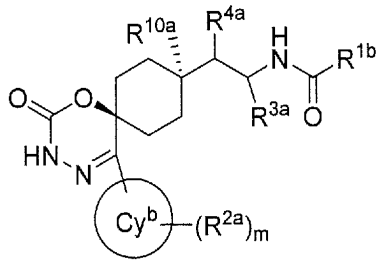

[ XIVa ]

wherein

Cy$^b$ is

(1) $C_{6-10}$ aryl, or
(2) 5- or 6-membered heteroaryl containing one or two nitrogen atoms as a ring constituting atom, besides carbon atom;

$R^{1b}$ is as defined in Item 12a; and
$R^{2a}$, $R^{3a}$, $R^{4a}$, $R^{10a}$ and m are as defined in Item 1a.

[Item 15a] The compound according to Item 12a or a pharmaceutically acceptable salt thereof, which is represented by Formula [XVa]:

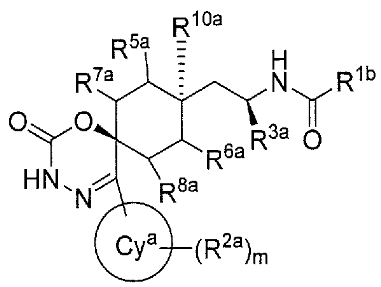

[ XVa ]

wherein

Cy$^a$, $R^{2a}$, $R^{3a}$, $R^{5a}$, $R^{6a}$, $R^{7a}$, $R^{8a}$, $R^{10a}$ and m are as defined in Item 1a; and
$R^{1b}$ is as defined in Item 12a.

[Item 16a] The compound according to Item 12a or a pharmaceutically acceptable salt thereof, which is represented by Formula [XVIa]:

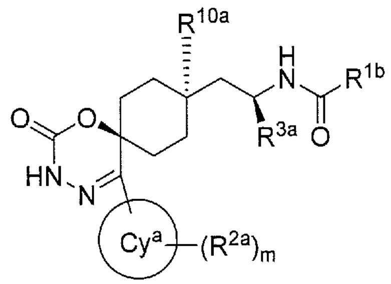

[ XVIa ]

wherein
$Cy^a$, $R^{2a}$, $R^{3a}$, $R^{10a}$ and m are as defined in Item 1a; and $R^{1b}$ is as defined in Item 12a.

[Item 17a] The compound according to Item 14a or a pharmaceutically acceptable salt thereof, which is represented by Formula [XVIIa]:

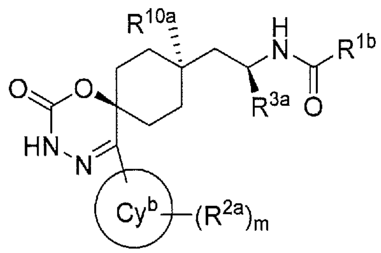

[ XVIIa ]

wherein

$Cy^b$ is as defined in Item 14a;
$R^{1b}$ is as defined in Item 12a; and
$R^{2a}$, $R^{3a}$, $R^{10a}$ and m are as defined in Item 1a.

[Item 18a] A compound selected from the group consisting of compounds represented by the following formulae:

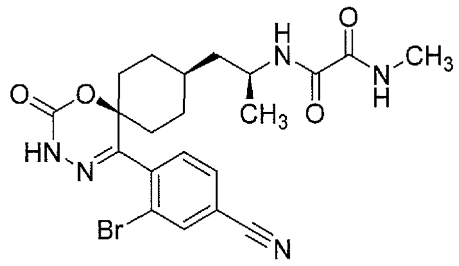

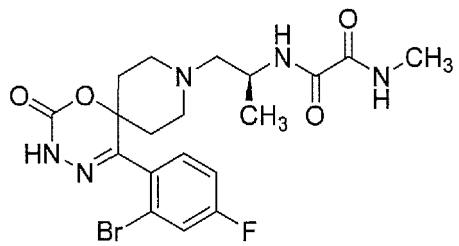

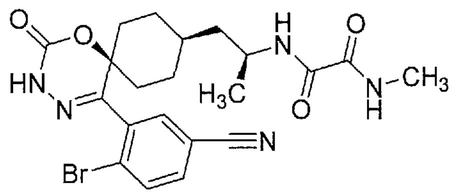

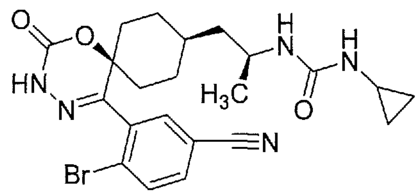

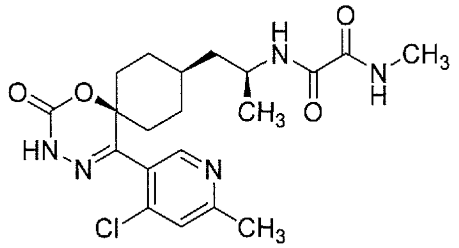

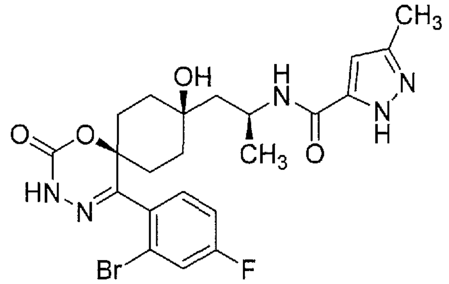

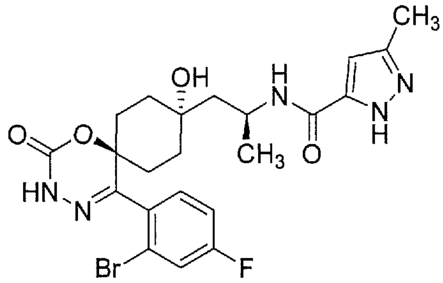

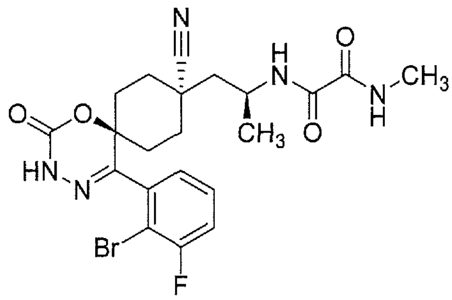

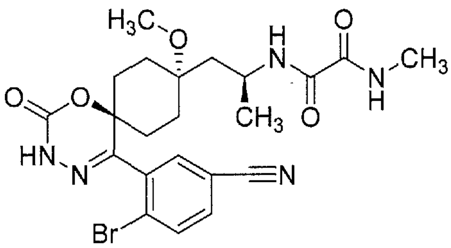

and

,

or a pharmaceutically acceptable salt thereof.

[Item 19a] A pharmaceutical composition comprising a compound as defined in any one of Items 1a to 18a, or a pharmaceutically acceptable salt thereof, and a pharmaceutically acceptable carrier.

[Item 20a] A pharmaceutical composition comprising a compound as defined in any one of Items 1a to 18a, or a pharmaceutically acceptable salt thereof, in combination with one or more other drugs, and a pharmaceutically acceptable carrier.

[Item 21a] A PLD inhibitor comprising a compound as defined in any one of Items 1a to 18a, or a pharmaceutically acceptable salt thereof.

[Item 22a] A PLD1 inhibitor comprising a compound as defined in any one of Items 1a to 18a, or a pharmaceutically acceptable salt thereof.

[Item 23a] A PLD1/2 inhibitor comprising a compound as defined in any one of Items 1a to 18a, or a pharmaceutically acceptable salt thereof.

[Item 24a] An agent for the treatment or prophylaxis of a disease selected from the group consisting of thrombosis and cancer, comprising a compound as defined in any one of Items 1a to 18a, or a pharmaceutically acceptable salt thereof.

[Item 25a] An agent for the treatment or prophylaxis of a disease selected from the group consisting of thrombosis and cancer, comprising a compound as defined in any one of Items 1a to 18a, or a pharmaceutically acceptable salt thereof, in combination with one or more other drugs.

[Item 26a] A method for inhibiting PLD in a mammal, comprising administering a therapeutically effective amount of a compound as defined in any one of Items 1a to 18a, or a pharmaceutically acceptable salt thereof to the mammal.

[Item 27a] A method for inhibiting PLD1 in a mammal, comprising administering a therapeutically effective amount of a compound as defined in any one of Items 1a to 18a, or a pharmaceutically acceptable salt thereof to the mammal.

[Item 28a] A method for inhibiting PLD1/2 in a mammal, comprising administering a therapeutically effective amount of a compound as defined in any one of Items 1a to 18a, or a pharmaceutically acceptable salt thereof to the mammal.

[Item 29a] A method for treating or preventing a disease selected from the group consisting of thrombosis and cancer in a mammal, comprising administering a therapeutically effective amount of a compound as defined in any one of Items 1a to 18a, or a pharmaceutically acceptable salt thereof to the mammal.

[Item 30a] A method for treating or preventing a disease selected from the group consisting of thrombosis and cancer in a mammal, comprising administering a therapeutically effective amount of a compound as defined in any one of Items 1a to 18a, or a pharmaceutically acceptable salt thereof, in combination with one or more other drugs, to the mammal.

[Item 31a] Use of a compound as defined in any one of Items 1a to 18a, or a pharmaceutically acceptable salt thereof, for the manufacture of a PLD inhibitor.

[Item 32a] Use of a compound as defined in any one of Items 1a to 18a, or a pharmaceutically acceptable salt thereof, for the manufacture of a PLD1 inhibitor.

[Item 33a] Use of a compound as defined in any one of Items 1a to 18a, or a pharmaceutically acceptable salt thereof, for the manufacture of a PLD1/2 inhibitor.

[Item 34a] Use of a compound as defined in any one of Items 1a to 18a, or a pharmaceutically acceptable salt thereof, for the manufacture of an agent for the treatment or prophylaxis of a disease selected from the group consisting of thrombosis and cancer.

[Item 35a] Use of a compound as defined in any one of Items 1a to 18a, or a pharmaceutically acceptable salt thereof, in combination with one or more other drugs, for the manufacture of an agent for the treatment or prophylaxis of a disease selected from the group consisting of thrombosis and cancer.

[Item 36a] A compound as defined in any one of Items 1a to 18a, or a pharmaceutically acceptable salt thereof, for use in the inhibition of PLD.

[Item 37a] A compound as defined in any one of Items 1a to 18a, or a pharmaceutically acceptable salt thereof, for use in the inhibition of PLD1.

[Item 38a] A compound as defined in any one of Items 1a to 18a, or a pharmaceutically acceptable salt thereof, for use in the inhibition of PLD1/2.

[Item 39a] A compound as defined in any one of Items 1a to 18a, or a pharmaceutically acceptable salt thereof, for use in the treatment or prophylaxis of a disease selected from the group consisting of thrombosis and cancer.

[Item 40a] A compound as defined in any one of Items 1a to 18a, or a pharmaceutically acceptable salt thereof, in combination with one or more other drugs, for use in the treatment or prophylaxis of a disease selected from the group consisting of thrombosis and cancer.

**Effect of the Invention**

Since the compound represented by Formula [I] or Formula [Ia] or a pharmaceutically acceptable salt thereof of the present invention has a PLD inhibitory activity, it may be useful for the treatment or prophylaxis of thrombosis and cancer.

**Embodiments of the Invention**

The definitions of the terms used herein are as follows.

Examples of the "halogen" include fluorine, chlorine, bromine and iodine.

The "$C_{1-4}$ alkyl" means a linear or branched saturated hydrocarbon group having 1 to 4 carbon atoms. Examples of the "$C_{1-4}$ alkyl" include methyl, ethyl, n-propyl, isopropyl, n-butyl, sec-butyl, isobutyl, and tert-butyl. The preferred "$C_{1-4}$ alkyl" are methyl, ethyl, n-propyl, n-butyl, isobutyl, and tert-butyl.

The "$C_{1-6}$ alkyl" means a linear or branched saturated hydrocarbon group having 1 to 6 carbon atoms. Examples of the "$C_{1-6}$ alkyl" include methyl, ethyl, n-propyl, isopropyl, n-butyl, sec-butyl, isobutyl, tert-butyl, n-pentyl, isopentyl, neopentyl, 1,1-dimethylpropyl, 1,2-dimethylpropyl, 1-ethylpropyl, n-hexyl, isohexyl, 1-ethyl-2-methylpropyl, and 3,3-dimethylbutyl. The preferred "$C_{1-6}$ alkyl" are methyl, ethyl, n-propyl, isopropyl, n-butyl, isobutyl, and tert-butyl.

The "$C_{2-4}$ alkenyl" means a linear or branched hydrocarbon group having 2 to 4 carbon atoms and a carbon-carbon double bond. Examples of the "$C_{2-4}$ alkenyl" include vinyl, allyl, prop-1-en-1-yl, prop-1-en-2-yl, 2-methylprop-1-ene-yl, but-3-en-1-yl, but-2-en-1-yl, and but-2-en-2-yl. The preferred "$C_{2-4}$ alkenyl" are vinyl, prop-1-en-1-yl, and 2-methylprop-1-ene-yl.

The "$C_{1-4}$ haloalkyl" means the above-mentioned "$C_{1-4}$ alkyl" substituted by 1 to 5 halogen independently selected from the group consisting of the above-mentioned "halogen". Examples of the "$C_{1-4}$ haloalkyl" include monofluoromethyl, difluoromethyl, trifluoromethyl, 2-fluoroethyl, 2-chloroethyl, 2-bromoethyl, 1,1-difluoroethyl, 1-fluoro-1-methylethyl, 2,2-difluoroethyl, 2,2,2-trifluoroethyl, pentafluoroethyl, 3,3,3-trifluoropropyl, and 4,4,4-trifluorobutyl. The preferred "$C_{1-4}$ haloalkyl" are difluoromethyl, trifluoromethyl, 2,2-difluoroethyl, 2,2,2-trifluoroethyl, and 3,3,3-trifluoropropyl.

The "$C_{1-4}$ alkoxy" means a group wherein the above-mentioned "$C_{1-4}$ alkyl" is bonded to an oxygen atom. Examples of the "$C_{1-4}$ alkoxy" include methoxy, ethoxy, n-propoxy, isopropoxy, n-butoxy, sec-butoxy, isobutoxy, and tert-butoxy. The preferred "$C_{1-4}$ alkoxy" are methoxy, ethoxy, n-propoxy, isobutoxy, and tert-butoxy.

The "$C_{1-6}$ alkoxy" means a group wherein the above-mentioned "$C_{1-6}$ alkyl" is bonded to an oxygen atom. Examples of the "$C_{1-6}$ alkoxy" include methoxy, ethoxy, n-propoxy, isopropoxy, n-butoxy, sec-butoxy, isobutoxy, tert-butoxy, n-pentyloxy, (3-methylbutan-2-yl)oxy, and n-hexyloxy. The preferred "$C_{1-6}$ alkoxy" are methoxy, ethoxy, n-propoxy, isobutoxy, and tert-butoxy.

The "$C_{3-4}$ cycloalkyl" means a monocyclic saturated hydrocarbon group having 3 or 4 carbon atoms. Examples of the "$C_{3-4}$ cycloalkyl" include cyclopropyl, and cyclobutyl. The preferred "$C_{3-4}$ cycloalkyl" is cyclopropyl.

The "$C_{5-8}$ bridged cycloalkyl" means a 5 to 8-membered bridged cyclic saturated hydrocarbon group. Examples of the "$C_{5-8}$ bridged cycloalkyl" include bicyclo[1.1.1]pentyl, bicyclo[2.2.1]heptyl, and bicyclo[2.2.2]octyl. The preferred "$C_{5-8}$ bridged cycloalkyl" is bicyclo[1.1.1]pentyl.

The "$C_{6-10}$ aryl" means an aromatic hydrocarbon group having 6 to 10 carbon atoms. Examples of the "$C_{6-10}$ aryl" include phenyl and naphthyl. The preferred "$C_{6-10}$ aryl" is phenyl.

The "4 to 7-membered heterocycloalkyl containing one or two heteroatoms selected from the group consisting of nitrogen and oxygen atoms as a ring constituting atom, besides carbon atom" means a 4 to 7-membered saturated heterocyclic group containing one or two of nitrogen and oxygen atoms as a ring constituting atom, besides carbon atom. Examples of the "4 to 7-membered heterocycloalkyl containing one or two heteroatoms selected from the group consisting of nitrogen and oxygen atoms as a ring constituting atom, besides carbon atom" include azetidinyl, oxetanyl, pyrrolidinyl, tetrahydrofuryl, piperidyl, tetrahydropyranyl, dioxanyl, piperazinyl, morpholinyl, and azepanyl. The preferred "4 to 7-membered heterocycloalkyl containing one or two heteroatoms selected from the group consisting of nitrogen and oxygen atoms as a ring constituting atom, besides carbon atom" are azetidinyl, oxetanyl, pyrrolidinyl, tetrahydrofuryl, piperidyl, dioxanyl, and morpholinyl.

The "5 to 9-membered bridged heterocycloalkyl containing one or two heteroatoms selected from the group consisting of nitrogen and oxygen atoms as a ring constituting atom, besides carbon atom" means a 5 to 9-membered bridged heterocyclic group containing one or two of nitrogen and oxygen atoms as a ring constituting atom, besides carbon atom. Examples of the "5 to 9-membered bridged heterocycloalkyl containing one or two heteroatoms selected from the group consisting of nitrogen and oxygen atoms as a ring constituting atom, besides carbon atom" include 6-azabicyclo [3.1.1]heptyl, 7-azabicyclo[2.2.1]heptyl, 3-oxa-6-azabicyclo[3.1.1]heptyl, 2-oxa-5-azabicyclo[2.2.1]heptyl, and 8-oxa-3-azabicyclo[3.2.1]octyl. The preferred "5 to 9-membered bridged heterocycloalkyl containing one or two heteroatoms selected from the group consisting of nitrogen and oxygen atoms as a ring constituting atom, besides carbon atom" are 2-oxa-5-azabicyclo[2.2.1]heptyl, and 8-oxa-3-azabicyclo[3.2.1]octyl.

The "6 to 11-membered spiro heterocycloalkyl containing one or two heteroatoms selected from the group consisting of nitrogen and oxygen atoms as a ring constituting atom, besides carbon atom" means a 6 to 11-membered spiro heterocyclic group containing one or two of nitrogen and oxygen atoms as a ring constituting atom, besides carbon atom. Examples of the "6 to 11-membered spiro heterocycloalkyl containing one or two heteroatoms selected from the group consisting of nitrogen and oxygen atoms as a ring constituting atom, besides carbon atom" include 2-azaspiro[3.3] heptyl, 2-oxa-6-azaspiro[3.3]heptyl, 5-azaspiro[2.4]heptyl, 4-oxa-7-azaspiro[2.5]octyl, 2,8-diazaspiro[4.5]decyl, and 3-oxa-9-azaspiro[5.5]undecyl. The preferred "6 to 11-membered spiro heterocycloalkyl containing one or two heteroatoms selected from the group consisting of nitrogen and oxygen atoms as a ring constituting atom, besides carbon atom" are 2-oxa-6-azaspiro[3.3]heptyl, 5-azaspiro[2.4]heptyl, and 4-oxa-7-azaspiro[2.5]octyl.

The "5- or 6-membered heteroaryl containing one or two nitrogen atoms as a ring constituting atom, besides carbon atom" means a 5- or 6-membered aromatic heterocyclic group containing one or two nitrogen atoms as a ring constituting atom, besides carbon atom. Examples of the "5- or 6-membered heteroaryl containing one or two nitrogen atoms as a ring constituting atom, besides carbon atom" include pyrrolyl, imidazolyl, pyrazolyl, pyridyl, pyrazinyl, pyrimidinyl and pyridazinyl. The preferred "5- or 6-membered heteroaryl containing one or two nitrogen atoms as a ring constituting atom, besides carbon atom" are pyrrolyl, imidazolyl, pyrazolyl, pyridyl, pyrazinyl, pyrimidinyl, and pyridazinyl.

The "5 to 10-membered heteroaryl containing one or two nitrogen atoms as a ring constituting atom, besides carbon atom" means a 5 to 10-membered aromatic heterocyclic group containing one or two nitrogen atoms as a ring constituting atom, besides carbon atom. Examples of the "5 to 10-membered heteroaryl containing one or two nitrogen atoms as a ring constituting atom, besides carbon atom" include pyrrolyl, imidazolyl, pyrazolyl, pyridyl, pyrazinyl, pyrimidinyl, pyridazinyl, indolyl, benzimidazolyl, indazolyl, indolizinyl, imidazo[1,2-a]pyridyl, pyrazolo[1,5-a]pyridyl, quinolyl, isoquinolyl, and quinoxalyl. The preferred "5 to 10-membered heteroaryl containing one or two nitrogen atoms as a ring constituting atom, besides carbon atom" are pyrrolyl, imidazolyl, pyrazolyl, pyridyl, pyrazinyl, pyrimidinyl, pyridazinyl, and pyrazolo[1,5-a]pyridyl.

The "5 to 10-membered heteroaryl containing 1 to 3 heteroatoms selected from the group consisting of nitrogen and oxygen atoms as a ring constituting atom, besides carbon atom" means a 5 to 10-membered aromatic heterocyclic group containing 1 to 3 of nitrogen and oxygen atoms as a ring constituting atom, besides carbon atom. Examples of the "5 to 10-membered heteroaryl containing 1 to 3 heteroatoms selected from the group consisting of nitrogen and oxygen atoms as a ring constituting atom, besides carbon atom" include pyrrolyl, furyl, imidazolyl, pyrazolyl, 1,2,3-triazolyl, 1,2,4-triazolyl, oxazolyl, isoxazolyl, 1,2,4-oxadiazolyl, pyridyl, pyrazinyl, pyrimidinyl, pyridazinyl, triazinyl, indolyl, benzofuranyl,

benzimidazolyl, indazolyl, benzoxazolyl, benzisoxazolyl, indolizinyl, imidazo[1,2-a]pyridyl, pyrazolo[1,5-a]pyridyl, quinolyl, isoquinolyl, and quinoxalyl. The preferred "5 to 10-membered heteroaryl containing 1 to 3 heteroatoms selected from the group consisting of nitrogen and oxygen atoms as a ring constituting atom, besides carbon atom" are pyrrolyl, imidazolyl, pyrazolyl, 1,2,3-triazolyl, 1,2,4-triazolyl, isoxazolyl, 1,2,4-oxadiazolyl, pyridyl, pyrimidinyl, pyridazinyl, pyrazinyl, indolyl, benzofuranyl, benzimidazolyl, indolizinyl, imidazo[1,2-a]pyridyl, and pyrazolo[1,5-a]pyridyl.

The "5 to 7-membered partially unsaturated cyclic group containing one or two nitrogen atoms as a ring constituting atom, besides carbon atom" means a 5 to 7-membered partially unsaturated cyclic group containing one or two nitrogen atoms as a ring constituting atom, besides carbon atom. Examples of the "5 to 7-membered partially unsaturated cyclic group containing one or two nitrogen atoms as a ring constituting atom, besides carbon atom" include dihydropyrrolyl, dihydropyridyl, and dihydropyrimidinyl. The preferred "5 to 7-membered partially unsaturated cyclic group containing one or two nitrogen atoms as a ring constituting atom, besides carbon atom" is dihydropyridyl.

The "8 to 10-membered saturated or partially unsaturated fused cyclic group containing 1 to 4 heteroatoms selected from the group consisting of nitrogen and oxygen atoms as a ring constituting atom, besides carbon atom" means a 8 to 10-membered fused heterocyclic group containing 1 to 4 heteroatoms selected from the group consisting of nitrogen and oxygen atoms as a ring constituting atom, besides carbon atom and containing at least one saturated ring as a constituent ring in the fused ring. Examples of the "8 to 10-membered saturated or partially unsaturated fused cyclic group containing 1 to 4 heteroatoms selected from the group consisting of nitrogen and oxygen atoms as a ring constituting atom, besides carbon atom" include octahydro-2H-isoindolyl, dihydrobenzofuranyl, benzo[d][1,3]dioxolyl, isoindolyl, dihydroisoquinolyl, dihydro-1,6-naphthyridinyl, dihydro-1,7-naphthyridinyl, dihydroisoxazolo[4,3-c]pyridyl, dihydropyrazolo[1,5-a]pyrazinyl, and dihydrotriazolo[1,5-a]pyrazinyl.

The "9- or 10-membered partially unsaturated fused cyclic group containing one or two oxygen atoms as a ring constituting atom, besides carbon atom" means a 9- or 10-membered fused heterocyclic group containing one or two oxygen atoms as a ring constituting atom, besides carbon atom, and containing at least one saturated ring as a constituent ring in the fused ring. Examples of the "9- or 10-membered partially unsaturated fused cyclic group containing one or two oxygen atoms as a ring constituting atom, besides carbon atom" include 2,3-dihydrobenzo[b][1,4]dioxiranyl, dihydrobenzofuranyl, and benzo[d][1,3]dioxolyl. The preferred "9- or 10-membered partially unsaturated fused cyclic group containing one or two oxygen atoms as a ring constituting atom, besides carbon atom" are dihydrobenzofuranyl, and benzo[d][1,3]dioxolyl.

The expression that Substituent $\alpha$ is "optionally substituted" by Substituent $\beta$ means that Substituent $\alpha$ is unsubstituted, or substituted by Substituent $\beta$ at any substitutable position thereof (any hydrogen is replaced with Substituent $\beta$). For example, the "$C_{1-6}$ alkyl optionally substituted by hydroxy" means that $C_{1-6}$ alkyl is unsubstituted, or substituted by hydroxy at any substitutable position thereof.

Specific embodiments of each substituent of the compound of Formula [I] (hereinafter, also to be referred to as "Compound [I]") and the compound of Formula [Ia] (hereinafter, also to be referred to as "Compound [Ia]") are exemplified below. Each group of Compound [I] and Compound [Ia] is not limited to its specific embodiments, and Compound [I] and Compound [Ia] also includes combinations of two or more embodiments selected appropriately from the specific embodiments of each substituent.

First, specific embodiments of each substituent of Compound [I] are exemplified below.

A is preferably $CR^{10}$ wherein $R^{10}$ is hydrogen, hydroxy, or halogen.

Cy is preferably

(1) $C_{6-10}$ aryl, or
(2) 5- or 6-membered heteroaryl containing one or two nitrogen atoms as a ring constituting atom, besides carbon atom.

$R^3$ is preferably $C_{1-4}$ alkyl.

$R^4$ is preferably hydrogen.

$R^5$ is preferably hydrogen.

$R^6$ is preferably hydrogen.

$R^7$ and $R^8$ are preferably both hydrogens.

$R^9$ is preferably hydrogen.

m is preferably one or two.

One of preferred embodiments of Compound [I] is a compound represented by Formula [II]:

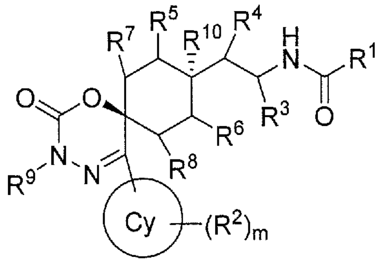

[ II ]

wherein

Cy is

(1) $C_{6-10}$ aryl, or
(2) 5- or 6-membered heteroaryl containing one or two nitrogen atoms as a ring constituting atom, besides carbon atom;

$R^1$ is

(1) $C_{1-6}$ alkyl wherein the alkyl is optionally substituted by

(a) hydroxy,
(b) cyano,
(c) $SO_2R^{11}$ wherein $R^{11}$ is $C_{1-4}$ alkyl, or
(d) $NHCOR^{12}$ wherein $R^{12}$ is $C_{1-4}$ alkyl,

(2) $C_{2-4}$ alkenyl wherein the alkenyl is optionally substituted by $NR^{13}R^{14}$ wherein $R^{13}$ and $R^{14}$ are each independently hydrogen or $C_{1-4}$ alkyl,
(3) $C_{1-4}$ haloalkyl,
(4) $C_{1-6}$ alkoxy wherein the alkoxy is optionally substituted by phenyl,
(5) $NR^{15}R^{16}$ wherein $R^{15}$ and $R^{16}$ are each independently

(a) hydrogen,
(b) $C_{1-4}$ alkyl wherein the alkyl is optionally substituted by

(i) phenyl wherein the phenyl is optionally substituted by halogen, or
(ii) pyridyl,

(c) $C_{1-4}$ alkoxy, or
(d) $C_{3-4}$ cycloalkyl,

(6) $COR^{17}$ wherein $R^{17}$ is $C_{1-4}$ alkyl,
(7) $CONR^{18}R^{19}$ wherein $R^{18}$ and $R^{19}$ are each independently

(a) hydrogen,
(b) $C_{1-4}$ alkyl,
(c) $C_{3-4}$ cycloalkyl, or
(d) $C_{5-8}$ bridged cycloalkyl,

(8) $C_{3-4}$ cycloalkyl wherein the cycloalkyl is optionally substituted by

(a) hydroxy,
(b) halogen, or
(c) phenyl,

(9) 4 to 7-membered heterocycloalkyl containing one or two heteroatoms selected from the group consisting of

nitrogen and oxygen atoms as a ring constituting atom, besides carbon atom, wherein the heterocycloalkyl is optionally substituted by

(a) hydroxy,
(b) oxo,
(c) $NR^{20}R^{21}$ wherein $R^{20}$ and $R^{21}$ are each independently hydrogen or $C_{1-4}$ alkyl, or
(d) phenyl,

(10) 6 to 11-membered spiro heterocycloalkyl containing one or two heteroatoms selected from the group consisting of nitrogen and oxygen atoms as a ring constituting atom, besides carbon atom,
(11) phenyl wherein the phenyl is optionally substituted by one or two halogens,
(12) 5 to 10-membered heteroaryl containing 1 to 3 heteroatoms selected from the group consisting of nitrogen and oxygen atoms as a ring constituting atom, besides carbon atom, wherein the heteroaryl is optionally substituted by one or two $R^{22}$, or
(13) a 8 to 10-membered saturated or partially unsaturated fused cyclic group containing 1 to 4 heteroatoms selected from the group consisting of nitrogen and oxygen atoms as a ring constituting atom, besides carbon atom, wherein the fused cyclic group is optionally substituted by $C_{1-4}$ haloalkyl;

$R^2$ in the number of m are each independently

(1) hydroxy,
(2) cyano,
(3) halogen,
(4) $C_{1-6}$ alkyl wherein the alkyl is optionally substituted by

(a) hydroxy, or
(b) $C_{3-4}$ cycloalkyl,

(5) $C_{2-4}$ alkenyl wherein the alkenyl is optionally substituted by $C_{1-4}$ alkoxy,
(6) $C_{1-4}$ haloalkyl,
(7) $C_{1-4}$ alkoxy wherein the alkoxy is optionally substituted by 1 to 3 substituents selected from the group consisting of

(a) hydroxy, and
(b) halogen,

(8) $SR^{23}$ wherein $R^{23}$ is $C_{1-4}$ alkyl,
(9) $COR^{24}$ wherein $R^{24}$ is hydroxy or $C_{1-4}$ alkyl,
(10) $CONR^{25}R^{26}$ wherein $R^{25}$ and $R^{26}$ are each independently

(a) hydrogen,
(b) $C_{1-6}$ alkyl, or
(c) $C_{3-4}$ cycloalkyl,

(11) $SO_2R^{27}$ wherein $R^{27}$ is $C_{1-6}$ alkyl,
(12) $C_{3-4}$ cycloalkyl,
(13) 4 to 7-membered heterocycloalkyl containing one or two heteroatoms selected from the group consisting of nitrogen and oxygen atoms as a ring constituting atom, besides carbon atom, wherein the heterocycloalkyl is optionally substituted by one or two substituents selected from the group consisting of

(a) halogen,
(b) $C_{1-4}$ alkyl, and
(c) $C_{1-4}$ haloalkyl,

(14) 5 to 9-membered bridged heterocycloalkyl containing one or two heteroatoms selected from the group consisting of nitrogen and oxygen atoms as a ring constituting atom, besides carbon atom,
(15) 6 to 11-membered spiro heterocycloalkyl containing one or two heteroatoms selected from the group consisting of nitrogen and oxygen atoms as a ring constituting atom, besides carbon atom, or

(16) phenyl;

$R^3$ is $C_{1-4}$ alkyl;
$R^4$, $R^5$, $R^6$, $R^7$, $R^8$ and $R^9$ are all hydrogen;
$R^{10}$ is

(1) hydrogen,
(2) hydroxy, or
(3) halogen;

one or two $R^{22}$ are each independently

(1) halogen,
(2) $C_{1-4}$ alkyl,
(3) $C_{1-4}$ haloalkyl,
(4) $C_{1-4}$ alkoxy,
(5) $NHCOR^{29}$ wherein $R^{29}$ is $C_{1-4}$ alkyl, or
(6) $SO_2R^{30}$ wherein $R^{30}$ is $C_{1-4}$ alkyl; and

m is one or two.

Preferred specific embodiments of Compound [I] include the compounds of Examples 1 to 339 listed in Table 1-1 to Table 1-43 described later.
Next, specific embodiments of each substituent of Compound [Ia] are exemplified below. Compound [Ia] includes Compound [I].
$A^a$ is preferably $CR^{10a}$.
In another embodiment, $A^a$ is preferably N.
$A^{2a}$ is preferably $CR^{5a}$.
$Cy^a$ is preferably

(1) $C_{6-10}$ aryl, or
(2) 5- or 6-membered heteroaryl containing one or two nitrogen atoms as a ring constituting atom, besides carbon atom.

$R^{1a}$ is preferably

(1) $C_{1-6}$ alkyl wherein the alkyl is optionally substituted by

(a) hydroxy,
(b) cyano,
(c) $SO_2R^{11}$ wherein $R^{11}$ is $C_{1-4}$ alkyl, or
(d) $NHCOR^{12}$ wherein $R^{12}$ is $C_{1-4}$ alkyl,

(2) $C_{2-4}$ alkenyl wherein the alkenyl is optionally substituted by 1 to 3 of

(a) $NR^{13}R^{14}$ wherein $R^{13}$ and $R^{14}$ are each independently hydrogen or $C_{1-4}$ alkyl,
(b) halogen,
(c) $COR^{35a}$ wherein $R^{35a}$ is hydroxy or $C_{1-4}$ alkoxy,
(d) $CONR^{36a}R^{37a}$ wherein $R^{36a}$ and $R^{37a}$ are each independently hydrogen or $C_{1-4}$ alkyl, or
(e) a partial structural formula:

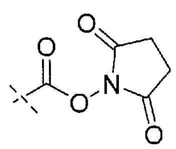,

(3) $C_{1-6}$ alkoxy wherein the alkoxy is optionally substituted by phenyl,

(4) $NR^{15}R^{16}$ wherein $R^{15}$ and $R^{16}$ are each independently

(a) hydrogen,
(b) $C_{1-4}$ alkyl wherein the alkyl is optionally substituted by

(i) phenyl wherein the phenyl is optionally substituted by halogen, or
(ii) pyridyl,

(c) $C_{1-4}$ alkoxy, or
(d) $C_{3-4}$ cycloalkyl,

(5) $CONR^{18a}R^{19a}$ wherein $R^{18a}$ and $R^{19a}$ are each independently

(a) hydrogen,
(b) $C_{1-4}$ alkyl wherein the alkyl is optionally substituted by hydroxy,
(c) $C_{3-4}$ cycloalkyl,
(d) $C_{5-8}$ bridged cycloalkyl,
(e) $C_{1-4}$ haloalkyl, or
(f) $C_{1-4}$ alkoxy,

(6) 6 to 11-membered spiro heterocycloalkyl containing one or two heteroatoms selected from the group consisting of nitrogen and oxygen atoms as a ring constituting atom, besides carbon atom,
(7) 5 to 10-membered heteroaryl containing 1 to 3 heteroatoms selected from the group consisting of nitrogen and oxygen atoms as a ring constituting atom, besides carbon atom, wherein the heteroaryl is optionally substituted by one or two $R^{22a}$ wherein each $R^{22a}$ is as defined above,
(8) a 8 to 10-membered saturated or partially unsaturated fused cyclic group containing 1 to 4 heteroatoms selected from the group consisting of nitrogen and oxygen atoms as a ring constituting atom, besides carbon atom, wherein the fused cyclic group is optionally substituted by $C_{1-4}$ haloalkyl, or
(9) a 5 to 7-membered partially unsaturated cyclic group containing one or two nitrogen atoms as a ring constituting atom, besides carbon atom, wherein the partially unsaturated cyclic group is optionally substituted by an oxo group and $C_{1-4}$ alkyl.

In another embodiment, $R^{1a}$ is preferably

(1) $NR^{15}R^{16}$ wherein $R^{15}$ and $R^{16}$ are each independently

(a) hydrogen,
(b) $C_{1-4}$ alkyl wherein the alkyl is optionally substituted by

(i) phenyl wherein the phenyl is optionally substituted by halogen, or
(ii) pyridyl,

(c) $C_{1-4}$ alkoxy, or
(d) $C_{3-4}$ cycloalkyl,

(2) $CONR^{18a}R^{19a}$ wherein $R^{18a}$ and $R^{19a}$ are each independently

(a) hydrogen,
(b) $C_{1-4}$ alkyl wherein the alkyl is optionally substituted by hydroxy,
(c) $C_{3-4}$ cycloalkyl,
(d) $C_{5-8}$ bridged cycloalkyl,
(e) $C_{1-4}$ haloalkyl, or
(f) $C_{1-4}$ alkoxy, or

(3) 5 to 10-membered heteroaryl containing 1 to 3 heteroatoms selected from the group consisting of nitrogen and oxygen atoms as a ring constituting atom, besides carbon atom, wherein the heteroaryl is optionally substituted by one or two $R^{22a}$ wherein each $R^{22a}$ is as defined above.

$R^{2a}$ in the number of m are each independently preferably

(1) cyano,
(2) halogen, or
(3) $C_{1-6}$ alkyl wherein the alkyl is optionally substituted by

(a) hydroxy, or
(b) $C_{3-4}$ cycloalkyl.

$R^{3a}$ is preferably $C_{1-4}$ alkyl.
In another embodiment, $R^{3a}$ is preferably methyl.
$R^{4a}$ is preferably hydrogen.
$R^{5a}$ is preferably hydrogen.
The combination of $R^{6a}$, $R^{7a}$ and $R^{8a}$ is preferably

(1) a combination where $R^{6a}$ is hydrogen, and $R^{7a}$ and $R^{8a}$ are both hydrogens,
(2) a combination where $R^{6a}$ is hydrogen, and $R^{7a}$ and $R^{8a}$ are bonded to each other to form a cyclopentane ring together with the spiro carbon atom and the carbon atoms to which they are bonded, or
(3) a combination where $R^{6a}$ and $R^{7a}$ are bonded to each other to form a cyclopentane ring together with the spiro carbon atom and the carbon atoms to which they are bonded, and $R^{8a}$ is hydrogen.

In another embodiment, $R^{6a}$, $R^{7a}$ and $R^{8a}$ are preferably all hydrogen.
$R^{9a}$ is preferably hydrogen.
$R^{10a}$ is preferably

(1) hydrogen,
(2) hydroxy, or
(3) cyano.

One or two $R^{22a}$ are each independently preferably

(1) halogen,
(2) $C_{1-4}$ alkyl,
(3) $C_{1-4}$ haloalkyl,
(4) $C_{1-4}$ alkoxy,
(5) $NHCOR^{29}$ wherein $R^{29}$ is $C_{1-4}$ alkyl,
(6) cyano, or
(7) $C_{3-4}$ cycloalkyl.

m is preferably one or two.
One of preferred embodiments of Compound [Ia] is a compound represented by Formula [IIa]:

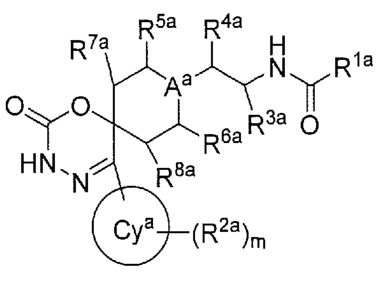

[ IIa ]

wherein
$A^a$, $Cy^a$, $R^{1a}$, $R^{2a}$, $R^{3a}$, $R^{4a}$, $R^{5a}$, $R^{6a}$, $R^{7a}$, $R^{8a}$ and m are as defined above.
Another preferred embodiment of Compound [Ia] is a compound represented by Formula [IIIa]:

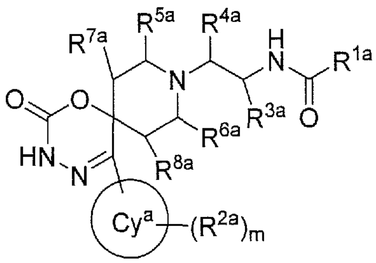

[ IIIa ]

wherein

$Cy^a$, $R^{1a}$, $R^{2a}$, $R^{3a}$, $R^{4a}$, $R^{5a}$, $R^{6a}$, $R^{7a}$, $R^{8a}$ and m are as defined above.

Another preferred embodiment of Compound [Ia] is a compound represented by Formula [IVa]:

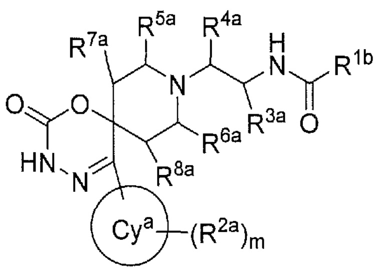

[ IVa ]

wherein

$Cy^a$, $R^{1b}$, $R^{2a}$, $R^{3a}$, $R^{4a}$, $R^{5a}$, $R^{6a}$, $R^{7a}$, $R^{8a}$ and m are as defined above.

Another preferred embodiment of Compound [Ia] is a compound represented by Formula [Va]:

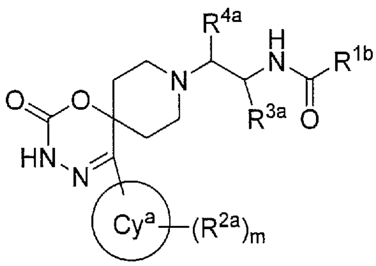

[ Va ]

wherein

$Cy^a$, $R^{1b}$, $R^{2a}$, $R^{3a}$, $R^{4a}$ and m are as defined above.

Another preferred embodiment of Compound [Ia] is a compound represented by Formula [VIa]:

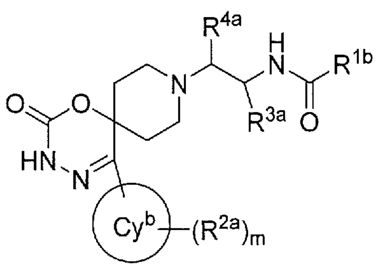

[ VIa ]

wherein
$Cy^b$, $R^{1b}$, $R^{2a}$, $R^{3a}$, $R^{4a}$ and m are as defined above.

Another preferred embodiment of Compound [Ia] is a compound represented by Formula [VIIa]:

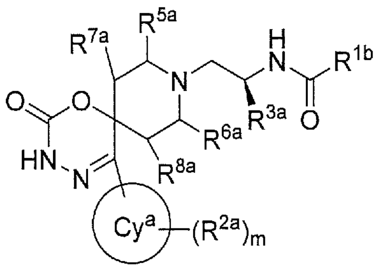

[ VIIa ]

wherein
$Cy^a$, $R^{1b}$, $R^{2a}$, $R^{3a}$, $R^{5a}$, $R^{6a}$, $R^{7a}$, $R^{8a}$ and m are as defined above.

Another preferred embodiment of Compound [Ia] is a compound represented by Formula [VIIIa]:

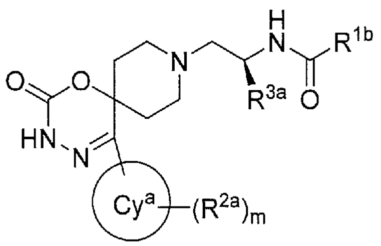

[ VIIIa ]

wherein
$Cy^a$, $R^{1b}$, $R^{2a}$, $R^{3a}$ and m are as defined above.

Another preferred embodiment of Compound [Ia] is a compound represented by Formula [IXa]:

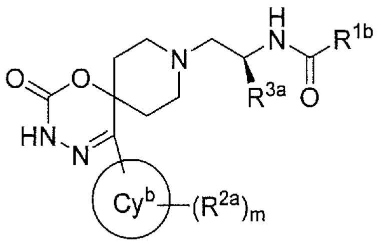

[ IXa ]

wherein
$Cy^b$, $R^{1b}$, $R^{2a}$, $R^{3a}$ and m are as defined above.

Another preferred embodiment of Compound [Ia] is a compound represented by Formula [Xa]:

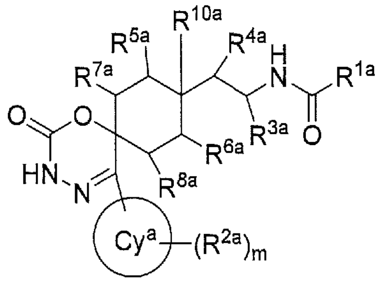

[ Xa ]

wherein

Cya, $R^{1a}$, $R^{2a}$, $R^{3a}$, R4a, R5a, $R^{6a}$, $R^{7a}$, $R^{8a}$, $R^{10a}$ and m are as defined above.

Another preferred embodiment of Compound [Ia] is a compound represented by Formula [XIa]:

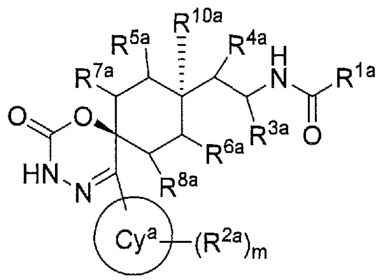

[ XIa ]

wherein

Cya, $R^{1a}$, R2a, $R^{3a}$, $R^{4a}$, $R^{5a}$, $R^{6a}$, $R^{7a}$, $R^{8a}$, $R^{10a}$ and m are as defined above.

Another preferred embodiment of Compound [Ia] is a compound represented by Formula [XIIa]:

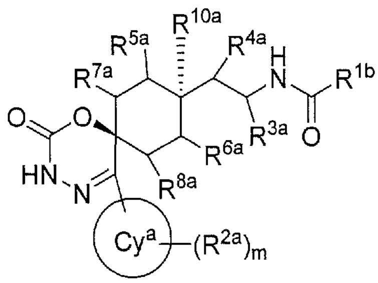

[ XIIa ]

wherein

$Cy^a$, $R^{1b}$, $R^{2a}$, $R^{3a}$, $R^{4a}$, $R^{5a}$, $R^{6a}$, $R^{7a}$, $R^{8a}$, $R^{10a}$ and m are as defined above.

Another preferred embodiment of Compound [Ia] is a compound represented by Formula [XIIIa]:

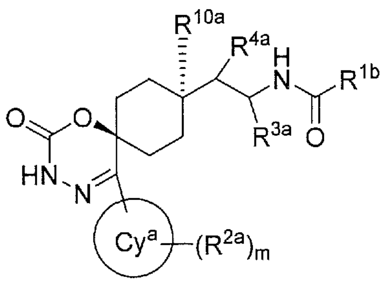

[ XIIIa ]

wherein
$Cy^a$, $R^{1b}$, $R^{2a}$, $R^{3a}$, $R^{4a}$, $R^{10a}$ and m are as defined above.

Another preferred embodiment of Compound [Ia] is a compound represented by Formula [XIVa]:

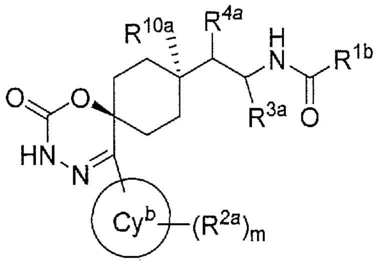

[ XIVa ]

wherein
$Cy^b$, $R^{1b}$, $R^{2a}$, $R^{3a}$, $R^{4a}$, $R^{10a}$ and m are as defined above.

Another preferred embodiment of Compound [Ia] is a compound represented by Formula [XVa]:

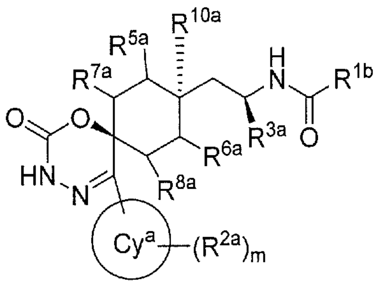

[ XVa ]

wherein
$Cy^a$, $R^{1b}$, $R^{2a}$, $R^{3a}$, $R^{5a}$, $R^{6a}$, $R^{7a}$, $R^{3a}$, $R^{10a}$ and m are as defined above.

Another preferred embodiment of Compound [Ia] is a compound represented by Formula [XVIa]:

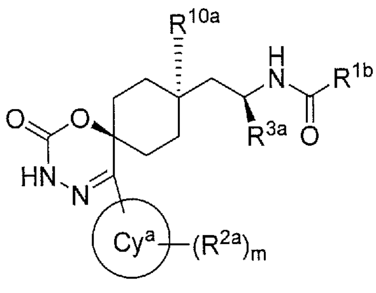

[ XVIa ]

wherein
Cya, $R^{1b}$, $R^{2a}$, $R^{3a}$, $R^{10a}$ and m are as defined above.

Another preferred embodiment of Compound [Ia] is a compound represented by Formula [XVIIa]:

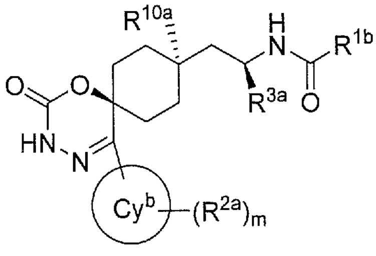

[ XVIIa ]

wherein
$Cy^b$, $R^{1b}$, $R^{2a}$, $R^{3a}$, $R^{10a}$ and m are as defined above.

Another preferred embodiment of Compound [Ia] is a compound represented by Formula [XVIIIa]:

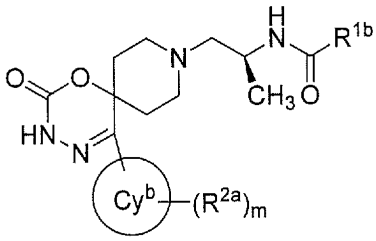

[ XVIIIa ]

wherein
$Cy^b$, $R^{1b}$, $R^{2a}$ and m are as defined above.

Another preferred embodiment of Compound [Ia] is a compound represented by Formula [XIXa]:

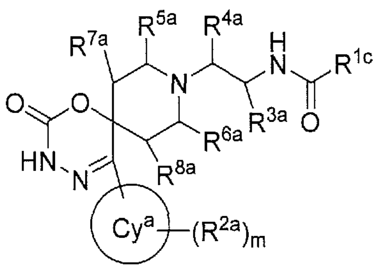

[ XIXa ]

wherein

$R^{1c}$ is

(1) $C_{1-4}$ haloalkyl wherein the haloalkyl is optionally substituted by hydroxy,
(2) $COR^{17a}$ wherein $R^{17a}$ is $C_{1-4}$ alkyl or hydroxy,
(3) $C_{3-4}$ cycloalkyl wherein the cycloalkyl is optionally substituted by

(a) hydroxy,
(b) halogen, or
(c) phenyl,

(4) 4 to 7-membered heterocycloalkyl containing one or two heteroatoms selected from the group consisting of nitrogen and oxygen atoms as a ring constituting atom, besides carbon atom, wherein the heterocycloalkyl is optionally substituted by

(a) hydroxy,
(b) oxo,
(c) $NR^{20}R^{21}$ wherein $R^{20}$ and $R^{21}$ are each independently hydrogen or $C_{1-4}$ alkyl, or
(d) phenyl, or

(5) phenyl wherein the phenyl is optionally substituted by one or two of

(a) halogen, or
(b) $C_{1-4}$ haloalkyl, and

Cya, $R^{2a}$, $R^{3a}$, $R^{4a}$, $R^{5a}$, $R^{6a}$, $R^{7a}$, $R^{8a}$ and m are as defined above.

Another preferred embodiment of Compound [Ia] is a compound represented by Formula [XXa]:

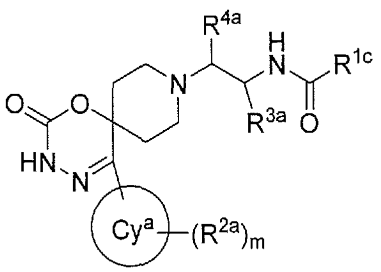

[ XXa ]

wherein
$Cy^a$, $R^{1c}$, $R^{2a}$, $R^{3a}$, $R^{4a}$ and m are as defined above.

Another preferred embodiment of Compound [Ia] is a compound represented by Formula [XXIa]:

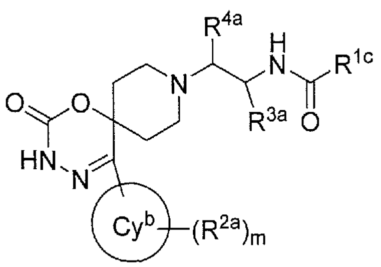

[ XXIa ]

wherein
$Cy^b$, $R^{1c}$, $R^{2a}$, $R^{3a}$, $R^{4a}$ and m are as defined above.

Another preferred embodiment of Compound [Ia] is a compound represented by Formula [XXIIa]:

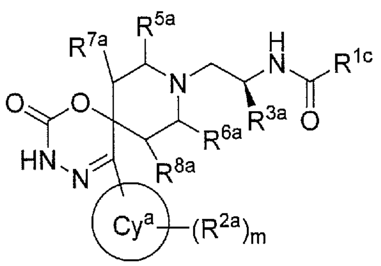

[ XXIIa ]

wherein

$Cy^a$, $R^{1c}$, $R^{2a}$, $R^{3a}$, $R^{5a}$, $R^{6a}$, $R^{7a}$, $R^{8a}$ and m are as defined above.

Another preferred embodiment of Compound [Ia] is a compound represented by Formula [XXIIIa]:

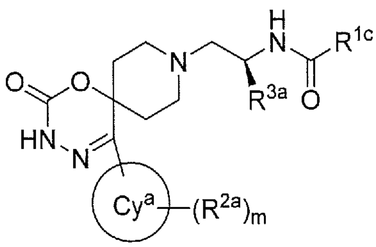

[ XXIIIa ]

wherein
$Cy^a$, $R^{1c}$, $R^{2a}$, $R^{3a}$ and m are as defined above.

Another preferred embodiment of Compound [Ia] is a compound represented by Formula [XXIVa]:

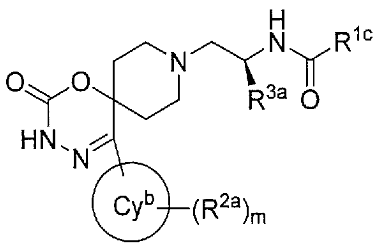

[ XXIVa ]

wherein
$Cy^b$, $R^{1c}$, $R^{2a}$, $R^{3a}$ and m are as defined above.

Another preferred embodiment of Compound [Ia] is a compound represented by Formula [XXVa]:

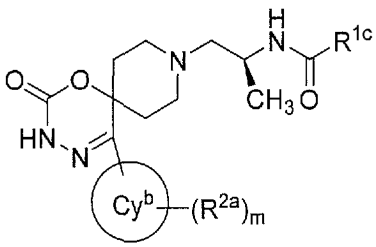

[ XXVa ]

wherein
$Cy^b$, $R^{1c}$, $R^{2a}$ and m are as defined above.

Another preferred embodiment of Compound [Ia] is a compound represented by Formula [XXVIa-1], Formula [XXVIa-2], Formula [XXVIa-3], Formula [XXVIa-4], Formula [XXVIa-5], Formula [XXVIa-6], Formula [XXVIa-7], Formula [XXVIa-8], or Formula [XXVIa-9]:

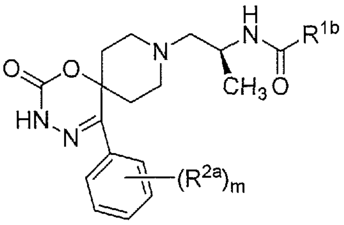

[ XXVIa-1 ]

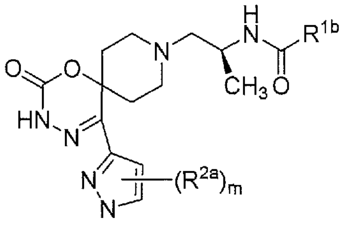

[ XXVIa-2 ]

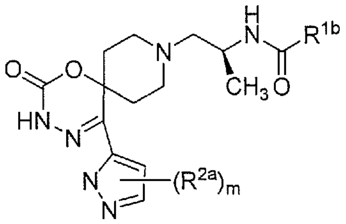

[ XXVIa-3 ]

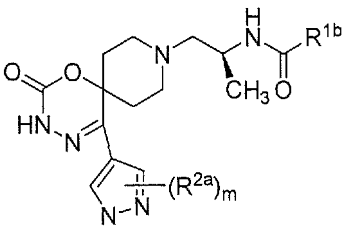

[ XXVIa-4 ]

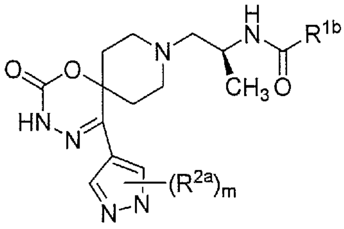

[ XXVIa-5 ]

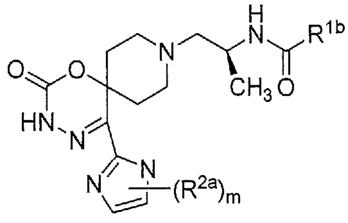

[ XXVIa-6 ]

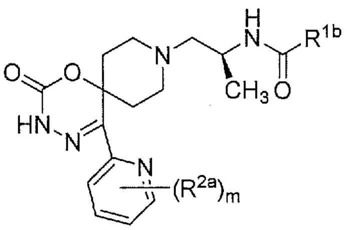

[ XXVIa-7 ]

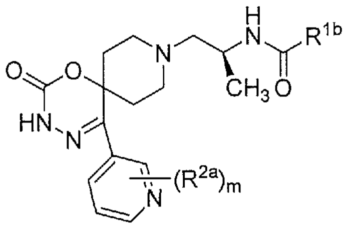

[ XXVIa-8 ]

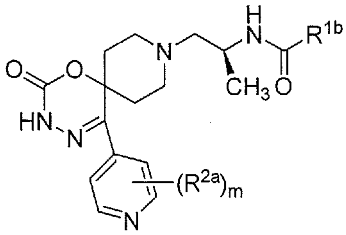

[ XXVIa-9 ]

wherein
$R^{1b}$, $R^{2a}$ and m are as defined above.

Another preferred embodiment of Compound [Ia] is a compound represented by Formula [XXVIIa-1], Formula [XXVIIa-2], Formula [XXVIIa-3], Formula [XXVIIa-4], Formula [XXVIIa-5], Formula [XXVIIa-6], Formula, [XXVIIa-7], Formula [XXVIIa-8], Formula [XXVIIa-9], Formula [XXVIIa-10], or Formula [XXVIIa-11]:

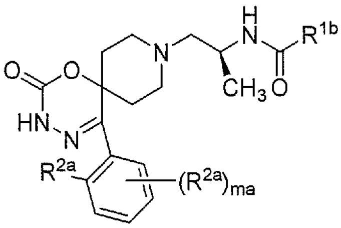

[ XXVIIa-1 ]

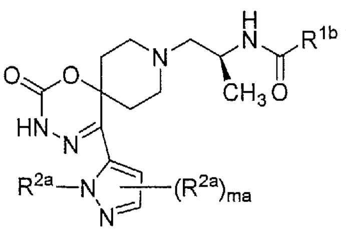

[ XXVIIa-2 ]

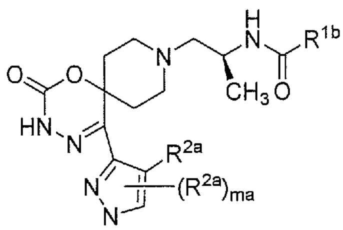

[ XXVIIa-3 ]

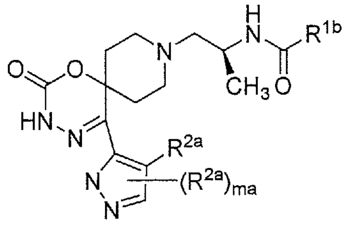

[ XXVIIa-4 ]

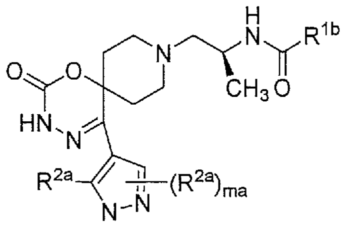

[ XXVIIa-5 ]

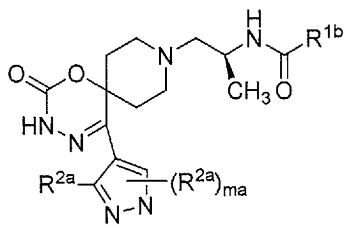

[ XXVIIa-6 ]

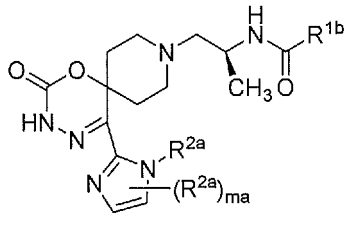

[ XXVIIa-7 ]

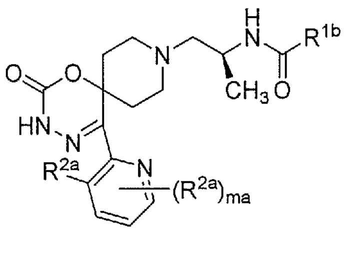

[ XXVIIa-8 ]

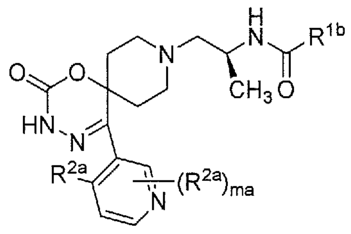

[ XXVIIa-9 ]

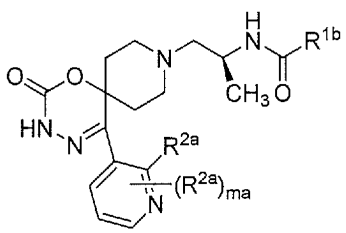

[ XXVIIa-10 ]

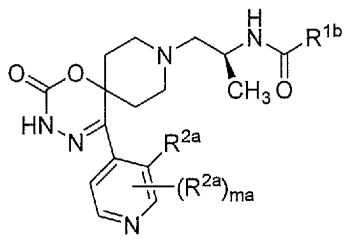

[ XXVIIa-11 ]

wherein

ma is 0, one or two, and
$R^{1b}$ and $R^{2a}$ are as defined above.

Another preferred embodiment of Compound [Ia] is a compound represented by Formula [XXVIIIa]:

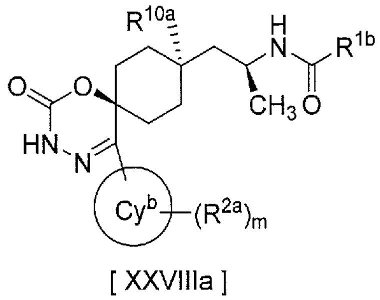

[ XXVIIIa ]

wherein
$Cy^b$, $R^{1b}$, $R^{2a}$, $R^{10a}$ and m are as defined above.
Another preferred embodiment of Compound [Ia] is a compound represented by Formula [XXIXa]:

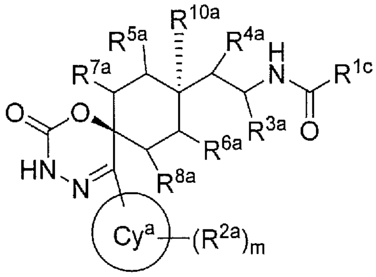

[ XXIXa ]

wherein

Cy$^a$, $R^{1c}$, $R^{2a}$, $R^{3a}$, $R^{4a}$, $R^{5a}$, $R^{6a}$, $R^{7a}$, $R^{8a}$, $R^{10a}$ and m are as defined above.

Another preferred embodiment of Compound [Ia] is a compound represented by Formula [XXXa]:

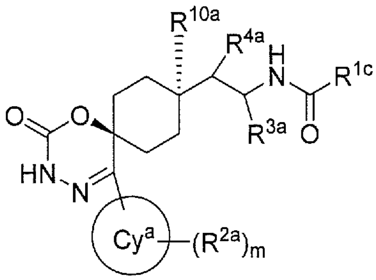

[ XXXa ]

wherein

Cy$^a$, $R^{1c}$, $R^{2a}$, $R^{3a}$, $R^{4a}$, $R^{10a}$ and m are as defined above.

Another preferred embodiment of Compound [Ia] is a compound represented by Formula [XXXIa]:

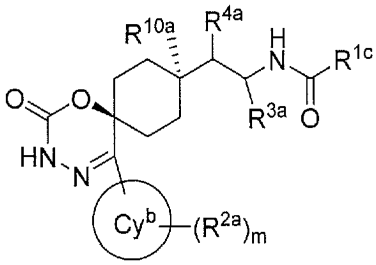

[ XXXIa ]

wherein

Cy$^b$, $R^{1c}$, $R^{2a}$, $R^{3a}$, $R^{4a}$, $R^{10a}$ and m are as defined above.

Another preferred embodiment of Compound [Ia] is a compound represented by Formula [XXXIIa]:

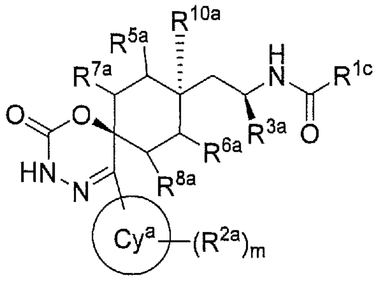

[ XXXIIa ]

wherein

Cya, $R^{1c}$, $R^{2a}$, $R^{3a}$, $R^{5a}$, $R^{6a}$, $R^{7a}$, $R^{8a}$, $R^{10a}$ and m are as defined above.

Another preferred embodiment of Compound [Ia] is a compound represented by Formula [XXXIIIa]:

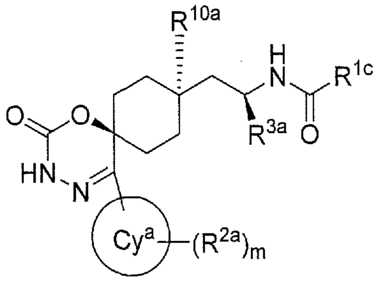

[ XXXIIIa ]

wherein

$Cy^a$, $R^{1c}$, $R^{2a}$, $R^{3a}$, $R^{10a}$ and m are as defined above.

Another preferred embodiment of Compound [Ia] is a compound represented by Formula [XXXIVa]:

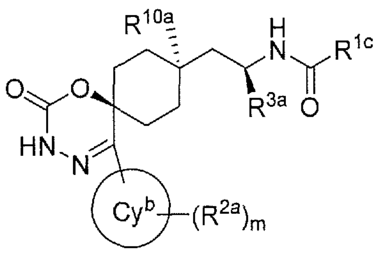

[ XXXIVa ]

wherein

$Cy^b$, $R^{1c}$, $R^{2a}$, $R^{3a}$, $R^{10a}$ and m are as defined above.

Another preferred embodiment of Compound [Ia] is a compound represented by Formula [XXXVa]:

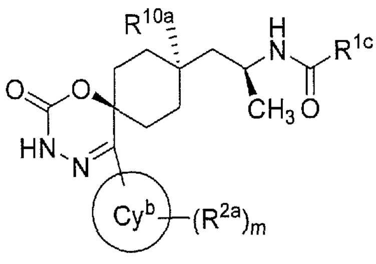

[ XXXVa ]

wherein
$Cy^b$, $R^{1c}$, $R^{2a}$, $R^{10a}$ and m are as defined above.

Another preferred embodiment of Compound [Ia] is a compound represented by Formula [XXXVIa-1], Formula [XXXVIa-2], Formula [XXXVIa-3], Formula [XXXVIa-4], Formula [XXXVIa-5], Formula [XXXVIa-6], Formula [XXXVIa-7], Formula [XXXVIa-8], or Formula [XXXVIa-9]:

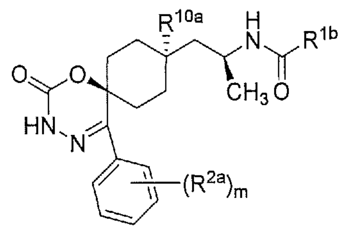
[ XXXVIa-1 ]

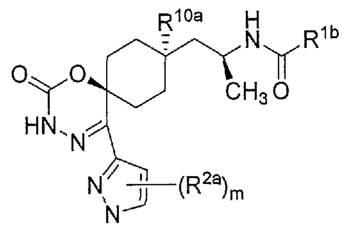
[ XXXVIa-2 ]

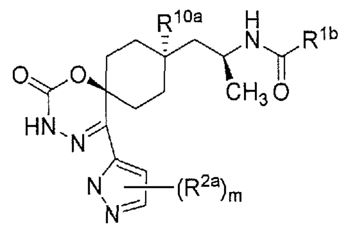
[ XXXVIa-3 ]

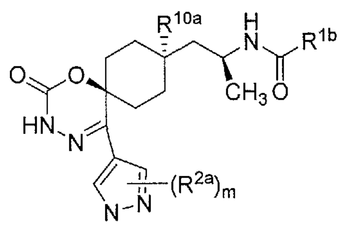
[ XXXVIa-4 ]

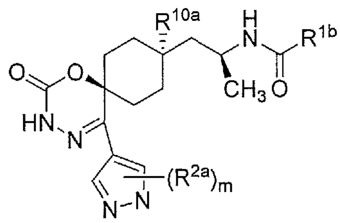
[ XXXVIa-5 ]

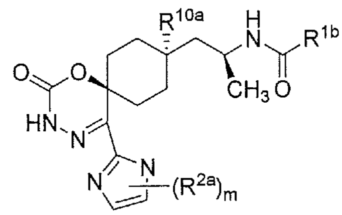
[ XXXVIa-6 ]

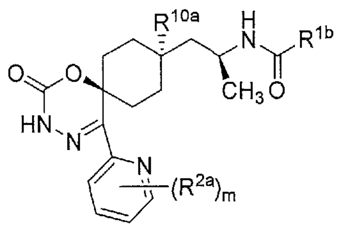
[ XXXVIa-7 ]

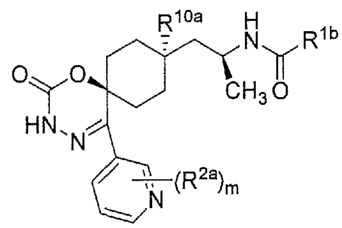
[ XXXVIa-8 ]

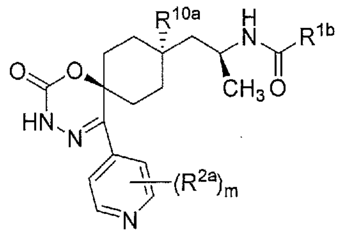
[ XXXVIa-9 ]

wherein
$R^{1b}$, $R^{2a}$, $R^{10a}$ and m are as defined above.

Another preferred embodiment of Compound [Ia] is a compound represented by Formula [XXXVIIa-1], Formula [XXXVIIa-2], Formula [XXXVIIa-3], Formula [XXXVIIa-4], Formula [XXXVIIa-5], Formula [XXXVIIa-6], Formula [XXXVIIa-7], Formula [XXXVIIa-8], Formula [XXXVIIa-9], Formula [XXXVIIa-10], or Formula [XXXVIIa-11]:

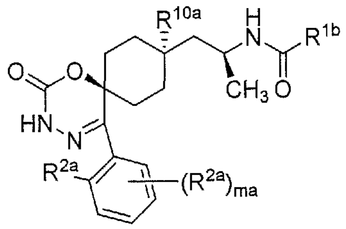
[ XXXVIIa-1 ]

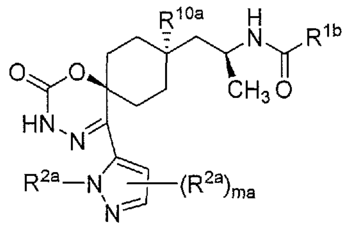
[ XXXVIIa-2 ]

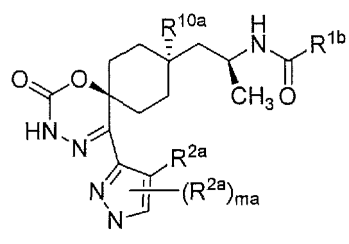
[ XXXVIIa-3 ]

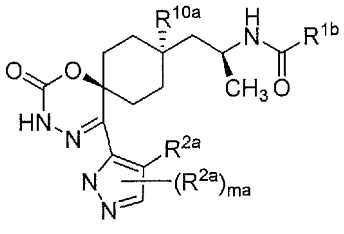

[ XXXVIIa-4 ]

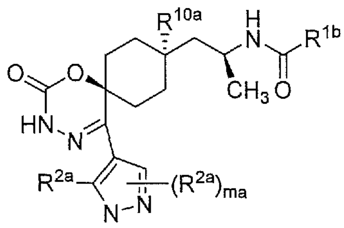

[ XXXVIIa-5 ]

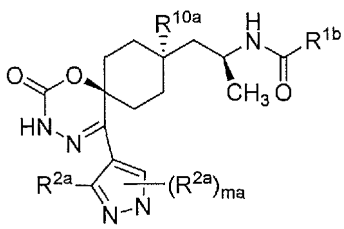

[ XXXVIIa-6 ]

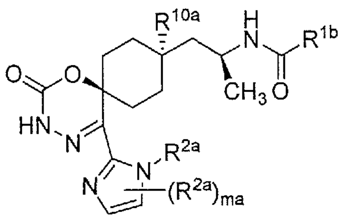

[ XXXVIIa-7 ]

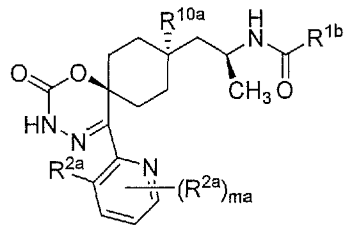

[ XXXVIIa-8 ]

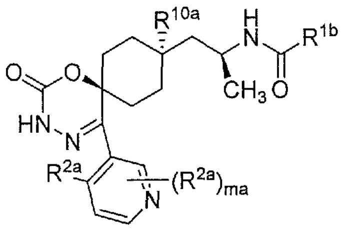

[ XXXVIIa-9 ]

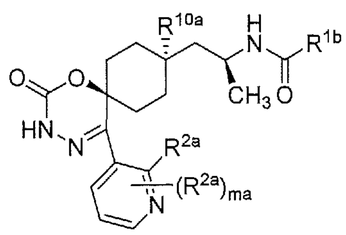

[ XXXVIIa-10 ]

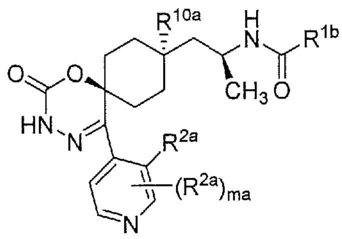

[ XXXVIIa-11 ]

wherein
$R^{1b}$, $R^{2a}$, $R^{10a}$ and ma are as defined above.

Preferred specific embodiments of Compound [Ia] include the compounds of Examples 1 to 339 and Examples 2-001 to 2-172 listed in Table 1-1 to Table 1-61 described later.

The "pharmaceutically acceptable salt" may be any salt known in the art as long as it is not associated with undue toxicity. Specific examples thereof include salts with inorganic acid, salts with organic acid, salts with inorganic base, and salts with organic base. Various forms of pharmaceutically acceptable salts are well known in the art, and they are described in the following documents.

(a) Berge et al., J. Pharm. Sci., 66, p 1-19 (1977),
(b) Stahl et al., "Handbook of Pharmaceutical Salt: Properties, Selection, and Use" (Wiley-VCH, Weinheim, Germany, 2002),
(c) Paulekuhn et al., J. Med. Chem., 50, p 6665-6672 (2007)

The pharmaceutically acceptable salt can be obtained by reacting Compound [I] or Compound [Ia] with an inorganic acid, an organic acid, an inorganic base or an organic base according to a known method.

Examples of the salt with inorganic acid include salts with hydrofluoric acid, hydrochloric acid, hydrobromic acid, hydriodic acid, nitric acid, phosphoric acid and sulfuric acid. Preferred are salts with hydrochloric acid, nitric acid, sulfuric acid, phosphoric acid and hydrobromic acid.

Examples of the salt with organic acid include salts with acetic acid, adipic acid, alginic acid, 4-aminosalicylic acid, anhydromethylenecitric acid, benzoic acid, benzenesulfonic acid, calcium edetate, camphoric acid, camphor-10-sulfonic acid, carbonic acid, citric acid, edetic acid, ethane-1,2-disulfonic acid, dodecylsulfuric acid, ethanesulfonic acid, fumaric acid, glucoheptonic acid, gluconic acid, glucuronic acid, glucoheptonic acid, glycollylarsanilic acid, hexylresorcinoic acid, hydroxynaphthoic acid, 2-hydroxy-1-ethanesulfonic acid, lactic acid, lactobionic acid, malic acid, maleic acid, mandelic acid, methanesulfonic acid, methylsulfuric acid, methylnitric acid, methylenebis(salicylic acid), galactaric acid,

naphthalene-2-sulfonic acid, 2-naphthoic acid, 1,5-naphthalenedisulfonic acid, oleic acid, oxalic acid, pamoic acid, pantothenic acid, pectic acid, picric acid, propionic acid, polygalacturonic acid, salicylic acid, stearic acid, succinic acid, tannic acid, tartaric acid, teoclic acid, thiocyanic acid, trifluoroacetic acid, p-toluenesulfonic acid, undecanoic acid, aspartic acid and glutamic acid. Preferred are salts with oxalic acid, maleic acid, citric acid, fumaric acid, lactic acid, malic acid, succinic acid, tartaric acid, acetic acid, trifluoroacetic acid, benzoic acid, glucuronic acid, oleic acid, pamoic acid, methanesulfonic acid, benzenesulfonic acid, p-toluenesulfonic acid and 2-hydroxy-1-ethanesulfonic acid.

Examples of the salt with inorganic base include salts with lithium, sodium, potassium, magnesium, calcium, barium, aluminium, zinc, bismass and ammonium. Preferred are salts with sodium, potassium, calcium, magnesium and zinc.

Examples of the salt with organic base include salts with arecoline, betaine, choline, clemizole, ethylene diamine, N-methylglucamine, N-benzyl phenethylamine, tris(hydroxymethyl)methylamine, arginine and lysine. Preferred are salts with tris(hydroxymethyl)methylamine, N-methylglucamine and lysine.

Compound [I] or Compound [Ia] or a pharmaceutically acceptable salt thereof may be present as a solvate. The term "solvate" refers to Compound [I] or Compound [Ia] or a pharmaceutically acceptable salt thereof with which a solvent molecule is coordinated. The solvate may be any pharmaceutically acceptable solvate, and include hydrates, ethanolates, acetic acid solvate, dimethyl sulfoxidates or the like of Compound [I] or Compound [Ia] or a pharmaceutically acceptable salt thereof.

Specific examples include semihydrate, monohydrate, dihydrate, mono acetic acid solvate and monoethanolate of Compound [I] or Compound [Ia], monohydrate of sodium salt of Compound [I] or Compound [Ia], and 2/3 ethanolate of monohydrochloride of Compound [I] or Compound [Ia]. These solvates can be obtained according to a known method.

Compound [I] or Compound [Ia] may be present as a tautomer. In this case, Compound [I] or Compound [Ia] can be a single tautomer or a mixture thereof.

Compound [I] or Compound [Ia] may have a carbon-carbon double bond. In this case, Compound [I] or Compound [Ia] can be present as an E form, a Z form, or a mixture thereof.

Compound [I] or Compound [Ia] may be present as a stereoisomer that should be recognized as a cis/trans isomer. In this case, Compound [I] or Compound [Ia] can be present as a cis form, a trans form, or a mixture thereof.

Compound [I] or Compound [Ia] may contain one or more asymmetric carbons. In this case, Compound [I] or Compound [Ia] may be present as a single enantiomer, a single diastereomer, a mixture of enantiomers or a mixture of diastereomers.

Compound [I] or Compound [Ia] may be present as an atropisomer. In this case, Compound [I] or Compound [Ia] may be present as a single atropisomer or a mixture thereof.

Compound [I] or Compound [Ia] may simultaneously contain a plurality of structural features that give rise to the above-mentioned isomers. Moreover, Compound [I] or Compound [Ia] may contain the above-mentioned isomers at any ratio.

The formulae, chemical structures and compound names indicated herein without specifying the stereochemistry thereof includes all of the above isomers that may be present, unless otherwise noted. For example, the structure represented by formula:

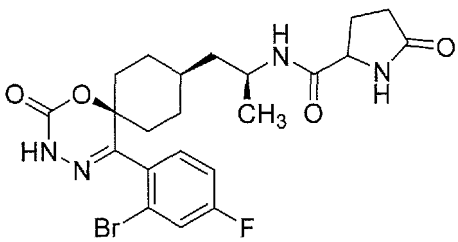

includes all of

(1) a mixture of two diastereomers (the stereochemistry of the asymmetric carbon marked with * is S or R) represented by the following formula:

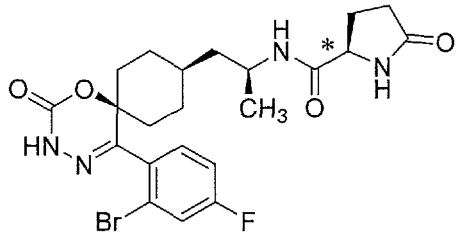 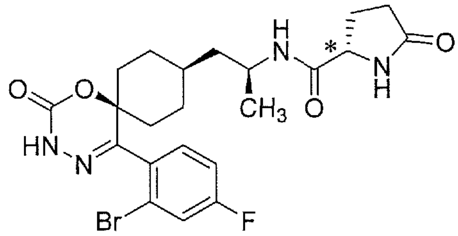 ,

(2) a diastereomer where the stereochemistry of the asymmetric carbon marked with * is S, and
(3) a diastereomer where the stereochemistry of the asymmetric carbon marked with * is R,

unless otherwise noted.

A diastereomeric mixture can be separated into each diastereomer by conventional methods such as chromatography and crystallization. Alternatively, each diastereomer can also be produced by using a stereochemically single starting material, or by a synthesis method employing a stereoselective reaction.

An enantiomeric mixture can be separated into each single enantiomer by a method well known in the art. For example, first, a diastereomeric mixture can be prepared by reacting an enantiomeric mixture with a substantially pure enantiomer compound known as a chiral auxiliary. Next, the obtained diastereomeric mixture can be separated into a single diastereomer having high isomer ratio or a substantially pure single diastereomer by a conventional method such as fractional crystallization and chromatography. Finally, the separated diastereomer can be converted to a desired enantiomer by removing the added chiral auxiliary by cleavage. Moreover, an enantiomeric mixture can also be directly separated by a chromatography method using a chiral solid phase well known in the art. Alternatively, one of enantiomers can also be obtained by using a substantially pure optically active starting material or by employing stereoselective synthesis (asymmetric induction) of a prochiral intermediate using a chiral auxiliary and an asymmetric catalyst.

The absolute steric configuration can be determined by the X-ray crystal analysis of the crystalline product or intermediate. In this case, a crystalline product or intermediate derivatized with a reagent having an asymmetric center with a known steric configuration may be used if necessary.

Compound [I] or Compound [Ia] may be labeled with an isotope [$^{2}$H(D), $^{3}$H, $^{11}$C, $^{13}$C, $^{14}$C, $^{13}$N, $^{15}$N, $^{15}$O, $^{18}$O, $^{18}$F, $^{35}$S, $^{123}$I etc.]. For example, when Compound [I] or Compound [Ia] has methyl, the methyl can be replaced by $-CD_3$. Such labeled Compound [I] or Compound [Ia] is also included in the present invention. Compound [I] or Compound [Ia] labeled with an isotope may be useful for medicaments, pharmacokinetic studies, in vitro and/or in vivo assays, and/or diagnostics (e.g. positron emission tomography (PET), single photon emission computed tomography (SPECT)). Compound [I] or Compound [Ia] labeled with an isotope can be produced using an isotope-labelling reagent instead of a non-isotope-labelling reagent, according to known methods or the methods described here.

Compound [I] or Compound [Ia] or a pharmaceutically acceptable salt thereof is preferably substantially purified, Compound [I] or Compound [Ia] or a pharmaceutically acceptable salt thereof. Further preferably, it is Compound [I] or Compound [Ia] or a pharmaceutically acceptable salt thereof that is purified to a purity of 80% or more.

The pharmaceutical composition of the present invention may be produced by appropriately admixing a therapeutically effective amount of an active ingredient (e.g., Compound [I] or Compound [Ia] or a pharmaceutically acceptable salt thereof) with at least one kind of a pharmaceutically acceptable carrier and the like, according to a method known in the art of pharmaceutical preparations. The content of the active ingredient in the pharmaceutical composition varies depending on the dosage form, the dose, and the like. It is, for example, 0.1 to 100 wt% of the whole composition.

Examples of the dosage form of the pharmaceutical composition containing Compound [I] or Compound [Ia] or a pharmaceutically acceptable salt thereof include oral preparations such as tablet, capsule, granule, powder, troche, syrup, emulsion and suspension, and parenteral preparations such as external preparation, suppository, injection, eye drop, nasal preparations and pulmonary preparation.

Examples of the "pharmaceutically acceptable carrier" include various organic or inorganic carrier substances conventionally used as preparation materials, and specifically include excipient, disintegrant, binder, glidant, lubricant and the like for solid preparations; solvent, solubilizing agent, suspending agent, isotonicity agent, buffering agent, soothing agent and the like for liquid preparations; and base, emulsifier, moistening agent, stabilizer, stabilizing agent, dispersant, plasticizer, pH adjuster, absorption enhancer, gelling agent, preservative, filler, solvent, solubilizing agent, suspending agent and the like for semi-solid preparations. Where necessary, additives such as preservative, antioxidant, colorant, sweetening agent and the like may be used.

Examples of the "excipient" include lactose, sucrose, D-mannitol, D-sorbitol, corn starch, dextrin, microcrystalline cellulose, crystalline cellulose, carmellose, carmellose calcium, sodium carboxymethyl starch, low-substituted hydroxypropylcellulose, gum arabic and the like.

Examples of the "disintegrant" include carmellose, carmellose calcium, carmellose sodium, sodium carboxymethyl starch, croscarmellose sodium, crospovidone, low-substituted hydroxypropylcellulose, hydroxypropylmethylcellulose, crystalline cellulose and the like.

Examples of the "binder" include hydroxypropylcellulose, hydroxypropylmethylcellulose, povidone, crystalline cellulose, sucrose, dextrin, starch, gelatin, carmellose sodium, gum arabic and the like.

Examples of the "glidant" include light anhydrous silicic acid, magnesium stearate and the like.

Examples of the "lubricant" include magnesium stearate, calcium stearate, talc and the like.

Examples of the "solvent" include purified water, ethanol, propylene glycol, macrogol, sesame oil, corn oil, olive oil and the like.

Examples of the "solubilizing agent" include propylene glycol, D-mannitol, benzyl benzoate, ethanol, triethanolamine, sodium carbonate, sodium citrate and the like.

Examples of the "suspending agent" include benzalkonium chloride, carmellose, hydroxypropylcellulose, propylene glycol, povidone, methylcellulose, glycerol monostearate and the like.

Examples of the "isotonic agent" include glucose, D-sorbitol, sodium chloride, D-mannitol and the like.

Examples of the "buffering agent" include sodium hydrogenphosphate, sodium acetate, sodium carbonate, sodium citrate and the like.

Examples of the "soothing agent" include benzyl alcohol and the like.

Examples of the "base" include water, animal and vegetable oils (olive oil, corn oil, arachis oil, sesame oil, castor oil etc.), lower alcohols (ethanol, propanol, propylene glycol, 1,3-butylene glycol, phenol etc.), higher fatty acids and esters thereof, wax, higher alcohols, polyalcohols, hydrocarbons (white vaseline, liquid paraffin, paraffin etc.), hydrophilic vaseline, purified lanolin, absorptive ointment, hydrous lanolin, hydrophilic ointment, starch, pullulan, gum arabic, tragacanth gum, gelatin, dextran, cellulose derivatives (methyl cellulose, carboxymethyl cellulose, hydroxyethyl cellulose, hydroxypropyl cellulose etc.), synthetic polymers (carboxyvinyl polymer, sodium polyacrylate, polyvinyl alcohol, polyvinyl pyrrolidone etc.), propylene glycol, Macrogol (Macrogol 200 to 600), and combinations of two or more types thereof.

Examples of the "preservative" include ethyl parahydroxybenzoate, chlorobutanol, benzyl alcohol, sodium dehydroacetate, sorbic acid and the like.

Examples of the "antioxidant" include sodium sulfite, ascorbic acid and the like.

Examples of the "colorant" include food colors (Food Color Red No. 2 or 3, and Food Color Yellow No. 4 or 5 etc.), β-carotene and the like.

Examples of the "sweetening agent" include saccharin sodium, dipotassium glycyrrhizinate, aspartame and the like.

The pharmaceutical composition can be administered orally or parenterally (topically, rectally, intravenously, intramuscularly, and subcutaneously etc.) to human as well as mammals other than human (mouse, rat, hamster, guinea pig, rabbit, cat, dog, swine, bovine, horse, sheep, monkey etc.). The dose (hereinafter, also to be referred to as "therapeutically effective amount") varies depending on administration subject, administration route, target disease, symptom, severity of disease, dosage form and the like. For example, when administered orally to a human (adult patient) (body weight 60 kg), the lower limit of the therapeutically effective amount is, for example, about 0.01 mg, about 0.1 mg, about 0.5 mg, about 1 mg, about 10 mg, about 20 mg or about 50 mg per day, and the upper limit of the therapeutically effective amount is, for example, about 1 mg, about 5 mg, about 10 mg, about 20 mg, about 50 mg, about 100 mg, about 200 mg, about 500 mg or about 1000 mg per day. This amount can be administered in once, twice, three or more times a day.

The expression "inhibiting PLD" means to eliminate or attenuate the activity of PLD by inhibiting its function. For example, it means to inhibit the function as PLD based on the conditions in the below-mentioned Experimental Example 1 and/or Experimental Example 2. Inhibition of the function of PLD or elimination or attenuation of the activity of PLD is preferably performed in human clinical indications.

One embodiment of the "inhibiting PLD" is "inhibiting PLD1"

The expression "inhibiting PLD1" means to eliminate or attenuate the activity of PLD1 by inhibiting its function. For example, it means to inhibit the function as PLD1 based on the conditions in the below-mentioned Experimental Example 1 and/or Experimental Example 2. Inhibition of the function of PLD1 or elimination or attenuation of the activity of PLD1 is preferably performed in human clinical indications.

One embodiment of the "inhibiting PLD" is "inhibiting PLD1/2"

The expression "inhibiting PLD1/2" means to eliminate or attenuate the activities of PLD1 and PLD2 by inhibiting their functions. For example, it means to inhibit the functions as PLD1 and PLD2 based on the conditions in the below-mentioned Experimental Example 1 and/or Experimental Example 2. Inhibition of the functions of PLD1 and PLD2 or elimination or attenuation of the activities of PLD1 and PLD2 is preferably performed in human clinical indications.

The "inhibiting PLD" is preferably "inhibiting human PLD".

The "PLD inhibitor" means a substance that binds to PLD and inhibits the function of PLD. The "PLD inhibitor" is preferably "human PLD inhibitor".

One embodiment of the "PLD inhibitor" is "PLD1 inhibitor".

The "PLD1 inhibitor" means a substance that binds to PLD1 and inhibits the function of PLD1. The "PLD1

inhibitor" is preferably "human PLD1 inhibitor".

One embodiment of the "PLD inhibitor" is "PLD1/2 inhibitor".

The "PLD1/2 inhibitor" means a substance that binds to PLD1 and PLD2 and inhibits the functions of PLD1 and PLD2. The "PLD1/2 inhibitor" is preferably "human PLD1/2 inhibitor".

Compound [I] or Compound [Ia] or a pharmaceutically acceptable salt thereof has a PLD inhibitory activity, and may be useful for the treatment and/or prophylaxis of various diseases or conditions expected to be improved by controlling PLD activity. Examples of various diseases or conditions expected to be improved by controlling PLD activity include thrombosis (e.g., arterial thrombosis, acute coronary syndrome, stable angina, unstable angina, non-ST elevation myocardial infarction, ST elevation myocardial infarction, ischemic stroke, non-cardioembolic stroke, atherosclerotic stroke, cryptogenic stroke, embolic stroke of undetermined source (ESUS), lacunar stroke, transient ischemic attack, peripheral arterial disease, etc.), thrombosis during perioperative and postoperative periods associated with revascularization (coronary artery bypass graft, percutaneous coronary intervention, carotid endarterectomy, carotid artery stenting, thrombolysis, lower extremity revascularization, etc.) or aortic valve replacement (surgical aortic valve replacement, transcatheter aortic valve replacement, etc.), and cancers (e.g., breast cancer, ovarian cancer, lung cancer, colorectal cancer, kidney cancer, pancreatic cancer, prostate cancer, brain tumor, etc.).

As used herein, the term "treatment" includes improvement of symptoms, prevention of severity, maintenance of remission, prevention of exacerbation, secondary prevention and prevention of relapse.

As used herein, the term "prophylaxis" includes suppressing the onset of symptoms.

In some embodiments, the PLD inhibitor or pharmaceutical composition may be provided in the form of a kit (administration, treatment and/or prevention kits, etc.), a package (packaging good, etc.) and a medicine set (and/or a container), associated therewith a written matter stating that it can or should be used for the prophylaxis or treatment of the above-mentioned diseases. Such kit, package and medicine set may contain one or more containers filled with PLD inhibitor and/or other medicines or drugs (or components). Examples of such kit, package and medicine set include commercial kits, commercial packages and commercial medicines, which are appropriately directed to the treatment and/or prevention of target diseases. Examples of the written matter contained therein include precautions or package inserts in the form directed by the government organization which regulates manufacture, use or sale of pharmaceutical or biological products, which show the approval of the government organization about manufacture, use or sale of the products related to human administration. In the above-mentioned kit, package and medicine set, packed products may also be included, and structures constructed for suitable administration step may also be included, and structures constructed to achieve more preferred medical treatment and/or prevention, including treatment and/or prevention of the target diseases, may also be included.

Compound [I] or Compound [Ia] or a pharmaceutically acceptable salt thereof of the present invention can be used in combination with one or a plurality of other medicaments (hereinafter to be also referred to as "concomitant drug") (hereinafter to be referred to as combined use), as long as the medicinal effects thereof are not impaired. The administration period is not limited, and they may be administered to an administration subject simultaneously or at given intervals. In addition, the combination of Compound [I] or Compound [Ia] or pharmaceutically acceptable salt thereof and the concomitant drug can also be administered as a single preparation. The administration form of the concomitant drug is not particularly limited, and it is only required that Compound [I] or Compound [Ia] or a pharmaceutically acceptable salt thereof of the present invention is combined with a concomitant drug.

The dose of the concomitant drug can be appropriately selected based on the dose used in clinical practice. The ratio of Compound [I] or Compound [Ia] or a pharmaceutically acceptable salt thereof of the present invention to the concomitant drug can be appropriately selected according to the subject of administration, route of administration, target disease, symptoms and combination.

The combination of PLD inhibitors with existing thrombotic agents is expected to suppress arterial thrombus formation without increasing a risk of bleeding.

Examples of the concomitant drug include anti-platelet agents, anti-coagulants, thrombolytic agents and the like.

Examples of the anti-platelet agent include aspirin, ticlopidine, cilostazol, clopidogrel, prasugrel, ticagrelor, selatogrel, glenzoshimab, sarunfiban, revacept, anfibatide, PZ-128, vikagrel, tirofiban, abciximab and the like.

Examples of the anti-coagulant include warfarin, rivaroxaban, apixaban, edoxaban, dabigatran, heparin, heparinoid, fondaparinux, argatroban, tecarfarin, abelacimab, milvexian, asundexian, BAY-2306001, TRx1, osocimab, AB-023, and dimolegin and the like.

Examples of the thrombolytic agent include recombinant tissue plasminogen activator, urokinase and the like.

The presentation herein of preferred embodiments and options of the compounds, methods, uses and compositions of the present invention also includes the presentation of combinations of such preferred embodiments and options, as long as they are combinable and consistent.

[General Production Method]

The general production method of Compound [I] or Compound [Ia] or a pharmaceutically acceptable salt thereof is explained in the following. However, the general production method of Compound [I] or Compound [Ia] or a pharmaceutically acceptable salt thereof is not limited to such production methods. Unless otherwise referred, the salt of each compound in general production methods can be selected appropriately from the above "pharmaceutically acceptable salt".

The compound obtained in each step can be, if necessary, isolated or purified according to a method known per se such as distillation, recrystallization and column chromatography, or directly used in the next step without isolation or purification.

As used herein, the room temperature refers to a temperature in an uncontrolled state, and one embodiment includes 1°C to 40°C.

The abbreviations used are as follows.

THF: tetrahydrofuran
DMF: N,N-dimethylformamide
$CDCl_3$: deuteration chloroform
Pt-C: platinum-activated carbon
Pd-C: palladium-activated carbon
DMSO: dimethyl sulfoxide
LiHMDS: lithium bis(trimethylsilyl)amide
NaHMDS: sodium bis(trimethylsilyl)amide
TBAF: tetra-n-butylammonium fluoride
DBU: diazabicycloundecene
CDI: 1,1'-carbonyldiimidazole
WSC: 1-ethyl-3-(3-dimethylaminopropyl)carbodiimide hydrochloride
HATU: 1-[bis(dimethylamino)methylene]-1H-1,2,3-triazole[4,5-b]pyridinium 3-oxide hexafluorophosphate
DMT-MM: 4-(4,6-dimethoxy-1,3,5-triazin-2-yl)-4-methylmorpholinium chloride
HOAt: 1-hydroxy-7-azabenzotriazole
DMAP: 4-dimethylaminopyridine
CPME: cyclopentyl methyl ether
DMA: dimethylacetamide
$Boc_2O$: di-tert-butyl dicarbonate
LDA: lithium diisopropylamide

Production Method A1: production method of Compound [I] or a salt thereof

Compound [I] or a salt thereof can be produced, for example, according to the following Production Method A1.

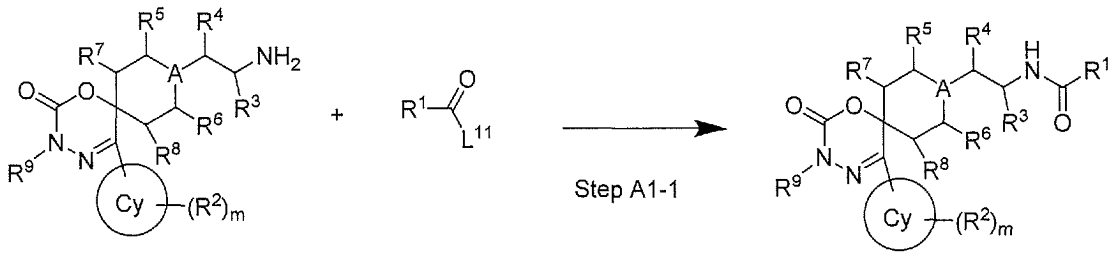


[A1-1] [A1-2] [I]

wherein

A, Cy, $R^1$, $R^2$, $R^3$, $R^4$, $R^5$, $R^6$, $R^7$, $R^8$, $R^9$ and m are as defined above, and
$L^{11}$ is hydroxy or a leaving group (for example, halogen, etc.).

(Step A1-1)

Compound [I] or a salt thereof can be produced by reacting Compound [A1-1] or a salt thereof with Compound

[A1-2] or a salt thereof.

For example, when $L^{11}$ is halogen, Compound [I] or a salt thereof can be produced by reacting Compound [A1-1] or a salt thereof with Compound [A1-2] or a salt thereof in the presence of a base, in a solvent.

Examples of the base include triethylamine, N,N-diisopropylethylamine and the like. The preferred base is N,N-diisopropylethylamine.

Examples of the solvent include DMF, acetonitrile, THF and the like. The preferred solvent is DMF.

The reaction temperature is, for example, 0°C to 60°C, preferably 20°C to 40°C.

Alternatively, for example, when $L^{11}$ is hydroxy, Compound [I] or a salt thereof can also be produced by reacting Compound [A1-1] or a salt thereof with Compound [A1-2] or a salt thereof in the presence of a condensing agent and a base, in a solvent.

Examples of the base include triethylamine, N,N-diisopropylethylamine and the like. The preferred base is N,N-diisopropylethylamine.

Examples of the condensing agent include WSC, HATU, DMT-MM and the like. The preferred condensing agent is WSC or HATU.

Examples of the solvent include DMF, acetonitrile, methanol, THF and the like. The preferred solvent is DMF.

The reaction temperature is, for example, 0°C to 60°C, preferably 20°C to 40°C.

Compound [A1-1] or a salt thereof may be a commercially available product, or can be produced from a commercially available product by a known method, for example, it can be also produced according to Production Methods B1 and B2 described below.

Compound [A1-2] or a salt thereof may be a commercially available product, or can be produced from a commercially available product by a known method.

Alternatively, Compound [I] or a salt thereof can also be produced by carrying out this production method using compound or a salt thereof having a functional group or a protected functional group on Cy that can be converted to $R^2$ by a known reaction, instead of Compound [A1-1] or a salt thereof to obtain a compound corresponding to Compound [I], or a salt thereof, and then converting the functional group of the compound or salt thereof to $R^2$.

<u>Production Method A2: production method of Compound [I-2] or a salt thereof</u>

Compound [I] or a salt thereof wherein $R^1$ is $NR^{31}R^{32}$, i.e., Compound [I-2] or a salt thereof, can be produced, for example, according to the following Production Method A2.

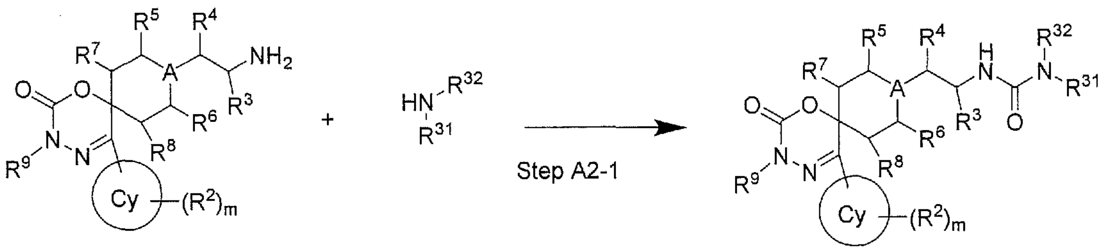


[A1-1] [A2-1] [ I-2 ]

wherein

A, Cy, $R^2$, $R^3$, $R^4$, $R^5$, $R^6$, $R^7$, $R^8$, $R^9$ and m are as defined above, and
$R^{31}$ and $R^{32}$ are each independently

(1) hydrogen,
(2) $C_{1-4}$ alkyl wherein the alkyl is optionally substituted by

(a) phenyl wherein the phenyl is optionally substituted by halogen, or
(b) pyridyl,

(3) $C_{1-4}$ alkoxy, or
(4) $C_{3-4}$ cycloalkyl, or
$R^{31}$ and $R^{32}$ are bonded to each other to form, together with the nitrogen atoms to which they are bonded,
(5) 4 to 7-membered heterocycloalkyl containing one or two heteroatoms selected from the group consisting of nitrogen and oxygen atoms as a ring constituting atom, besides carbon atom, wherein the heterocycloalkyl is

optionally substituted by

(a) hydroxy,
(b) oxo,
(c) $NR^{20}R^{21}$ wherein $R^{20}$ and $R^{21}$ are each independently hydrogen or $C_{1-4}$ alkyl, or
(d) phenyl,

(6) 6 to 11-membered spiro heterocycloalkyl containing one or two heteroatoms selected from the group consisting of nitrogen and oxygen atoms as a ring constituting atom, besides carbon atom, or
(7) a 8 to 10-membered saturated or partially unsaturated fused cyclic group containing 1 to 4 heteroatoms selected from the group consisting of nitrogen and oxygen atoms as a ring constituting atom, besides carbon atom, wherein the fused cyclic group is optionally substituted by $C_{1-4}$ haloalkyl.

(Step A2-1)

Compound [I-2] or a salt thereof can be produced by reacting Compound [A1-1] or a salt thereof with Compound [A2-1] or a salt thereof in the presence of an ureating agent and a base, in a solvent.
Examples of the ureating agent include CDI, triphosgene and the like. The preferred ureating agent is CDI.
Examples of the base include N-methylmorpholine, triethylamine, N,N-diisopropylethylamine and the like. The preferred base is N-methylmorpholine.
Examples of the solvent include THF, acetonitrile, DMF, water, mixed solvent thereof and the like. The preferred solvent is THF or a mixed solvent of THF and water.
The reaction temperature is, for example, 0°C to 80°C, preferably 30°C to 60°C.
Compound [A2-1] or a salt thereof may be a commercially available product, or can be produced from a commercially available product by a known method.
Alternatively, Compound [I-2] or a salt thereof can also be produced by carrying out this production method using compound or a salt thereof having a functional group or a protected functional group on Cy that can be converted to $R^2$ by a known reaction, instead of Compound [A1-1] or a salt thereof to obtain a compound corresponding to Compound [I-2], or a salt thereof, and then converting the functional group of the compound or salt thereof to $R^2$.

Production Method A3: production method of Compound [Ia] or a salt thereof

Compound [Ia] or a salt thereof can be produced, for example, according to the following Production Method A3.

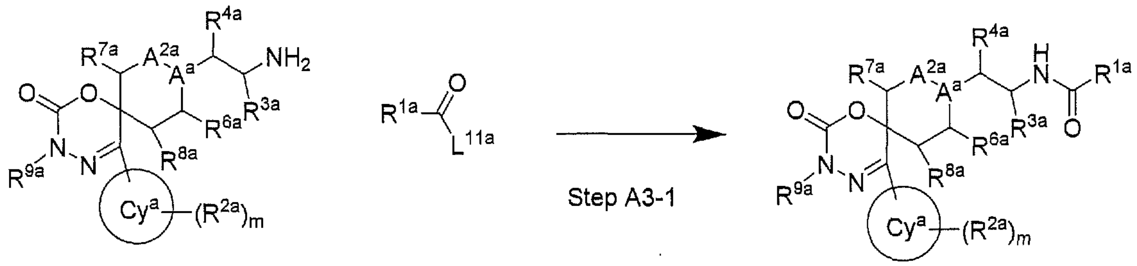


[A3-1]   [A3-2]   [ Ia ]

wherein

$A^a$, $A^{2a}$, $Cy^a$, $R^{1a}$, $R^{2a}$, R3a, $R^{4a}$, $R^{6a}$, $R^{7a}$, $R^{8a}$, $R^{9a}$ and m are as defined above, and
$L^{11a}$ is hydroxy or a leaving group (for example, halogen, etc.).

(Step A3-1)

Compound [I] or a salt thereof can be produced by reacting Compound [A3-1] or a salt thereof with Compound [A3-2] or a salt thereof.
For example, when $L^{11a}$ is halogen, Compound [Ia] or a salt thereof can be produced by reacting Compound [A3-1] or a salt thereof with Compound [A3-2] or a salt thereof in the presence of a base, in a solvent.
Examples of the base include triethylamine, N,N-diisopropylethylamine and the like. The preferred base is N,N-diisopropylethylamine.

Examples of the solvent include DMF, acetonitrile, THF and the like. The preferred solvent is DMF.

The reaction temperature is, for example, 0°C to 60°C, preferably 20°C to 40°C.

Alternatively, for example, when $L^{11a}$ is hydroxy, Compound [Ia] or a salt thereof can also be produced by reacting Compound [A3-1] or a salt thereof with Compound [A3-2] or a salt thereof in the presence of a condensing agent and a base, in a solvent.

Examples of the base include triethylamine, N,N-diisopropylethylamine and the like. The preferred base is N,N-diisopropylethylamine.

Examples of the condensing agent include WSC, HATU, DMT-MM and the like. The preferred condensing agent is WSC or HATU.

Examples of the solvent include DMF, acetonitrile, methanol, THF and the like. The preferred solvent is DMF.

The reaction temperature is, for example, 0°C to 60°C, preferably 20°C to 40°C.

Compound [A3-1] or a salt thereof may be a commercially available product, or can be produced from a commercially available product by a known method, for example, it can be also produced according to Production Methods B3 and B4 described below.

Compound [A3-2] or a salt thereof may be a commercially available product, or can be produced from a commercially available product by a known method.

Alternatively, Compound [Ia] or a salt thereof can also be produced by carrying out this production method using compound or a salt thereof having a functional group or a protected functional group on $Cy^a$ that can be converted to $R^{2a}$ by a known reaction, instead of Compound [A3-1] or a salt thereof to obtain a compound corresponding to Compound [Ia], or a salt thereof, and then converting the functional group of the compound or salt thereof to $R^{2a}$,

Production Method A4: production method of Compound [Ia-2] or a salt thereof

Compound [Ia] or a salt thereof wherein $R^{1a}$ is $NR^{31}R^{32}$, i.e., Compound [Ia-2] or a salt thereof, can be produced, for example, according to the following Production Method A4.

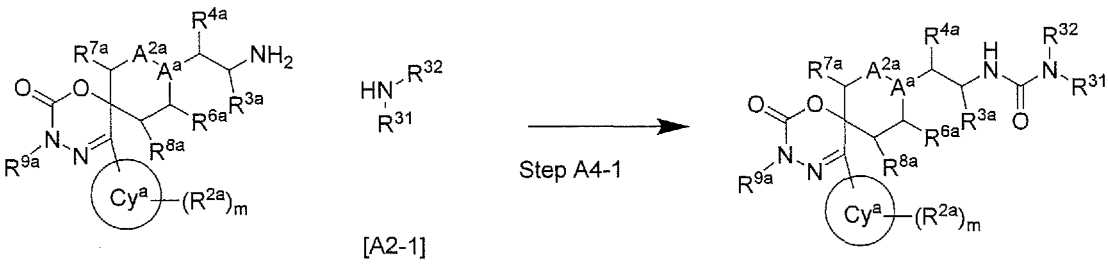

[A3-1] [A2-1] [ Ia-2 ]

wherein

$A^a$, $Cy^a$, $R^{2a}$, $R^{3a}$, $R^{4a}$, $R^{5a}$, $R^{6a}$, $R^{7a}$, $R^{8a}$, $R^{9a}$, $R^{31}$, $R^{32}$ and m are as defined above.

(Step A4-1)

Compound [Ia-2] or a salt thereof can be produced by reacting Compound [A3-1] or a salt thereof with Compound [A2-1] or a salt thereof in the presence of an ureating agent and a base, in a solvent.

Examples of the ureating agent include CDI, triphosgene and the like. The preferred ureating agent is CDI.

Examples of the base include N-methylmorpholine, triethylamine, N,N-diisopropylethylamine and the like. The preferred base is N-methylmorpholine.

Examples of the solvent include THF, acetonitrile, DMF, water, mixed solvent thereof and the like. The preferred solvent is THF or a mixed solvent of THF and water.

The reaction temperature is, for example, 0°C to 80°C, preferably 30°C to 60°C.

Alternatively, Compound [Ia-2] or a salt thereof can also be produced by carrying out this production method using compound or a salt thereof having a functional group or a protected functional group on $Cy^2$ that can be converted to $R^{2a}$ by a known reaction, instead of Compound [A3-1] or a salt thereof to obtain a compound corresponding to Compound [Ia-2], or a salt thereof, and then converting the functional group of the compound or salt thereof to $R^{2a}$.

Production Method B1: production method of Compound [B1-7] or a salt thereof

Compound [A1-1] or a salt thereof (used in Production Methods A1 and A2) wherein A is $CR^{10}$ and $R^9$ is hydrogen, i.e., Compound [B1-7] or a salt thereof, can be produced, for example, according to the following Production Method B1.

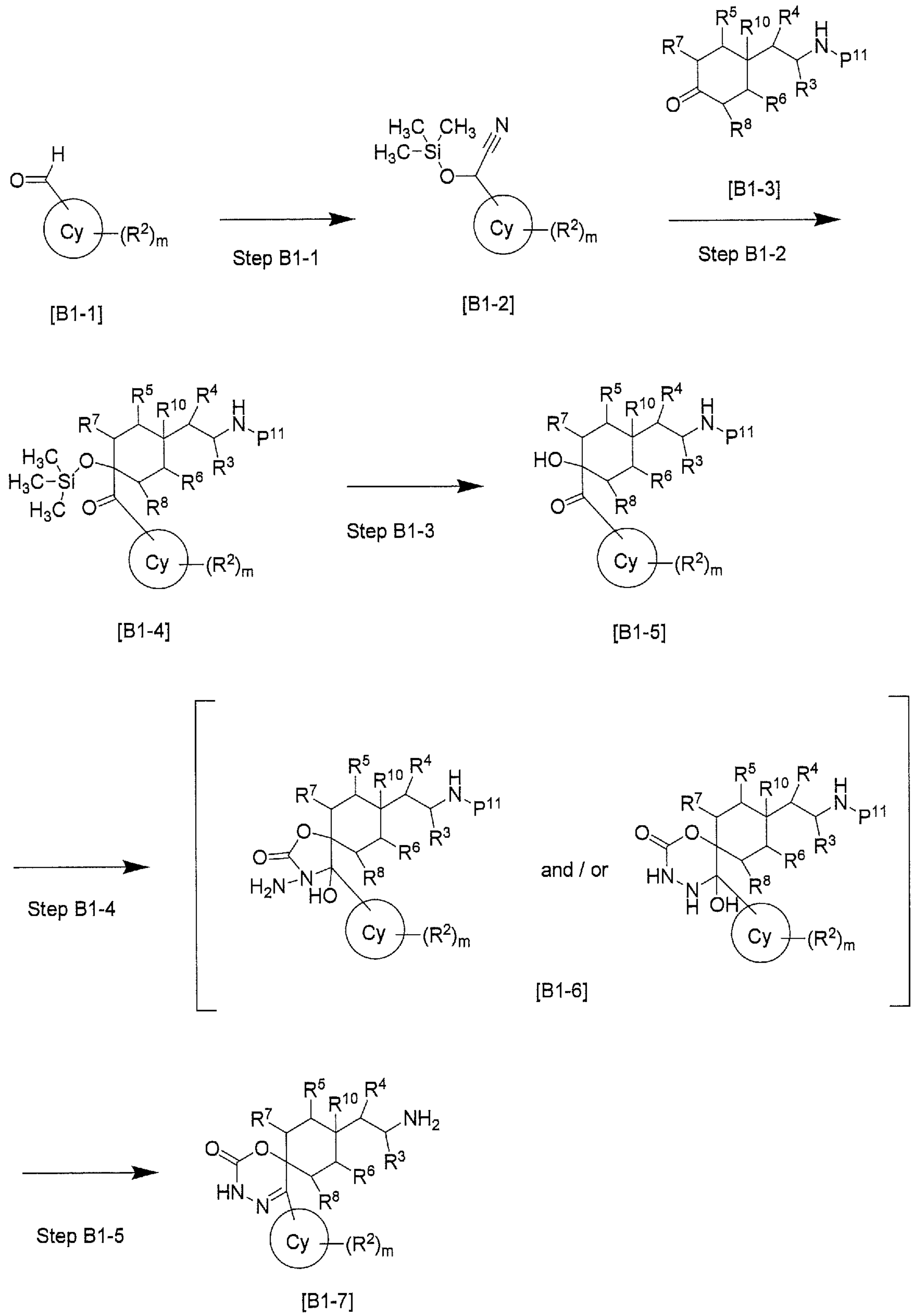

wherein

Cy, $R^2$, $R^3$, $R^4$, $R^5$, $R^6$, $R^7$, $R^8$, $R^{10}$ and m are as defined above, and
$P^{11}$ is a protecting group for amine (for example, tert-butoxycarbonyl, benzyloxycarbonyl, etc.).

(Step B1-1)

Compound [B1-2] or a salt thereof can be produced by reacting Compound [B1-1] or a salt thereof with trimethylsilylcyanide in the presence of a catalyst, in a solvent.
Examples of the catalyst include lithium chloride, DMAP and the like. The preferred catalyst is lithium chloride or DMAP.
Examples of the solvent include THF, acetonitrile and the like. The preferred solvent is THF.
The reaction temperature is, for example, 0°C to 60°C, preferably 15°C to 30°C.
Compound [B1-1] or a salt thereof may be a commercially available product, or can be produced from a commercially available product by a known method.

(Step B1-2)

Compound [B1-4] or a salt thereof can be produced by reacting Compound [B1-2] or a salt thereof with Compound [B1-3] or a salt thereof in the presence of a catalyst and a base, in a solvent. Step B1-1 and Step B1-2 can also be carried out in one-pot.
Examples of the catalyst include lithium chloride, DMAP and the like. The preferred catalyst is lithium chloride or DMAP.
Examples of the base include LiHMDS, NaHMDS, LDA, n-butyllithium and the like. The preferred base is LiHMDS or NaHMDS.
Examples of the solvent include THF, n-hexane, toluene and the like. The preferred solvent is THF or toluene.
The reaction temperature is, for example, -100°C to 0°C, preferably -78°C to -50°C.
Compound [B1-3] or a salt thereof may be a commercially available product, or can be produced from a commercially available product by a known method, for example, it can be also produced according to Production Method C1 described below.

(Step B1-3)

Compound [B1-5] or a salt thereof can be produced by reacting Compound [B1-4] or a salt thereof in the presence of a desilylating agent and an acid, in a solvent.
Examples of the desilylating agent include TBAF, hydrogen fluoride-pyridine and the like. The preferred desilylating agent is TBAF.
Examples of the acid include acetic acid and the like. The preferred acid is acetic acid.
Examples of the solvent include THF, acetonitrile and the like. The preferred solvent is THF.
The reaction temperature is, for example, 0°C to 60°C, preferably 15°C to 30°C.

(Step B1-4)

Compound [B1-6] or a salt thereof can be produced by reacting Compound [B1-5] or a salt thereof in the presence of a base, CDI and hydrazine monohydrate, in a solvent.
Examples of the base include DBU, N-methylimidazole and the like. The preferred base is DBU.
Examples of the solvent include THF, DMF, acetonitrile and the like. The preferred solvent is THF or DMF.
The reaction temperature is, for example, 0°C to 60°C, preferably 20°C to 40°C.

(Step B1-5)

Compound [B1-7] or a salt thereof can be produced by reacting Compound [B1-6] or a salt thereof in the presence of an acid, in a solvent.
Examples of the acid include hydrochloric acid, acetic acid and the like. The preferred acid is hydrochloric acid.
Examples of the solvent include THF, water, toluene and the like. The preferred solvent is THF.
The reaction temperature is, for example, 15°C to 120°C, preferably 20°C to 60°C.
Alternatively, Compound [B1-7] or a salt thereof can also be produced by carrying out this production method using compound or a salt thereof having a functional group or a protected functional group on Cy that can be converted to

$R^2$ by a known reaction, instead of Compound [B1-1] or a salt thereof to obtain a compound corresponding to Compound [B1-7], or a salt thereof, and then converting the functional group of the compound or salt thereof to $R^2$.

Production Method B2: production method of Compound [B2-8] or a salt thereof

Compound [A1-1] or a salt thereof (used in Production Methods A1 and A2) wherein A is N and $R^9$ is hydrogen, i.e., Compound [B2-8] or a salt thereof, can be produced, for example, according to the following Production Method B2.

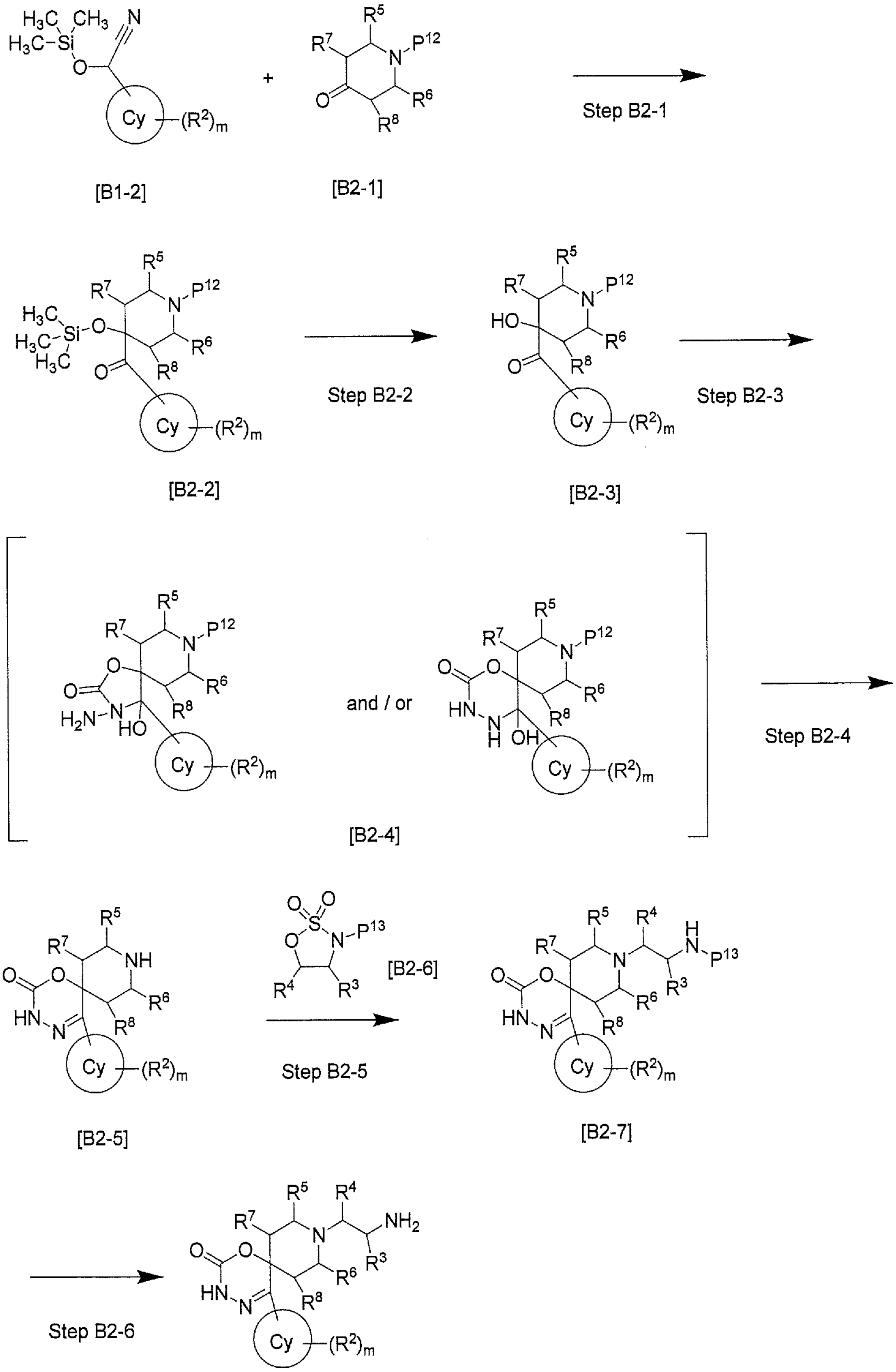


[B1-2] [B2-1]

[B2-2] [B2-3]

and / or

[B2-4]

[B2-6]

[B2-5] [B2-7]

[B2-8]

wherein

Cy, $R^2$, $R^3$, $R^4$, $R^5$, $R^6$, $R^7$, $R^8$ and m are as defined above, and
$P^{12}$ and $P^{13}$ are each independently a protecting group for amine (for example, tert-butoxycarbonyl, etc.).

(Step B2-1)

Compound [B2-2] or a salt thereof can be produced by reacting Compound [B1-2] or a salt thereof with Compound [B2-1] or a salt thereof according to Step B1-2.

Compound [B2-1] or a salt thereof may be a commercially available product, or can be produced from a commercially available product by a known method.

(Step B2-2)

Compound [B2-3] or a salt thereof can be produced by reacting Compound [B2-2] or a salt thereof according to Step B1-3.

(Step B2-3)

Compound [B2-4] or a salt thereof can be produced by reacting Compound [B2-3] or a salt thereof according to Step B1-4.

(Step B2-4)

Compound [B2-5] or a salt thereof can be produced by reacting Compound [B2-4] or a salt thereof according to Step B1-5.

(Step B2-5)

Compound [B2-7] or a salt thereof can be produced by reacting Compound [B2-5] or a salt thereof with Compound [B2-6] or a salt thereof in the presence of a base, in a solvent.

Examples of the base include triethylamine, N,N-diisopropylethylamine and the like. The preferred base is triethylamine.

Examples of the solvent include methanol, acetonitrile, THF and the like. The preferred solvent is methanol.

The reaction temperature is, for example, 0°C to 60°C, preferably 20°C to 40°C.

Compound [B2-6] or a salt thereof may be a commercially available product, or can be produced from a commercially available product by a known method.

(Step B2-6)

Compound [B2-8] or a salt thereof can be produced by removing the protecting group $P^{13}$ of Compound [B2-7] or a salt thereof employing a deprotection reaction. The deprotection reaction can be carried out under a condition suitable for the type of $P^{13}$.

For example, when $P^{13}$ is tert-butoxycarbonyl, Compound [B2-8] or a salt thereof can be produced by reacting Compound [B2-7] or a salt thereof with an acid in a solvent.

Examples of the acid include hydrochloric acid, trifluoroacetic acid and the like. The preferred acid is hydrochloric acid.

Examples of the solvent include CPME, ethyl acetate and the like. The preferred solvent is CPME or ethyl acetate.

The reaction temperature is, for example, 0°C to 60°C, preferably 20°C to 40°C.

Alternatively, Compound [B2-8] or a salt thereof can also be produced by carrying out this production method using compound or a salt thereof having a functional group or a protected functional group on Cy that can be converted to $R^2$ by a known reaction, instead of Compound [B1-2] or a salt thereof to obtain a compound corresponding to Compound [B2-8], or a salt thereof, and then converting the functional group of the compound or salt thereof to $R^2$.

Production Method B3: production method of Compound [B3-7] or a salt thereof

Compound [A3-1] or a salt thereof (used in Production Methods A3 and A4) wherein $A^a$ is $CR^{10a}$ and $R^{9a}$ is hydrogen, i.e., Compound [B3-7] or a salt thereof, can be produced, for example, according to the following Production Method B3.

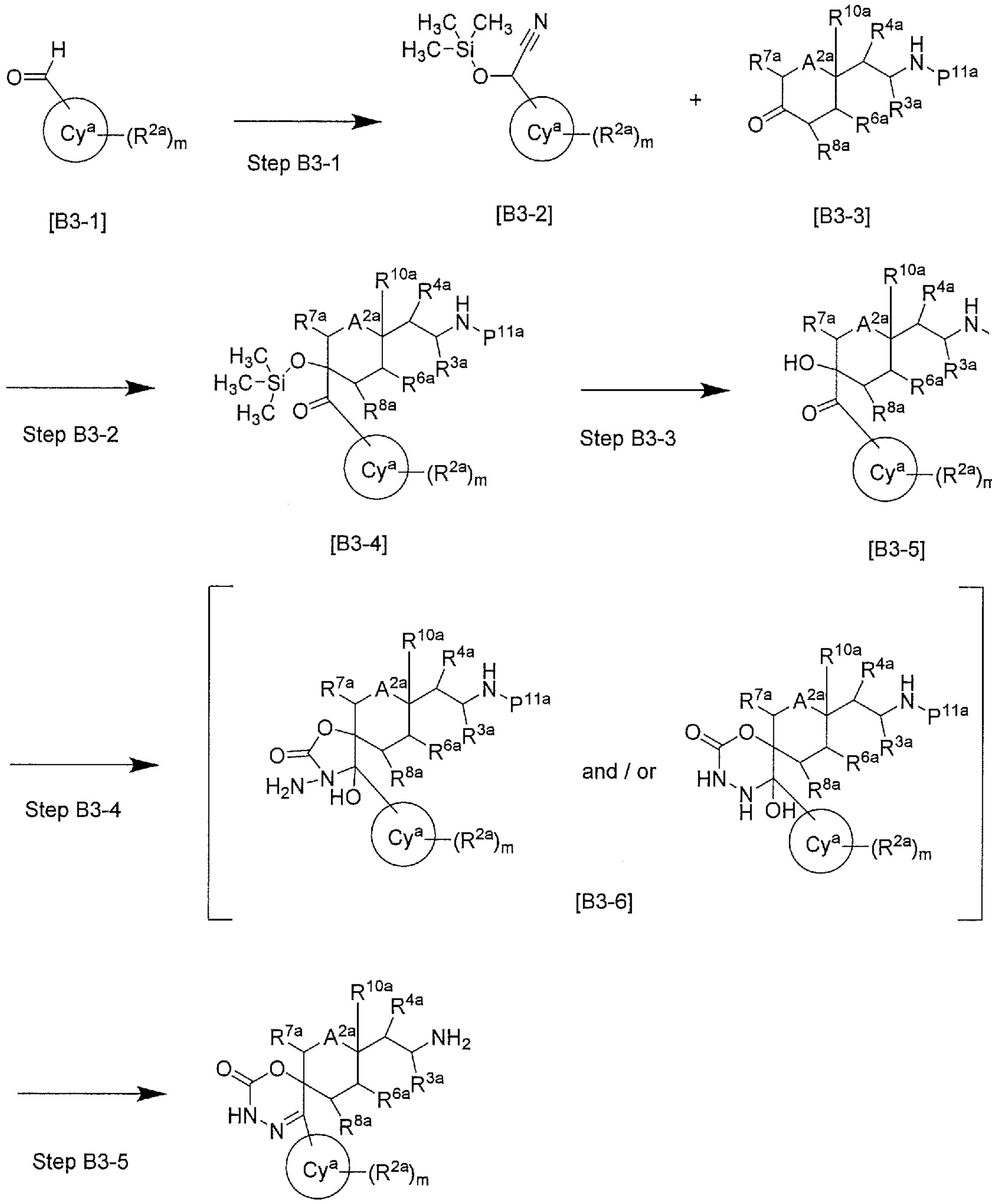


[B3-1] [B3-2] + [B3-3] [B3-4] [B3-5] [B3-6] (and / or) [B3-7]

wherein

$A^{2a}$, $Cy^a$, $R^{2a}$, $R^{3a}$, $R^{4a}$, $R^{6a}$, $R^{7a}$, $R^{8a}$, $R^{10a}$ and m are as defined above, and
$P^{11a}$ is a protecting group for amine (for example, tert-butoxycarbonyl, benzyloxycarbonyl, etc.).

(Step B3-1)

Compound [B3-2] or a salt thereof can be produced by reacting Compound [B3-1] or a salt thereof with trimethylsilylcyanide in the presence of a catalyst, in a solvent.

Examples of the catalyst include lithium chloride, DMAP and the like. The preferred catalyst is lithium chloride or DMAP.

Examples of the solvent include THF, acetonitrile and the like. The preferred solvent is THF.

The reaction temperature is, for example, 0°C to 60°C, preferably 15°C to 30°C.

Compound [B3-1] or a salt thereof may be a commercially available product, or can be produced from a

commercially available product by a known method.

(Step B3-2)

Compound [B3-4] or a salt thereof can be produced by reacting Compound [B3-2] or a salt thereof with Compound [B3-3] or a salt thereof in the presence of a catalyst and a base, in a solvent. Step B3-1 and Step B3-2 can also be carried out in one-pot.

Examples of the catalyst include lithium chloride, DMAP and the like. The preferred catalyst is lithium chloride or DMAP.

Examples of the base include LiHMDS, NaHMDS, LDA, n-butyllithium and the like. The preferred base is LiHMDS or NaHMDS.

Examples of the solvent include THF, n-hexane, toluene and the like. The preferred solvent is THF or toluene.

The reaction temperature is, for example, -100°C to 0°C, preferably -78°C to -50°C.

Compound [B3-3] or a salt thereof may be a commercially available product, or can be produced from a commercially available product by a known method, for example, it can be also produced according to Production Method C2 described below.

(Step B3-3)

Compound [B3-5] or a salt thereof can be produced by reacting Compound [B3-4] or a salt thereof in the presence of a desilylating agent and an acid, in a solvent.

Examples of the desilylating agent include TBAF, hydrogen fluoride-pyridine and the like. The preferred desilylating agent is TBAF.

Examples of the acid include acetic acid and the like. The preferred acid is acetic acid.

Examples of the solvent include THF, acetonitrile and the like. The preferred solvent is THF.

The reaction temperature is, for example, 0°C to 60°C, preferably 15°C to 30°C.

(Step B3-4)

Compound [B3-6] or a salt thereof can be produced by reacting Compound [B3-5] or a salt thereof in the presence of a base, CDI and hydrazine monohydrate, in a solvent.

Examples of the base include DBU, N-methylimidazole and the like. The preferred base is DBU.

Examples of the solvent include THF, DMF, acetonitrile and the like. The preferred solvent is THF or DMF.

The reaction temperature is, for example, 0°C to 60°C, preferably 20°C to 40°C.

(Step B3-5)

Compound [B3-7] or a salt thereof can be produced by reacting Compound [B3-6] or a salt thereof in the presence of an acid, in a solvent.

Examples of the acid include hydrochloric acid, acetic acid and the like. The preferred acid is hydrochloric acid.

Examples of the solvent include THF, water, toluene and the like. The preferred solvent is THF.

The reaction temperature is, for example, 15°C to 120°C, preferably 20°C to 60°C.

Alternatively, Compound [B3-7] or a salt thereof can also be produced by carrying out this production method using compound or a salt thereof having a functional group or a protected functional group on $Cy^a$ that can be converted to $R^{2a}$ by a known reaction, instead of Compound [B3-1] or a salt thereof to obtain a compound corresponding to Compound [B3-7], or a salt thereof, and then converting the functional group of the compound or salt thereof to $R^{2a}$.

Production Method B4: production method of Compound [B4-8] or a salt thereof

Compound [A3-1] or a salt thereof (used in Production Methods A3 and A4) wherein $A^2$ is N and $R^{9a}$ is hydrogen, i.e., Compound [B4-8] or a salt thereof, can be produced, for example, according to the following Production Method B4.

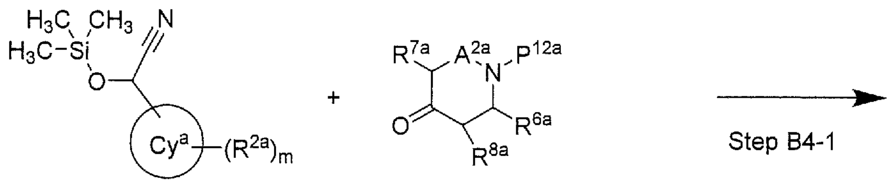


[B3-2] [B4-1]

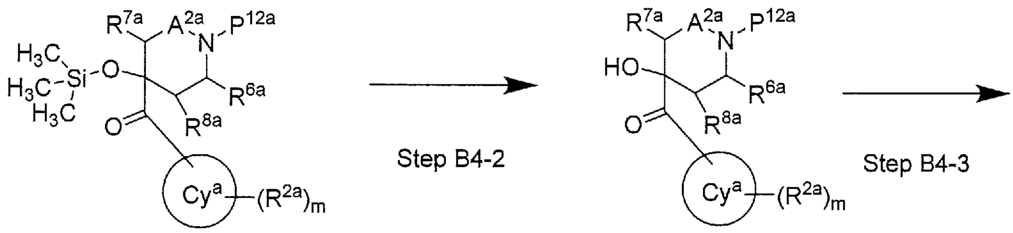


[B4-2] [B4-3]

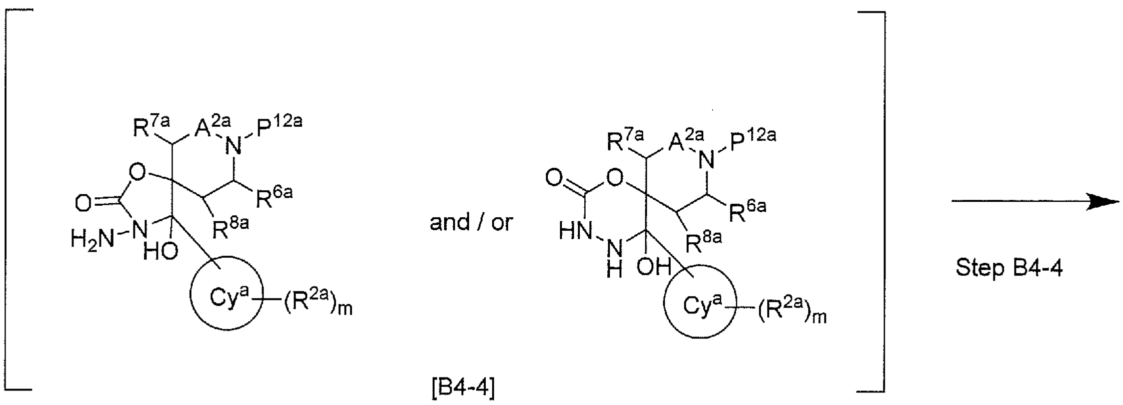


[B4-4]

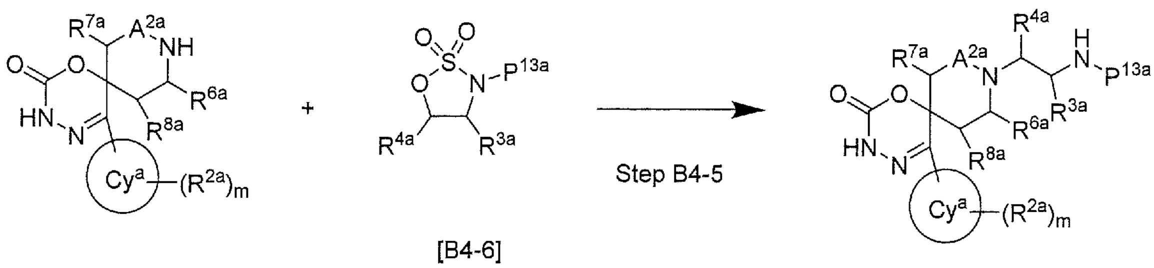


[B4-5] [B4-6] [B4-7]

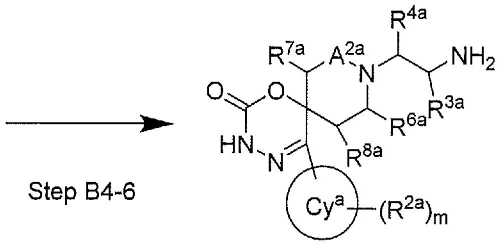


[B4-8]

wherein

$A^{2a}$, $Cy^a$, $R^{2a}$, $R^{3a}$, $R^{4a}$, $R^{6a}$, $R^{7a}$, $R^{8a}$ and m are as defined above, and
$P^{12a}$ and $P^{13a}$ are each independently a protecting group for amine (for example, tert-butoxycarbonyl, benzyloxycarbonyl, etc.).

(Step B4-1)

Compound [B4-2] or a salt thereof can be produced by reacting Compound [B3-2] or a salt thereof with Compound [B4-1] or a salt thereof according to Step B3-2.
Compound [B4-1] or a salt thereof may be a commercially available product, or can be produced from a commercially available product by a known method.

(Step B4-2)

Compound [B4-3] or a salt thereof can be produced by reacting Compound [B4-2] or a salt thereof according to Step B3-3.

(Step B4-3)

Compound [B4-4] or a salt thereof can be produced by reacting Compound [B4-3] or a salt thereof according to Step B3-4.

(Step B4-4)

Compound [B4-5] or a salt thereof can be produced by reacting Compound [B4-4] or a salt thereof according to Step B3-5.

(Step B4-5)

Compound [B4-7] or a salt thereof can be produced by reacting Compound [B4-5] or a salt thereof with Compound [B4-6] or a salt thereof in the presence of a base, in a solvent.
Examples of the base include triethylamine, N,N-diisopropylethylamine and the like. The preferred base is triethylamine.
Examples of the solvent include methanol, acetonitrile, THF and the like. The preferred solvent is methanol.
The reaction temperature is, for example, 0°C to 60°C, preferably 20°C to 40°C.
Compound [B4-6] or a salt thereof may be a commercially available product, or can be produced from a commercially available product by a known method.

(Step B4-6)

Compound [B4-8] or a salt thereof can be produced by removing the protecting group $P^{13a}$ of Compound [B4-7] or a salt thereof employing a deprotection reaction. The deprotection reaction can be carried out under a condition suitable for the type of $P^{13a}$.
For example, when $P^{13a}$ is tert-butoxycarbonyl, Compound [B4-8] or a salt thereof can be produced by reacting Compound [B4-7] or a salt thereof with an acid in a solvent.
Examples of the acid include hydrochloric acid, trifluoroacetic acid and the like. The preferred acid is hydrochloric acid.
Examples of the solvent include CPME, ethyl acetate and the like. The preferred solvent is CPME or ethyl acetate.
The reaction temperature is, for example, 0°C to 60°C, preferably 20°C to 40°C.
Alternatively, Compound [B4-8] or a salt thereof can also be produced by carrying out this production method using compound or a salt thereof having a functional group or a protected functional group on $Cy^a$ that can be converted to $R^{2a}$ by a known reaction, instead of Compound [B3-2] or a salt thereof to obtain a compound corresponding to Compound [B4-8], or a salt thereof, and then converting the functional group of the compound or salt thereof to $R^{2a}$.

Production Method C1: production method of Compound [C1-9] or a salt thereof

Compound [B1-3] or a salt thereof (used in Production Method B1) wherein $R^{10}$ is hydrogen, i.e., Compound [C1-9] or a salt thereof, can be produced, for example, according to the following Production Method C1.

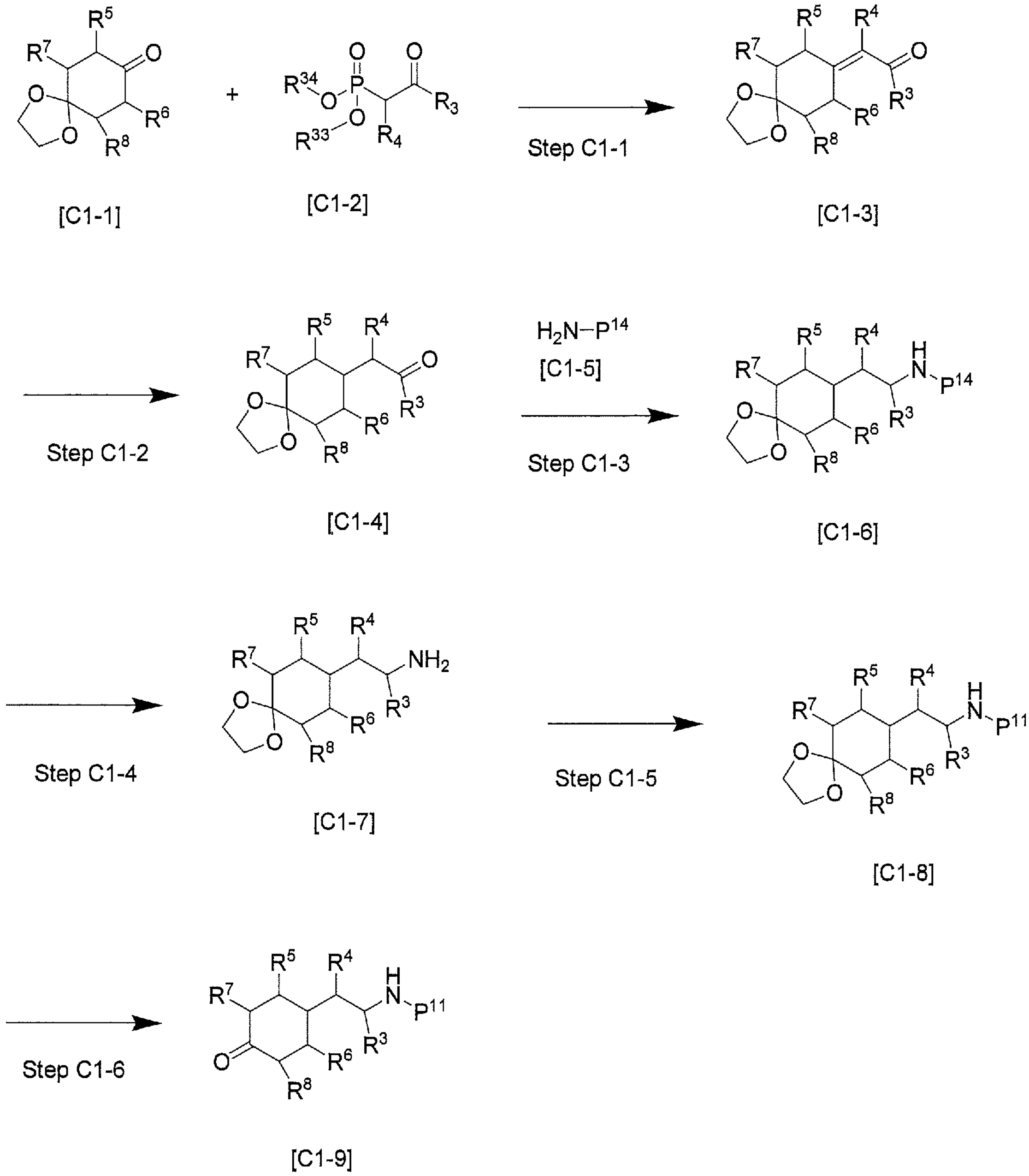


[C1-1] [C1-2] [C1-3] [C1-4] [C1-6] [C1-7] [C1-8] [C1-9]

wherein

$R^3$, $R^4$, $R^5$, $R^6$, $R^7$, $R^8$ and $P^{11}$ are as defined above,
$R^{33}$ and $R^{34}$ are each independently $C_{1-4}$ alkyl, and
$P^{14}$ is a protecting group for amine (for example, benzyl, (S)-1-phenylethyl, etc.).

(Step C1-1)

Compound [C1-3] or a salt thereof can be produced by reacting Compound [C1-1] or a salt thereof with Compound [C1-2] or a salt thereof in the presence of a base, in a solvent.

Examples of the base include sodium ethoxide, sodium hydride, DBU and the like. The preferred base is sodium

ethoxide.

Examples of the solvent include ethanol, THF, DMF and the like. The preferred solvent is ethanol.

The reaction temperature is, for example, 0°C to 60°C, preferably 20°C to 40°C.

Compound [C1-1] or a salt thereof may be a commercially available product, or can be produced from a commercially available product by a known method.

Compound [C1-2] or a salt thereof may be a commercially available product, or can be produced from a commercially available product by a known method.

(Step C1-2)

Compound [C1-4] or a salt thereof can be produced by subjecting Compound [C1-3] or a salt thereof to catalytic hydrogenation in the presence of a metal catalyst, in a solvent.

Examples of the metal catalyst include Pd-C, Pt-C, palladium hydroxide and the like. The preferred metal catalyst is Pd-C or palladium hydroxide.

Examples of the solvent include methanol, ethanol, THF and the like. The preferred solvent is THF.

The reaction temperature is, for example, 0°C to 60°C, preferably 20°C to 40°C.

(Step C1-3)

Compound [C1-6] or a salt thereof can be produced by subjecting Compound [C1-4] or a salt thereof to a reductive amination reaction with Compound [C1-5] or a salt thereof in a solvent. The reductive amination reaction includes, for example, a reaction using an acid and a reducing agent.

Examples of the reducing agent include sodium triacetoxyborohydride, sodium cyanoborohydride, sodium borohydride and the like. The preferred reducing agent is sodium triacetoxyborohydride.

Examples of the acid include acetic acid, trifluoroacetic acid, hydrochloric acid and the like. The preferred acid is acetic acid.

Examples of the solvent include THF, chloroform, toluene, N-methylpyrrolidone and the like. The preferred solvent is THF.

The reaction temperature is, for example, 0°C to 60°C, preferably 20°C to 40°C.

Compound [C1-5] or a salt thereof may be a commercially available product, or can be produced from a commercially available product by a known method.

Compound [C1-6] or a salt thereof wherein $R^3$ is methyl, and $R^4$, $R^5$, $R^6$, $R^7$ and $R^8$ are all hydrogens, i.e., the following Compound [C1-10]

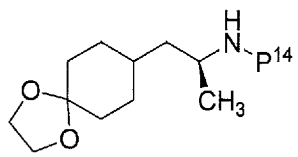

[C1-10]

wherein

$P^{14}$ are as defined above,
or a salt thereof can be produced by, for example, carrying out Step C1-3 using (S)-1-phenethylamine as Compound [C1-5] or a
salt thereof, and stirring the obtained diastereomeric mixture or a salt thereof in a solvent, and then collecting the precipitated solid by filtration.

Examples of the solvent include toluene, THF, ethyl acetate and the like. The preferred solvent is ethyl acetate.

(Step C1-4)

Compound [C1-7] or a salt thereof can be produced by removing the protecting group $P^{14}$ of Compound [C1-6] or a salt thereof employing a deprotection reaction. The deprotection reaction can be carried out under a condition suitable for the type of $P^{14}$.

For example, when $P^{14}$ is (S)-1-phenylethyl, Compound [C1-7] or a salt thereof can be produced by reacting Compound [C1-6] or a salt thereof according to Step C1-2.

(Step C1-5)

Compound [C1-8] or a salt thereof can be produced by introducing the protecting group $P^{11}$ to Compound [C1-7] or a salt thereof. The introduction of the protecting group can be carried out under a condition suitable for the type of $P^{11}$.
For example, when $P^{11}$ is tert-butoxycarbonyl, Compound [C1-8] or a salt thereof can be produced by reacting Compound [C1-7] or a salt thereof with the corresponding carbamating agent in a solvent. Bases may be added according to conditions.
Examples of the corresponding carbamating agent include $Boc_2O$ and the like. The preferred corresponding carbamating agent is $Boc_2O$.
Examples of the base include sodium hydroxide, sodium bicarbonate and the like.
Examples of the solvent include THF, water, mixed solvent thereof and the like. The preferred solvent is a mixed solvent of THF and water.
The reaction temperature is, for example, 0°C to 60°C, preferably 20°C to 40°C.

(Step C1-6)

Compound [C1-9] or a salt thereof can be produced by reacting Compound [C1-8] or a salt thereof in the presence of a catalyst, in a solvent.
Examples of the catalyst include trifluoroacetic acid, p-toluenesulfonic acid, p-toluenesulfonic acid pyridine salt and the like. The preferred catalyst is p-toluenesulfonic acid pyridine salt.
Examples of the solvent include THF, acetone, water, mixed solvent thereof and the like. The preferred solvent is a mixed solvent of acetone and water.
The reaction temperature is, for example, 20°C to 100°C, preferably 40°C to 80°C.

Production Method C2: production method of Compound [C2-9] or a salt thereof

Compound [B3-3] or a salt thereof (used in Production Method B3) wherein $R^{10a}$ is hydrogen and $A^{2a}$ is $CR^{5a}$, i.e., Compound [C2-9] or a salt thereof, can be produced, for example, according to the following Production Method C2.

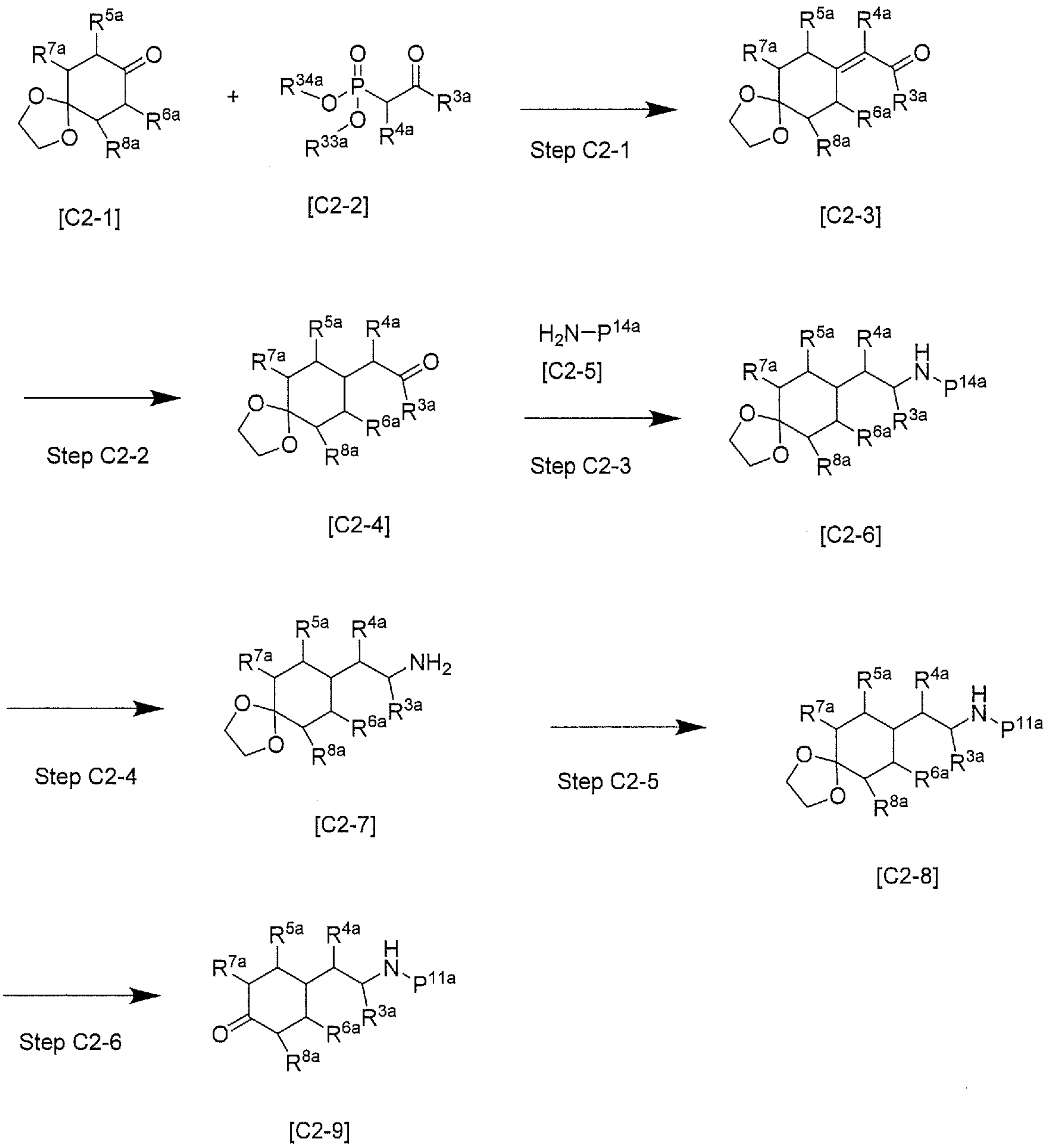


[C2-1] [C2-2] [C2-3] [C2-4] [C2-6] [C2-7] [C2-8] [C2-9]

wherein

$R^{3a}$, $R^{4a}$, $R^{5a}$, $R^{6a}$, $R^{7a}$, $R^{8a}$ and $P^{11a}$ are as defined above,
$R^{33a}$ and $R^{34a}$ are each independently $C_{1-4}$ alkyl, and
$P^{14a}$ is a protecting group for amine (for example, benzyl, (S)-1-phenylethyl, etc.).

(Step C2-1)

Compound [C2-3] or a salt thereof can be produced by reacting Compound [C2-1] or a salt thereof with Compound [C2-2] or a salt thereof in the presence of a base, in a solvent.

Examples of the base include sodium ethoxide, sodium hydride, DBU and the like. The preferred base is sodium ethoxide.

Examples of the solvent include ethanol, THF, DMF and the like. The preferred solvent is ethanol.

The reaction temperature is, for example, 0°C to 60°C, preferably 20°C to 40°C.

Compound [C2-1] or a salt thereof may be a commercially available product, or can be produced from a commercially available product by a known method.

Compound [C2-2] or a salt thereof may be a commercially available product, or can be produced from a commercially available product by a known method.

(Step C2-2)

Compound [C2-4] or a salt thereof can be produced by subjecting Compound [C2-3] or a salt thereof to catalytic hydrogenation in the presence of a metal catalyst, in a solvent.
Examples of the metal catalyst include Pd-C, Pt-C, palladium hydroxide and the like. The preferred metal catalyst is Pd-C or palladium hydroxide.
Examples of the solvent include methanol, ethanol, THF and the like. The preferred solvent is THF.
The reaction temperature is, for example, 0°C to 60°C, preferably 20°C to 40°C.

(Step C2-3)

Compound [C2-6] or a salt thereof can be produced by subjecting Compound [C2-4] or a salt thereof to a reductive amination reaction with Compound [C2-5] or a salt thereof in a solvent. The reductive amination reaction includes, for example, a reaction using an acid and a reducing agent.
Examples of the reducing agent include sodium triacetoxyborohydride, sodium cyanoborohydride, sodium borohydride and the like. The preferred reducing agent is sodium triacetoxyborohydride.
Examples of the acid include acetic acid, trifluoroacetic acid, hydrochloric acid and the like. The preferred acid is acetic acid.
Examples of the solvent include THF, chloroform, toluene, N-methylpyrrolidone and the like. The preferred solvent is THF.
The reaction temperature is, for example, 0°C to 60°C, preferably 20°C to 40°C.
Compound [C2-5] or a salt thereof may be a commercially available product, or can be produced from a commercially available product by a known method.
Compound [C2-6] or a salt thereof wherein $R^{3a}$ is methyl, and $R^{4a}$, $R^{5a}$, $R^{6a}$, $R^{7a}$ and $R^{8a}$ are all hydrogens, i.e., the following Compound [C2-10]

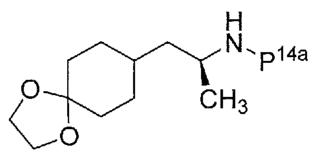

[C2-10]

wherein

$P^{14a}$ are as defined above
or a salt thereof can be produced by, for example, carrying out Step C2-3 using (S)-1-phenethylamine as Compound [C2-5] or a salt thereof, and stirring the obtained diastereomeric mixture or a salt thereof in a solvent, and then collecting the precipitated solid by filtration.

Examples of the solvent include toluene, THF, ethyl acetate and the like. The preferred solvent is ethyl acetate.

(Step C2-4)

Compound [C2-7] or a salt thereof can be produced by removing the protecting group $P^{14a}$ of Compound [C2-6] or a salt thereof employing a deprotection reaction. The deprotection reaction can be carried out under a condition suitable for the type of $P^{14a}$.
For example, when $P^{14a}$ is (S)-1-phenylethyl, Compound [C2-7] or a salt thereof can be produced by reacting Compound [C2-6] or a salt thereof according to Step C2-2.

(Step C2-5)

Compound [C2-8] or a salt thereof can be produced by introducing the protecting group $P^{11a}$ to Compound [C2-7] or a salt thereof. The introduction of the protecting group can be carried out under a condition suitable for the type of $P^{11a}$.
For example, when $P^{11a}$ is tert-butoxycarbonyl, Compound [C2-8] or a salt thereof can be produced by reacting Compound [C2-7] or a salt thereof with the corresponding carbamating agent in a solvent. Bases may be added according

to conditions.

Examples of the corresponding carbamating agent include $Boc_2O$ and the like. The preferred corresponding carbamating agent is $Boc_2O$.

Examples of the base include sodium hydroxide, sodium bicarbonate and the like.

Examples of the solvent include THF, water, mixed solvent thereof and the like. The preferred solvent is a mixed solvent of THF and water.

The reaction temperature is, for example, 0°C to 60°C, preferably 20°C to 40°C.

(Step C2-6)

Compound [C2-9] or a salt thereof can be produced by reacting Compound [C2-8] or a salt thereof in the presence of a catalyst in a solvent.

Examples of the catalyst include trifluoroacetic acid, p-toluenesulfonic acid, p-toluenesulfonic acid pyridine salt and the like. The preferred catalyst is p-toluenesulfonic acid pyridine salt.

Examples of the solvent include THF, acetone, water, mixed solvent thereof and the like. The preferred solvent is a mixed solvent of acetone and water.

The reaction temperature is, for example, 20°C to 100°C, preferably 40°C to 80°C.

**Examples**

Next, the production method of Compound [I] or Compound [Ia] or a pharmaceutically acceptable salt thereof of the present invention will be concretely explained with reference to the following Production Examples. However, the production method of Compound [I] or Compound [Ia] or a pharmaceutically acceptable salt thereof of the present invention should not be limited to such production examples.

The compound obtained in each step can be, if necessary, isolated or purified according to a method known per se such as distillation, recrystallization and column chromatography, or directly used in the next step without isolation or purification.

As used herein, the room temperature refers to a temperature in an uncontrolled state, and one embodiment includes 1°C to 40°C.

[Production Example 1]: Synthesis of tert-butyl (S)-(1-(4-oxocyclohexyl)propan-2-yl) carbamate

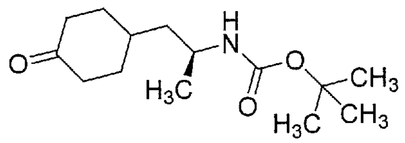

(1) ethyl 4-(trans-4-((tert-butoxycarbonyl)amino)cyclohexyl)-3-oxobutanoate

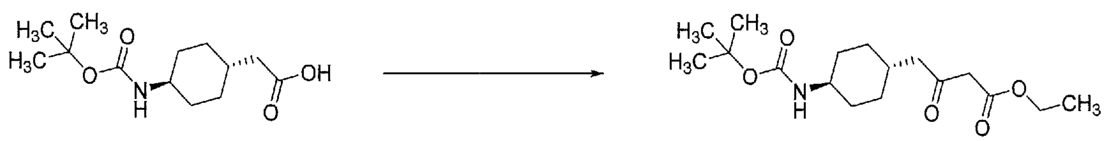

Under argon atmosphere, to a solution of 2-(trans-4-((tert-butoxycarbonyl)amino)cyclohexyl)acetic acid (1.4 kg) in THF (6.2 kg) was added dropwise a solution of carbonyldiimidazole(1 kg) in DMF (4.7 kg) at room temperature, and the mixture was stirred. The washing with DMF (0.7 kg) is combined therewith, and the mixture was stirred for 3 hr. Monoethyl potassium malonate (1.3 kg) was added thereto, and then magnesium chloride (0.6 kg) was added thereto in divided portions. After stirring all day, water (4.2 kg) and 6M hydrochloric acid (3.1 kg) were added thereto at an internal temperature of 5°C, and the mixture was stirred at room temperature for 1 hr. Toluene (6.1 kg) was added thereto, the mixture was subjected to extraction, and the aqueous layer was subjected to re-extraction with toluene (2.4 kg). The combined organic layers were washed twice with 20% brine (7 kg). The organic layer was concentrated under reduced pressure, followed by azeotropy with methanol (2.2 kg). To the obtained residue was added methanol (3.3 kg) to give a

methanol solution. The above methanol solution was combined with a methanol solution obtained by the same procedures from 2-(trans-4-((tert-butoxycarbonyl)amino)cyclohexyl)acetic acid (8 kg) to give a methanol solution (34 kg) of the title compound. A part of it was concentrated to give an NMR sample.
1H-NMR (CDCl3) δ: 4.44-4.31 (1H, m), 4.19 (2H, q, J = 7.1 Hz), 3.46-3.27 (1H, m), 3.40 (2H, s), 2.43 (2H, d, J = 6.3 Hz), 2.04-1.93 (2H, m), 1.88-1.69 (3H, m), 1.44 (9H, s), 1.28 (3H, t, J = 7.1 Hz), 1.19-0.97 (4H, m).

(2) tert-butyl (trans-4-(2-oxopropyl)cyclohexyl)carbamate

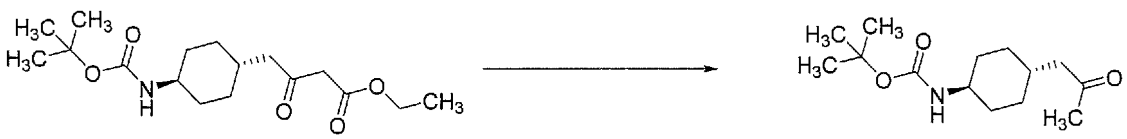

The methanol solution obtained in (1) (34 kg) of ethyl 4-(trans-4-((tert-butoxycarbonyl)amino)cyclohexyl)-3-oxobutanoate was warmed to 35°C, 4M aqueous sodium hydroxide solution (20 kg) was added thereto, and the mixture was stirred. After stirring at 40°C for 2 hr, a solution of citric acid (8.4 kg) in water (19 kg) was added dropwise thereto at 45°C. Ethyl acetate (25 kg) was added thereto, the mixture was stirred for 20 min, and a solution of citric acid (8.4 kg) in water (19 kg) was added dropwise thereto. After stirring for 3 hr, the mixture was allowed to cool to room temperature, ethyl acetate (25 kg) was added thereto, and the mixture was subjected to liquid separation. The organic layer was washed with 10% brine (47 kg), and concentrated under reduced pressure. Ethyl acetate (17 kg) was added thereto, and the mixture was concentrated under reduced pressure to give the title compound (8.1 kg) in 87% yield.
1H-NMR (CDCl3) δ: 4.36 (1H, br s), 3.35 (1H, br s), 2.31 (2H, d, J = 6.4 Hz), 2.12 (3H, s), 2.02-1.95 (2H, m), 1.83-1.71 (3H, m), 1.44 (9H, s), 1.18-0.95 (4H, m).

(3) tert-butyl (trans-4-((S)-2-(((S)-1-phenylethyl)amino)propyl)cyclohexyl)carbamate acetate

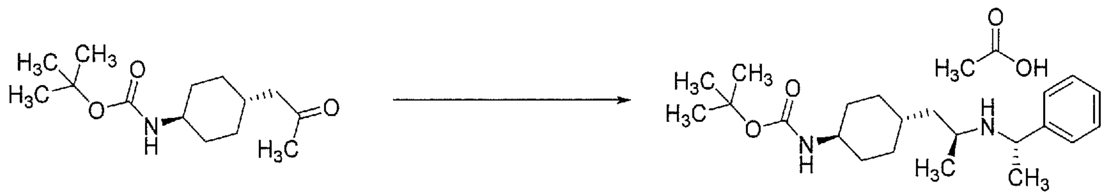

A solution of tert-butyl (trans-4-(2-oxopropyl)cyclohexyl)carbamate obtained in (2) (8.1 kg) in methanol (67 kg) was prepared, and under Ar atmosphere, (S)-1-phenethylamine (4.4 kg), acetic acid (1.2 kg) and a suspension of 2% Pt-C (0.42 kg) in water (1.7 kg) were added thereto at room temperature, and the mixture was stirred. The mixture was stirred under hydrogen atmosphere (4 atm) at 40°C for 20 hr. After cooling to room temperature, the 2% Pt-C was removed by filtration with KC flock (registered trademark) as a filter aid, and washed twice with methanol (15 kg). The obtained solution was concentrated under reduced pressure, isopropyl alcohol (20 kg) was added thereto, and the mixture was concentrated under reduced pressure. The same operation was performed again, and to the obtained solid was added isopropyl alcohol (43 kg) to prepare a suspension. This suspension was heated to 70°C, and acetic acid (1 kg) was added dropwise thereto. After stirring for 2 hr, the mixture was allowed to cool to room temperature, and stirred for 17 hr. The mixture was stirred for additional 2 hr at an internal temperature of 5°C, and the obtained solid was collected by filtration, washed with ice-cooled isopropyl alcohol (20 kg), and dried at 30°C for 19 hr to give the title compound (9 kg) in 65% yield.
1H-NMR (DMSO-D6) δ: 7.35-7.25 (4H, m), 7.21-7.15 (1H, m), 6.62 (1H, d, J = 7.5 Hz), 3.80 (1H, q, J = 6.5 Hz), 3.17-3.01 (1H, m), 2.48-2.38 (1H, m), 1.90 (3H, s), 1.77-1.59 (3H, m), 1.54-1.28 (11H, m), 1.23-0.66 (12H, m).

(4) tert-butyl (S)-(trans-4-(2-aminopropyl)cyclohexyl)carbamate acetate

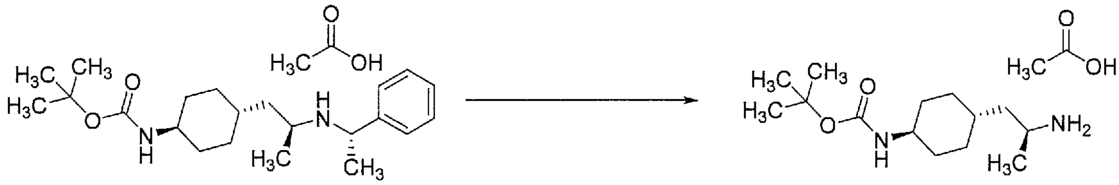

To a solution of tert-butyl (trans-4-((S)-2-(((S)-1-phenylethyl)amino)propyl)cyclohexyl)carbamate acetate obtained in (3) (8.9 kg) in methanol (85 kg) was added a suspension of 5% Pd-C (PE type) (0.89 kg) in water (1.8 kg) under Ar atmosphere at room temperature, and the mixture was stirred. The mixture was stirred under hydrogen atmosphere (4 atm) at 35°C for 2 hr. After gradually cooling to room temperature, the 5% Pd-C was removed by filtration with KC flock (registered trademark) as a filter aid, and washed with methanol (21 kg). The filtrate was concentrated under reduced pressure, followed by azeotropy with acetonitrile (21 kg). To the obtained solid was added methanol (42 kg) to give a methanol solution of the title compound. A part of it was concentrated to give an NMR sample.
1H-NMR (DMSO-D6) δ: 6.71-6.64 (1H, m), 6.14 (3H, br s), 3.21-3.02 (1H, m), 2.99-2.86 (1H, m), 1.84-1.58 (7H, m), 1.37 (9H, s), 1.32-1.04 (5H, m), 1.03-0.96 (3H, m), 0.94-0.75 (2H, m).

(5) benzyl (S)-(1-(trans-4-((tert-butoxycarbonyl)amino)cyclohexyl)propan-2-yl)carbamate

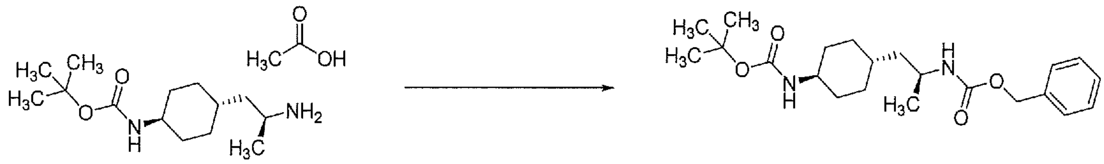

To a solution of tert-butyl (S)-(trans-4-(2-aminopropyl)cyclohexyl)carbamate acetate (obtained in the same manner as in (4)) (218 g) in DMF (1080 ml) was added triethylamine (100 ml) at room temperature, and the mixture was stirred. Under water-cooling, N-carbobenzoxyoxysuccinimide (179 g) was added thereto, and the mixture was stirred at room temperature for 6 hr. Under water-cooling, water (2160 ml) was added thereto, and the mixture was stirred for 1 hr. The obtained solid was collected by filtration, washed with water (1200 ml), and dried at room temperature for 48 hr to give seed crystals (257 g) of the title compound in 97% yield.

To a methanol solution obtained in (4) of tert-butyl (S)-(trans-4-(2-aminopropyl)cyclohexyl)carbamate acetate were added triethylamine (5.1 kg) and a solution of N-carbobenzoxyoxysuccinimide(5.8 kg) in acetonitrile (14 kg) at 10°C, and the mixture was stirred. The washing with acetonitrile (3.5 kg) is combined therewith, and the mixture was stirred for 3 hr. Water (8.9 kg) was added thereto, the seed crystals obtained in the same manner as above were added thereto, and the mixture was stirred for 2 hr. The obtained solid was collected by filtration, washed with 50% methanol aqueous solution (24 kg), and dried for 69 hr to give the title compound (8 kg) in 97% yield.
1H-NMR (DMSO-D6) δ: 7.41-7.27 (5H, m), 7.06 (1H, d, J = 8.2 Hz), 6.66 (1H, d, J = 8.2 Hz), 5.03 (1H, d, J = 12.0 Hz), 4.98 (1H, d, J = 12.0 Hz), 3.66-3.52 (1H, m), 3.19-3.03 (1H, m), 1.80-1.51 (4H, m), 1.43-1.26 (1H, m), 1.37 (9H, s), 1.23-0.75 (6H, m), 1.01 (3H, d, J = 6.7 Hz).

(6) benzyl (S)-(1-(trans-4-aminocyclohexyl)propan-2-yl)carbamate p-toluenesulfonate

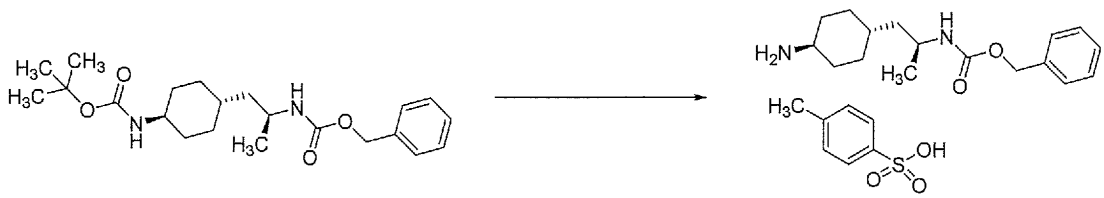

A suspension of benzyl (S)-(1-(trans-4-((tert-butoxycarbonyl)amino)cyclohexyl)propan-2-yl)carbamate (obtained in the same manner as in (5)) (257 g) in ethyl acetate (2570 ml) was heated to 80°C, p-toluenesulfonic acid monohydrate (188 g) was added thereto, and the mixture was stirred for 3 hr. After gradually cooling to room temperature,

the mixture was allowed to stand all day. The obtained solid was collected by filtration, and washed with ethyl acetate (236 ml). The solid was dried at 50°C for 8 hr dried under reduced pressure, and then dried under reduced pressure at room temperature all day to give seed crystals (281 g) of the title compound in 92% yield.

A suspension of benzyl (S)-(1-(trans-4-((tert-butoxycarbonyl)amino)cyclohexyl)propan-2-yl)carbamate obtained in (5) (8 kg) in ethyl acetate (58 kg) was prepared, p-toluenesulfonic acid monohydrate (5.1 kg) was added thereto at room temperature, and the mixture was stirred. The washing with ethyl acetate (1.4 kg) is combined therewith, and the mixture was stirred for 1 hr. the seed crystals obtained in the same manner as above were added thereto, and the mixture was stirred for 1 hr, warmed to 55°C, and stirred for 4 hr. After gradually cooling to room temperature, the mixture was stirred for 14 hr. The obtained solid was collected by filtration, washed with ethyl acetate (22 kg), and dried at 50°C for 23 hr to give the title compound (9 kg) in 96% yield.

1H-NMR (DMSO-D6) δ: 7.67 (3H, br s), 7.47 (2H, d, J = 8.2 Hz), 7.41-7.28 (5H, m), 7.14-7.06 (3H, m), 5.02 (1H, d, J = 12.7 Hz), 4.98 (1H, d, J = 12.7 Hz), 3.66-3.53 (1H, m), 2.98-2.83 (1H, m), 2.29 (3H, s), 1.95-1.60 (4H, m), 1.39-0.81 (7H, m), 1.02 (3H, d, J = 6.7 Hz).

(7) benzyl (S)-(1-trans-(4-aminocyclohexyl)propan-2-yl)carbamate

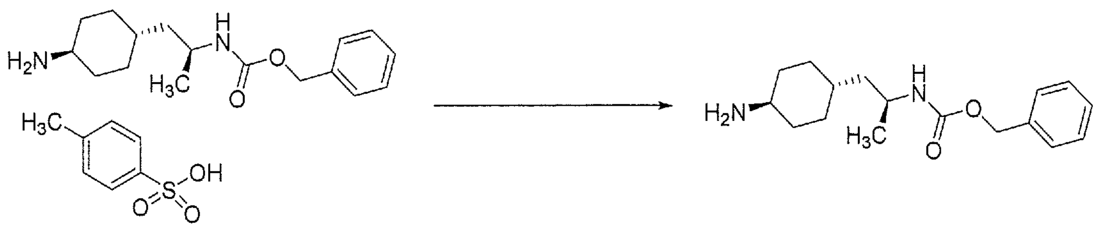

To a solution of benzyl (S)-(1-(trans-4-aminocyclohexyl)propan-2-yl)carbamate p-toluenesulfonate obtained in (6) (30 g) in 2-methyl-tetrahydrofuran (120 ml) was added 4M aqueous sodium hydroxide solution (17 ml) at room temperature, and the mixture was stirred for 1.5 hr. Water (120 ml) was added thereto, the mixture was subjected to extraction, and the organic layer was washed with water (120 ml, twice) and saturated brine (120 ml), and dried over sodium sulfate. The sodium sulfate was removed by filtration, and the filtrate was concentrated under reduced pressure. Methanol (90 ml) was added thereto, and the mixture was concentrated under reduced pressure to give the title compound (18 g) in 96% yield.

1H-NMR (DMSO-D6) δ: 7.41-7.25 (5H, m), 7.05 (1H, d, J = 8.5 Hz), 5.03 (1H, d, J = 12.5 Hz), 4.97 (1H, d, J = 12.5 Hz), 3.68-3.50 (1H, m), 2.46-2.33 (1H, m), 1.81-0.71 (13H, m), 1.01 (3H, d, J = 6.5 Hz).

(8) benzyl (S)-(1-(4-oxocyclohexyl)propan-2-yl)carbamate

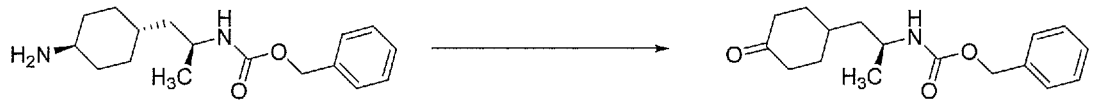

To a solution of benzyl (S)-(1-trans-(4-aminocyclohexyl)propan-2-yl)carbamate obtained in (7) (18 g) in methanol (90 ml) was added 3,5-di-tert-butyl-1,2-benzoquinone (14 g) at room temperature, and the mixture was stirred for 1.5 hr. Water (18 ml), acetone (72 ml) and DOWEX (45 g) were added thereto, and the mixture was stirred for 1.5 hr. Additional DOWEX (9 g) was added thereto, and the mixture was stirred for 30 min. The mixture was filtered with ethyl acetate, and the filtrate was concentrated under reduced pressure. Ethyl acetate (72 ml) and water (36 ml) were added thereto, the mixture was subjected to extraction, and the organic layer was dried over sodium sulfate. The sodium sulfate was removed by filtration, and the filtrate was concentrated under reduced pressure. Ethyl acetate (36 ml) and silica gel (18 g) were added thereto, and the mixture was stirred for 30 min. The mixture was filtered with ethyl acetate, and the filtrate was concentrated under reduced pressure. To the obtained residue was added diisopropyl ether (18 ml), and the mixture was warmed to an internal temperature of 48°C. Heptane (72 ml) was added dropwise thereto, and the mixture was stirred for 1.5 hr. The obtained solid was collected by filtration, washed with heptane, and dried under reduced pressure at 60°C for 3 hr and then at room temperature all day to give the title compound (15 g) in 83% yield.

1H-NMR (DMSO-D6) δ: 7.41-7.25 (5H, m), 7.15 (1H, d, J = 8.6 Hz), 5.03 (1H, d, J = 12.4 Hz), 4.99 (1H, d, J = 12.4 Hz),

3.70-3.55 (1H, m), 2.40-2.10 (4H, m), 2.08-1.66 (3H, m), 1.49-1.18 (4H, m), 1.06 (3H, d, J = 6.5 Hz).

(9) tert-butyl (S)-(1-(4-oxocyclohexyl)propan-2-yl)carbamate

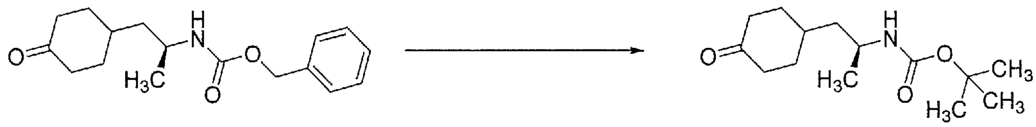

To a solution of benzyl (S)-(1-(4-oxocyclohexyl)propan-2-yl)carbamate obtained in (8) (15 g) in methanol (145 ml) were added 10% Pd-C (1.5 g) and $Boc_2O$ (12 g) under argon atmosphere at room temperature. The mixture was stirred under hydrogen atmosphere (1 atm) for 4 hr. After purging with nitrogen, the 10% Pd-C was removed by Celite filtration, and washed with methanol. The filtrate was concentrated under reduced pressure, to the obtained residue was added acetone (27 ml), and the mixture was stirred at room temperature. Water (82 ml) was added thereto, and the mixture was stirred all day. The obtained solid was collected by filtration, washed with water, and dried under reduced pressure at 60°C for 3 hr and then at room temperature all day to give the title compound (12 g) in 88% yield. The same procedures as in (7)-(9) above were performed with benzyl (S)-(1-(trans-4-aminocyclohexyl)propan-2-yl)carbamate p-toluenesulfonate (211 g) to give the title compound (60 g).
1H-NMR (DMSO-D6) δ: 6.65 (1H, d, J = 8.6 Hz), 3.63-3.46 (1H, m), 2.42-2.10 (4H, m), 2.08-1.97 (1H, m), 1.94-1.83 (1H, m), 1.80-1.66 (1H, m), 1.46-1.16 (4H, m), 1.38 (9H, s), 1.02 (3H, d, J = 6.5 Hz).

[Production Example 2]: Synthesis of $N^1$-((S)-1-(cis-5-(2-bromo-4-cyanophenyl)-2-oxo-1-oxa-3,4-diazaspiro[5.5]undec-4-en-9-yl)propan-2-yl)-$N^2$-methyloxamide (Example 57)

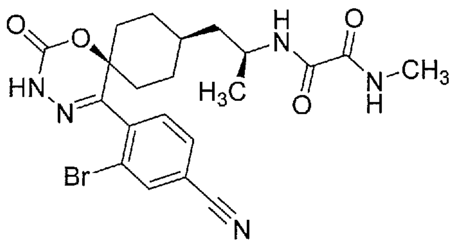

(1) tert-butyl ((S)-1-(cis-4-(2-bromo-4-cyanobenzoyl)-4-((trimethylsilyl)oxy)cyclohexyl)propan-2-yl)carbamate

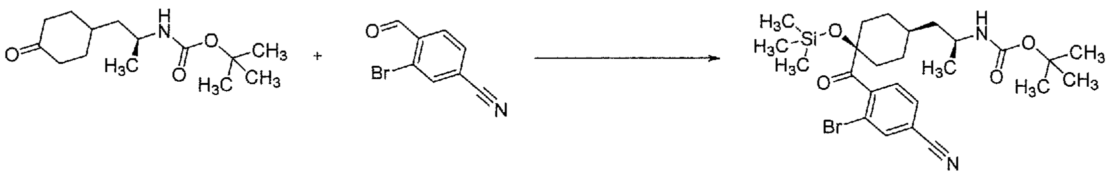

To a solution of 3-bromo-4-formylbenzonitrile (2.1 g) in THF (4 ml) were added lithium chloride (0.043 g) and trimethylsilylcyanide (1.5 ml) at room temperature, and the mixture was stirred at room temperature for 1 hr. THF (26 ml) was added thereto, and the mixture was cooled to -78°C. 1M NaHMDS-THF solution (10.2 ml) was added thereto, and the mixture was stirred for 3 min. A solution of tert-butyl (S)-(1-(4-oxocyclohexyl)propan-2-yl)carbamate (obtained in the same manner as in (9) of [Production Example 1]) (2 g) in THF (10 ml) was added thereto, and the mixture was stirred for 30 min, and warmed to -10°C over 1 hr. Water and ethyl acetate were added thereto, and the mixture was subjected to extraction. The aqueous layer was subjected to re-extraction twice with ethyl acetate, and the combined organic layers were washed with saturated brine, and dried over sodium sulfate. The sodium sulfate was removed by filtration, and the filtrate was concentrated under reduced pressure. The obtained residue was purified by silica gel chromatography (developing solvent: ethyl acetate/n-hexane) to give the title compound (1.2 g) in 28% yield.

1H-NMR (CDCl3) δ: 7.89 (1H, s), 7.62 (1H, d, J = 8.1 Hz), 7.39 (1H, d, J = 8.1 Hz), 4.30-4.15 (1H, m), 3.84-3.56 (1H, m), 2.01-1.92 (2H, m), 1.86-1.70 (3H, m), 1.64-1.52 (1H, m), 1.43 (9H, s), 1.40-1.18 (5H, m), 1.11 (3H, d, J = 6.5 Hz), 0.04 (9H, s).

(2) tert-butyl ((S)-1-(cis-4-(2-bromo-4-cyanobenzoyl)-4-hydroxycyclohexyl)propan-2-yl)carbamate

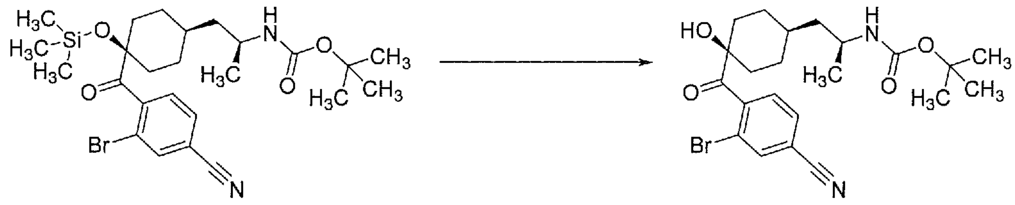

To a solution of tert-butyl ((S)-1-(cis-4-(2-bromo-4-cyanobenzoyl)-4-((trimethylsilyl)oxy)cyclohexyl)propan-2-yl) carbamate obtained in (1) and the same compound obtained in the same manner as above (total 1.4 g) in THF (2.7 ml) were added acetic acid (0.42 ml) and 1M TBAF-THF solution (3.7 ml) at room temperature, and the mixture was stirred for 15 min. Saturated aqueous ammonium chloride solution, water and ethyl acetate were added thereto, and the mixture was subjected to extraction. The aqueous layer was subjected to re-extraction twice with ethyl acetate, and the combined organic layers were washed with saturated aqueous sodium bicarbonate solution and saturated brine, and dried over sodium sulfate. The sodium sulfate was removed by filtration, and the filtrate was concentrated under reduced pressure. The obtained residue was purified by silica gel chromatography (developing solvent: ethyl acetate/n-hexane) to give the title compound (1.1 g) in 93% yield.
1H-NMR (CDCl3) δ: 7.89 (1H, s), 7.65 (1H, d, J = 8.1 Hz), 7.32 (1H, d, J = 7.5 Hz), 4.30-4.17 (1H, m), 3.84-3.67 (1H, m), 2.66 (1H, s), 1.93-1.74 (5H, m), 1.69-1.60 (1H, m), 1.50-1.22 (5H, m), 1.43 (9H, s), 1.10 (3H, d, J = 5.9 Hz).

(3) 4-(cis-9-((S)-2-aminopropyl)-2-oxo-1-oxa-3,4-diazaspiro[5.5]undec-4-en-5-yl)-3-bromobenzonitrile hydrochloride

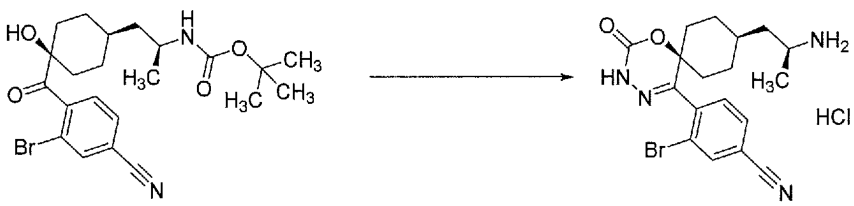

To a solution of tert-butyl ((S)-1-(cis-4-(2-bromo-4-cyanobenzoyl)-4-hydroxycyclohexyl)propan-2-yl)carbamate obtained in (2) (1.1 g) in THF (11 ml) were added DBU (0.052 ml) and CDI (0.55 g) at room temperature, and the mixture was stirred for 1 hr. Hydrazine monohydrate was added thereto, and the mixture was stirred for 30 min. 10% Aqueous citric acid solution and ethyl acetate were added thereto, and the mixture was subjected to extraction. The aqueous layer was subjected to re-extraction twice with ethyl acetate, and the combined organic layers were washed with saturated brine, and dried over sodium sulfate. The sodium sulfate was removed by filtration, and the filtrate was concentrated under reduced pressure. The obtained residue was purified by silica gel chromatography (developing solvent: ethyl acetate/n-hexane, ethyl acetate/methanol). To the obtained solid were added THF (6.5 ml) and 6M hydrochloric acid (3.2 ml) at room temperature, and the mixture was stirred all day and then at 50°C for 5 hr. The reaction solution was concentrated under reduced pressure, followed by azeotropy twice with THF (2 ml) to give the title compound (0.61 g).
1H-NMR (DMSO-D6) δ: 11.37 (1H, s), 8.39 (1H, s), 7.99 (1H, d, J = 8.1 Hz), 7.90-7.72 (3H, m), 7.70 (1H, d, J = 8.1 Hz), 3.27-3.13 (1H, m), 2.23-2.10 (2H, m), 1.62-1.17 (9H, m), 1.13 (3H, d, J = 5.4 Hz).

(4) $N^1$-((S)-1-(cis-5-(2-bromo-4-cyanophenyl)-2-oxo-1-oxa-3,4-diazaspiro[5.5]undec-4-en-9-yl)propan-2-yl)-$N^2$-methyloxamide

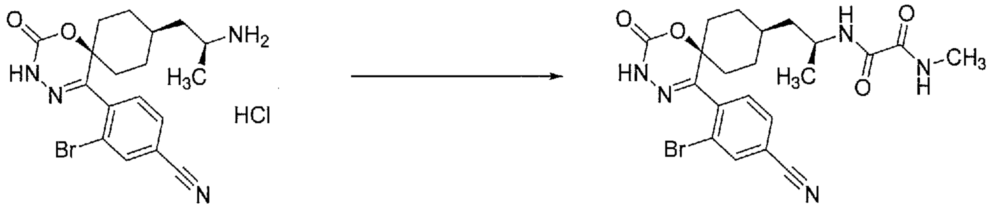

To a solution of 4-(cis-9-((S)-2-aminopropyl)-2-oxo-1-oxa-3,4-diazaspiro[5.5]undec-4-en-5-yl)-3-bromobenzonitrile hydrochloride obtained in (3) (0.57 g) in DMF (8.4 ml) were added 2-methylamino-2-oxoacetic acid (0.24 g), HOAt (0.33 g) and N,N-diisopropylethylamine (1.0 ml) at room temperature. WSC (0.47 g) was added thereto, and the mixture was stirred at room temperature all day. Water, saturated aqueous sodium bicarbonate solution and ethyl acetate were added thereto, and the mixture was subjected to extraction. The aqueous layer was subjected to re-extraction twice with ethyl acetate, and the combined organic layers were washed with saturated brine, and dried over sodium sulfate. The sodium sulfate was removed by filtration, and the filtrate was concentrated under reduced pressure. The residue was subjected to azeotropy twice with toluene (10 ml), and the obtained residue was purified by silica gel chromatography (developing solvent: ethyl acetate/n-hexane), and then reverse-phase silica gel chromatography (developing solvent: acetonitrile/water). The obtained solid was subjected to recrystallization in ethyl acetate (8.4 ml)/isopropyl ether (8.4 ml) at 50°C, and the mixture was stirred at room temperature all day. The obtained suspension was filtered, and the obtained solid was washed threetimes with a mixed solvent (1 ml) of ice-cooled ethyl acetate:isopropyl ether=1:1, and dried under reduced pressure at 50°C for 4 hr to give the title compound (0.30 g) in 57% yield.
1H-NMR (CDCl3) δ: 8.24 (1H, s), 7.97 (1H, d, J = 1.6 Hz), 7.68 (1H, dd, J = 7.9, 1.6 Hz), 7.44-7.39 (1H, m), 7.33 (1H, d, J = 7.9 Hz), 7.18-7.14 (1H, m), 4.08-4.00 (1H, m), 2.89 (3H, d, J = 5.3 Hz), 2.34-2.28 (2H, m), 1.79-1.72 (1H, m), 1.60-1.51 (2H, m), 1.50-1.31 (5H, m), 1.30-1.21 (1H, m), 1.16 (3H, d, J = 6.5 Hz).

[Production Example 3]: Synthesis of (S)-$N^1$-(1-(5-(2-bromo-4-fluorophenyl)-2-oxo-1-oxa-3,4,9-triazaspiro[5.5]undec-4-en-9-yl)propan-2-yl)-$N^2$-methyloxamide (Example 179)

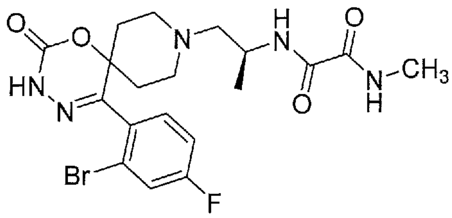

(1) tert-butyl 4-(2-bromo-4-fluorobenzoyl)-4-((trimethylsilyl)oxy)piperidine-1-carboxylate

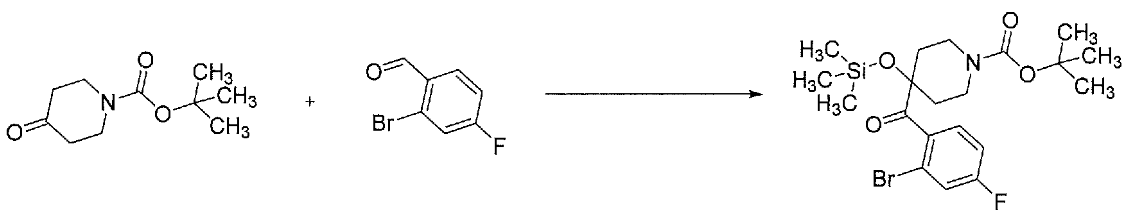

To a solution of 2-bromo-4-fluorobenzaldehyde (1.2 ml) and DMAP (0.015 g) in acetonitrile (20 ml) was added trimethylsilylcyanide (1.5 ml) at room temperature, and the mixture was stirred for 3 hr. The reaction solution was concentrated, THF (18 ml) was added thereto, and the mixture was cooled to -78°C. 1.1M LiHMDS-n-hexane solution (10 ml) was added thereto, and the mixture was stirred for 30 min. A solution of tert-butyl 4-oxopiperidine-1-carboxylate (2 g) in THF (9 ml) was added thereto, and the mixture was stirred for 1 hr. 2M Hydrochloric acid (12 ml) was added thereto, and the mixture was stirred at room temperature all day. The reaction solution was ice-cooled, 2M aqueous sodium hydroxide solution (12.5 ml) was added thereto, and the mixture was stirred at room temperature for 30 min. Water and ethyl acetate were added thereto, and the mixture was subjected to extraction. The aqueous layer was subjected to re-extraction with

ethyl acetate, and the combined organic layers were dried over magnesium sulfate. The magnesium sulfate was removed by filtration, and the filtrate was concentrated under reduced pressure. The obtained residue was purified by silica gel chromatography (developing solvent: ethyl acetate/n-hexane=15/85 to 35/65) to give the title compound (1.8 g) in 34% yield.

1H-NMR (DMSO-D6) δ: 7.74 (1H, dd, J = 8.7, 2.4 Hz), 7.62 (1H, dd, J = 8.6, 6.0 Hz), 7.38 (1H, td, J = 8.6, 2.5 Hz), 3.66 (2H, dt, J = 13.9, 4.0 Hz), 3.24-3.14 (2H, m), 1.92-1.83 (2H, m), 1.82-1.75 (2H, m), 1.38 (9H, s), 0.06 (9H, s).

(2) tert-butyl 4-(2-bromo-4-fluorobenzoyl)-4-hydroxypiperidine-1-carboxylate

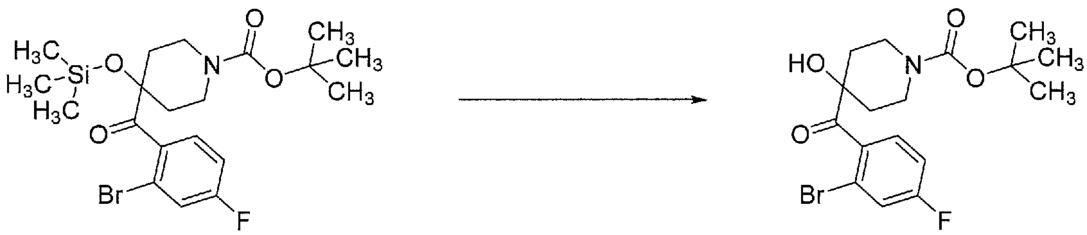

To a solution of tert-butyl 4-(2-bromo-4-fluorobenzoyl)-4-((trimethylsilyl)oxy)piperidine-1-carboxylate obtained in (1) (1.0 g) in THF (8.8 ml) was added 1M TBAF-THF solution (1.9 ml) at room temperature, and the mixture was stirred for 1 hr. Saturated aqueous ammonium chloride solution and ethyl acetate were added thereto, and the mixture was subjected to extraction. The organic layer was concentrated under reduced pressure, and the obtained residue was purified by silica gel chromatography (developing solvent: ethyl acetate/n-hexane=20/80 to 40/60) to give the title compound (0.74 g) in 92% yield.

1H-NMR (DMSO-D6) δ: 7.67 (1H, dd, J = 8.8, 2.5 Hz), 7.58 (1H, dd, J = 8.6, 6.0 Hz), 7.34 (1H, td, J = 8.6, 2.4 Hz), 5.70 (1H, s), 3.84-3.73 (2H, m), 3.15-2.92 (2H, m), 1.81-1.66 (4H, m), 1.40 (9H, s).

(3) tert-butyl 5-(2-bromo-4-fluorophenyl)-2-oxo-1-oxa-3,4,9-triazaspiro[5.5]undec-4-ene-9-carboxylate

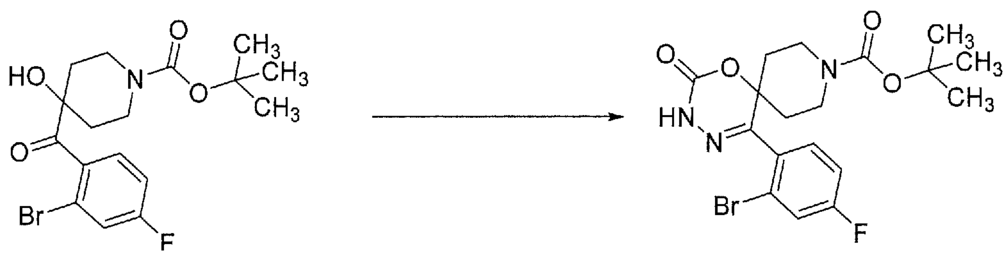

To a solution of tert-butyl 4-(2-bromo-4-fluorobenzoyl)-4-hydroxypiperidine-1-carboxylate obtained in (2) (0.74 g) in THF (6.9 ml) were added DBU (0.026 ml) and CDI (0.42 g) at room temperature, and the mixture was stirred for 1 hr. Hydrazine monohydrate (0.25 ml) was added thereto, and the mixture was stirred for 1 hr. 1M Hydrochloric acid and ethyl acetate were added thereto, and the mixture was subjected to extraction. The aqueous layer was subjected to re-extraction with ethyl acetate, and the combined organic layers were washed with saturated aqueous sodium bicarbonate solution and saturated brine, and dried over magnesium sulfate. The sodium sulfate was removed by filtration, and the filtrate was concentrated under reduced pressure. To the obtained residue were added THF (7.3 ml) and 6M hydrochloric acid (3.7 ml) at room temperature, and the mixture was stirred for 20 hr. The mixture was neutralized with 4M aqueous sodium hydroxide solution (6.0 ml), $Boc_2O$ (0.44 ml) was added thereto, and the mixture was stirred for 1 hr. Ethyl acetate was added thereto, and the mixture was subjected to extraction. The organic layer was concentrated under reduced pressure, and the obtained residue was purified by silica gel chromatography (developing solvent: ethyl acetate/n-hexane=30/70 to 50/50) to give the title compound (0.60 g) in 79% yield.

1H-NMR (DMSO-D6) δ: 11.35 (1H, s), 7.76 (1H, dd, J = 8.6, 2.5 Hz), 7.57 (1H, dd, J = 8.7, 5.9 Hz), 7.36 (1H, td, J = 8.5, 2.6 Hz), 3.90-3.78 (2H, m), 3.10-2.91 (2H, m), 2.16-2.06 (2H, m), 1.59-1.46 (2H, m), 1.35 (9H, s).

(4) 5-(2-bromo-4-fluorophenyl)-1-oxa-3,4,9-triazaspiro[5.5]undec-4-en-2-one hydrochloride

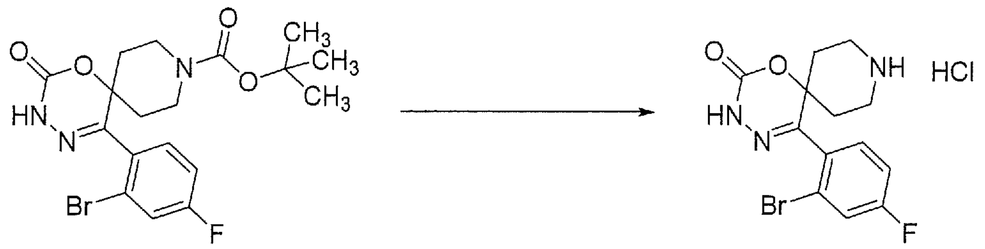

To tert-butyl 5-(2-bromo-4-fluorophenyl)-2-oxo-1-oxa-3,4,9-triazaspiro[5,5]undec-4-ene-9-carboxylate obtained in (3) (0.60 g) was added 4M hydrogen chloride-CPME solution (5.6 ml) at room temperature, and the mixture was stirred for 3 hr. The obtained reaction solution was concentrated under reduced pressure to give the title compound (0.53 g) in 100% yield.
1H-NMR (DMSO-D6) δ: 11.48 (1H, s), 8.87-8.70 (1H, m), 8.56-8.40 (1H, m), 7.81 (1H, dd, J = 8.6, 2.5 Hz), 7.61 (1H, dd, J = 8.7, 5.9 Hz), 7.44 (1H, td, J = 8.5, 2.6 Hz), 3.28-3.20 (2H, m), 3.11-2.99 (2H, m), 2.36-2.26 (2H, m), 1.99-1.85 (2H, m).

(5) tert-butyl (S)-(1-(5-(2-bromo-4-fluorophenyl)-2-oxo-1-oxa-3,4,9-triazaspiro[5.5]undec-4-en-9-yl)propan-2-yl)carbamate

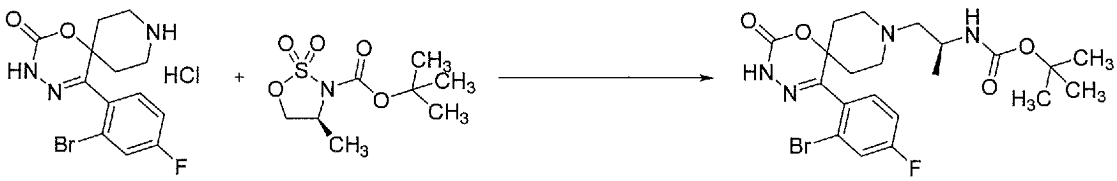

To a solution of 5-(2-bromo-4-fluorophenyl)-1-oxa-3,4,9-triazaspiro[5.5]undec-4-en-2-one hydrochloride obtained in (4) (0.53 g) in methanol (5.3 ml) were added triethylamine (0.89 ml) and tert-butyl (S)-4-methyl-1,2,3-oxathiazolidine-3-carboxylate 2,2-dioxide (0.36 g) under ice-cooled, and the mixture was stirred for 5 hr. The methanol was evaporated, THF (5.3 ml), water (5.3 ml) and potassium hydrogen sulfate (0.52 g) were added thereto, and the mixture was stirred at room temperature for 2 days. Saturated aqueous sodium bicarbonate solution and ethyl acetate were added thereto, and the mixture was subjected to extraction. The organic layer was concentrated under reduced pressure, and the obtained residue was purified by thin layer chromatography (developing solvent: ethyl acetate/n-hexane=67/33) to give the title compound (0.57 g) in 84% yield.
1H-NMR (DMSO-D6) δ: 11.24 (1H, s), 7.75 (1H, dd, J = 8.7, 2.7 Hz), 7.53 (1H, dd, J = 8.6, 6.0 Hz), 7.36 (1H, td, J = 8.5, 2.6 Hz), 6.45-6.38 (1H, m), 3.64-3.51 (1H, m), 2.81-2.71 (1H, m), 2.63-2.57 (1H, m), 2.35-2.24 (1H, m), 2.24-2.01 (5H, m), 1.68-1.44 (2H, m), 1.28 (9H, s), 0.94 (3H, d, J = 6.5 Hz).

(6) (S)-9-(2-aminopropyl)-5-(2-bromo-4-fluorophenyl)-1-oxa-3,4,9-triazaspiro[5.5]undec-4-en-2-one dihydrochloride

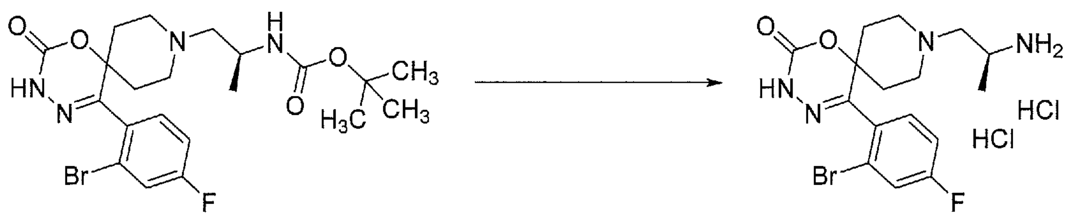

To tert-butyl (S)-(1-(5-(2-bromo-4-fluorophenyl)-2-oxo-1-oxa-3,4,9-triazaspiro[5.5]undec-4-en-9-yl)propan-2-yl)carbamate obtained in (5) (0.12 g) was added 4M hydrogen chloride-CPME solution (1.1 ml) at room temperature,

and the mixture was stirred for 3 hr. The obtained reaction solution was concentrated under reduced pressure to give the title compound (0.13 g).
1H-NMR (DMSO-D6) δ: 11.59-11.30 (1H, m), 10.86-10.59 (1H, m), 8.46-8.13 (2H, m), 7.79 (1H, dd, J = 8.6, 2.5 Hz), 7.58 (1H, dd, J = 8.6, 6.0 Hz), 7.41 (1H, td, J = 8.5, 2.5 Hz), 3.90-3.53 (5H, m), 3.49-3.30 (1H, m), 2.44-2.10 (4H, m), 1.68-1.42 (2H, m), 1.31-1.13 (3H, m).

(7) (S)-N1-(1-(5-(2-bromo-4-fluorophenyl)-2-oxo-1-oxa-3,4,9-triazaspiro[5.5]undec-4-en-9-yl)propan-2-yl)-N2-methyloxamide

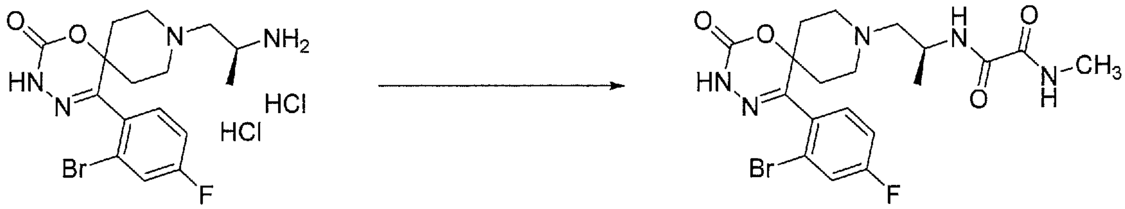

To a solution of (S)-9-(2-aminopropyl)-5-(2-bromo-4-fluorophenyl)-1-oxa-3,4,9-triazaspiro[5.5]undec-4-en-2-one dihydrochloride obtained in (6) (0.04 g) in acetonitrile (0.34 ml) were added 2-methylamino-2-oxoacetic acid (0.014 g), HOAt (0.018 g) and N,N-diisopropylethylamine (0.059 ml) at room temperature. WSC (0.026 g) was added thereto, and the mixture was stirred at room temperature for 3 hr. Saturated aqueous sodium bicarbonate solution and ethyl acetate were added thereto, and the mixture was subjected to extraction. The organic layer was concentrated under reduced pressure, and the obtained residue was purified by thin layer chromatography (developing solvent: ethyl acetate) to give the title compound (0.029 g) in 87% yield.
1H-NMR (DMSO-D6) δ: 11.25 (1H, s), 8.63 (1H, q, J = 4.7 Hz), 8.35 (1H, d, J = 8.8 Hz), 7.75 (1H, dd, J = 8.6, 2.5 Hz), 7.54 (1H, dd, J = 8.6, 5.8 Hz), 7.36 (1H, td, J = 8.6, 2.5 Hz), 3.96-3.89 (1H, m), 2.72-2.60 (2H, m), 2.64 (3H, d, J = 4.7 Hz), 2.42 (1H, dd, J = 12.5, 8.3 Hz), 2.33-2.19 (3H, m), 2.10-2.02 (2H, m), 1.64-1.48 (2H, m), 1.04 (3H, d, J = 6.5 Hz).

[Production Example 4]: Synthesis of N1-((S)-1-(cis-5-(2-bromo-5-cyanophenyl)-2-oxo-1-oxa-3,4-diazaspiro[5.5]undec-4-en-9-yl)propan-2-yl)-N2-methyloxamide (Example 198)

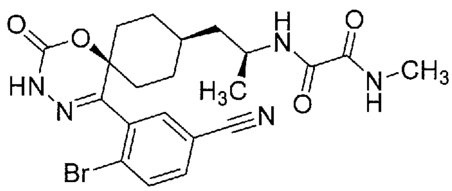

(1) tert-butyl ((S)-1-(cis-4-(2-bromo-5-cyanobenzoyl)-4-((trimethylsilyl)oxy)cyclohexyl)propan-2-yl)carbamate

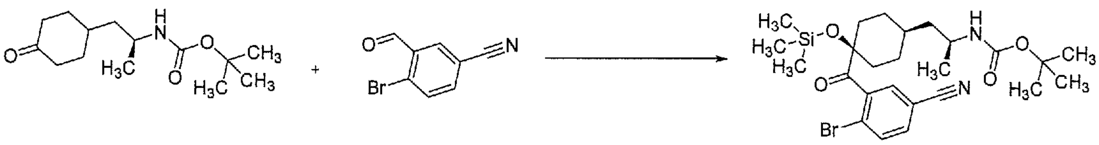

To a solution of 4-bromo-3-formylbenzonitrile (54 g) and lithium chloride (1.1 g) in THF (350 ml) was added trimethylsilylcyanide (34 ml) under water-cooling, and the mixture was stirred for 1 hr. The mixture was cooled to -78°C, 1.1M LiHMDS-n-hexane solution (225 ml) was added thereto, and the mixture was stirred for 2 hr. The mixture was warmed to - 60°C, a solution of tert-butyl (S)-(1-(4-oxocyclohexyl)propan-2-yl)carbamate (obtained in (9) of [Production Example 1]) (50 g) in THF (100 ml) was added thereto, and the mixture was stirred for 1.5 hr. Acetic acid (15 ml) was added thereto, the mixture was warmed to -20°C, and water (250 ml) was added thereto. After stirring at room temperature for 10 min,

water (250 ml) and toluene (500 ml) were added thereto, and the mixture was subjected to extraction. The organic layer was washed with water (500 ml, threetimes) and 20% brine (500 ml), and dried over sodium sulfate. The sodium sulfate was removed by filtration, and the filtrate was concentrated under reduced pressure at room temperature to give the title compound (150 g) containing small amount of toluene.
1H-NMR (DMSO-D6) δ: 8.01-7.98 (1H, m), 7.96 (1H, d, J = 8.3 Hz), 7.89-7.85 (1H, m), 6.59 (1H, d, J = 8.6 Hz), 3.62-3.48 (1H, m), 2.01-1.91 (2H, m), 1.77-1.50 (4H, m), 1.40-1.07 (5H, m), 1.36 (9H, s), 0.99 (3H, d, J = 6.5 Hz), 0.00 (9H, s).

(2) tert-butyl ((S)-1-(cis-4-(2-bromo-5-cyanobenzoyl)-4-hydroxycyclohexyl)propan-2-yl)carbamate

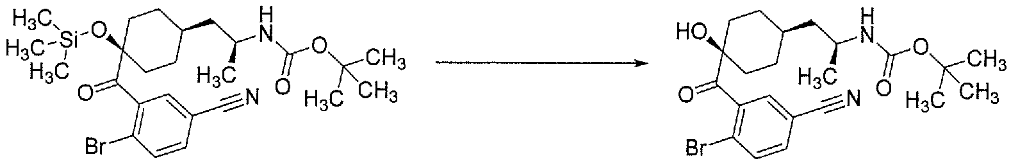

To tert-butyl ((S)-1-(cis-4-(2-bromo-5-cyanobenzoyl)-4-((trimethylsilyl)oxy)cyclohexyl)propan-2-yl)carbamate obtained in (1) (150 g) were added THF (310 ml), acetic acid (14 ml) and 1M TBAF-THF solution (230 ml) at room temperature, and the mixture was stirred at room temperature for 1.5 hr. Water (500 ml) and toluene (500 ml) were added thereto, and the mixture was subjected to extraction. The obtained organic layer was washed with 5% aqueous sodium bicarbonate solution (500 ml), water (500 ml) and saturated brine (500 ml), and dried over sodium sulfate. The sodium sulfate was removed by filtration, and the filtrate was concentrated under reduced pressure. Methanol (150 ml) was added thereto, and the mixture was concentrated again under reduced pressure. To the obtained residue were added methanol (270 ml) and water (90 ml), and the mixture was stirred for two days. Under ice-cooled, the mixture was stirred again all day, and the obtained solid was collected by filtration. The solid was washed with a mixed solvent of ice-cooled methanol (68 ml) and water (22 ml), and dried under reduced pressure at 60°C for 3 hr and then at room temperature all day to give the title compound (52 g) in 58% yield.
1H-NMR (DMSO-D6) δ: 7.92-7.88 (2H, m), 7.83-7.79 (1H, m), 6.59 (1H, d, J = 8.1 Hz), 5.28 (1H, s), 3.64-3.48 (1H, m), 1.88-1.78 (2H, m), 1.71-1.09 (9H, m), 1.38 (9H, s), 0.99 (3H, d, J = 6.5 Hz).

(3) 3-(cis-9-((S)-2-aminopropyl)-2-oxo-1-oxa-3,4-diazaspiro[5.5]undec-4-en-5-yl)-4-bromobenzonitrile

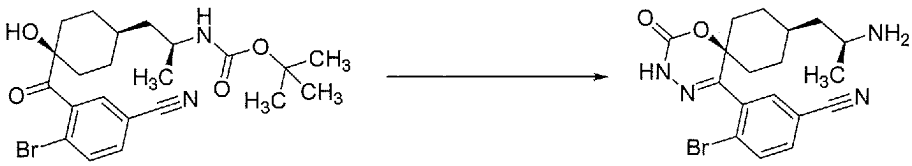

To a solution of tert-butyl ((S)-1-(cis-4-(2-bromo-5-cyanobenzoyl)-4-hydroxycyclohexyl)propan-2-yl)carbamate obtained in (2) (48 g) in THF (240 ml) were added DBU (2.4 ml) and CDI (25 g) at room temperature, and the mixture was stirred for 1 hr. Hydrazine monohydrate (10 ml) was added thereto, and the mixture was stirred for 30 min. Water (240 ml) and ethyl acetate (240 ml) were added thereto, and the mixture was subjected to extraction. The obtained organic layer was washed with 10% aqueous citric acid solution (240 ml), water (240 ml) and 20% brine (240 ml), and dried over sodium sulfate. The sodium sulfate was removed by filtration, and the filtrate was concentrated under reduced pressure. To the obtained residue was added ethyl acetate (530 ml), and the mixture was stirred at room temperature for 1 hr. The solid was removed by filtration, and the filtrate was concentrated under reduced pressure. To concentrate were added THF (240 ml) and 6M hydrochloric acid (120 ml) at room temperature, and the mixture was stirred at 50°C for 5 hr. The mixture was gradually cooled to room temperature, water (242 ml) and 8M aqueous sodium hydroxide solution (91 ml) were added thereto, and the mixture was stirred for 5 min. THF (120 ml) and toluene (240 ml) were added thereto, and the mixture was subjected to extraction. The aqueous layer was subjected to re-extraction with THF (96 ml) and toluene (120 ml). The combined organic layers were washed with 20% brine (240 ml), and dried over sodium sulfate. The sodium sulfate was removed by filtration, and the filtrate was concentrated under reduced pressure. Ethyl acetate (144 ml) was added thereto, and the mixture was concentrated again under reduced pressure. To the obtained residue was added ethyl acetate (54 ml) at room temperature, and the mixture was stirred all day. The obtained solid was collected by filtration, washed with ice-cooled ethyl acetate (36 ml), and dried under reduced pressure 60°C for 3 hr and then at room temperature all day to give the title compound (32 g) in 89% yield.
1H-NMR (DMSO-D6) δ: 11.29 (1H, br s), 8.04-7.96 (2H, m), 7.91-7.84 (1H, m), 2.87-2.75 (1H, m), 2.19-2.05 (2H, m), 1.80-1.03 (11H, m), 0.92 (3H, d, J = 5.9 Hz).

(4) $N^1$-((S)-1-(cis-5-(2-bromo-5-cyanophenyl)-2-oxo-1-oxa-3,4-diazaspiro[5.5]undec-4-en-9-yl)propan-2-yl)-$N^2$-methyloxamide

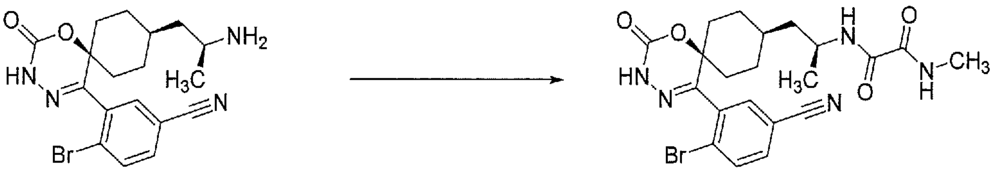

To a solution of 3-(cis-9-((S)-2-aminopropyl)-2-oxo-1-oxa-3,4-diazaspiro[5.5]undec-4-en-5-yl)-4-bromobenzonitrile (obtained in the same manner as in (3)) (0.02 g) in DMF (0.5 ml) were added 2-methylamino-2-oxoacetic acid (0.008 g), HOAt (0.007 g), WSC (0.014 g) and N,N-diisopropylethylamine (0.026 ml) at room temperature, and the mixture was stirred all day. Water and ethyl acetate were added thereto, and the mixture was subjected to extraction. The aqueous layer was subjected to re-extraction with ethyl acetate. The combined organic layers were washed with water and saturated brine, and dried over sodium sulfate. The sodium sulfate was removed by filtration, and the filtrate was concentrated under reduced pressure. The residue was purified by thin layer chromatography (developing solvent: ethyl acetate) to give the title compound (0.021 g) in 87% yield.
1H-NMR (DMSO-D6) δ: 11.29 (1H, s), 8.65-8.59 (1H, m), 8.41 (1H, d, J = 9.0 Hz), 8.00 (1H, d, J = 1.8 Hz), 7.98 (1H, d, J = 8.3 Hz), 7.86 (1H, dd, J = 8.3, 1.8 Hz), 3.98-3.85 (1H, m), 2.64 (3H, d, J = 4.9 Hz), 2.16-2.04 (2H, m), 1.69-1.59 (1H, m), 1.59-1.13 (8H, m), 1.06 (3H, d, J = 6.5 Hz).

(5) crystal of $N^1$-((S)-1-(cis-5-(2-bromo-5-cyanophenyl)-2-oxo-1-oxa-3,4-diazaspiro[5.5]undec-4-en-9-yl)propan-2-yl)-$N^2$-methyloxamide

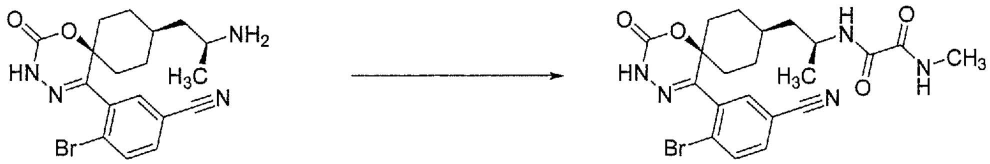

Under nitrogen atmosphere, to a solution of 3-(cis-9-((S)-2-aminopropyl)-2-oxo-1-oxa-3,4-diazaspiro[5.5]undec-4-en-5-yl)-4-bromobenzonitrile (obtained in the same manner as in (3)) (42 g), 2-methylamino-2-oxoacetic acid (13 g) and WSC (24 g) in DMF (210 ml) was added HOAt (7.1 g) at room temperature, and the mixture was stirred for 1 hr. 5% Aqueous sodium bicarbonate solution (420 ml) and ethyl acetate (420 ml) were added thereto, and the mixture was subjected to extraction. The aqueous layer was subjected to re-extraction with ethyl acetate (210 ml), and the combined organic layers were washed with 5% aqueous sodium bicarbonate solution (210 ml), water (210 ml×2) and saturated brine (210 ml). Sodium sulfate and activated carbon (1.3 g) were added thereto, the mixture was stirred for 1.5 hr, and the solid was removed by filtration. The obtained solution was concentrated under reduced pressure, and to the obtained amorphous solid was added DMF (115 ml). The obtained solution was concentrated under reduced pressure, the ethyl acetate was evaporated, and the obtained solution was subjected to dust removal filtration, followed by washing with DMF (139 ml). To the obtained DMF solution was added water (150 ml) at room temperature. After confirming the solid precipitation, water (612 ml) was added dropwise thereto, and the obtained suspension was stirred at room temperature for 5 days. The suspension was warmed to 50°C, stirred overnight, and allowed to cool to room temperature. The suspension was filtered, and the solid was washed with water. The obtained wet crystals were air-dried overnight, and then dried under reduced pressure at 60°C for 3 hr to give the title compound (45 g).

[Production Example 5]: Synthesis of 1-((S)-1-(cis-5-(2-bromo-5-cyanophenyl)-2-oxo-1-oxa-3,4-diazaspiro[5.5]undec-4-en-9-yl)propan-2-yl)-3-cyclopropylurea (Example 222)

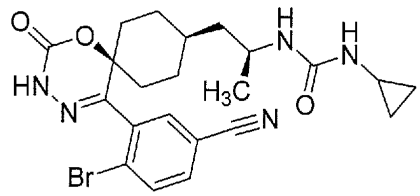

(1) 1-((S)-1-(cis-5-(2-bromo-5-cyanophenyl)-2-oxo-1-oxa-3,4-diazaspiro[5.5]undec-4-en-9-yl)propan-2-yl)-3-cyclopropylurea

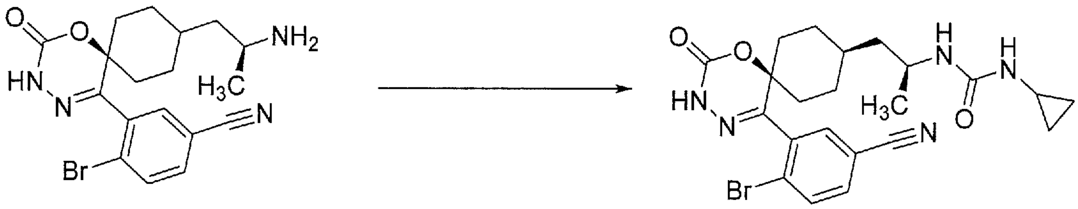

To a solution of 3-(cis-9-((S)-2-aminopropyl)-2-oxo-1-oxa-3,4-diazaspiro[5.5]undec-4-en-5-yl)-4-bromobenzonitrile (obtained in the same manner as in (3) of [Production Example 4]) (0.020 g) in THF (0.4 ml) was added cyclopropylisocyanate (0.005 ml) at room temperature, and the mixture was stirred for 3 hr. Water and ethyl acetate were added thereto, and the mixture was subjected to extraction. The aqueous layer was subjected to re-extraction with ethyl acetate, and the combined organic layers were washed with water and saturated brine, and dried over sodium sulfate. The sodium sulfate was removed by filtration, and the filtrate was concentrated under reduced pressure. The obtained residue was purified by thin layer chromatography (developing solvent: ethyl acetate) to give the title compound (0.019 g) in 79% yield.
1H-NMR (DMSO-D6) δ: 11.30 (1H, s), 8.03 (1H, d, J = 1.8 Hz), 7.99 (1H, d, J = 8.3 Hz), 7.87 (1H, dd, J = 8.4, 2.0 Hz), 5.89 (1H, d, J = 2.8 Hz), 5.46 (1H, d, J = 8.6 Hz), 3.72-3.61 (1H, m), 2.40-2.31 (1H, m), 2.19-2.07 (2H, m), 1.70-1.57 (1H, m), 1.56-1.06 (8H, m), 0.97 (3H, d, J = 6.5 Hz), 0.56-0.46 (2H, m), 0.31-0.22 (2H, m).

[Production Example 6]: Synthesis of $N^1$-((S)-1-(cis-5-(4-chloro-6-methylpyridin-3-yl)-2-oxo-1-oxa-3,4-diazaspiro[5.5]undec-4-en-9-yl)propan-2-yl)-$N^2$-methyloxamide

(Example 230)

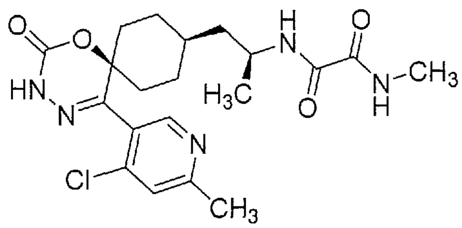

(1) tert-butyl ((S)-1-(cis-4-(4-chloro-6-methylnicotinoyl)-4-((trimethylsilyl)oxy)cyclohexyl)propan-2-yl)carbamate

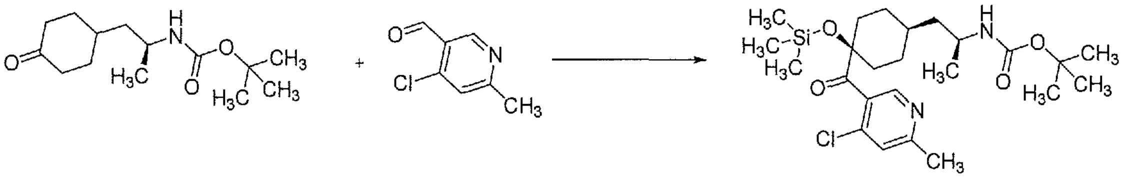

To a mixture of 4-chloro-6-methylnicotinaldehyde (0.55 g) and lithium chloride (0.015 g) was added trimethylsilylcyanide (0.55 ml), and the mixture was stirred at room temperature for 10 min. THF (7 ml) was added thereto, and the mixture was cooled to -78°C. 1M NaHMDS-THF solution (1.9 ml) was added thereto, and the mixture was stirred for 5 min. tert-Butyl (S)-(1-(4-oxocyclohexyl)propan-2-yl)carbamate (obtained in the same manner as in (9) of [Production Example 1]) (0.7 g) was added thereto, and the mixture was stirred for 30 min, and warmed to 0°C over 1 hr. Water and ethyl acetate were added thereto, and the mixture was subjected to extraction. The aqueous layer was subjected to re-extraction twice with ethyl acetate, and the combined organic layers were washed with saturated brine, and dried over sodium sulfate. The sodium sulfate was removed by filtration, and the filtrate was concentrated under reduced pressure. The obtained residue was purified by silica gel chromatography (developing solvent: ethyl acetate/n-hexane) to give the title compound (1.1 g) in 83% yield.

1H-NMR (CDCl3) δ: 8.58 (1H, s), 7.25 (1H, s), 4.32-4.15 (1H, m), 3.84-3.65 (1H, m), 2.57 (3H, s), 1.99-1.88 (2H, m), 1.83-1.68 (3H, m), 1.66-1.55 (1H, m), 1.43 (9H, s), 1.41-1.21 (5H, m), 1.11 (3H, d, J = 6.5 Hz), 0.06 (9H, s).

(2) tert-butyl ((S)-1-(cis-4-(4-chloro-6-methylnicotinoyl)-4-hydroxycyclohexyl)propan-2-yl)carbamate

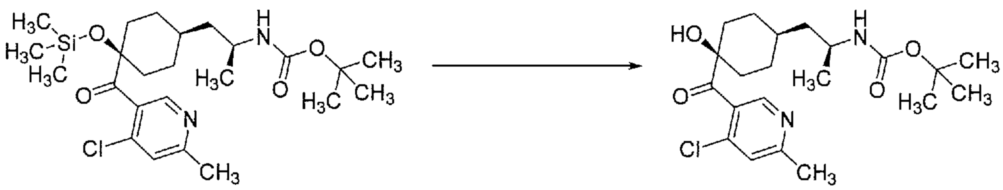

To a solution of tert-butyl ((S)-1-(cis-4-(4-chloro-6-methylnicotinoyl)-4-((trimethylsilyl)oxy)cyclohexyl)propan-2-yl)carbamate obtained in (1) (1.1 g) in THF (2.2 ml) were added acetic acid (0.39 ml) and 1M TBAF-THF solution (3.4 ml) at room temperature, and the mixture was stirred for 15 min, and allowed to stand all day. Saturated aqueous ammonium chloride solution, water and ethyl acetate were added thereto, and the mixture was subjected to extraction. The aqueous layer was subjected to re-extraction twice with ethyl acetate, and the combined organic layers were washed with saturated brine, and dried over sodium sulfate. The sodium sulfate was removed by filtration, and the filtrate was concentrated under reduced pressure. The obtained residue was purified by silica gel chromatography (developing solvent: ethyl acetate/n-hexane) to give the title compound (0.80 g) in 86% yield.

1H-NMR (CDCl3) δ: 8.39 (1H, s), 7.26 (1H, s), 4.29-4.16 (1H, m), 3.83-3.65 (1H, m), 2.93 (1H, s), 2.58 (3H, s), 1.87-1.73 (5H, m), 1.67-1.59 (1H, m), 1.43 (9H, s), 1.41-1.22 (5H, m), 1.10 (3H, d, J = 6.5 Hz).

(3) cis-9-((S)-2-aminopropyl)-5-(4-chloro-6-methylpyridin-3-yl)-1-oxa-3,4-diazaspiro[5.5]undec-4-en-2-one dihydrochloride

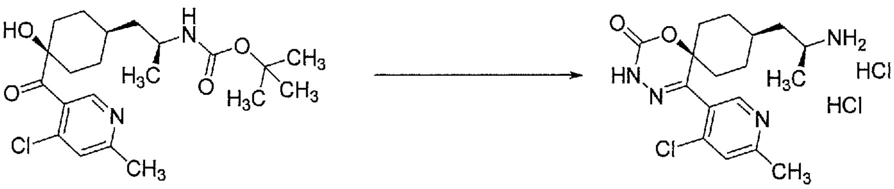

To a solution of tert-butyl ((S)-1-(cis-4-(4-chloro-6-methylnicotinoyl)-4-hydroxycyclohexyl)propan-2-yl)carbamate obtained in (2) (0.8 g) in THF (8 ml) were added DBU (0.044 ml) and CDI (0.47 g) at room temperature, and the mixture was stirred for 1 hr. Hydrazine monohydrate (0.19 ml) was added thereto, and the mixture was stirred for 1 hr. 10% Aqueous citric acid solution and ethyl acetate were added thereto, and the mixture was subjected to extraction. The

aqueous layer was subjected to re-extraction twice with ethyl acetate, and the combined organic layers were washed with saturated brine, and dried over sodium sulfate. The sodium sulfate was removed by filtration, and the filtrate was concentrated under reduced pressure. The obtained residue was purified by silica gel chromatography (developing solvent: ethyl acetate/n-hexane). To the obtained solid were added THF (9.0 ml) and 6M hydrochloric acid (4.5 ml) at room temperature, and the mixture was stirred all day and then at 50°C for 6 hr. The reaction solution was concentrated under reduced pressure, followed by azeotropy twice with THF (10 ml) to give the title compound (0.98 g).
1H-NMR (DMSO-D6) δ: 11.40 (1H, s), 8.64 (1H, s), 8.08-7.97 (3H, m), 7.81 (1H, s), 3.25-3.14 (1H, m), 2.59 (3H, s), 2.18-2.08 (2H, m), 1.62-1.19 (9H, m), 1.15 (3H, d, J = 6.5 Hz).

(4) $N^1$-((S)-1-(cis-5-(4-chloro-6-methylpyridin-3-yl)-2-oxo-1-oxa-3,4-diazaspiro[5.5]undec-4-en-9-yl)propan-2-yl)-$N^2$-methyloxamide

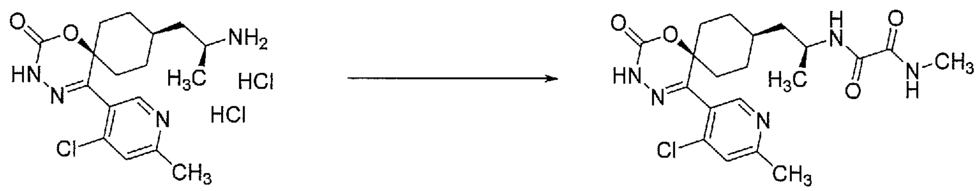

To a solution of cis-9-((S)-2-aminopropyl)-5-(4-chloro-6-methylpyridin-3-yl)-1-oxa-3,4-diazaspiro[5.5]undec-4-en-2-one dihydrochloride obtained in (3) (0.1 g) in DMF (1.5 ml) were added 2-methylamino-2-oxoacetic acid (0.045 g), HOAt (0.062 g) and N,N-diisopropylethylamine (0.23 ml) at room temperature. WSC (0.087 g) was added thereto, and the mixture was stirred at room temperature for 15 min, and allowed to stand all day. Water, saturated aqueous sodium bicarbonate solution and ethyl acetate were added thereto, and the mixture was subjected to extraction. The aqueous layer was subjected to re-extraction twice with ethyl acetate, and the combined organic layers were washed with saturated brine, and dried over sodium sulfate. The sodium sulfate was removed by filtration, and the filtrate was concentrated under reduced pressure, followed by azeotropy with toluene (5 ml). The obtained residue was purified by silica gel chromatography (developing solvent: ethyl acetate/n-hexane) to give the title compound (0.067 g) in 71% yield.
1H-NMR (DMSO-D6) δ: 11.28 (1H, s), 8.62 (1H, q, J = 4.8 Hz), 8.48 (1H, s), 8.41 (1H, d, J = 9.2 Hz), 7.58 (1H, s), 3.98-3.83 (1H, m), 2.64 (3H, d, J = 4.8 Hz), 2.51 (3H, s), 2.15-2.02 (2H, m), 1.71-1.59 (1H, m), 1.57-1.15 (8H, m), 1.05 (3H, d, J = 6.5 Hz).

[Production Example 7]: Synthesis of N-((S)-1-(cis-5-(2-bromo-4-fluorophenyl)-9-hydroxy-2-oxo-1-oxa-3,4-diazaspiro [5.5]undec-4-en-9-yl)propan-2-yl)-3-methyl-1H-pyrazole-5-carboxamide

(Example 320)

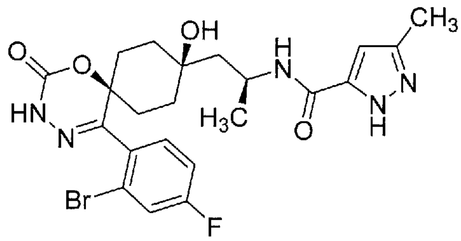

(1) 1,4-dioxaspiro[4.5]dec-7-en-8-yl trifluoromethanesulfonate

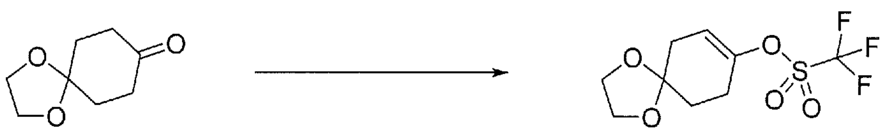

A solution of 1,4-dioxaspiro[4.5]decan-8-one (5 g) in THF (50 ml) was cooled to -78°C, 1.1M LiHMDS-n-hexane solution (31 ml) was added thereto, and the mixture was stirred for 10 min. The mixture was warmed to 0°C, stirred for 20 min, and cooled to -78°C, and Comins reagent (14 g) was added thereto. The mixture was stirred for 30 min, warmed to 0°C, and stirred for additional 1.5 hr. Saturated aqueous sodium bicarbonate solution, water and ethyl acetate were added thereto, and the mixture was subjected to extraction. The aqueous layer was subjected to re-extraction with ethyl acetate, and the combined organic layers were washed with saturated brine, and dried over sodium sulfate. The sodium sulfate was removed by filtration, and the filtrate was concentrated under reduced pressure. The obtained residue was purified by silica gel chromatography (developing solvent: ethyl acetate/n-hexane) to give the title compound (7.5 g) in 81% yield.
1H-NMR (CDC13) δ: 5.69-5.65 (1H, m), 4.00 (4H, t, J = 2.4 Hz), 2.54 (2H, t, J = 6.5 Hz), 2.41 (2H, d, J = 2.7 Hz), 1.91 (2H, t, J = 6.7 Hz).

(2) (S)-2-((tert-butoxycarbonyl)amino)propyl methanesulfonate

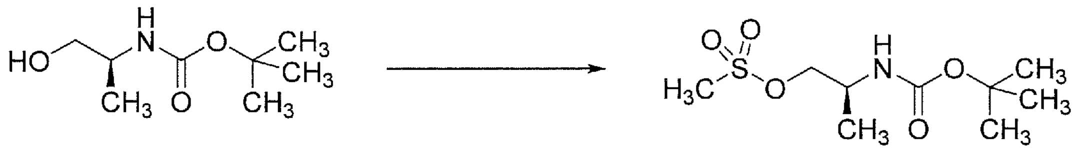

To a solution of tert-butyl (S)-(1-hydroxypropan-2-yl)carbamate (10 g) in THF (100 ml) was added triethylamine (16 ml) at room temperature. The mixture was cooled to 0°C, methanesulfonic anhydride (11 g) was added thereto, and the mixture was stirred at room temperature for 15 min. The reaction solution was concentrated under reduced pressure, and the obtained residue was purified by silica gel chromatography (developing solvent: ethyl acetate/n-hexane) to give the title compound (13.7 g) in 95% yield.
1H-NMR (CDCl3) δ: 4.68-4.49 (1H, m), 4.28-4.19 (1H, m), 4.15 (1H, dd, J = 9.7, 4.3 Hz), 4.04-3.88 (1H, m), 3.04 (3H, s), 1.45 (9H, s), 1.24 (3H, d, J = 7.0 Hz).

(3) tert-butyl (S)-(1-(1,4-dioxaspiro[4.5]dec-7-en-8-yl)propan-2-yl)carbamate

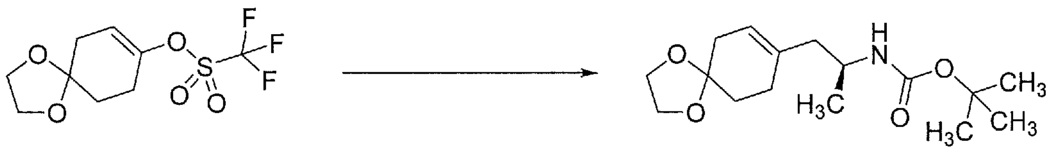

To a solution of 1,4-dioxaspiro[4.5]dec-7-en-8-yl trifluoromethanesulfonate obtained in (1) (5 g) in DMA (100 ml) were added (S)-2-((tert-butoxycarbonyl)amino)propyl methanesulfonate obtained in (2) (7.9 g), nickel(II) iodide (0.093 ml), 2,2':6',2"-terpyridine (0.61 g), manganese (2.9 g) and sodium iodide (1.3 g) at room temperature, and the mixture was stirred at 80°C for 1.5 hr. Water and ethyl acetate were added thereto at room temperature, and the mixture was subjected to extraction. The aqueous layer was subjected to re-extraction twice with ethyl acetate, and the combined organic layers were washed with saturated brine, and dried over sodium sulfate. The sodium sulfate was removed by filtration, and the filtrate was concentrated under reduced pressure. The obtained residue was purified by silica gel chromatography (developing solvent: ethyl acetate/n-hexane) to give the title compound (1.9 g) in 37% yield.
1H-NMR (CDCl3) δ: 5.34 (1H, s), 4.40-4.21 (1H, m), 3.97 (4H, s), 3.88-3.71 (1H, m), 2.30-1.98 (6H, m), 1.75 (2H, t, J = 6.5 Hz), 1.43 (9H, s), 1.10 (3H, d, J = 6.5 Hz).

(4) tert-butyl (S)-(1-(4-oxocyclohex-1-en-1-yl)propan-2-yl)carbamate

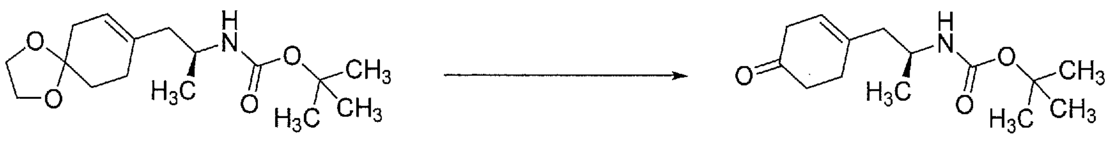

To a solution of tert-butyl (S)-(1-(1,4-dioxaspiro[4,5]dec-7-en-8-yl)propan-2-yl)carbamate obtained in (3) (0.5 g) in water (2 ml) was added acetic acid (8 ml) at room temperature, and the mixture was stirred for 15 min, and allowed to stand all day. The reaction solution was concentrated under reduced pressure, followed by azeotropy threetimes with toluene (2 ml), and the obtained residue was purified by silica gel chromatography (developing solvent: ethyl acetate/n-hexane) to give the title compound (0.37 g) in 87% yield.
1H-NMR (CDCl3) δ: 5.49 (1H, s), 4.37-4.22 (1H, m), 3.91-3.76 (1H, m), 2.85 (2H, s), 2.60-2.30 (4H, m), 2.22-2.09 (2H, m), 1.42 (9H, s), 1.14 (3H, d, J = 6.5 Hz).

(5) tert-butyl ((2S)-1-(4-(2-bromo-4-fluorobenzoyl)-4-((trimethylsilyl)oxy)cyclohex-1-en-1-yl)propan-2-yl)carbamate

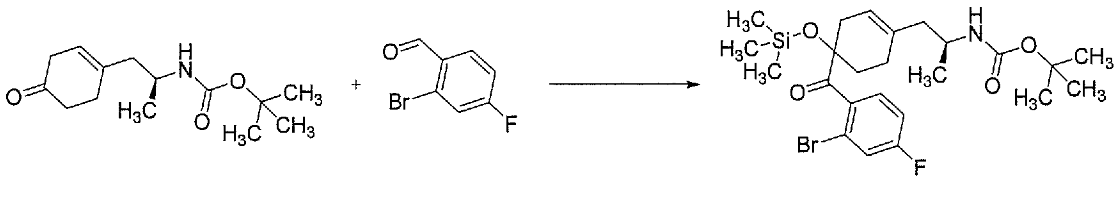

To 2-bromo-4-fluorobenzaldehyde (0.16 g) and lithium chloride (0.003 g) was added trimethylcyanide (0.12 ml) at room temperature, and the mixture was stirred for 10 min. THF (1.5 ml) was added thereto, and the mixture was cooled to -78°C. 1M NaHMDS-THF solution (0.77 ml) was added thereto, and the mixture was stirred for 5 min. tert-Butyl (S)-(1-(4-oxocyclohex-1-en-1-yl)propan-2-yl)carbamate obtained in (4) (0.15 g) was added to the reaction solution, and the mixture was stirred for 30 min. The mixture was warmed to 0°C over 1 hr, water and ethyl acetate were added thereto, and the mixture was subjected to extraction. The aqueous layer was subjected to re-extraction twice with ethyl acetate, and the combined organic layers were washed with saturated brine, and dried over sodium sulfate. The sodium sulfate was removed by filtration, and the filtrate was concentrated under reduced pressure. The obtained residue was purified by silica gel chromatography (developing solvent: ethyl acetate/n-hexane) to give the title compound (0.22 g) in 66% yield.
1H-NMR (CDCl3) δ: 7.65 (0.3H, dd, J = 8.6, 6.5 Hz), 7.56 (0.7H, dd, J = 8.1, 6.5 Hz), 7.36 (1H, d, J = 8.1 Hz), 7.04 (1H, t, J = 8.4 Hz), 5.36 (1H, s), 4.37-4.21 (1H, m), 3.88-3.74 (1H, m), 2.81-2.67 (1H, m), 2.34-1.92 (7H, m), 1.45 (3H, s), 1.43 (6H, s), 1.14 (2.1H, d, J = 6.5 Hz), 1.12 (0.9H, d, J = 6.5 Hz), 0.01 (6.3H, s), 0.00 (2.7H, s).

(6) tert-butyl ((2S)-1-(4-(2-bromo-4-fluorobenzoyl)-4-hydroxycyclohex-1-en-1-yl)propan-2-yl)carbamate

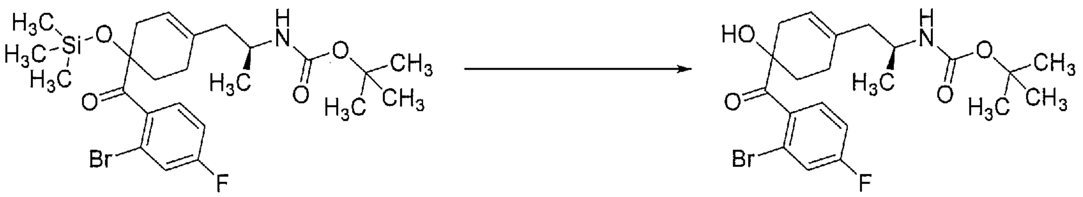

To a solution of tert-butyl ((2S)-1-(4-(2-bromo-4-fluorobenzoyl)-4-((trimethylsilyl)oxy)cyclohex-1-en-1-yl)propan-2-yl)carbamate obtained in (5) (0.22 g) in THF (0.41 ml) were added acetic acid (0.067 ml) and 1M TBAF-THF solution (0.59 ml) at room temperature, and the mixture was stirred for 15 min, and allowed to stand at room temperature all day. Saturated aqueous ammonium chloride solution, water and ethyl acetate were added thereto, and the mixture was subjected to extraction. The aqueous layer was subjected to re-extraction twice with ethyl acetate, and the combined organic layers were washed with saturated aqueous sodium bicarbonate solution and saturated brine, and dried over sodium sulfate. The sodium sulfate was removed by filtration, and the filtrate was concentrated under reduced pressure. The obtained residue was purified by silica gel chromatography (developing solvent: ethyl acetate/n-hexane) to give the title compound (0.17 g) in 66% yield.
1H-NMR (CDCl3) δ: 7.50-7.41 (0.3H, m), 7.40-7.29 (1.7H, m), 7.12-6.96 (1H, m), 5.43-5.35 (1H, m), 4.37-4.18 (1H, m), 4.01 (0.3H, s), 3.90-3.73 (1H, m), 3.10 (0.7H, s), 2.82-2.50 (1H, m), 2.30-1.88 (7H, m), 1.39-1.38 (2.7H, s), 1.37-1.35 (6.3H, s), 1.14 (0.9H, d, J = 7.5 Hz), 1.12 (2.1H, d, J = 6.5 Hz).

(7) tert-butyl ((2S)-1-(5-(2-bromo-4-fluorophenyl)-2-oxo-1-oxa-3,4-diazaspiro[5.5]undeca-4,8-dien-9-yl)propan-2-yl) carbamate

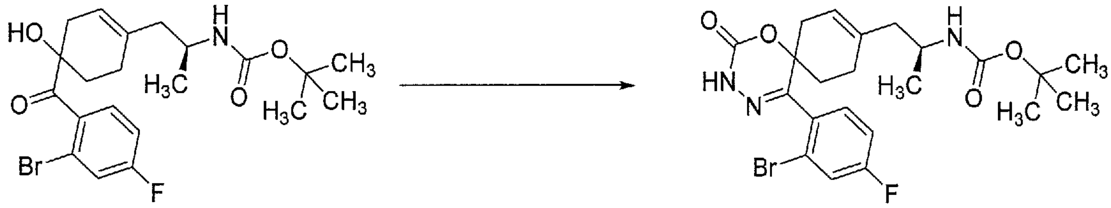

To a solution of tert-butyl ((2S)-1-(4-(2-bromo-4-fluorobenzoyl)-4-hydroxycyclohex-1-en-1-yl)propan-2-yl)carbamate obtained in (6) (0.17 g) in THF (1.7 ml) were added DBU (0.009 ml) and CDI (0.093 g) at room temperature, and the mixture was stirred for 30 min. Hydrazine monohydrate (0.037 ml) was added thereto, and the mixture was stirred at room temperature for 30 min. 10% Aqueous citric acid solution and ethyl acetate were added thereto, and the mixture was subjected to extraction. The aqueous layer was subjected to re-extraction twice with ethyl acetate, and the combined organic layers were washed with saturated brine, and dried over sodium sulfate. The sodium sulfate was removed by filtration, and the filtrate was concentrated under reduced pressure. The obtained residue was purified by silica gel chromatography (developing solvent: ethyl acetate/n-hexane). To the obtained solid were added THF (1.5 ml) and 6M hydrochloric acid (0.73 ml) at room temperature, and the mixture was stirred for 15 min, and allowed to stand all day. The mixture was stirred at 50°C for 1.5 hr, and the reaction solution was concentrated under reduced pressure, followed by azeotropy twice with THF (2 ml). To the obtained solid were added saturated aqueous sodium bicarbonate solution (0.65 mL), THF (1.3 ml), water (0.65 ml) and $Boc_2O$ (0.19 ml) at room temperature, and the mixture was stirred all day. Water and ethyl acetate were added thereto, and the mixture was subjected to extraction. The aqueous layer was subjected to re-extraction twice with ethyl acetate, and the combined organic layers were washed with saturated brine, and dried over sodium sulfate. The sodium sulfate was removed by filtration, and the filtrate was concentrated under reduced pressure. The obtained residue was purified by silica gel chromatography (developing solvent: ethyl acetate/n-hexane) to give the title compound (0.12 g) in 88% yield.
1H-NMR (CDCl3) $\delta$: 8.13 (1H, s), 7.44-7.39 (1H, m), 7.24-7.16 (1H, m), 7.13-7.02 (1H, m), 5.31-5.18 (1H, m), 4.40-4.14 (1H, m), 3.86-3.67 (1H, m), 2.71-2.60 (1H, m), 2.44-2.08 (4H, m), 2.07-1.91 (2H, m), 1.78-1.66 (1H, m), 1.43 (2.7H, s), 1.32 (6.3H, s), 1.12 (0.9H, d, J = 7.0 Hz), 1.07 (2.1H, d, J = 7.0 Hz).

(8) tert-butyl ((S)-1-(cis-5-(2-bromo-4-fluorophenyl)-9-hydroxy-2-oxo-1-oxa-3,4-diazaspiro[5.5]undec-4-en-9-yl)propan-2-yl)carbamate (cis isomer), and tert-butyl ((S)-1-(trans-5-(2-bromo-4-fluorophenyl)-9-hydroxy-2-oxo-1-oxa-3,4-diazaspiro[5.5]undec-4-en-9-yl)propan-2-yl)carbamate (trans isomer)

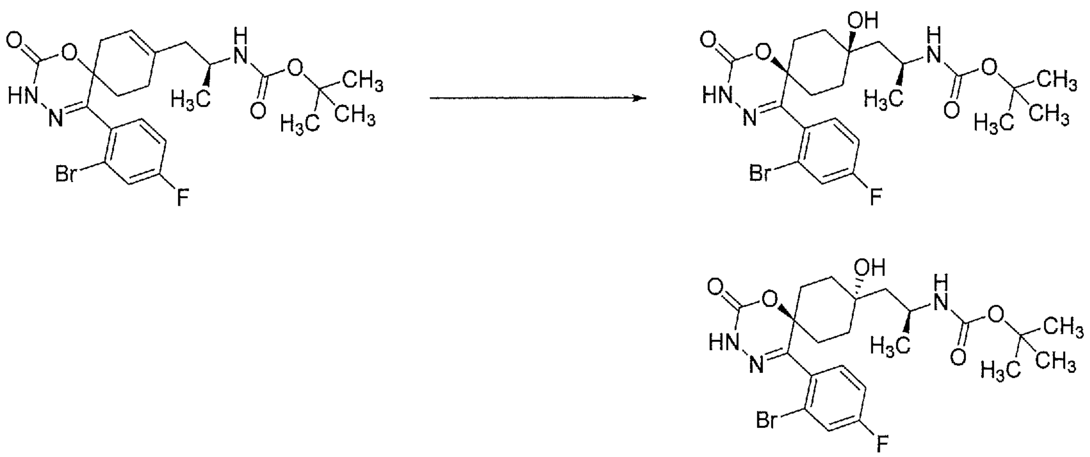

To a solution of tert-butyl ((2S)-1-(5-(2-bromo-4-fluorophenyl)-2-oxo-1-oxa-3,4-diazaspiro[5.5]undeca-4,8-dien-9-yl)propan-2-yl)carbamate obtained in (7) (0.12 g) in THF (1.8 ml) were added cobalt(II) acetyl acetone (0.063 g) and phenylsilane (0.30 ml) at room temperature, and the mixture was stirred under oxygen atmosphere all day.

Saturated aqueous sodium thiosulfate solution, water and ethyl acetate were added thereto, and the mixture was subjected to extraction. The aqueous layer was subjected to re-extraction twice with ethyl acetate, and the combined organic layers were washed with saturated brine, and dried over sodium sulfate. The sodium sulfate was removed by filtration, and the filtrate was concentrated under reduced pressure. The obtained residue was purified by silica gel chromatography (developing solvent: ethyl acetate/n-hexane) to give a cis isomer (0.047 g) of the title compound in 27% yield, and a trans isomer (0.060 g) of the title compound in 30% yield.

(cis isomer)
1H-NMR (CDCl3) δ: 8.22-8.08 (1H, m), 7.43 (1H, dd, J = 8.1, 2.2 Hz), 7.27-7.22 (1H, m), 7.11 (1H, td, J = 8.1, 2.5 Hz), 4.63 (1H, d, J = 6.5 Hz), 3.81-3.67 (1H, m), 2.32-2.22 (2H, m), 2.19 (1H, s), 2.00-1.84 (3H, m), 1.84-1.48 (5H, m), 1.42 (9H, s), 1.15 (3H, d, J = 6.5 Hz).
(trans isomer)
1H-NMR (CDCl3) δ: 8.10 (1H, s), 7.40 (1H, dd, J = 8.1, 2.2 Hz), 7.28-7.22 (1H, m), 7.10 (1H, t, J = 8.6 Hz), 4.59-4.44 (1H, m), 3.99-3.81 (1H, m), 3.81-3.66 (1H, m), 2.19 (1H, s), 2.15-2.03 (1H, m), 2.03-1.89 (2H, m), 1.85-1.73 (2H, m), 1.73-1.50 (4H, m), 1.41 (9H, s), 1.17 (3H, d, J = 7.0 Hz).

(9) cis-9-((S)-2-aminopropyl)-5-(2-bromo-4-fluorophenyl)-9-hydroxy-1-oxa-3,4-diazaspiro[5.5]undec-4-en-2-one

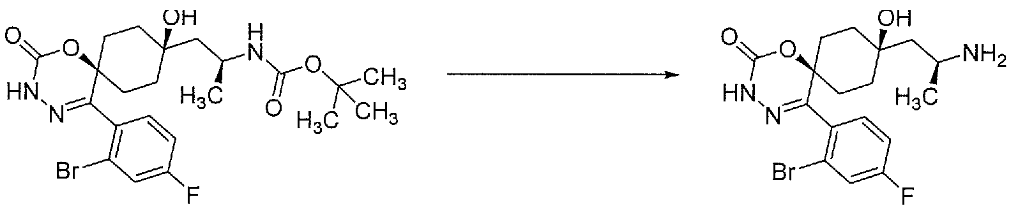

To a solution of tert-butyl ((S)-1-(cis-5-(2-bromo-4-fluorophenyl)-9-hydroxy-2-oxo-1-oxa-3,4-diazaspiro[5.5]undec-4-en-9-yl)propan-2-yl)carbamate obtained in (8) (0.039 g) in chloroform (0.39 ml) was added trifluoroacetic acid (0.39 ml) at room temperature, and the mixture was stirred for 30 min. The reaction solution was concentrated under reduced pressure, and the residue was purified by silica gel chromatography (InertSep (registered trademark) SCX, developing solvent: methanol/2M ammonia-methanol solution) to give the title compound (0.030 g) in 85% yield.
1H-NMR (DMSO-D6) δ: 11.30-11.21 (1H, m), 7.75 (1H, dd, J = 8.6, 2.7 Hz), 7.56 (1H, dd, J = 8.9, 5.7 Hz), 7.40-7.32 (1H, m), 2.97-2.85 (1H, m), 2.10-1.97 (2H, m), 1.81-1.27 (9H, m), 1.09-0.99 (2H, m), 0.96 (3H, d, J = 6.5 Hz).

(10) N-((S)-1-(cis-5-(2-bromo-4-fluorophenyl)-9-hydroxy-2-oxo-1-oxa-3,4-diazaspiro[5.5]undec-4-en-9-yl)propan-2-yl)-3-methyl-1H-pyrazole-5-carboxamide

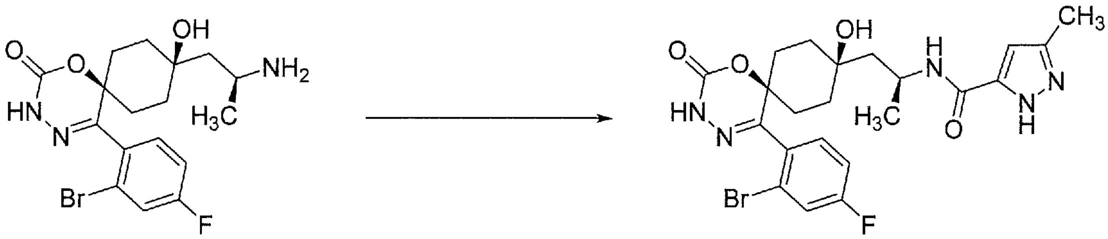

To a solution of cis-9-((S)-2-aminopropyl)-5-(2-bromo-4-fluorophenyl)-9-hydroxy-1-oxa-3,4-diazaspiro[5.5]undec-4-en-2-one obtained in (9) (0.013 g) in DMF (0.2 ml) were added 3-methyl-1H-pyrazole-5-carboxylic acid (0.005 g), HOAt (0.006 g) and N,N-diisopropylethylamine (0.017 ml) at room temperature. WSC (0.008 g) was added thereto, and the mixture was stirred at room temperature for 15 min, and allowed to stand all day. Water, saturated aqueous sodium bicarbonate solution and ethyl acetate were added thereto, and the mixture was subjected to extraction. The aqueous layer was subjected to re-extraction twice with ethyl acetate, and the combined organic layers were washed with saturated brine, and dried over sodium sulfate. The sodium sulfate was removed by filtration, and the filtrate was concentrated under reduced pressure, followed by azeotropy twice with toluene (2 ml). The obtained residue was purified by silica gel chromatography (developing solvent: ethyl acetate/n-hexane) and then reverse-phase silica gel chromatography

(developing solvent: acetonitrile/water) to give the title compound (0.004 g) in 26% yield.
1H-NMR (DMSO-D6) δ: 12.81 (1H, s), 11.24 (1H, s), 7.90-7.79 (1H, m), 7.74 (1H, d, J = 8.1 Hz), 7.59-7.48 (1H, m), 7.39-7.26 (1H, m), 6.29 (1H, s), 4.59 (1H, s), 4.07-3.93 (1H, m), 2.25 (3H, s), 2.11-1.94 (2H, m), 1.78-1.28 (8H, m), 1.12 (3H, d, J = 5.9 Hz).

[Production Example 8]: Synthesis of N-((S)-1-(trans-5-(2-bromo-4-fluorophenyl)-9-hydroxy-2-oxo-1-oxa-3,4-diazaspiro[5,5]undec-4-en-9-yl)propan-2-yl)-3-methyl-1H-pyrazole-5-carboxamide (Example 322)

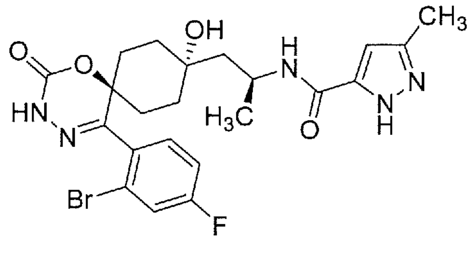

(1) trans-9-((S)-2-aminopropyl)-5-(2-bromo-4-fluorophenyl)-9-hydroxy-1-oxa-3,4-diazaspiro[5.5]undec-4-en-2-one

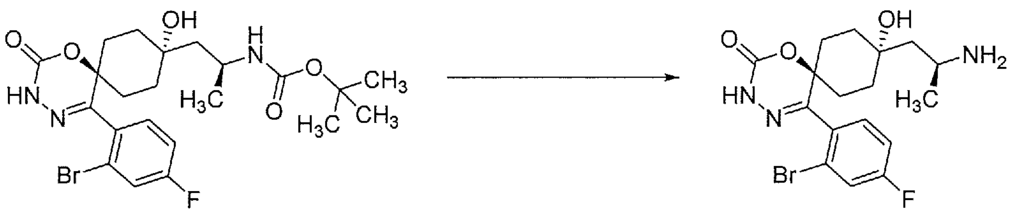

To a solution of tert-butyl ((S)-1-(trans-5-(2-bromo-4-fluorophenyl)-9-hydroxy-2-oxo-1-oxa-3,4-diazaspiro[5,5]undec-4-en-9-yl)propan-2-yl)carbamate (obtained in (8) of [Production Example 7]) (0.056 g) in chloroform (0.44 ml) was added trifluoroacetic acid (0.44 ml) at room temperature, and the mixture was stirred for 30 min. The reaction solution was concentrated under reduced pressure, and the residue was purified by silica gel chromatography (InertSep (registered trademark) SCX, developing solvent: methanol/2M ammonia-methanol solution) to give the title compound (0.036 g) in 100% yield.
LC-MS(M+1): 414

(2) N-((S)-1-(trans-5-(2-bromo-4-fluorophenyl)-9-hydroxy-2-oxo-1-oxa-3,4-diazaspiro[5.5]undec-4-en-9-yl)propan-2-yl)-3-methyl-1H-pyrazole-5-carboxamide

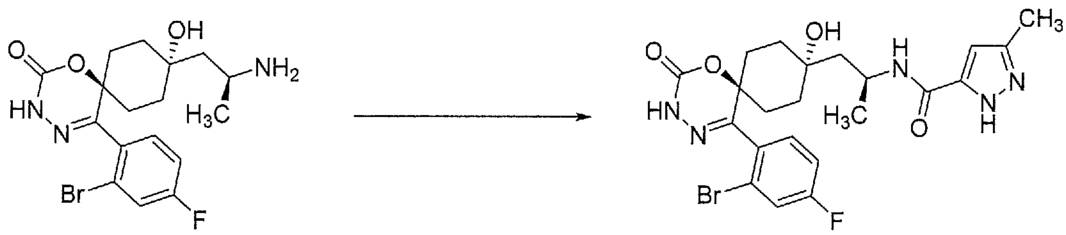

To a solution of trans-9-((S)-2-aminopropyl)-5-(2-bromo-4-fluorophenyl)-9-hydroxy-1-oxa-3,4-diazaspiro[5,5]undec-4-en-2-one obtained in (1) (0.016 g) in DMF (0.24 ml) were added 3-methyl-1H-pyrazole-5-carboxylic acid (0.006 g), HOAt (0.007 g) and N,N-diisopropylethylamine (0.020 ml) at room temperature. WSC (0.010 g) was added thereto, and the mixture was stirred at room temperature for 15 min, and allowed to stand all day. Water, saturated aqueous sodium bicarbonate solution and ethyl acetate were added thereto, and the mixture was subjected to extraction. The aqueous layer was subjected to re-extraction twice with ethyl acetate, and the combined organic layers were washed with saturated

brine, and dried over sodium sulfate. The sodium sulfate was removed by filtration, and the filtrate was concentrated under reduced pressure, followed by azeotropy twice with toluene (2 ml). The obtained residue was purified by silica gel chromatography (developing solvent: ethyl acetate/n-hexane) and then reverse-phase silica gel chromatography (developing solvent: acetonitrile/water) to give the title compound (0.007 g) in 34% yield.
1H-NMR (DMSO-D6) δ: 12.80 (1H, s), 11.18 (1H, s), 7.85 (1H, d, J = 7.5 Hz), 7.74 (1H, dd, J = 8.6, 2.7 Hz), 7.51 (1H, dd, J = 8.6, 5.9 Hz), 7.37 (1H, ddd, J = 8.6, 8.1, 2.7 Hz), 6.31 (1H, s), 4.25-4.08 (1H, m), 4.21 (1H, s), 2.24 (3H, s), 1.97-1.82 (2H, m), 1.82-1.66 (3H, m), 1.66-1.34 (5H, m), 1.12 (3H, d, J = 6.5 Hz).

[Production Example 9]: Synthesis of $N^1$-((S)-1-(trans-5-(2-bromo-3-fluorophenyl)-9-cyano-2-oxo-1-oxa-3,4-diazaspiro[5.5]undec-4-en-9-yl)propan-2-yl)-$N^2$-methyloxamide

(Example 2-153)

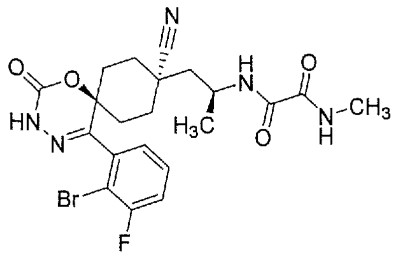

(1) tert-butyl (S)-(1-(8-cyano-1,4-dioxaspiro[4.5]decan-8-yl)propan-2-yl)carbamate

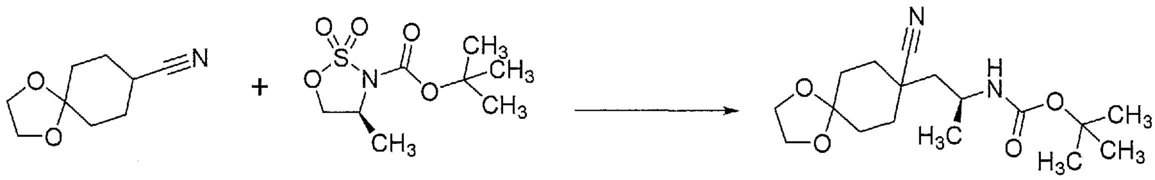

A solution of 1,4-dioxaspiro[4.5]decane-8-carbonitrile (7.5 g) in THF (75 ml) was cooled to -78°C, 1.11M LDA-THF/n-hexane solution (48.5 ml) was added thereto, and the mixture was stirred for 1 hr. tert-Butyl (S)-4-methyl-1,2,3-oxathiazolidine-3-carboxylate 2,2-dioxide (12.8 g) was added thereto, and the mixture was warmed to 0°C, and stirred for 1 hr. 1M Hydrochloric acid (135 ml) was added thereto, and the mixture was stirred at room temperature for 30 min. Ethyl acetate and water were added thereto, and the mixture was subjected to extraction. The organic layer was washed with saturated brine, and dried over sodium sulfate. The sodium sulfate was removed by filtration, and the filtrate was concentrated under reduced pressure. The obtained residue was purified by silica gel chromatography (developing solvent: ethyl acetate/n-hexane=25/75 to 55/45), and the fractions were concentrated under reduced pressure to give the title compound (14.3 g) in 98% yield.
1H-NMR (DMSO-D6) δ: 6.78 (1H, d, J = 8.6 Hz), 3.87 (4H, s), 3.78-3.63 (1H, m), 2.06-1.44 (10H, m), 1.37 (9H, s), 1.06 (3H, d, J = 6.5 Hz).

(2) tert-butyl (S)-(1-(1-cyano-4-oxocyclohexyl)propan-2-yl)carbamate

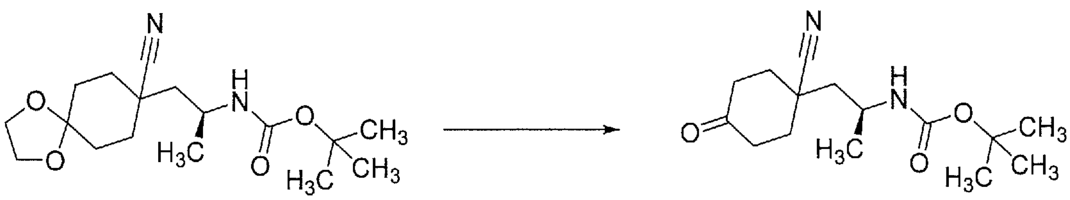

To a solution of tert-butyl (S)-(1-(8-cyano-1,4-dioxaspiro[4.5]decan-8-yl)propan-2-yl)carbamate obtained in (1) (10.9 g) in THF (54.5 ml) was added 6M hydrochloric acid (28 ml) at room temperature, and the mixture was stirred at 60°C for 6 hr. After standing at room temperature for 3 days, 10M aqueous sodium hydroxide solution (16.8 ml) and $Boc_2O$ (23.4 ml) were added thereto, and the mixture was stirred for 2 hr. Ethyl acetate and water were added thereto, and the mixture was subjected to extraction. The organic layer was washed with saturated brine, and dried over sodium sulfate. The sodium sulfate was removed by filtration, and the filtrate was concentrated under reduced pressure. The obtained residue was purified by silica gel chromatography (developing solvent: ethyl acetate/n-hexane=30/70 to 65/35), and the fractions were concentrated under reduced pressure to give the title compound (5.59 g) in 59% yield.
1H-NMR (DMSO-D6) δ: 6.84 (1H, d, J = 9.2 Hz), 3.83-3.67 (1H, m), 2.55-2.39 (2H, m), 2.36-2.10 (4H, m), 1.92-1.75 (3H, m), 1.65 (1H, dd, J = 14.3, 3.5 Hz), 1.38 (9H, s), 1.09 (3H, d, J = 6.5 Hz).

(3) tert-butyl ((S)-1-(trans-4-(2-bromo-3-fluorobenzoyl)-1-cyano-4-((trimethylsilyl)oxy)cyclohexyl)propan-2-yl)carbamate

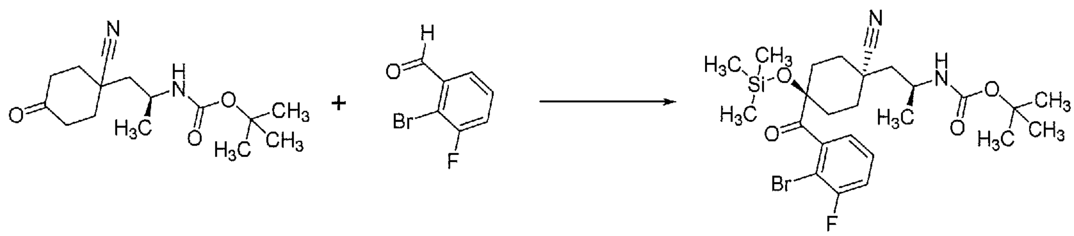

Under argon atmosphere, to a solution of 2-bromo-3-fluorobenzaldehyde (0.38 g) and lithium chloride (0.008 g) in THF (0.8 ml) was added trimethylsilylcyanide (0.27 ml) at room temperature, and the mixture was stirred for 30 min. THF (5.2 ml) was added thereto, and the mixture was cooled to -78°C, and stirred for 5 min. 1.13M LiHMDS-n-hexane solution (1.6 ml) was added thereto, and the mixture was stirred for 10 min. tert-Butyl (S)-(1-(1-cyano-4-oxocyclohexyl)propan-2-yl)carbamate obtained in (2) (0.40 g) was added thereto, and the mixture was stirred for 30 min. Then, and the mixture was warmed to 0°C over 1 hr, water and ethyl acetate were added thereto, and the mixture was subjected to extraction. The organic layer was washed with saturated brine, and dried over sodium sulfate. The sodium sulfate was removed by filtration, and the filtrate was concentrated under reduced pressure. The obtained residue was purified by silica gel chromatography (developing solvent: ethyl acetate/n-hexane=3/97 to 30/70), and the fractions were concentrated under reduced pressure to give the title compound (0.62 g) in 78% yield.
1H-NMR (DMSO-D6) δ: 7.59-7.41 (2H, m), 7.39-7.33 (1H, m), 6.84 (1H, d, J = 8.6 Hz), 3.82-3.67 (1H, m), 2.07-1.49 (10H, m), 1.37 (9H, s), 1.08 (3H, d, J = 6.5 Hz), 0.04 (9H, s).

(4) tert-butyl ((S)-1-(trans-4-(2-bromo-3-fluorobenzoyl)-1-cyano-4-hydroxycyclohexyl)propan-2-yl)carbamate

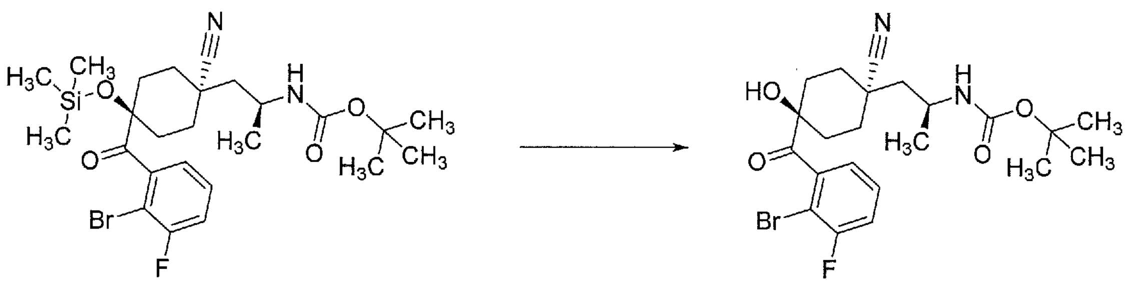

To a solution of tert-butyl ((S)-1-(trans-4-(2-bromo-3-fluorobenzoyl)-1-cyano-4-((trimethylsilyl)oxy)cyclohexyl) propan-2-yl)carbamate obtained in (3) (0.62 g) in THF (3.1 ml) were added acetic acid (0.19 ml) and 1M TBAF-THF solution (1.7 ml) at room temperature, and the mixture was stirred for 2 hr. Saturated aqueous sodium bicarbonate solution and ethyl acetate were added thereto, the mixture was subjected to extraction, and the organic layer was dried over sodium sulfate. The sodium sulfate was removed by filtration, and the filtrate was concentrated under reduced pressure. The obtained residue was purified by silica gel chromatography (developing solvent: ethyl acetate/n-hexane=30/70 to 60/40), and the fractions were concentrated under reduced pressure to give the title compound (0.25 g) in 46% yield.

1H-NMR (DMSO-D6) δ: 7.55-7.39 (2H, m), 7.32-7.27 (1H, m), 6.81 (1H, d, J = 8.6 Hz), 5.53 (1H, s), 3.83-3.69 (1H, m), 2.02-1.49 (10H, m), 1.37 (9H, s), 1.07 (3H, d, J = 6.5 Hz).

(5) trans-9-((S)-2-aminopropyl)-5-(2-bromo-3-fluorophenyl)-2-oxo-1-oxa-3,4-diazaspiro[5.5]undec-4-ene-9-carbonitrile hydrochloride

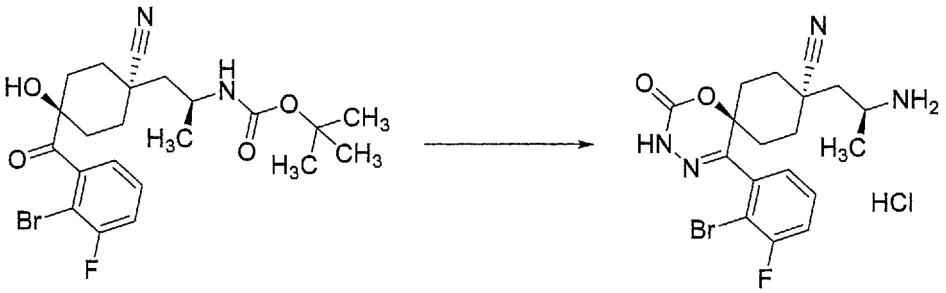

To a solution of tert-butyl ((S)-1-(trans-4-(2-bromo-3-fluorobenzoyl)-1-cyano-4-hydroxycyclohexyl)propan-2-yl) carbamate obtained in (4) (0.25 g) in THF (2.5 ml) were added CDI (0.13 g) and DBU (0.012 ml) at room temperature, and the mixture was stirred for 30 min. Hydrazine monohydrate (0.069 ml) was added, and the mixture was stirred for 30 min. 10% Aqueous citric acid solution and ethyl acetate were added thereto, and the mixture was subjected to extraction. The organic layer was dried over sodium sulfate, the sodium sulfate was removed by filtration, and the filtrate was concentrated under reduced pressure. The obtained residue was purified by silica gel chromatography (developing solvent: ethyl acetate/n-hexane=50/50 to 90/10), and the fractions were concentrated under reduced pressure. To the obtained solid were added THF (2.3 ml) and 6M hydrochloric acid (1.1 ml), and the mixture was stirred at 50°C for 2 hr. The reaction solution was concentrated under reduced pressure, followed by azeotropy with THF to give the title compound (0.17 g) in 72% yield.
1H-NMR (DMSO-D6) δ: 11.45 (1H, s), 7.93 (3H, br s), 7.62-7.50 (2H, m), 7.42-7.36 (1H, m), 3.44-3.34 (1H, m), 2.31-2.19 (2H, m), 2.03-1.81 (4H, m), 1.78-1.58 (4H, m), 1.31 (3H, d, J = 6.5 Hz).

(6) $N^1$-((S)-1-(trans-5-(2-bromo-3-fluorophenyl)-9-cyano-2-oxo-1-oxa-3,4-diazaspiro[5.5]undec-4-en-9-yl)propan-2-yl)-$N^2$-methyloxamide

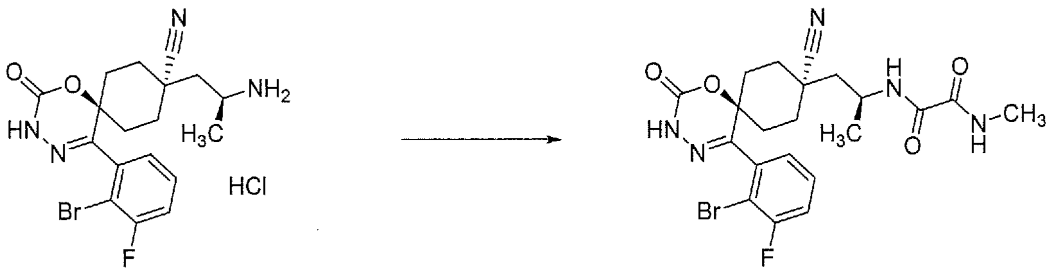

To a solution of trans-9-((S)-2-aminopropyl)-5-(2-bromo-3-fluorophenyl)-2-oxo-1-oxa-3,4-diazaspiro[5.5]undec-4-ene-9-carbonitrile hydrochloride obtained in (5) (0.025 g) in DMF (0.4 ml) were added 2-methylamino-2-oxoacetic acid (0.007 g), N,N-diisopropylethylamine (0.048 ml) and HATU (0.025 g) at room temperature, and the mixture was stirred for 1 hr. The reaction solution was purified by reverse-phase silica gel chromatography (developing solvent: acetonitrile/-water=5/95 to 70/30) and then thin layer chromatography (developing solvent: ethyl acetate), and the fractions were concentrated under reduced pressure to give the title compound (0.018 g) in 65% yield.
1H-NMR (DMSO-D6) δ: 11.39 (1H, s), 8.72 (1H, d, J = 9.2 Hz), 8.63-8.57 (1H, m), 7.58-7.47 (2H, m), 7.38-7.34 (1H, m), 4.17-4.04 (1H, m), 2.64 (3H, d, J = 4.8 Hz), 2.27-1.95 (4H, m), 1.81-1.53 (6H, m), 1.12 (3H, d, J = 6.5 Hz).

[Production Example 10]: Synthesis of $N^1$-((S)-1-(trans-5-(2-bromo-5-cyanophenyl)-9-methoxy-2-oxo-1-oxa-3,4-diazaspiro[5.5]undec-4-en-9-yl)propan-2-yl)-$N^2$-methyloxamide

(Example 2-123)

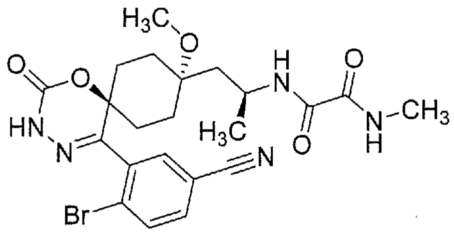

(1) 1-(8-methoxy-1,4-dioxaspiro[4.5]decan-8-yl)propan-2-one

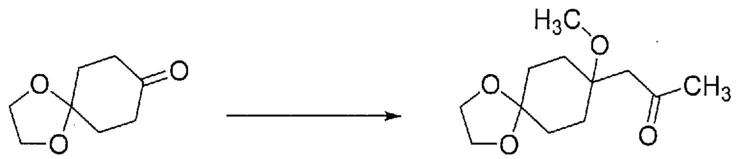

To a solution of 1,4-dioxaspiro[4.5]decan-8-one (5.0 g) in methanol (50 ml) were added dimethyl (2-oxopropyl) phosphonate (5.9 g) and 28% sodium methoxide-methanol solution (23.5 ml) at room temperature, and the mixture was stirred all day. Water (20 ml) was added thereto, and the mixture was concentrated under reduced pressure to evaporate the methanol. The mixture was diluted with ethyl acetate, and washed with saturated brine. The obtained organic layer was dried over sodium sulfate, the sodium sulfate was removed by filtration, and the filtrate was concentrated under reduced pressure. The obtained residue was purified by silica gel chromatography (developing solvent: ethyl acetate/n-hexane=20/80 to 50/50), and the fractions were concentrated under reduced pressure to give the title compound (2.1 g) in 28% yield.
1H-NMR (DMSO-D6) δ: 3.83 (4H, s), 3.15 (3H, s), 2.57 (2H, s), 2.11 (3H, s), 1.78-1.68 (2H, m), 1.66-1.41 (6H, m).

(2) (S)-1-(8-methoxy-1,4-dioxaspiro[4.5]decan-8-yl)-N-((S)-1-phenylethyl)propan-2-amine

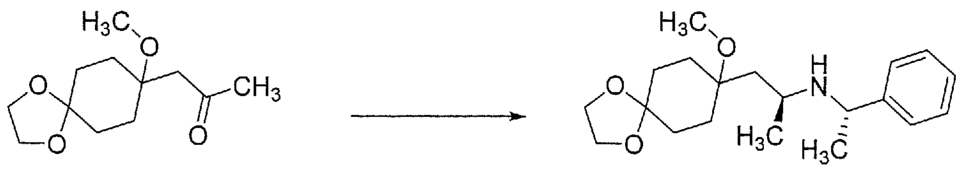

To a solution of 1-(8-methoxy-1,4-dioxaspiro[4.5]decan-8-yl)propan-2-one obtained in (1) (2.1 g) in THF (31 ml) were added (S)-1-phenethylamine (1.3 ml), acetic acid (0.52 ml) and sodium triacetoxyborohydride (3.6 g) at room temperature, and the mixture was stirred for 3 hr. Under ice-cooled, 4M aqueous sodium hydroxide solution (11.3 ml) and water were added thereto, and the mixture was diluted with ethyl acetate. The obtained organic layer was washed with saturated brine. The organic layer was dried over sodium sulfate, the sodium sulfate was removed by filtration, and the filtrate was concentrated under reduced pressure. The obtained residue was purified by silica gel chromatography (developing solvent: methanol/ethyl acetate/n-hexane=0/25/75 to 0/100/0 to 5/95/0), and the fractions were concentrated under reduced pressure to give the title compound (1.4 g) in 46% yield.
1H-NMR (DMSO-D6) δ: 7.36-7.23 (4H, m), 7.20-7.15 (1H, m), 3.82 (4H, s), 3.78 (1H, q, J = 6.7 Hz), 3.04 (3H, s), 2.66-2.56 (1H, m), 1.89-1.29 (11H, m), 1.24-1.10 (3H, m), 0.90 (3H, d, J = 6.6 Hz).

(3) tert-butyl (S)-(1-(8-methoxy-1.4-dioxaspiro[4.5]decan-8-yl)propan-2-yl)carbamate

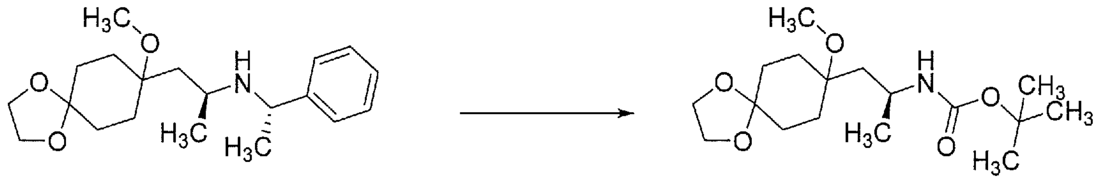

To a solution of (S)-1-(8-methoxy-1,4-dioxaspiro[4.5]decan-8-yl)-N-((S)-1-phenylethyl)propan-2-amine obtained in (2) (1.4 g) in THF (14 ml) were added acetic acid (0.48 ml) and 20% palladium hydroxide-activated carbon at room temperature, and the mixture was stirred under hydrogen atmosphere (1 atm) all day. The reaction solution was filtered through Celite, followed by washing with THF (14 ml). To the obtained THF solution were added water (4.8 ml), sodium bicarbonate (1.1 g) and $Boc_2O$ (1.1 ml) at room temperature, and the mixture was stirred for 2 hr. The mixture was diluted with ethyl acetate, and washed with saturated brine. The obtained organic layer was dried over sodium sulfate, the sodium sulfate was removed by filtration, and the filtrate was concentrated under reduced pressure. The obtained residue was purified by silica gel chromatography (developing solvent: ethyl acetate/n-hexane=15/85 to 50/50), and the fractions were concentrated under reduced pressure to give the title compound (1.0 g) in 76% yield.
1H-NMR (DMSO-D6) δ: 6.62 (1H, d, J = 8.1 Hz), 3.83 (4H, s), 3.65-3.48 (1H, m), 3.02 (3H, s), 1.79-1.27 (10H, m), 1.36 (9H, s), 1.03 (3H, d, J = 6.5 Hz).

(4) tert-butyl (S)-(1-(1-methoxy-4-oxocyclohexyl)propan-2-yl)carbamate

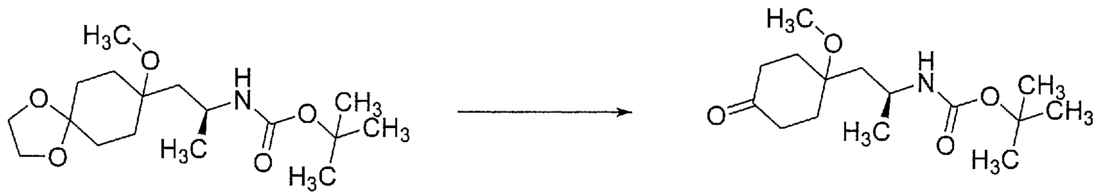

To a solution of tert-butyl (S)-(1-(8-methoxy-1.4-dioxaspiro[4.5]decan-8-yl)propan-2-yl)carbamate obtained in (3) (1.0 g) in a mixed solvent of acetone (10 ml) and water (4.2 ml) was added pyridinium p-toluenesulfonate (0.16 g) at room temperature, and the mixture was stirred at 60°C for 6 hr. Saturated aqueous sodium bicarbonate solution (5 ml) was added thereto at room temperature, and the acetone was evaporated. Ethyl acetate was added thereto, and the mixture was subjected to extraction. The obtained organic layer was washed with saturated brine, and dried over sodium sulfate, the sodium sulfate was removed by filtration, and the filtrate was concentrated under reduced pressure. The obtained residue was purified by silica gel chromatography (developing solvent: ethyl acetate/n-hexane=20/80 to 55/45), and the fractions were concentrated under reduced pressure to give the title compound (0.77 g) in 85% yield.
1H-NMR (DMSO-D6) δ: 6.68 (1H, d, J = 8.6 Hz), 3.70-3.54 (1H, m), 3.14 (3H, s), 2.48-2.31 (2H, m), 2.11-1.92 (4H, m), 1.78-1.54 (4H, m), 1.36 (9H, s), 1.06 (3H, d, J = 6.5 Hz).

(5) tert-butyl ((S)-1-(trans-4-(2-bromo-5-cyanobenzoyl)-1-methoxy-4-((trimethylsilyl)oxy)cyclohexyl)propan-2-yl)carbamate

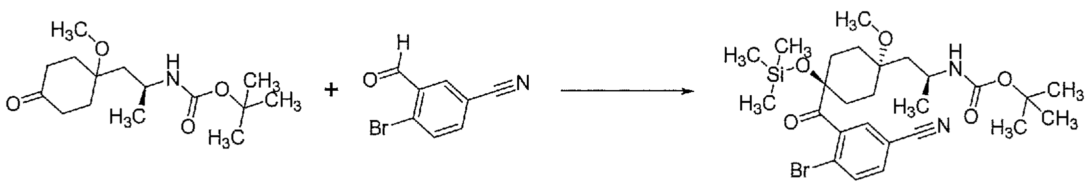

Under argon atmosphere, to a solution of 4-bromo-3-formylbenzonitrile (0.24 g) and lithium chloride (0.005 g) in THF (0.5 ml) was added trimethylsilylcyanide (0.16 ml), and the mixture was stirred at room temperature for 30 min. THF (3.3 ml) was added thereto, and the mixture was cooled to -78°C, and stirred for 5 min. 1.13M LiHMDS-n-hexane solution (1.0 ml) was added thereto, and the mixture was stirred for 10 min. tert-Butyl (S)-(1-(1-methoxy-4-oxocyclohexyl) propan-2-yl)carbamate obtained in (4) (0.25 g) was added thereto, and the mixture was stirred for 30 min. Then the

mixture was warmed to 0°C over 1 hr, water and ethyl acetate were added thereto, and the mixture was subjected to extraction. The organic layer was washed with saturated brine, and dried over sodium sulfate. The sodium sulfate was removed by filtration, and the filtrate was concentrated under reduced pressure. The obtained residue was purified by silica gel chromatography (developing solvent: ethyl acetate/n-hexane=5/95 to 33/67), and the fractions were concentrated under reduced pressure to give the title compound (0.23 g) in 45% yield.

1H-NMR (DMSO-D6) δ: 8.05-7.82 (3H, m), 6.69 (1H, d, J = 8.1 Hz), 3.67-3.51 (1H, m), 3.00 (3H, s), 2.01-1.31 (10H, m), 1.36 (9H, s), 1.04 (3H, d, J = 6.5 Hz), 0.00-0.03 (9H, m).

(6) tert-butyl ((S)-1-(trans-4-(2-bromo-5-cyanobenzoyl)-4-hydroxy-1-methoxycyclohexyl)propan-2-yl)carbamate

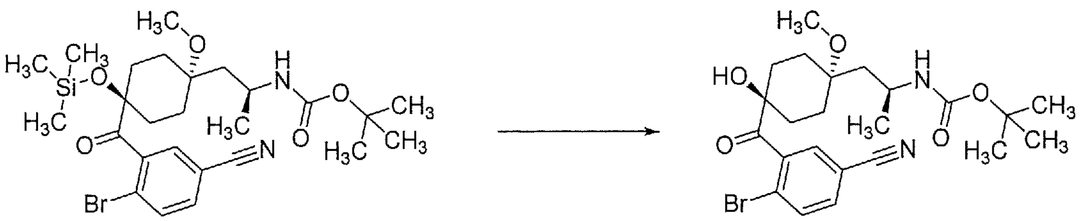

To a solution of tert-butyl ((S)-1-(trans-4-(2-bromo-5-cyanobenzoyl)-1-methoxy-4-((trimethylsilyl)oxy)cyclohexyl)propan-2-yl)carbamate obtained in (5) (0.22 g) in THF (2.2 ml) were added acetic acid (0.067 ml) and 1M TBAF-THF solution (0.58 ml) at room temperature, and the mixture was stirred for 2 hr. Saturated aqueous sodium bicarbonate solution and ethyl acetate were added thereto, and the mixture was subjected to extraction. The organic layer was washed with saturated brine, and dried over sodium sulfate. The sodium sulfate was removed by filtration, and the filtrate was concentrated under reduced pressure. The obtained residue was purified by silica gel chromatography (developing solvent: ethyl acetate/n-hexane=30/70 to 60/40), and the fractions were concentrated under reduced pressure to give the title compound (0.15 g) in 78% yield.

1H-NMR (DMSO-D6) δ: 7.93-7.88 (2H, m), 7.84-7.79 (1H, m), 6.64 (1H, d, J = 8.1 Hz), 5.31 (1H, s), 3.69-3.56 (1H, m), 3.04 (3H, s), 1.97-1.31 (10H, m), 1.36 (9H, s), 1.04 (3H, d, J = 6.5 Hz).

(7) 3-(trans-9-((S)-2-aminopropyl)-9-methoxy-2-oxo-1-oxa-3,4-diazaspiro[5.5]undec-4-en-5-yl)-4-bromobenzonitrile hydrochloride

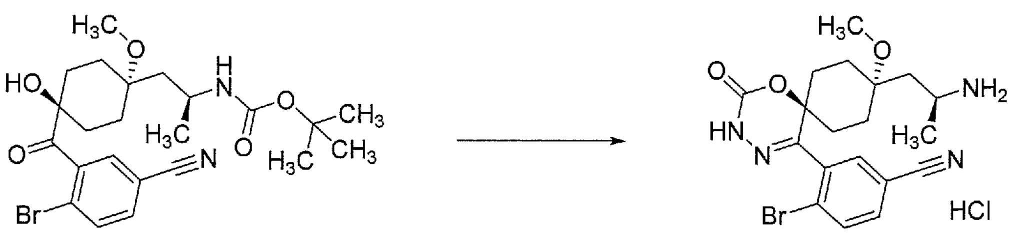

To a solution of tert-butyl ((S)-1-(trans-4-(2-bromo-5-cyanobenzoyl)-4-hydroxy-1-methoxycyclohexyl)propan-2-yl)carbamate obtained in (6) (0.15 g) in THF (1.5 ml) were added CDI (0.073 g) and DBU (0.007 ml), and the mixture was stirred at room temperature for 30 min. Hydrazine monohydrate (0.045 ml) was added thereto, and the mixture was stirred for 30 min. 10% Aqueous citric acid solution and ethyl acetate were added thereto, the mixture was subjected to extraction, and organic layer was dried over sodium sulfate. The sodium sulfate was removed by filtration, and the filtrate was concentrated under reduced pressure. The obtained residue was purified by silica gel chromatography (developing solvent: ethyl acetate/n-hexane=50/50 to 90/10), and the fractions were concentrated under reduced pressure. To the obtained solid were added THF (1.6 ml) and 6M hydrochloric acid (0.8 ml), and the mixture was stirred at 50°C for 2 hr. The mixture was allowed to cool to room temperature, and the reaction solution was concentrated, followed by azeotropy with THF to give the title compound (0.14 g) in 96% yield.

1H-NMR (DMSO-D6) δ: 11.38 (1H, s), 8.12-8.08 (1H, m), 8.05-8.00 (1H, m), 7.93-7.86 (1H, m), 7.71 (3H, br s), 3.68-3.49 (1H, m), 2.98 (3H, s), 2.07-1.93 (2H, m), 1.84-1.45 (8H, m), 1.22 (3H, d, J = 6.5 Hz).

(8) $N^1$-((S)-1-(trans-5-(2-bromo-5-cyanophenyl)-9-methoxy-2-oxo-1-oxa-3,4-diazaspiro[5.5]undec-4-en-9-yl)propan-2-yl)-$N^2$-methyloxamide

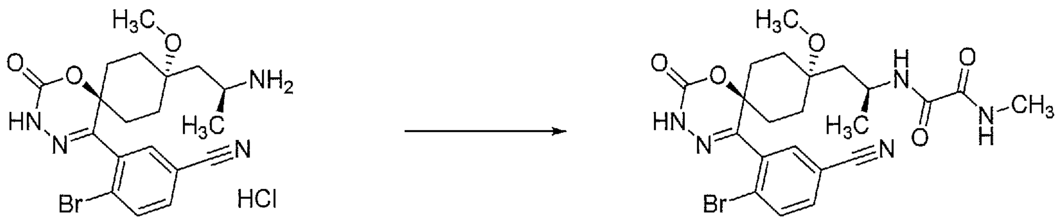

To a solution of 3-(trans-9-((S)-2-aminopropyl)-9-methoxy-2-oxo-1-oxa-3,4-diazaspiro[5.5]undec-4-en-5-yl)-4-bromobenzonitrile hydrochloride obtained in (7) (0.050 g) in DMF (0.75 ml) were added 2-methylamino-2-oxoacetic acid (0.016 g), N,N-diisopropylethylamine (0.074 ml) and HATU (0.060 g) at room temperature, and the mixture was stirred for 1 hr. The reaction solution was purified by reverse-phase silica gel chromatography (developing solvent: acetonitrile/-water=5/95 to 65/35) and then thin layer chromatography (developing solvent: ethyl acetate/n-hexane=67/33), and the fractions were concentrated under reduced pressure to give the title compound (0.049 g) in 89% yield.
1H-NMR (DMSO-D6) δ: 11.32 (1H, s), 8.65-8.54 (2H, m), 8.09-8.05 (1H, m), 8.03-7.98 (1H, m), 7.91-7.86 (1H, m), 4.01-3.92 (1H, m), 2.92 (3H, s), 2.67-2.63 (3H, m), 1.99-1.42 (10H, m), 1.08 (3H, d, J = 6.5 Hz).

The compounds of the other Examples were obtained by employing the same methods as in the above Production Methods or Production Examples, and, where necessary, known methods. The structural formulae and physical property data of the compounds of Examples 1 to 339 and 2-001 to 2-172 are shown in Table 1-1 to Table 1-61.

Table 1-1

| Ex. No. | Structure | Notes | 1H-NMR (400 MHz) | MS (M+1) | MS (M-1) |
|---|---|---|---|---|---|
| 1 | 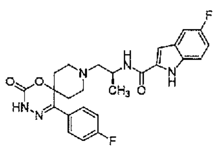 | | 1H-NMR (DMSO-D6) δ: 11.65 (1H, s), 11.16 (1H, s), 8.17 (1H, d, J = 8.3 Hz), 7.44-7.37 (4H, m), 7.16 (2H, t, J = 8.3 Hz), 7.11 (1H, s), 7.04 (1H, td, J = 8.3, 2.6 Hz), 4.24-4.16 (1H, m), 2.82-2.71 (2H, m), 2.39-2.30 (4H, m), 1.97 (2H, d, J = 13.6 Hz), 1.81 (2H, td, J = 13.6, 5.2 Hz), 1.15 (3H, d, J = 6.5 Hz). | 482 | 480 |
| 2 | 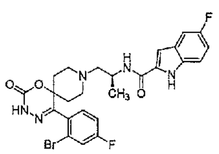 | | 1H-NMR (DMSO-D6) δ: 11.63 (1H, s), 11.30 (1H, s), 8.20 (1H, d, J = 6.5 Hz), 7.73 (1H, d, J = 6.5 Hz), 7.51 (1H, t, J = 7.6 Hz), 7.42-7.36 (2H, m), 7.30 (1H, t, J = 8.9 Hz), 7.08 (1H, s), 7.03 (1H, t, J = 8.9 Hz), 4.29-4.18 (1H, m), 2.48-2.35 (6H, m), 2.17-2.09 (2H, m), 1.69-1.57 (2H, m), 1.13 (3H, d, J = 6.7 Hz). | 560 | 558 |
| 3 | 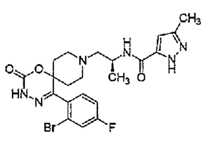 | | 1H-NMR (DMSO-D6) δ: 12.80 (1H, s), 11.26 (1H, s), 7.74 (1H, d, J = 8.0 Hz), 7.58 (1H, d, J = 7.4 Hz), 7.53 (1H, t, J = 7.4 Hz), 7.35 (1H, t, J = 8.0 Hz), 6.29 (1H, s), 4.12-4.02 (1H, m), 2.68 (2H, d, J = 12.3 Hz), 2.44-2.39 (1H, m), 2.33-2.25 (3H, m), 2.24 (3H, s), 2.07 (2H, d, J = 12.3 Hz), 1.55 (2H, s), 1.07 (3H, d, J = 6.2 Hz). | 507 | 505 |
| 4 | 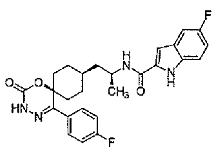 | | 1H-NMR (DMSO-D6) δ: 11.62 (1H, s), 11.11 (1H, s), 8.20 (1H, d, J = 8.3 Hz), 7.50 (2H, dd, J = 8.8, 5.5 Hz), 7.42-7.36 (2H, m), 7.22 (2H, t, J = 8.8 Hz), 7.12 (1H, d, J = 1.6 Hz), 7.02 (1H, td, J = 8.8, 2.5 Hz), 4.21-4.12 (1H, m), 2.04-1.95 (2H, m), 1.78-1.70 (2H, tm), 1.68-1.61 (1H, m), 1.61-1.51 (2H, m), 1.48-1.23 (4H, m), 1.15 (3H, d, J = 6.5 Hz). | 481 | 479 |

(continued)

| Ex. No. | Structure | Notes | 1H-NMR (400 MHz) | MS (M+1) | MS (M-1) |
|---|---|---|---|---|---|
| 5 | 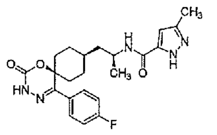 | | 1H-NMR (DMSO-D6) δ: 12.82 (1H, s), 11.11 (1H, s), 7.62 (1H, s), 7.51 (2H, dd, J = 8.6, 5.3 Hz), 7.23 (2H, dd, J = 8.6, 5.3 Hz), 6.33 (1H, s), 4.12-4.02 (1H, m), 2.24 (3H, s), 2.02-1.95 (2H, m), 1.74-1.66 (2H, m), 1.66-1.60 (1H, m), 1.59-1.50 (2H, m), 1.42-1.22 (4H, m), 1.10 (3H, d, J = 6.2 Hz). | 428 | 426 |
| 6 | 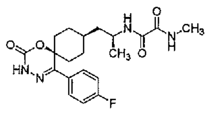 | | 1H-NMR (DMSO-D6) δ: 11.11 (1H, s), 8.66 (1H, d, J = 5.3 Hz), 8.45 (1H, d, J = 9.2 Hz), 7.52 (2H, dd, J = 8.9, 5.3 Hz), 7.25 (2H, t, J = 8.9 Hz), 3.99-3.89 (1H, m), 2.66 (3H, d, J = 5.3 Hz), 2.02-1.95 (2H, m), 1.74-1.46 (5H, m), 1.35-1.21 (4H, m), 1.08 (3H, d, J = 6.5 Hz). | 405 | 403 |
| 7 | 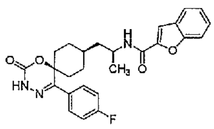 | | 1H-NMR (DMSO-D6) δ: 11.12 (1H, s), 8.45 (1H, d, J = 8.3 Hz), 7.76 (1H, d, J = 8.3 Hz), 7.65 (1H, d, J = 8.3 Hz), 7.52 (1H, s), 7.51 (2H, t, J = 7.5 Hz), 7.46 (1H, t, J = 7.5 Hz), 7.33 (1H, t, J = 8.3 Hz), 7.22 (2H, t, J = 8.3 Hz), 4.20-4.10 (1H, m), 2.04-1.94 (2H, m), 1.74-1.53 (5H, m), 1.46-1.26 (4H, m), 1.16 (3H, d, J = 6.5 Hz). | 464 | 462 |
| 8 | 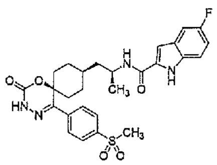 | | 1H-NMR (DMSO-D6) δ: 11.62 (1H, s), 11.29 (1H, s), 8.20 (1H, d, J = 9.0 Hz), 7.93 (2H, d, J = 8.3 Hz), 7.74 (2H, d, J = 8.3 Hz), 7.42-7.36 (2H, m), 7.13 (1H, d, J = 2.1 Hz), 7.02 (1H, td, J = 9.0, 2.3 Hz), 4.21-4.13 (1H, m), 3.24 (3H, s), 2.08-1.98 (2H, m), 1.78-1.30 (9H, m), 1.16 (3H, d, J = 6.5 Hz). | 541 | 539 |

Table 1-2

| | | | | | |
|---|---|---|---|---|---|
| 9 | 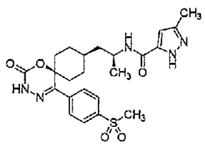 | | 1H-NMR (DMSO-D6) δ: 12.83 (1H, s), 11.28 (1H, s), 7.94 (2H, d, J = 8.3 Hz), 7.74 (2H, d, J = 8.3 Hz), 7.63 (1H, d, J = 8.8 Hz), 6.32 (1H, s), 4.12-4.05 (1H, m), 3.25 (3H, s), 2.24 (3H, s), 2.06-1.98 (2H, m), 1.75-1.68 (2H, m), 1.65-1.54 (3H, m), 1.43-1.23 (4H, m), 1.10 (3H, d, J = 6.7 Hz). | 488 | 486 |
| 10 | 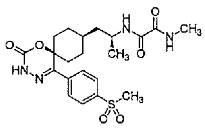 | | 1H-NMR (DMSO-D6) δ: 11.29 (1H, s), 8.66 (1H, d, J = 5.1 Hz), 8.46 (1H, q, J = 5.1 Hz), 7.96 (2H, d, J = 8.3 Hz), 7.76 (2H, d, J = 8.3 Hz), 3.98-3.90 (1H, m), 3.27 (3H, s), 2.66 (3H, d, J = 5.1 Hz), 2.06-1.98 (2H, m), 1.77-1.50 (5H, m), 1.38-1.23 (4H, m), 1.08 (3H, d, J = 6.5 Hz). | 465 | 463 |
| 11 | 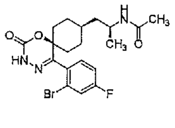 | | 1H-NMR (DMSO-D6) δ: 11.20 (1H, s), 7.74 (1H, dd, J = 8.4, 2.4 Hz), 7.58-7.50 (2H, m), 7.35 (1H, td, J = 8.4, 2.4 Hz), 3.86-3.76 (1H, m), 2.16-2.08 (2H, m), 1.73 (3H, s), 1.65-1.58 (1H, m), 1.51-1.14 (8H, m), 0.97 (3H, d, J = 6.5 Hz). | 440 | 438 |

(continued)

| | | | | | |
|---|---|---|---|---|---|
| 12 | 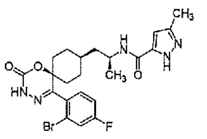 | | 1H-NMR (DMSO-D6) δ: 12.96 (0.18H, s), 12.81 (0.82H, s), 11.20 (1H, s), 7.97 (0.18H, d, J = 8.1 Hz), 7.71 (1H, dd, J = 8.6, 2.5 Hz), 7.60 (0.82H, d, J = 8.1 Hz), 7.51 (1H, dd, J = 8.6, 6.0 Hz), 7.31 (1H, td, J = 8.1, 2.5 Hz), 6.59 (0.18H, s), 6.31 (0.82H, s), 4.10-4.00 (1H, m), 2.23 (3H, s), 2.17-2.07 (2H, m), 1.73-1.65 (1H, m), 1.55-1.45 (2H, m), 1.40-1.20 (6H, m), 1.07 (3H, d, J = 6.5 Hz). | 506 | 504 |
| 13 | 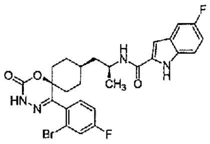 | | 1H-NMR (DMSO-D6) δ: 11.60 (1H, s), 11.20 (1H, s), 8.17 (1H, d, J = 8.7 Hz), 7.70 (1H, dd, J = 8.7, 2.7 Hz), 7.49 (1H, dd, J = 8.7, 6.1 Hz), 7.38 (1H, s), 7.40-7.32 (1H,m), 7.30 (1H, td, J = 8.7, 2.7 Hz), 7.10 (1H, s), 7.02 (1H, td, J = 9.2, 2.7 Hz), 4.20-4.08 (1H, m), 2.18-2.08 (2H, m), 1.78-1.70 (1H, m), 1.57-1.49 (2H, m), 1.43-1.23 (6H, m), 1.13 (3H, d, J = 6.5 Hz), | 559 | 557 |
| 14 | 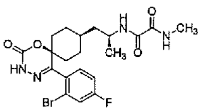 | | 1H-NMR (DMSO-D6) δ: 11.20 (1H, s), 8.64 (1H, q, J = 5.0 Hz), 8.43 (1H, d, J = 8.6 Hz), 7.73 (1H, dd, J = 8.6, 2.5 Hz), 7.52 (1H, dd, J = 8.6, 5,0 Hz), 7.34 (1H, td, J = 8.6, 2.5 Hz), 3.96-3.85 (1H, m), 2.64 (3H, d, J = 5.0 Hz), 2.17-2.05 (2H, m), 1.67-1.63 (1H, m), 1.55-1.15 (8H, m), 1.05 (3H, d, J = 6.5 Hz). | 483 | 481 |
| 15 | 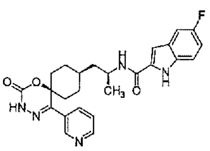 | | 1H-NMR (DMSO-D6) δ: 11.62 (1H, s), 11.25 (1H, s), 8.63 (1H, d, J = 2.4 Hz), 8.62 (1H, dd, J = 4.9, 1.8 Hz), 8.20 (1H, d, J = 8.3 Hz), 7.92 (1H, dt, J = 8.3, 1.8 Hz), 7.44-7.36 (3H, m), 7.12 (1H, d, J = 1.8 Hz), 7.02 (1H, td, J = 9.1, 2.4 Hz), 4.22-4.12 (1H, m), 2.06-1.98 (2H, m), 1.77-1.45 (6H, m), 1.41-1.28 (3H, m), 1.16 (3H, d, J = 6.5 Hz). | 464 | 462 |
| 16 | 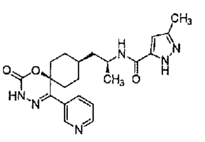 | | 1H-NMR (DMSO-D6) δ: 12.82 (1H, s), 11.24 (1H, s), 8.64 (1H, s), 8.63 (1H, dd, J = 4.7, 1.3 Hz), 7.93 (1H, d, J = 8.1 Hz), 7.65-7.58 (1H, m), 7.44 (1H, dd, J = 8.1, 4.7 Hz), 6.32 (1H, s), 4.14-4.04 (1H, m), 2.24 (3H, s), 2.06-1.99 (2H, m), 1.74-1.51 (5H, m), 1.46-1.23 (4H, m), 1.10 (3H, d, J = 6.0 Hz). | 411 | 409 |

Table 1-3

| | | | | | |
|---|---|---|---|---|---|
| 17 | 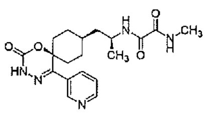 | | 1H-NMR (DMSO-D6) δ: 11.24 (1H, s), 8.67-8.63 (3H, m), 8.44 (1H, d, J = 9.2 Hz), 7.94 (1H, dt, J = 8.0, 1.8 Hz), 7.46 (1H, dd, J = 8.0, 4.7 Hz), 3.97-3.87 (1H, m), 2.65 (3H, d, J = 4.7 Hz), 2.04-1.97 (2H, m), 1.77-1.48 (5H, m), 1.35-1.24 (4H, m), 1.08 (3H, d, J = 6.7 Hz). | 388 | 386 |
| 18 | 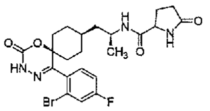 | "Mixture of two diastereomers due to 5-position of pyrrolidone ring | 1H-NMR (DMSO-D6) δ: 11.21 (1H, s), 7.76-7.73 (2H, m), 7.68 (1H, dd, J = 8.6, 4.6 Hz), 7.53 (1H, dd, J = 8.6, 6.0 Hz), 7.36 (1H, tt, J = 8.6, 2.3 Hz), 3.92-3.86 (1H, m), 3.85-3.81 (1H, m), 2.20-2.02 (5H, m), 1.82-1.73 (1H, m), 1.63-1.58 (1H, m), 1.51-1.18 (8H, m), 1.01-0.98 (3H, m). | 509 | 507 |
| 19 | 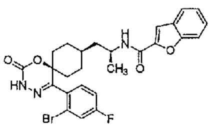 | | 1H-NMR (DMSO-D6) δ: 11.20 (1H, s), 8.41 (1H, d, J = 8.6 Hz), 7.75 (1H, d, J = 7.6 Hz), 7.70 (1H, dd, J = 8.6, 2.2 Hz), 7.63 (1H, d, J = 7.6 Hz), 7.50 (1H, dd, J = 8.6, 5.9 Hz), 7.49 (1H, s), 7.45 (1H, td, J = 7.6, 2.2 Hz), 7.33 (1H, t, J = 4.7 Hz), 7.31-7.27 (1H, m), 4.19-4.09 (1H, m), 2.18-2.07 (2H, m), 1.75-1.67 (1H, m), 1.60-1.50 (2H, m), 1.46-1.19 (6H, m), 1.13 (3H, d, J = 6.5 Hz). | 542 | 540 |

(continued)

| | | | | | |
|---|---|---|---|---|---|
| 20 | 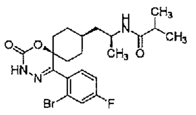 | | 1H-NMR (DMSO-D6) δ: 11.20 (1H, s), 7.74 (1H, dd, J = 8.6, 2.5 Hz), 7.53 (1H, dd, J = 8.6, 6.0 Hz), 7.42 (1H, d, J = 8.4 Hz), 7.35 (1H, td, J = 8.4, 2.5 Hz), 3.86-3.76 (1H, m), 2.30-2.23 (1H, m), 2.16-2.08 (2H, m), 1.63-1.56 (1H, m), 1.53-1.46 (1H, m), 1.43-1.18 (7H, m), 0.97-0.92 (9H, m). | 468 | 466 |
| 21 | 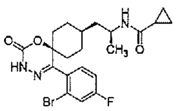 | | 1H-NMR (DMSO-D6) δ: 11.20 (1H, s), 7.77 (1H, d, J = 7.9 Hz), 7.74 (1H, dd, J = 8.7, 2.5 Hz), 7.54 (1H, dd, J = 8.7, 5.9 Hz), 7.35 (1H, td, J = 8.7, 2.5 Hz), 3.87-3.78 (1H, m), 2.17-2.07 (2H, m), 1.64-1.56 (1H, m), 1.52-1.17 (9H, m), 0.98 (3H, d, J = 6.5 Hz), 0.62-0.56 (4H, m). | 466 | 464 |
| 22 | 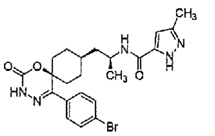 | | 1H-NMR (DMSO-D6) δ: 12.82 (1H, s), 11.15 (1H, s), 7.63-7.59 (1H, m), 7.60 (2H, d, J = 8.6 Hz), 7.41 (2H, d, J = 8.6 Hz), 6.33 (1H, s), 4.07 (1H, s), 2.25 (3H, s), 2.01-1.96 (2H, m), 1.72-1.51 (5H, m), 1.40-1.24 (4H, m), 1.10 (3H, d, J = 6.5 Hz). | 488 | 486 |
| 23 | 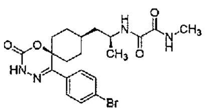 | | 1H-NMR (DMSO-D6) δ: 11.15 (1H, s), 8.65 (1H, q, J = 4.9 Hz), 8.44 (1H, d, J = 9.2 Hz), 7.62 (2H, d, J = 8.6 Hz), 7.43 (2H, d, J = 8.6 Hz), 3.98-3.88 (1H, m), 2.66 (3H, d, J = 4.9 Hz), 2.02-1.95 (2H, m), 1.74-1.47 (5H, m), 1.34-1.21 (4H, m), 1.08 (3H, d, J = 6.5 Hz). | 465 | 463 |
| 24 | 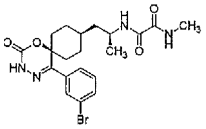 | | 1H-NMR (DMSO-D6) δ: 11.16 (1H, s), 8.65 (1H, q, J =4.5 Hz), 8.43 (1H, d, J = 9.0 Hz), 7.66 (1H, d, J = 7.5 Hz), 7.62 (1H, s), 7.50 (1H, d, J = 7.5 Hz), 7.39 (1H, t, J = 7.5 Hz), 3.98-3.88 (1H, m), 2.65 (3H, d, J = 4.5 Hz), 2.03-1.96 (2H, m), 1.83-1.48 (5H, m), 1.33-1.21 (4H, m), 1.08 (3H, d, J = 6.7 Hz). | 465 | 463 |

Table 1-4

| | | | | | |
|---|---|---|---|---|---|
| 25 | 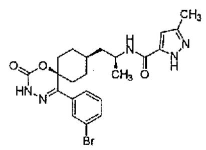 | | 1H-NMR (DMSO-D6) δ: 12.81 (1H, s), 11.16 (1H, s), 7.66-7.58 (3H, m), 7.48 (1H, d, J = 7.9 Hz), 7.38 (1H, d, J = 7.9 Hz), 6.32 (1H, s), 4.12-4.02 (1H, m), 2.24 (3H, s), 2.04-1.97 (2H, m), 1.78-1.48 (5H, m), 1.44-1.20 (4H, m), 1.10 (3H, d, J = 6.0 Hz). | 488 | 486 |
| 26 | 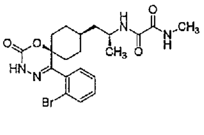 | | 1H-NMR (DMSO-D6) δ: 11.16 (1H, s), 8.63 (1H, q, J = 4.8 Hz), 8.41 (1H, d, J = 8.6 Hz), 7.73 (1H, d, J = 8.6 Hz), 7.48-7.37 (3H, m), 3.95-3.85 (1H, m), 2.63 (3H, d, J = 4.8 Hz), 2.18-2.08 (2H, m), 1.69-1.61 (1H, m), 1.54-1.13 (8H, m), 1.05 (3H, d, J = 6.7 Hz). | 465 | 463 |

(continued)

| | | | | | |
|---|---|---|---|---|---|
| 27 | 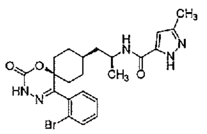 | | 1H-NMR (DMSO-D6) δ: 12.94 (0.18H, s), 12.80 (0.82H, s), 11.16 (1H, s), 7.94 (0.18H, d, J = 9.0 Hz), 7.72 (1H, d, J = 7.6 Hz), 7.58 (0.82H, d, J = 9.0 Hz), 7.44-7.38 (3H, m), 6.56 (0,18H, s), 6.30 (0.82H, s), 4.10-4.00 (1H, m), 2.23 (3H, s), 2.17-2.08 (2H, m), 1.75-1.63 (1H, m), 1.55-1.45 (2H, m), 1.39-1.20 (6H, m), 1.07 (3H, d, J = 6.0 Hz). | 488 | 486 |
| 28 | 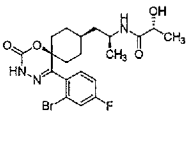 | | 1H-NMR (DMSO-D6) δ: 11.20 (1H, s), 7.74 (1H, dd, J = 8.6, 2.5 Hz), 7.53 (1H, dd, J = 8.6, 6.0 Hz), 7.35 (1H, td, J = 8.6, 2.5 Hz), 7.29 (1H, d, J = 8.6 Hz), 5.35 (1H, d, J = 5.3 Hz), 3.92-3.84 (2H, m), 2.16-2.09 (2H, m), 1.65-1.58 (1H, m), 1.52-1.18 (8H, m), 1.14 (3H, d, J = 6.7 Hz), 1.01 (3H, d, J = 6.5 Hz). | 470 | 468 |
| 29 | 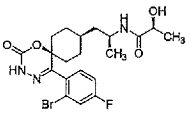 | | 1H-NMR (DMSO-D6) δ: 11.19 (1H, s), 7.74 (1H, dd, J = 8.8, 2.6 Hz), 7.53 (1H, dd, J = 8.6, 6.0 Hz), 7.35 (1H, td, J = 8.6, 2.6 Hz), 7.27 (1H, d, J = 8.8 Hz), 5.32 (1H, d, J = 5.1 Hz), 3.91-3.82 (2H, m), 2.17-2.09 (2H, m), 1.67-1.60 (1H, m), 1.51-1.19 (8H, m), 1.15 (3H, d, J = 6.7 Hz), 1.00 (3H, d, J = 6.5 Hz). | 470 | 468 |
| 30 | 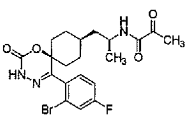 | | 1H-NMR (DMSO-D6) δ: 11.19 (1H, s), 8.27 (1H, d, J = 9.0 Hz), 7.73 (1H, dd, J = 8.6, 2.6 Hz), 7.52 (1H, dd, J = 8.6, 6.1 Hz), 7.34 (1H, td, J = 8.6, 2.6 Hz), 3.91-3.82 (1H, m), 2.30 (3H, s), 2.15-2,08 (2H, m), 1.64-1.59 (1H, m), 1.50-1.18 (8H, m), 1.04 (3H, d, J = 6.5 Hz). | 468 | 466 |
| 31 | 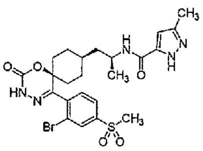 | | 1H-NMR (DMSO-D6) δ: 12.80 (1H, s), 11.31 (1H, s), 8.23 (1H, d, J = 1.8 Hz), 7.95 (1H, dd, J = 8.2, 1.8 Hz), 7.74 (1H, d, J = 8.2 Hz), 7.61-7.56 (1H, m), 6.31-6.29 (1H, m), 4.10-4.02 (1H, m), 3.33 (3H, s), 2.23 (3H, s), 2.20-2.10 (2H, m), 1.73-1.67 (1H, m), 1.55-1.46 (2H, m), 1.45-1.21 (6H, m), 1.07 (3H, d, J = 6.5 Hz). | 566 | 564 |
| 32 | 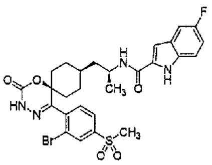 | | 1H-NMR (DMSO-D6) δ: 11.58 (1H, s), 11.32 (1H, s), 8.22 (1H, d, J = 1.8 Hz), 8.16 (1H, d, J = 8.6 Hz), 7.94 (1H, dd, J = 8.0, 1.8 Hz), 7.72 (1H, d, J = 8.0 Hz), 7.40-7.34 (2H, m), 7.10-7.08 (1H, m), 7.04-6.98 (1H, m), 4.18-4.09 (1H, m), 3.31 (3H, s), 2.20-2.12 (2H, m), 1.77-1.71 (1H, m), 1.57-1.49 (2H, m), 1.47-1.22 (6H, m), 1.13 (3H, d, J = 6.5 Hz). | 619 | 617 |

Table 1-5

| | | | | | |
|---|---|---|---|---|---|
| 33 | 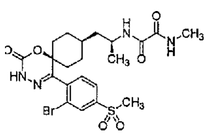 | | 1H-NMR (DMSO-D6) δ: 11.31 (1H, s), 8.65-8.60 (1H, m), 8.41 (1H, d, J = 9.2 Hz), 8.25 (1H, d, J = 1.8 Hz), 7.97 (1H, dd, J = 8.1, 1.8 Hz), 7.75 (1H, d, J = 8.1 Hz), 3.95-3.86 (1H, m), 3.34 (3H, s), 2.63 (3H, d, J = 4.9 Hz), 2.17-2.11 (2H, m), 1.69-1.63 (1H, m), 1.56-1.44 (2H, m), 1.40-1.18 (6H, m), 1.05 (3H, d, J = 6.5 Hz). | 543 | 541 |
| 34 | 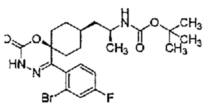 | | 1H-NMR (DMSO-D6) δ: 11.20 (1H, s), 7.74 (1H, dd, J = 8.5, 2.5 Hz), 7.53 (1H, dd, J = 8.5, 6.0 Hz), 7.35 (1H, td, J = 8.5, 2.5 Hz), 6.56 (1H, d, J = 9.0 Hz), 3.55-3.45 (1H, m), 2.19-2.08 (2H, m), 1.68-1.60 (1H, m), 1.50-1.12 (8H, m), 1.32 (9H, s), 0.96 (3H, d, J = 6.5 Hz). | 498 | 496 |

(continued)

| | | | | | |
|---|---|---|---|---|---|
| 35 | 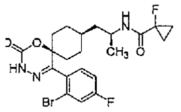 | | 1H-NMR (DMSO-D6) δ: 11.20 (1H, s), 8.05 (1H, d, J = 8.4 Hz), 7.74 (1H, dd, J = 8.4, 2.5 Hz), 7.54 (1H, dd, J = 8.4, 6.1 Hz), 7.36 (1H, td, J = 8.4, 2.5 Hz), 4.00-3.92 (1H, m), 2.18-2.09 (2H, m), 1.65-1.59 (1H, m), 1.51-1.19 (10H, m), 1.16-1,10 (2H, m), 1.06 (3H, d, J = 6.7 Hz). | 484 | 482 |
| 36 | 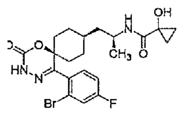 | | 1H-NMR (DMSO-D6) δ: 11.20 (1H, s), 7.74 (1H, dd, J = 8.7, 2.5 Hz), 7.54 (1H, dd, J = 8.7, 5.9 Hz), 7.42 (1H, d, J = 8.7 Hz), 7.36 (1H, td, J = 8.7, 2.5 Hz), 6.11 (1H, s), 3.93-3.85 (1H, m), 2.18-2.09 (2H, m), 1.66-1.59 (1H, m), 1.53-1.22 (8H, m), 1.03 (3H, d, J = 6.5 Hz), 0.96 (2H, q, J = 3.8 Hz), 0.77 (2H, q, J = 3.8 Hz). | 482 | 480 |
| 37 | 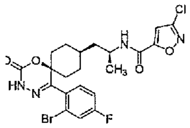 | | 1H-NMR (CDCl3) δ: 8.19 (1H, br s), 7.41 (1H, dd, J = 8.1, 2.5 Hz), 7.21-7.17 (1H, m), 7.12-7.07 (1H, m), 6.87 (1H, s), 6.22-6.18 (1H, m), 4.31-4.21 (1H, m), 2.35-2.28 (2H, m), 1.82-1.77 (1H, m), 1.63-1.52 (2H, m), 1.51-1.36 (5H, m), 1.34-1.25 (1H, m), 1.23 (3H, d, J = 6.5 Hz). | 527 | 525 |
| 38 | 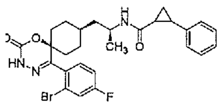 | Mixture of two diastereomers due to 1, 2-posltlons of cyclopropane ring (relative configuration: trans) | 1H-NMR (CDCl3) δ: 8.12-8.08 (1H, m), 7.43-7.40 (1H, m), 7.30-7.24 (2H, m), 7.23-7.16 (2H, m), 7.13-7.02 (3H, m), 5.29-5.23 (1H, m), 4.16-4.07 (1H, m), 2.47-2.40 (1H, m), 2.35-2.26 (2H, m), 1.80-1.75 (1H, m), 1.62-1.38 (7H, m), 1.37-1.17 (4H, m), 1.14-1.10 (3H, m). | 542 | 540 |
| 39 | 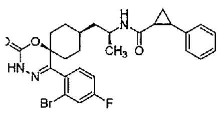 | Mixture of two diastereomers due to 1,2-positions of cyclopropane ring (relative configuration: cis) | 1H-NMR (CDCl3) δ: 8.11 (1H, s), 7.50-7.41 (1H, m), 7.23-7.09 (5H, m), 7.08-7.03 (1H, m), 6.90-6.86 (1H, m), 4.97-4.89 (1H, m), 3.89-3.80 (1H, m), 2.44-2.35 (1H, m), 2.27-2.12 (2H, m), 1.91-1.83 (1H, m), 1.69-1.61 (2H, m), 1.50-1.00 (9H, m), 0.94-0.74 (3H, m), | 542 | 540 |
| 40 | 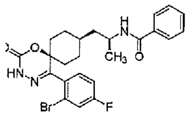 | | 1H-NMR (CDCl3) δ: 8.15 (1H, s), 7.74-7.71 (2H, m), 7.51-7.38 (4H, m), 7.20-7.16 (1H, m), 7.10-7.05 (1H, m), 5.77 (1H, d, J = 9.0 Hz), 4.38-4.28 (1H, m), 2.33-2.27 (2H, m), 1.90-1.85 (1H, m), 1.62-1.52 (3H, m), 1.51-1.38 (4H, m), 1.37-1.28 (1H, m), 1.22 (3H, d, J = 6.5 Hz). | 502 | 500 |

Table 1-6

| | | | | | |
|---|---|---|---|---|---|
| 41 | 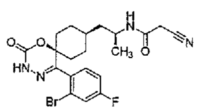 | | 1H-NMR (CDCl3) δ: 8.11 (1H, s), 7.42 (1H, dd, J = 8.1, 2.5 Hz), 7.23-7.19 (1H, m), 7.14-7.09 (1H, m), 5.74-5.70 (1H, m), 4.15-4.07 (1H, m), 3.33 (2H, s), 2.35-2.29 (2H, m), 1.78-1.73 (1H, m), 1.59-1.52 (2H, m), 1.50-1.37 (4H, m), 1.29-1.21 (2H, m), 1.16 (3H, d, J = 6.5 Hz). | 465 | 463 |

(continued)

| | | | | | |
|---|---|---|---|---|---|
| 42 | 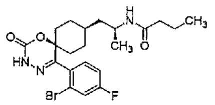 | | 1H-NMR (CDCl3) δ: 8.10 (1H, s), 7.41 (1H, dd, J = 8.1, 2.5 Hz), 7.22-7.18 (1H, m), 7.13-7.07 (1H, m), 5.08-5.02 (1H, m), 4.16-4.08 (1H, m), 2.32-2.27 (2H, m), 2.09 (2H, t, J = 7.5 Hz), 1.84-1.79 (1H, m), 1.67-1.59 (2H, m), 1.57-1.40 (5H, m), 1.33-1.23 (3H, m), 1.10 (3H, d, J = 6.5 Hz), 0.91 (3H, t, J = 7.5 Hz). | 468 | 466 |
| 43 | 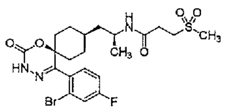 | | 1H-NMR (CDCl3) δ: 8.12 (1H, s), 7.42 (1H, dd, J = 8.2, 2.7 Hz), 7.23-7.20 (1H, m), 7.13-7.09 (1H, m), 5.31-5.28 (1H, m), 4.15-4.07 (1H, m), 3.46-3.31 (2H, m), 2.91 (3H, s), 2.69-2.65 (2H, m), 2.33-2.27 (2H, m), 1.77-1.72 (1H, m), 1.56-1.42 (5H, m), 1.36-1.24 (3H, m), 1.12 (3H, d, J = 6.5 Hz). | 532 | 530 |
| 44 | 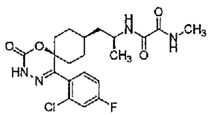 | | 1H-NMR (DMSO-D6) δ: 11.20 (1H, s), 8.66-8.60 (1H, m), 8.41 (1H, d, J = 9.0 Hz), 7.59 (1H, dd, J = 8.8, 2.5 Hz), 7.53 (1H, dd, J = 8.6, 6.1 Hz), 7.30 (1H, td, J = 6.6, 2.8 Hz), 3.97-3.85 (1H, m), 2.64 (3H, d, J = 5.0 Hz), 2.09 (2H, d, J = 13.0 Hz), 1.70-1.12 (9H, m), 1.05 (3H, d, J = 6.5 Hz). | 439 | 437 |
| 45 | 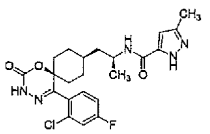 | | 1H-NMR (DMSO-D6) δ: 12.80 (1H, s), 11.19 (1H, s), 7.63-7.47 (3H, m), 7.28 (1H, td, J = 8.5, 2.2 Hz), 6.30 (1H, s), 4.14-3.99 (1H, m), 2.29-2.04 (5H, m), 1.79-1.16 (9H, m), 1.07 (3H, d, J = 6.5 Hz). | 462 | 460 |
| 46 | 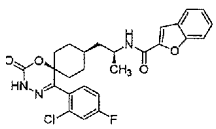 | | 1H-NMR (DMSO-D6) δ: 11.20 (1H, s), 8.40 (1H, d, J = 8.5 Hz), 7.75 (1H, d, J = 7.8 Hz), 7.63 (1H, d, J = 8.3 Hz), 7.57 (1H, dd, J = 8.8, 2.5 Hz), 7.52 (1H, dd, J = 8.8, 6.0 Hz), 7.49 (1H, d, J = 0.8 Hz), 7.47-7.42 (1H, m), 7.32 (1H, t, J = 7.5 Hz), 7.26 (1H, td, J = 8.5, 2.6 Hz), 4.18-4.08 (1H, m), 2.16-2.05 (2H, m), 1.75-1.66 (1H, m), 1.63-1.21 (8H, m), 1.13 (3H, d, J = 6.5 Hz). | 498 | 496 |
| 47 | 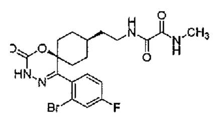 | | 1H-NMR (DMSO-D6) δ: 11.20 (1H, s), 8,70-8,61 (2H, m), 7.74 (1H, dd, J = 8.0, 2.5 Hz), 7.53 (1H, dd, J = 8.0, 6.6 Hz), 7.36 (1H, td, J = 8.0, 2.5 Hz), 3.18-3.00 (2H, m), 2.64 (3H, d, J = 4.9 Hz), 2.17-2.09 (2H, m), 1.62-1.56 (2H, m), 1.45-1.33 (4H, m), 1.28-1.20 (3H, m). | 469 | 467 |
| 48 | 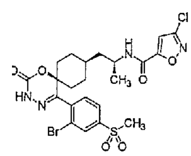 | | 1H-NMR (CDCl3) δ: 8.24 (1H, d, J = 1.8 Hz), 8.21 (1H, s), 7.94 (1H, dd, J = 8.1, 1.8 Hz), 7.42 (1H, d, J = 8.1 Hz), 6.86 (1H, s), 6.20-6.17 (1H, m), 4.31-4.22 (1H, m), 3.11 (3H, s), 2.37-2.32 (2H, m), 1.87-1.80 (1H, m), 1.64-1.27 (8H, m), 1.23 (3H, d, J = 6.5 Hz). | 587 | 585 |

Table 1-7

| | | | | | |
|---|---|---|---|---|---|
| 49 | 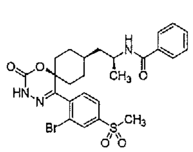 | | 1H-NMR (CDCl3) δ: 8.23 (1H, d, J = 1.8 Hz), 8.18 (1H, s), 7.92 (1H, dd, J = 8.2, 1.8 Hz), 7.73-7.70 (2H, m), 7.52-7.48 (1H, m), 7.45-7.40 (3H, m), 5.78-5.73 (1H, m), 4.38-4.31 (1H, m), 3.10 (3H, s), 2.36-2.30 (2H, m), 1.96-1.90 (1H, m), 1.64-1.49 (2H, m), 1.48-1.26 (6H, m), 1.22 (3H, d, J =6.7 Hz). | 562 | 560 |

(continued)

| | | | | | |
|---|---|---|---|---|---|
| 50 | 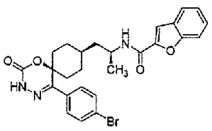 | | 1H-NMR (DMSO-D6) δ: 11.15 (1H, s), 8.43 (1H, d, J = 9.0 Hz), 7.76 (1H, d, J = 7.3 Hz), 7.65 (1H, d, J = 8.6 Hz), 7.59 (2H, d, J = 8.6 Hz), 7.51 (1H, s), 7.46 (1H, t, J = 7.3 Hz), 7.41 (2H, d, J = 8.6 Hz), 7.33 (1H, t, J = 7.3 Hz), 4.19-4.12 (1H, m), 2.04-1.96 (2H, m), 1.74-1.53 (5H, m), 1.47-1.24 (4H, m), 1.16 (3H, d, J = 6.5 Hz). | 524 | 522 |
| 51 | 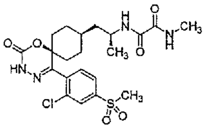 | | 1H-NMR (DMSO-D6) δ: 11.32 (1H, s), 8.65-8.58 (1H, m), 8.41 (1H, d, J = 9.0 Hz), 8.12 (1H, d, J = 1.8 Hz), 7.93 (1H, dd, J = 8.0, 1.8 Hz), 7.78 (1H, d, J = 8.0 Hz), 3.95-3.85 (1H, m), 3.34 (3H, s), 2.63 (3H, d, J = 4.8 Hz), 2.18-2.08 (2H, m), 1.69-1.61 (1H, m), 1.59-1.14 (8H, m), 1.05 (3H, d, J = 6.5 Hz). | 499 | 497 |
| 52 | 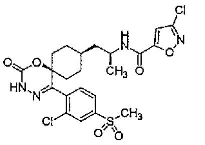 | | 1H-NMR (DMSO-D6) δ: 11.32 (1H, s), 8.77 (1H, d, J = 8.5 Hz), 8.11 (1H, d, J = 1.8 Hz), 7.93 (1H, dd, J = 8.0, 1.8 Hz), 7.78 (1H, d, J = 8.0 Hz), 7.32 (1H, s), 4.12-4.00 (1H, m), 3.33 (3H, s), 2.14 (2H, d, J = 11.3 Hz), 1.66 (1H, d, J = 11.0 Hz), 1.56-1.19 (8H, m), 1.10 (3H, d, J = 6.5 Hz). | 543 | 541 |
| 53 | 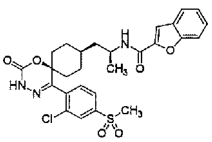 | | 1H-NMR (DMSO-D6) δ: 11.32 (1H, s), 8.40 (1H, d, J = 8.5 Hz), 8.09 (1H, d, J = 1.8 Hz), 7.90 (1H, dd, J = 8.0, 1.8 Hz), 7.75 (2H, t, J = 7.5 Hz), 7.62 (1H, d, J = 8.8 Hz), 7.48 (1H, s), 7.44 (1H, t, J = 7.3 Hz), 7.32 (1H, t, J = 7.3 Hz), 4.15 (1H, bs), 3.36 (3H, s), 2.14 (2H, t, J = 11.0 Hz), 1.71 (1H, d, J = 9.0 Hz), 1.60-1.30 (8H, m), 1.13 (3H, d, J = 6.5 Hz). | 558 | 556 |
| 54 | 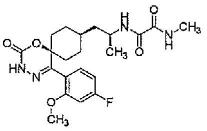 | | 1H-NMR (DMSO-D6) δ: 11.00 (1H, s), 8.64 (1H, q, J = 4.7 Hz), 8.42 (1H, d, J = 9.8 Hz), 7.21 (1H, dd, J = 8.4, 6.8 Hz), 7.01 (1H, dd, J = 9.8, 2.4 Hz), 6.81 (1H, td, J = 8.4, 2.4 Hz), 3.96-3.86 (1H, m), 3.79 (3H, s), 2.64 (3H, d, J = 4.7 Hz), 2.00-1.93 (2H, m), 1.68-1.60 (1H,m), 1.50-1.38 (4H, m), 1.28-1.13 (4H, m), 1.06 (3H, d, J = 6.8 Hz). | 435 | 433 |
| 55 | 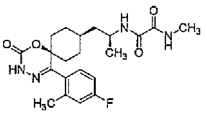 | | 1H-NMR (DMSO-D6) δ: 11.08 (1H, s), 8.62 (1H, q, J = 4.9 Hz), 8.41 (1H, d, J = 9.0 Hz), 7.32 (1H, dd, J = 8.6, 6.0 Hz), 7.17 (1H, dd, J = 10.3, 2.7 Hz), 7.06 (1H, td, J = 8.6, 2.7 Hz), 3.95-3.86 (1H, m), 2.64 (3H, d, J = 4.9 Hz), 2.23 (3H, s), 2.06-1.99 (2H, m), 1.68-1.15 (9H, m), 1.05 (3H, d, J = 6.5 Hz). | 419 | 417 |
| 56 | 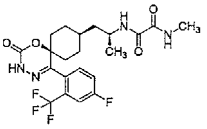 | | 1H-NMR (DMSO-D6) δ: 11.26 (1H, s), 8.64 (1H, q, J = 4.9 Hz), 8.43 (1H, d, J = 9.2 Hz), 7.80 (1H, dd, J = 9.2, 2.5 Hz), 7.71 (1H, dd, J = 8.8, 5.3 Hz), 7.65-7.58 (1H, m), 3.97-3.84 (1H, m), 2.65 (3H, d, J = 4.9 Hz), 2.07-1.88 (2H, m), 1.70-1.60 (1H, m), 1.60-1.14 (8H, m), 1.06 (3H, d, J = 6.5 Hz). | 473 | 471 |

Table 1-8

| | | | | | |
|---|---|---|---|---|---|
| 57 | 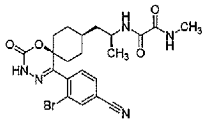 | | 1H-NMR (CDCl3) δ: 8.24 (1H, s), 7.97 (1H, d, J = 1.6 Hz), 7.68 (1H, dd, J = 7.9, 1.6 Hz), 7.44-7.39 (1H, m), 7.33 (1H, d, J = 7.9 Hz), 7.18-7.14 (1H, m), 4.08-4.00 (1H, m), 2.89 (3H, d, J = 5.3 Hz), 2.34-2.28 (2H, m), 1.79-1.72 (1H, m), 1.60-1.51 (2H, m), 1.50-1.31 (5H, m), 1.30-1.21 (1H, m), 1.16 (3H, d, J = 6.5 Hz). | 490 | 488 |
| 58 | 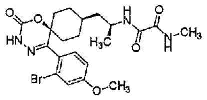 | | 1H-NMR (DMSO-D6) δ: 11.10 (1H, s), 8.65-8.60 (1H, m), 8.41 (1H, d, J = 9.5 Hz), 7.34 (1H, d, J = 8.6 Hz), 7.28 (1H, d, J = 2.5 Hz), 7.01 (1H, dd, J = 8.6, 2.5 Hz), 3.95-3.85 (1H, m), 3.81 (3H, s), 2.64 (3H, d, J = 4.9 Hz), 2.14-2.05 (2H, m), 1.68-1.62 (1H, m), 1.56-1.11 (8H, m), 1.05 (3H, d, J = 6.5 Hz). | 495 | 493 |

(continued)

| | | | | | |
|---|---|---|---|---|---|
| 59 | 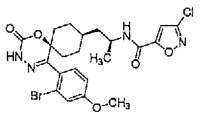 | | 1H-NMR (DMSO-D6) δ: 11.10 (1H, s), 8.77 (1H, d, J = 8.3 Hz), 7.34 (1H, d, J = 8.6 Hz), 7.32 (1H, s), 7.28 (1H, d, J = 2.5 Hz), 7.00 (1H, dd, J = 8.6, 2.8 Hz), 4.11-4.01 (1H, m), 3.80 (3H, s), 2.15-2.06 (2H, m), 1.69-1.61 (1H, m), 1.55-1.20 (8H, m), 1.11 (3H, d, J = 6.5 Hz). | 539 | 537 |
| 60 | 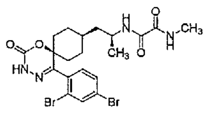 | | 1H-NMR (DMSO-D6) δ: 11.21 (1H, s), 8.62 (1H, q, J = 5.2 Hz), 8.41 (1H, d, J = 9.7 Hz), 8.02 (1H, d, J = 1.8 Hz), 7.66 (1H, dd, J = 8.3, 1.8 Hz), 7.41 (1H, d, J = 8.3 Hz), 3.95-3.86 (1H, m), 2.64 (3H, d, J = 5.2 Hz), 2.13-2.06 (2H, m), 1.68-1.61 (1H, m), 1.53-1.15 (8H, m), 1.05 (3H, d, J = 6.5 Hz), | 543 | 541 |
| 61 | 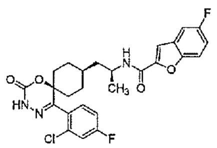 | | 1H-NMR (DMSO-D6) δ: 11.20 (1H, s), 8.46 (1H, d, J = 8.5 Hz), 7.66 (1H, dd, J = 9.0, 4.3 Hz), 7.57 (2H, dd, J = 8.8, 2.8 Hz), 7.52 (1H, dd, J = 8.6, 6.1 Hz), 7.49 (1H, s), 7.33-7.23 (2H, m), 4.18-4.07 (1H, m), 2.16-2.05 (2H, m), 1.75-1.66 (1H, m), 1.62-1.21 (8H, m), 1.13 (3H, d, J = 6.5 Hz). | 516 | 514 |
| 62 | 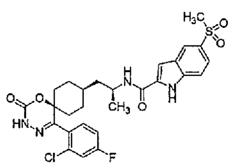 | | 1H-NMR (DMSO-D6) δ: 12.07 (1H, s), 11.20 (1H, s), 8.34 (1H, d, J = 8.5 Hz), 8.24 (1H, s), 7.68 (1H, dd, J = 8.8, 1,8 Hz), 7.61-7.55 (2H, m), 7.51 (1H, dd, J = 8.6, 6.1 Hz), 7.33 (1H, d, J = 1.5 Hz), 7.26 (1H, td, J = 8.6, 2.6 Hz), 4.22-4.10 (1H, m), 3.17 (3H, s), 2.15-2.06 (2H, m), 1.77-1.69 (1H, m), 1.59-1.21 (8H, m), 1.15 (3H, d, J = 6.5 Hz). | 575 | 573 |
| 63 | 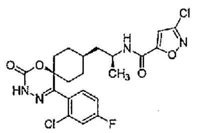 | | 1H-NMR (DMSO-D6) δ: 11.20 (1H, s), 8.77 (1H, d, J = 8.3 Hz), 7.59 (1H, dd, J = 9.0, 2.5 Hz), 7.53 (1H, dd, J = 8.6, 6.1 Hz), 7.32 (1H, s), 7.29 (1H, td, J = 8.5, 2.5 Hz), 4.12-4.01 (1H, m), 2.10 (2H, d, J = 13.0 Hz), 1.69-1.60 (1H, m), 1.57-1.20 (8H, m), 1.11 (3H, d, J = 6.8 Hz). | 483 | 481 |
| 64 | 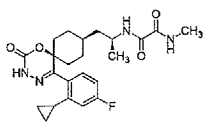 | | 1H-NMR (CDCl3) δ: 8.09 (1H, s), 7.44-7.38 (1H, m), 7.18-7.13 (1H, m), 7.08-7.04 (1H, m), 6.90-6.85 (1H, m), 6,52 (1H, dd, J = 10.6, 2.5 Hz), 4.08-3.98 (1H, m), 2.89 (3H, d, J = 5.3 Hz), 2.27-2.19 (2H, m), 1.89-1.82 (1H, m), 1.76-1.70 (1H, m), 1.54-1.35 (7H, m), 1.29-1.21 (1H, m), 1.16 (3H, d, J = 6.5 Hz), 1.06-1.02 (2H, m), 0.75-0.71 (2H, m), | 445 | 443 |

Table 1-9

| | | | | | |
|---|---|---|---|---|---|
| 65 | 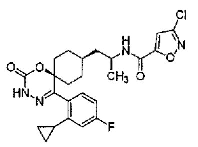 | | 1H-NMR (CDCl3) δ: 8.07 (1H, s), 7.05 (1H, dd, J = 8.6, 5.8 Hz), 6.89-6.84 (2H, m), 6.52 (1H, dd, J = 10.4, 2.5 Hz), 6.20-6.16 (1H, m), 4.30-4.21 (1H, m), 2.28-2.22 (2H, m), 1,89-1.77 (2H, m), 1.60-1.39 (7H, m), 1.35-1.26 (1H, m), 1.23 (3H, d, J = 6.5 Hz), 1.06-1.02 (2H, m), 0.75-0.71 (2H, m). | 489 | 487 |
| 66 | 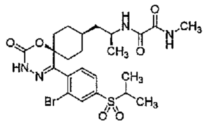 | | 1H-NMR (CDCl3) δ: 8.21 (1H, s), 8.18 (1H, d, J = 1.7 Hz), 7.88 (1H, dd, J = 8.0, 1.7 Hz), 7.43-7.39 (2H, m), 7.17-7.14 (1H, m), 4.11-4.01 (1H, m), 3.29-3.21 (1H, m), 2.89 (3H, d, J = 5.3 Hz), 2.36-2.29 (2H, m), 1.79-1.74 (1H, m), 1.58-1.37 (7H, m), 1.35 (6H, d, J = 7.2 Hz), 1.29-1.21 (1H, m), 1.16 (3H, d, J = 6.5 Hz). | 571 | 569 |
| 67 | 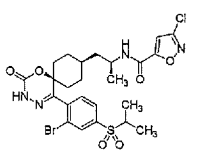 | | 1H-NMR (CDCl3) δ: 8.19 (1H, s), 8.17 (1H, d, J = 1.8 Hz), 7.87 (1H, dd, J = 8.0, 1.8 Hz), 7.41 (1H, d, J = 8.0 Hz), 6.86 (1H, s), 6.21-6.17 (1H, m), 4.32-4.22 (1H, m), 3.27-3.20 (1H, m), 2.38-2.31 (2H, m), 1.86-1.77 (1H, m), 1.61-1.39 (7H, m), 1.34 (6H, d, J = 6.9 Hz), 1.29-1.21 (4H, m). | 615 | 613 |

(continued)

| | | | | | |
|---|---|---|---|---|---|
| 68 | 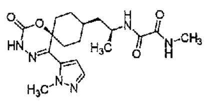 | | 1H-NMR (DMSO-D6) δ: 11.35 (1H, s), 8.68-8.63 (1H, m), 8.44 (1H, d, J = 9.2 Hz), 7.48 (1H, d, J = 1.8 Hz), 6.62 (1H, d, J = 2.1 Hz), 3.97-3.87 (1H, m), 3.81 (3H, s), 2.66 (3H, d, J = 5.1 Hz), 2.03-1.96 (2H, m), 1.77-1.46 (5H, m), 1.37-1.17 (4H, m), 1.08 (3H, d, J = 6.5 Hz). | 391 | 389 |
| 69 | 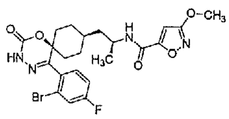 | | 1H-NMR (DMSO-D6) δ: 11.19 (1H, s), 8.58 (1H, d, J = 8.3 Hz), 7.72 (1H, dd, J = 8.7, 2.7 Hz), 7.52 (1H, dd, J = 8.6, 6.0 Hz), 7.33 (1H, td, J = 8.5, 2.5 Hz), 6.75 (1H, s), 4.09-3.99 (1H, m), 3.92 (3H, s), 2.16-2.07 (2H, m), 1.69-1.61 (1H, m), 1.55-1.20 (8H, m), 1.09 (3H, d, J = 6.7 Hz). | 523 | 521 |
| 70 | 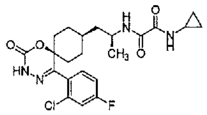 | | 1H-NMR (DMSO-D6) δ: 11.20 (1H, s), 8.68 (1H, d, J = 5.2 Hz), 8.41 (1H, d, J = 9.0 Hz), 7.60 (1H, dd, J = 8.6, 2.6 Hz), 7.53 (1H, dd, J = 9.0, 6.0 Hz), 7.30 (1H, td, J = 8.4, 2.6 Hz), 3.93-3.85 (1H, m), 2.77-2.70 (1H, m), 2.12-2.04 (2H, m), 1.68-1.61 (1H, m), 1.54-1.16 (8H, m), 1.05 (3H, d, J = 6.7 Hz), 0.64-0,58 (4H, m), | 465 | 463 |
| 71 | 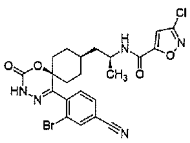 | | 1H-NMR (CDCl3) δ: 8.17 (1H, s), 7.96 (1H, d, J = 1.6 Hz), 7.67 (1H, dd, J = 8.0, 1.6 Hz), 7.33 (1H, d, J = 8.0 Hz), 6.86 (1H, s), 6.19-6.15 (1H, m), 4.30-4.22 (1H, m), 2.35-2.31 (2H, m), 1.85-1.79 (1H, m), 1.60-1.25 (8H, m), 1.23 (3H, d, J = 6.5 Hz). | 534 | 532 |
| 72 | 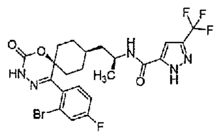 | | 1H-NMR (DMSO-D6) δ: 14.40-14.28 (1H, m), 11.20 (1H, s), 8.30-8.25 (1H, m), 7.72 (1H, dd, J = 8.7, 2.7 Hz), 7.50 (1H, dd, J = 8.6, 6.0 Hz), 7.32 (1H, td, J = 8.4, 2.5 Hz), 7.26 (1H, s), 4.14-4.03 (1H, m), 2.16-2.07 (2H, m), 1.75-1.67 (1H, m), 1.54-1.21 (8H, m), 1.11 (3H, d, J = 6.5 Hz). | 560 | 558 |

Table 1-10

| | | | | | |
|---|---|---|---|---|---|
| 73 | 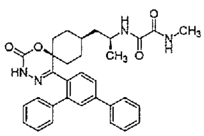 | | 1H-NMR (DMSO-D6) δ: 11.06 (1H, s), 8.68 (1H, q, J = 5.2 Hz), 8.38 (1H, d, J = 9.0 Hz), 7.78 (2H, d, J = 7.6 Hz), 7.74 (1H, dd, J = 8.1, 2.0 Hz), 7.66 (1H, d, J = 2.0 Hz), 7.51-7.37 (9H, m), 3.90-3.80 (1H, m), 2.67 (3H, d, J = 5.0 Hz), 1.63-1.07 (11H, m), 1.02 (3H, d, J = 6.7 Hz). | 539 | 537 |
| 74 | 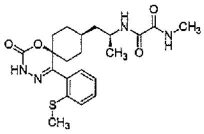 | | 1H-NMR (DMSO-D6) δ: 11.09 (1H, s), 8.63 (1H, q, J = 4.7 Hz), 8.41 (1H, d, J = 9.0 Hz), 7.46-7.36 (2H, m), 7.26-7.15 (2H, m), 3.95-3.85 (1H, m), 2.64 (3H, d, J = 4.7 Hz), 2.43 (3H, s), 2.16-2.07 (2H, m), 1.66-1.60 (1H, m), 1.53-1.37 (3H, m), 1.34-1.13 (5H, m), 1.05 (3H, d, J = 6.5 Hz). | 433 | 431 |
| 75 | 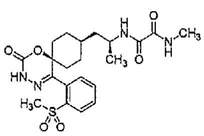 | | 1H-NMR (DMSO-D6) δ: 11.12 (1H, s), 8.64 (1H, q, J = 4.6 Hz), 8.42 (1H, d, J = 8.8 Hz), 8.07 (1H, d, J = 7.8 Hz), 7.82-7.75 (2H, m), 7.66 (1H, d, J = 7.8 Hz), 3.96-3.86 (1H, m), 3.24 (3H, s), 2.65 (3H, d, J = 4.6 Hz), 2.20-2.09 (2H, m), 1.66-1.45 (4H, m), 1.34-1.17 (5H, m), 1.06 (3H, d, J = 6.7 Hz). | 465 | 463 |
| 76 | 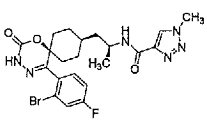 | | 1H-NMR (DMSO-D6) δ: 11.12 (1H, s), 8.78 (1H, d, J = 8.1 Hz), 8.06 (1H, d, J = 7.6 Hz), 7.77 (2H, dt, J = 18.1, 7.5 Hz), 7.65 (1H, d, J = 6.5 Hz), 7.33 (1H, s), 4.07 (1H, s), 3.24 (3H, s), 2.21-2.06 (2H, m), 1.68-1.42 (4H, m), 1.39-1.20 (5H, m), 1.12 (3H, d, J = 6.2 Hz). | 509 | 507 |

(continued)

| | | | | | |
|---|---|---|---|---|---|
| 77 | 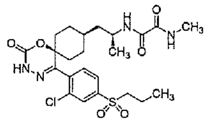 | | 1H-NMR (DMSO-D6) δ: 11.32 (1H, s), 8.66-8.59 (1H, m), 8.41 (1H, d, J = 9.3 Hz), 8.06 (1H, d, J = 1.8 Hz), 7.90 (1H, dd, J = 8.0, 1.8 Hz), 7.77 (1H, d, J = 8.0 Hz), 3.96-3.84 (1H, m), 3.45-3.39 (2H, m), 2.63 (3H, d, J = 5.3 Hz), 2.16-2.06 (2H, m), 1.69-1.13 (11H, m), 1.05 (3H, d, J = 6.5 Hz), 0.94 (3H, t, J = 7.5 Hz). | 527 | 525 |
| 78 | 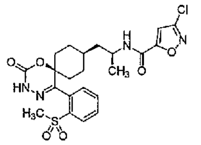 | | 1H-NMR (DMSO-D6) δ: 11.12 (1H, s), 8.78 (1H, d, J = 8.1 Hz), 8.06 (1H, d, J = 7.0 Hz), 7,82-7.72 (2H, m), 7.65 (1H, d, J = 7.0 Hz), 7.33 (1H, s), 4.10-4.02 (1H, m), 3.24 (3H, s), 2.21-2.06 (2H, m), 1.68-1.42 (4H, m), 1.39-1.20 (5H, m), 1.12 (3H, d, J = 6.2 Hz). | 509 | 507 |
| 79 | 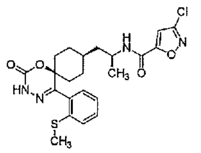 | | 1H-NMR (DMSO-D6) δ: 11.09 (1H, s), 8.76 (1H, d, J = 9.0 Hz), 7.45-7.37 (2H, m), 7.32 (1H, s), 7.25-7.18 (2H, m), 4.10-4.03 (1H, m), 2.43 (3H, s), 2.17-2.09 (2H, m), 1.66-1.61 (1H, m), 1.54-1.21 (8H, m), 1.11 (3H, d, J = 6.5 H). | 477 | 475 |
| 80 | 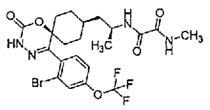 | | 1H-NMR (DMSO-D6) δ: 11.25 (1H, s), 8.66-8.60 (1H, m), 8.41 (1H, d, J = 9.2 Hz), 7.85-7.83 (1H, m), 7.61 (1H, d, J = 8.3 Hz), 7.51-7.46 (1H, m), 3.95-3.85 (1H, m), 2.64 (3H, d, J = 4.9 Hz), 2.16-2.08 (2H, m), 1.70-1.61 (1H, m), 1.56-1.16 (8H, m), 1.05 (3H, d, J = 6.5 Hz). | 549 | 547 |

Table 1-11

| | | | | | |
|---|---|---|---|---|---|
| 81 | 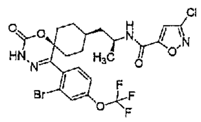 | | 1H-NMR (DMSO-D6) δ: 11.25 (1H, s), 8.77 (1H, d, J = 8.6 Hz), 7.85-7.83 (1H, m), 7.61 (1H, d, J = 8.6 Hz), 7.50-7.46 (1H, m), 7.33-7.31 (1H, m), 4.11-4.01 (1H, m), 2.18-2.09 (2H, m), 1.70-1.62 (1H, m), 1.57-1.21 (8H, m), 1.11 (3H, d, J = 6.5 Hz). | 593 | 591 |
| 82 | 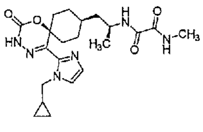 | | 1H-NMR (DMSO-D6) δ: 11.23 (1H, s), 8.71-8.65 (1H, m), 8.49 (1H, d, J = 9.5 Hz), 7.39 (1H, s), 7.03 (1H, s), 4.01-3.90 (1H, m), 3.98 (2H, d, J = 7.6 Hz), 2.68 (3H, d, J = 4.9 Hz), 2.37-2.20 (2H, m), 1.93-1.82 (2H, m), 1.73-1.64 (1H, m), 1.62-1.45 (2H, m), 1.37-1.15 (5H, m), 1.09 (3H, d, J = 6.5 Hz), 0.52-0.45 (2H, m), 0.37-0.30 (2H, m). | 431 | 429 |
| 83 | 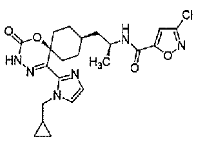 | | 1H-NMR (DMSO-D6) δ: 11.24 (1H, s), 8.86-8.82 (1H, m), 7.38 (2H, d, J = 9.0 Hz), 7.01 (1H, s), 4.16-4.06 (1H, m), 3.98 (2H, d, J = 7.4 Hz), 2.37-2.22 (2H, m), 1.93-1.84 (2H, m), 1.72-1.65 (1H, m), 1.60-1.50 (2H, m), 1.44-1.17 (5H, m), 1.15 (3H, d, J = 6.9 Hz), 0.51-0.45 (2H, m), 0.37-0.31 (2H, m). | 475 | 473 |
| 84 | 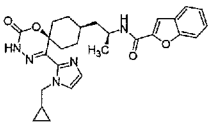 | | 1H-NMR (DMSO-D6) δ: 11.23 (1H, s), 8.47 (1H, d, J = 8.3 Hz), 7.79-7.75 (1H, m), 7.66 (1H, d, J = 8.6 Hz), 7.54 (1H, s), 7.50-7.43 (1H, m), 7.37 (1H, s), 7.34 (1H, t, J = 8.2 Hz), 6.98 (1H, s), 4.24-4.13 (1H, m), 3.97 (2H, d, J = 7.2 Hz), 2.38-2.21 (2H, m), 1.93-1.85 (2H, m), 1.79-1.69 (1H, m), 1.67-1.51 (2H, m), 1.48-1.14 (5H, m), 1.17 (3H, d, J = 6.2 Hz), 0.51-0.44 (2H, m), 0.36-0.29 (2H, m). | 490 | 488 |
| 85 | 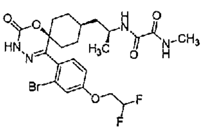 | | 1H-NMR (DMSO-D6) δ: 11.12 (1H, s), 8.67-8.59 (1H, m), 8.44-8.39 (1H, m), 7.41-7.35 (2H, m), 7.11-7.07 (1H, m), 6.55-6.23 (1H, m), 4.47-4.36 (2H, m), 3.98-3.82 (1H, m), 2.64 (3H, d, J = 4.9 Hz), 2.14-2.05 (2H, m), 1.71-1.61 (1H, m), 1.59-1,10 (8H, m), 1.05 (3H, d, J = 6.5 Hz). | 545 | 543 |

(continued)

| | | | | | |
|---|---|---|---|---|---|
| 86 | 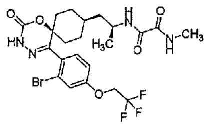 | | 1H-NMR (DMSO-D6) δ: 11.14 (1H, d, J = 2.3 Hz), 8.65-8.60 (1H, m), 8.41 (1H, d, J = 9.0 Hz), 7.46 (1H, d, J = 2.8 Hz), 7.41 (1H, d, J = 8.1 Hz), 7.14 (1H, dd, J = 8.6, 2.5 Hz), 4.87 (2H, q, J = 8.8 Hz), 3.96-3.85 (1H, m), 2.64 (3H, d, J = 4.6 Hz), 2.15-2.05 (2H, m), 1.70-1.60 (1H, m), 1.58-1.09 (8H, m), 1.05 (3H, d, J = 6.5 Hz). | 563 | 561 |
| 87 | 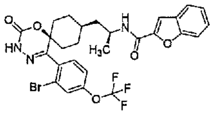 | | 1H-NMR (DMSO-D6) δ: 11.24 (1H, s), 8.40 (1H, d, J = 9.2 Hz), 7.82 (1H, d, J = 1.8 Hz), 7.74 (1H, d, J = 8.1 Hz), 7.63 (1H, d, J = 8.3 Hz), 7.59 (1H, d, J = 8.6 Hz), 7.49 (1H, d, J = 0.9 Hz), 7.47-7.42 (2H, m), 7.34-7.30 (1H, m), 4.18-4.07 (1H, m), 2.18-2.09 (2H, m), 1.75-1.66 (1H, m), 1.65-1.20 (8H, m), 1.13 (3H, d, J = 6.7 Hz). | 608 | 606 |
| 88 | 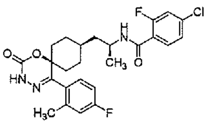 | | 1H-NMR (DMSO-D6) δ: 11.09 (1H, s), 8.13 (1H, d, J = 8.3 Hz), 7.56-7.48 (2H, m), 7.36-7.30 (2H, m), 7.18 (1H, dd, J = 10.2, 2.8 Hz), 7.08 (1H, td, J = 8.6, 2.8 Hz), 4.08-4.00 (1H, m), 2.24 (3H, s), 2.10-2.02 (2H, m), 1.70-1.63 (1H, m), 1.56-1.23 (8H, m), 1.09 (3H, d, J = 6.5 Hz). | 490 | 488 |

Table 1-12

| | | | | | |
|---|---|---|---|---|---|
| 89 | 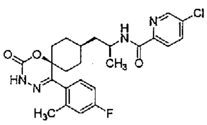 | | 1H-NMR (DMSO-D6) δ: 11.08 (1H, s), 8.66 (1H, d, J = 2.3 Hz), 8.45 (1H, d, J = 9.2 Hz), 8.09 (1H, dd, J = 8.6, 2.3 Hz), 8.01 (1H, d, J = 8.6 Hz), 7.30 (1H, dd, J = 8.4, 5.9 Hz), 7.16 (1H, dd, J = 10.2, 2.5 Hz), 7.03 (1H, td, J = 8.4, 2.5 Hz), 4.16-4.07 (1H, m), 2.22 (3H, s), 2.08-1.98 (2H, m), 1.72-1.21 (9H, m), 1.13 (3H, d, J = 6.7 Hz). | 473 | 471 |
| 90 | 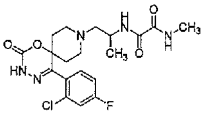 | | 1H-NMR (DMSO-D6) δ: 11.26 (1H, s), 8.66-8.59 (1H, m), 8.35 (1H, d, J = 8.8 Hz), 7.62 (1H, dd, J = 9.0, 2.5 Hz), 7.56 (1H, dd, J = 8.6, 6.1 Hz), 7.33 (1H, td, J = 8.6, 2.6 Hz), 3.98-3.86 (1H, m), 2.74-2.59 (2H, m), 2.64 (3H, d, J = 5.0 Hz), 2.42 (1H, dd, J = 12.5, 8.3 Hz), 2.35-2.18 (3H, m), 2.10-2,00 (2H, m), 1,63-1,46 (2H, m), 1.04 (3H, d, J = 6.5 Hz). | 440 | 438 |
| 91 | 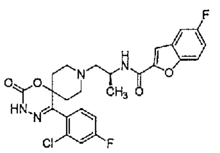 | | 1H-NMR (DMSO-D6) δ: 11.25 (1H, s), 8.39 (1H, d, J = 8.3 Hz), 7.65 (1H, dd, J = 9.1, 4.1 Hz), 7.59 (1H, d, J = 2.5 Hz), 7.56 (1H, d, J = 2.5 Hz), 7.52 (1H, dd, J = 8.6, 5.9 Hz), 7.45 (1H, s), 7.31 (1H, td, J = 8.6, 2.7 Hz), 7.26 (1H, td, J = 8.6, 2.5 Hz), 4.20-4.09 (1H, m), 2.77-2.64 (2H, m), 2.49-2.43 (1H, m), 2.37-2.25 (3H, m), 2.06 (2H, d, J = 14.3 Hz), 1.61-1.48 (2H, m), 1.11 (3H, d, J = 6.8 Hz). | 517 | 515 |
| 92 | 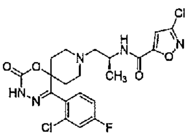 | | 1H-NMR (DMSO-D6) δ: 11.26 (1H, s), 8.72 (1H, d, J = 8.5 Hz), 7.61 (1H, dd, J = 9.0, 2.5 Hz), 7.54 (1H, dd, J = 8.6, 6.1 Hz), 7.34-7.28 (1H, m), 7.28 (1H, s), 4.14-4.04 (1H, m), 2.76-2.61 (2H, m), 2.42 (1H, dd, J = 12.5, 8.0 Hz), 2.37-2.23 (3H, m), 2.09-2.00 (2H, m), 1.60-1.48 (2H, m), 1.09 (3H, d, J = 6.8 Hz). | 484 | 482 |
| 93 | 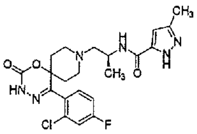 | | 1H-NMR (DMSO-D6) δ: 12.80 (1H, s), 11.25 (1H, s), 7.66-7.50 (3H, m), 7.31 (1H, td, J = 8.5, 2.6 Hz), 6.28 (1H, s), 4.12-4.01 (1H, m), 2.75-2.61 (2H, m), 2.46-2.38 (1H, m), 2.35-2.14 (6H, m), 2.10-2.01 (2H, m), 1.63-1.49 (2H, m), 1.07 (3H, d, J = 6.5 Hz). | 463 | 461 |

(continued)

| | | | | | |
|---|---|---|---|---|---|
| 94 | 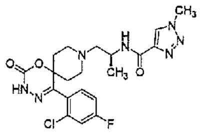 | | 1H-NMR (DMSO-D6) δ: 11.26 (1H, s), 8.41 (1H, s), 8.10 (1H, d, J = 8.5 Hz), 7.61 (1H, dd, J = 8.8, 2.5 Hz), 7.54 (1H, dd, J = 8.5, 6.0 Hz), 7.31 (1H, td, J = 8.5, 2.6 Hz), 4.16-4.01 (1H, m), 4.07 (3H, s), 2.75-2.65 (2H, m), 2.49-2.42 (1H, m), 2.35-2.23 (3H, m), 2.10-2.00 (2H, m), 1.64-1.48 (2H, m), 1.09 (3H, d, J = 6.5 Hz), | 464 | 462 |
| 95 | 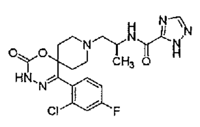 | | 1H-NMR (DMSO-D6) δ: 14.62 (1H, brs), 11.26 (1H, s), 8.46-8.09 (1H, m), 7.95 (1H, s), 7.60 (1H, dd, J = 8.8, 2.5 Hz), 7.54 (1H, dd, J = 8.8, 6.0 Hz), 7.31 (1H, td, J = 8.8, 2.5 Hz), 4.17-4.04 (1H, m), 2.77-2.61 (2H, m), 2.54-2.43 (1H, m), 2.37-2.21 (3H, m), 2.10-2.00 (2H, m), 1.65-1.48 (2H, m), 1.10 (3H, d, J = 6.5 Hz). | 450 | 448 |
| 96 | 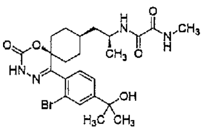 | | 1H-NMR (DMSO-D6) δ: 11.14 (1H, s), 8.63 (1H, q, J = 4.7 Hz), 8.41 (1H, d, J = 9.7 Hz), 7.78 (1H, s), 7.50 (1H, d, J = 8.2 Hz), 7.36 (1H, d, J = 8.2 Hz), 5.23 (1H, s), 3.97-3.83 (1H, m), 2.64 (3H, d, J = 4.7 Hz), 2.19-2.05 (2H, m), 1.72-1.61 (1H, m), 1.56-1.14 (8H, m), 1.43 (6H, s), 1.05 (3H, d, J = 6.7 Hz). | 523 | 521 |

Table 1-13

| | | | | | |
|---|---|---|---|---|---|
| 97 | 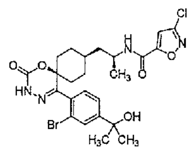 | | 1H-NMR (DMSO-D6) δ: 11.14 (1H, s), 8.76 (1H, d, J = 9.0 Hz), 7.78 (1H, s), 7.49 (1H, d, J = 8.2 Hz), 7.35 (1H, d, J = 8.2 Hz), 7.32 (1H, s), 5.22 (1H, s), 4.11-3.99 (1H, m), 2.19-2.07 (2H, m), 1.71-1.60 (1H, m), 1.57-1.20 (8H, m), 1.42 (6H, s), 1.11 (3H, d, J = 6.7 Hz). | 567 | 565 |
| 98 | 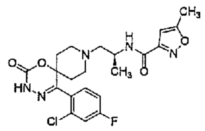 | | 1H-NMR (DMSO-D6) δ: 11.25 (1H, s), 8.30 (1H, d, J = 8.5 Hz), 7.62 (1H, dd, J = 9.0, 2.5 Hz), 7.55 (1H, dd, J = 8.6, 6.1 Hz), 7.32 (1H, td, J = 8.5, 2.5 Hz), 6.42 (1H, s), 4.14-4.03 (1H, m), 2.74-2.61 (2H, m), 2.47-2.38 (1H, m), 2.44 (3H, s), 2.36-2.23 (3H, m), 2.10-2.00 (2H, m), 1.62-1.47 (2H, m), 1.07 (3H, d, J = 6.8 Hz). | 464 | 462 |
| 99 | 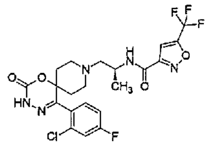 | | 1H-NMR (DMSO-D6) δ: 11.51 (1H, s), 9,12-9,07 (1H, m), 7.83 (1H, s), 7.72-7.58 (2H, m), 7.45-7.36 (1H, m), 4.56-4.32 (1H, m), 2.55-2.24 (6H, m), 2.12-1.81 (2H, m), 1.67-1.43 (2H, m), 1.21 (3H, d, J = 6.5 Hz). | 520 (M+3) | 516 |
| 100 | 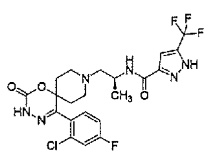 | | 1H-NMR (DMSO-D6) δ: 14.36 (1H, s), 11.26 (1H, s), 8.26 (1H, d, J = 8.0 Hz), 7.60 (1H, dd, J = 8.8, 2,5 Hz), 7.52 (1H, dd, J = 8.6, 6.1 Hz), 7.29 (1H, td, J = 8.6, 2.6 Hz), 7.23 (1H, s), 4.16-4.06 (1H, m), 2.78-2.64 (2H, m), 2.41 (1H, dd, J = 12.4, 7.6 Hz), 2.36-2.25 (3H, m), 2.05 (2H, d, J = 13.5 Hz), 1.62-1.50 (2H, m), 1.10 (3H, d, J = 6.5 Hz). | 517 | 515 |
| 101 | 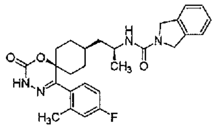 | | 1H-NMR (DMSO-D6) δ: 11,08 (1H, s), 7.34-7.24 (5H, m), 7.16 (1H, dd, J = 10.3, 2.7 Hz), 7.04 (1H, td, J = 8.5, 2.5 Hz), 5.88 (1H, d, J = 8.3 Hz), 4.60-4.50 (4H, m), 3.84-3.75 (1H, m), 2.23 (3H, s), 2.09-2.00 (2H, m), 1.69-1.61 (1H, m), 1.55-1.17 (8H, m), 1.04 (3H, d, J = 6.5 Hz). | 479 | 477 |

(continued)

| | | | | | |
|---|---|---|---|---|---|
| 102 | 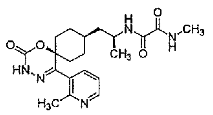 | | 1H-NMR (DMSO-D6) δ: 11.17 (1H, s), 8.62 (1H, q, J = 4.5 Hz), 8.51 (1H, d, J = 5.2 Hz), 8.41 (1H, d, J = 9.0 Hz), 7.73 (1H, d, J = 7.5 Hz), 7.28 (1H, dd, J = 7.5, 5.2 Hz), 3.97-3.83 (1H, m), 2.64 (3H, d, J = 4.5 Hz), 2.41 (3H, s), 2.14-2.01 (2H, m), 1.71-1.60 (1H, m), 1.58-1.40 (3H, m), 1.40-1.15 (5H, m), 1.05 (3H, d, J = 6.7 Hz). | 402 | 400 |
| 103 | 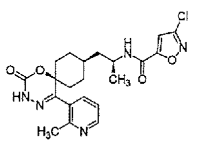 | | 1H-NMR (DMSO-D6) δ: 11.17 (1H, s), 8.77 (1H, d, J = 8.2 Hz), 8.50 (1H, d, J = 4.5 Hz), 7.72 (1H, d, J = 7.5 Hz), 7.32 (1H, s), 7.27 (1H, dd, J = 7.5, 4.5 Hz), 4.12-4.00 (1H, m), 2.41 (3H, s), 2.14-2.03 (2H, m), 1.71-1.61 (1H, m), 1.56-1.21 (8H, m), 1.11 (3H, d, J = 6.0 Hz). | 446 | 444 |
| 104 | 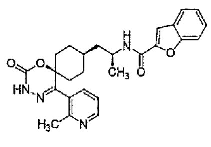 | | 1H-NMR (DMSO-D6) δ: 11.17 (1H, s), 8.48 (1H, d, J = 3.0 Hz), 8.40 (1H, d, J = 9.0 Hz), 7.74 (1H, d, J = 8.2 Hz), 7.71 (1H, dd, J = 8,2, 1.5 Hz), 7.63 (1H, d, J = 8.2 Hz), 7.49 (1H, s), 7.45 (1H, t, J = 7.5 Hz), 7.32 (1H, t, J = 7.5 Hz), 7.24 (1H, dd, J = 7.9, 4.9 Hz), 4.19-4.07 (1H, m), 2.41 (3H, s), 2.14-2.01 (2H, m), 1.75-1.66 (1H, m), 1.63-1.21 (8H, m), 1.13 (3H, d, J = 6.7 Hz). | 461 | 459 |

Table 1-14

| | | | | | |
|---|---|---|---|---|---|
| 105 | 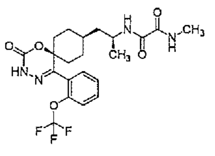 | | 1H-NMR (DMSO-D6) δ: 11.22 (1H, s), 8.63 (1H, q, J = 4.5 Hz), 8.42 (1H, d, J = 9.7 Hz), 7.64-7.58 (1H, m), 7.55-7.43 (3H, m), 3.97-3.83 (1H, m), 2.64 (3H, d, J = 4.5 Hz), 2.07-1.96 (2H, m), 1.71-1.58 (1H, m), 1.58-1.14 (8H, m), 1.05 (3H, d, J = 6.7 Hz). | 471 | 469 |
| 106 | 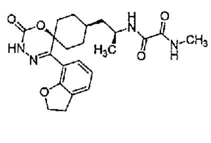 | | 1H-NMR (DMSO-D6) δ: 11.03 (1H, s), 8.70-8.65 (1H, m), 8.44 (1H, d, J = 9.0 Hz), 7.31 (1H, d, J = 6.9 Hz), 7.03 (1H, d, J = 7.6 Hz), 6.87 (1H, t, J = 7.5 Hz), 4.57-4.49 (2H, m), 4.00-3.87 (1H, m), 3.21 (2H, t, J = 8.4 Hz), 2.66 (3H, d, J = 4.9 Hz), 1.99-1.87 (2H, m), 1.79-1.42 (5H, m), 1.35-1.14 (4H, m), 1.07 (3H, d, J = 6.7 Hz). | 429 | 427 |
| 107 | 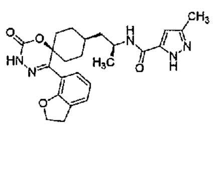 | | 1H-NMR (DMSO-D6) δ: 12.87-12.77 (1H, m), 11.02 (1H, s), 7.65-7.57 (1H, m), 7.31-7.27 (1H, m), 7.03-7.00 (1H, m), 6.85 (1H, t, J = 7.5 Hz), 6.35-6.29 (1H, m), 4.53-4.39 (2H, m), 4.13-4,02 (1H, m), 3.18 (2H, t, J = 8.7 Hz), 2.29-2.16 (3H, m), 1.98-1.87 (2H, m), 1.76-1.43 (5H, m), 1.33-1.17 (4H, m), 1.09 (3H, d, J = 6.5 Hz). | 452 | 450 |
| 108 | 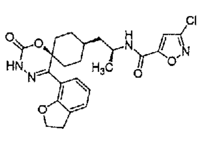 | | 1H-NMR (DMSO-D6) δ: 11.03 (1H, s), 8.80 (1H, d, J = 9.0 Hz), 7.35 (1H, s), 7.30 (1H, d, J = 7.4 Hz), 7.02 (1H, d, J = 7.4 Hz), 6.86 (1H, t, J = 7.5 Hz), 4.57-4.43 (2H, m), 4.13-4.01 (1H, m), 3.20 (2H, t, J = 8.7 Hz), 1.99-1.90 (2H, m), 1.77-1.44 (5H, m), 1.37-1.16 (4H, m), 1.13 (3H, d, J = 6.5 Hz). | 473 | 471 |
| 109 | 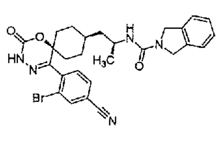 | | 1H-NMR (DMSO-D6) δ: 11.30 (1H, s), 8.33 (1H, d, J = 1.4 Hz), 7.92 (1H, dd, J = 7.9, 1.6 Hz), 7.67 (1H, d, J = 7.9 Hz), 7.32-7.25 (4H, m), 5.88 (1H, d, J = 8.1 Hz), 4.61-4.47 (4H, m), 3.86-3.75 (1H, m), 2.20-2.10 (2H, m), 1.73-1.60 (1H, m), 1.58-1.13 (8H, m), 1.04 (3H, d, J = 6.5 Hz). | 550 | 548 |

(continued)

| | | | | | |
|---|---|---|---|---|---|
| 110 | 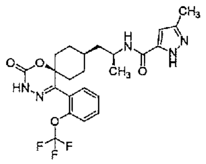 | | 1H-NMR (DMSO-D6) δ: 12.80 (1H, s), 11.22 (1H, s), 7.63-7.56 (2H, m), 7.53-7.49 (1H, m), 7.49-7.42 (2H, m), 6.30 (1H, s), 4.12-3.99 (1H, m), 2.23 (3H, s), 2.08-1.96 (2H, m), 1.74-1.62 (1H, m), 1.58-1.18 (8H, m), 1.07 (3H, d, J = 6.7 Hz). | 494 | 492 |
| 111 | 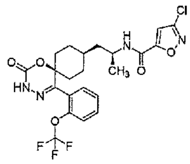 | | 1H-NMR (DMSO-D6) δ: 11.22 (1H, s), 8.77 (1H, d, J = 8.2 Hz), 7.65-7.56 (1H, m), 7.55-7.42 (3H, m), 7.32 (1H, s), 4.13-3.99 (1H, m), 2.10-1.97 (2H, m), 1.71-1.61 (1H, m), 1.57-1.20 (8H, m), 1.11 (3H, d, J = 6.7 Hz). | 515 | 513 |
| 112 | 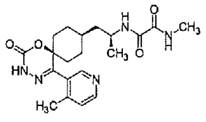 | | 1H-NMR (DMSO-D6) δ: 11.20 (1H, s), 8.62 (1H, q, J = 4.5 Hz), 8.51-8.44 (2H, m), 8.41 (1H, d, J = 9.0 Hz), 7.35 (1H, d, J = 5.2 Hz), 3.98-3.83 (1H, m), 2.64 (3H, d, J = 4.5 Hz), 2.25 (3H, s), 2.13-2.00 (2H, m), 1.72-1.60 (1H, m), 1.58-1.42 (3H, m), 1.42-1.17 (5H, m), 1.06 (3H, d, J = 6.0 Hz). | 402 | 400 |

Table 1-15

| | | | | | |
|---|---|---|---|---|---|
| 113 | 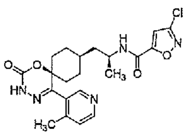 | | 1H-NMR (DMSO-D6) δ: 11.21 (1H, s), 8.77 (1H, d, J = 8.2 Hz), 8.47 (1H, d, J = 5.2 Hz), 8.45 (1H, s), 7.35 (1H, d, J = 5.2 Hz), 7.32 (1H, s), 4.13-3.99 (1H, m), 2.25 (3H, s), 2.14-2.00 (2H, m), 1.71-1.59 (1H, m), 1.57-1.20 (8H, m), 1.11 (3H, d, J = 6.7 Hz). | 446 | 444 |
| 114 | 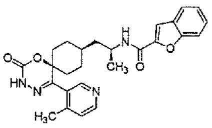 | | 1H-NMR (DMSO-D6) δ: 11.21 (1H, s), 8.49-8.37 (3H, m), 7.74 (1H, d, J = 8.2 Hz), 7.63 (1H, d, J = 8.2 Hz), 7.49 (1H, s), 7.44 (1H, t, J = 7.9 Hz), 7.37-7.28 (2H, m), 4.21-4.05 (1H, m), 2.24 (3H, s), 2.15-2.02 (2H, m), 1.79-1.65 (1H, m), 1.65-1.20 (8H, m), 1.13 (3H, d, J = 6.7 Hz). | 461 | 459 |
| 115 | 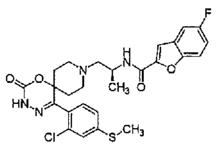 | | 1H-NMR (DMSO-D6) δ: 11.21 (1H, s), 8.40 (1H, d, J = 8.5 Hz), 7.66 (1H, dd, J = 9.0, 4.0 Hz), 7.58 (1H, dd, J = 8.6, 2.9 Hz), 7.46 (1H, s), 7.40 (1H, d, J = 1.8 Hz), 7.34 (1H, d, J = 8.3 Hz), 7.29 (1H, dd, J = 9.1, 2.9 Hz), 7.22 (1H, dd, J = 8.1, 1.9 Hz), 4.20-4.08 (1H, m), 3.32 (3H, s), 2.78-2.65 (2H, m), 2.49-2.41 (1H, m), 2.37-2.24 (3H, m), 2.09-1.99 (2H, m), 1.64-1.49 (2H, m), 1.11 (3H, d, J = 6.5 Hz). | 545 | 543 |
| 116 | 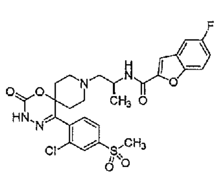 | | 1H-NMR (DMSO-D6) δ: 11.37 (1H, s), 8.40 (1H, d, J = 8.5 Hz), 8.12 (1H, d, J = 1.8 Hz), 7.93 (1H, dd, J = 8.3, 1.8 Hz), 7.79 (1H, d, J = 7.8 Hz), 7.68-7.63 (1H, m), 7.60-7.55 (1H, m), 7.44 (1H, s), 7.33-7.26 (1H, m), 4,19-4.08 (1H, m), 3.32 (3H, s), 2.80-2.65 (3H, m), 2.38-2.25 (3H, m), 2.15-2.05 (2H, m), 1.66-1.51 (2H, m), 1.11 (3H, d, J = 6.5 Hz). | 577 | 575 |

(continued)

| | | | | | |
|---|---|---|---|---|---|
| 117 | 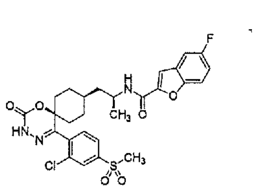 | | 1H-NMR (DMSO-D6) δ: 11.32 (1H, s), 8.46 (1H, d, J = 8.8 Hz), 8.09 (1H, d, J = 1.8 Hz), 7.90 (1H, dd, J = 8.0, 1.8 Hz), 7.76 (1H, d, J = 8.0 Hz), 7.66 (1H, dd, J = 9.1, 4.1 Hz), 7.57 (1H, dd, J = 8.8, 2.5 Hz), 7.48 (1H, s), 7.29 (1H, td, J = 9.1, 2.7 Hz), 4.18-4.06 (1H, m), 3.31 (3H, s), 2.14 (2H, t, J = 9.9 Hz), 1.75-1.67 (1H, m), 1.62-1.21 (8H, m), 1.12 (3H, d, J = 6.5 Hz). | 576 | 574 |
| 118 | 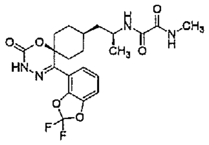 | | 1H-NMR (DMSO-D6) δ: 11.35 (1H, s), 8.63 (1H, q, J = 4.9 Hz), 8.44 (1H, d, J = 9.2 Hz), 7.51 (1H, dd, J = 6.2, 3.0 Hz), 7.31-7.25 (2H, m), 3.99-3.88 (1H, m), 2.65 (3H, d, J = 4.9 Hz), 2.07-1.98 (2H, m), 1.74-1.46 (5H, m), 1.36-1.18 (4H, m), 1.07 (3H, d, J = 6.5 Hz). | 467 | 465 |
| 119 | 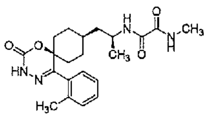 | | 1H-NMR (DMSO-D6) δ: 11.05 (1H, s), 8.63 (1H, q, J = 4.5 Hz), 8.41 (1H, d, J = 9.0 Hz), 7.38-7.18 (4H, m), 3.98-3.82 (1H, m), 2.64 (3H, d, J = 4.5 Hz), 2.22 (3H, s), 2.10-1.97 (2H, m), 1.70-1.60 (1H, m), 1.58-1.38 (3H, m), 1.38-1.15 (5H, m), 1.05 (3H, d, J = 6.7 Hz). | 401 | 399 |
| 120 | 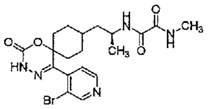 | Mixture (4:1) of two stereoisomers due to 1,4-posistions of cyclohexane ring (cis form is presumed to be major.) | 1H-NMR (DMSO-D6) δ: 11.37 (0.2H, s), 11.34 (0.8H, s), 8.91 (0.2H, s), 8.88 (0.8H, s), 8.68-8.58 (2H, m), 8.47-8.43 (0,2H, m), 8.41 (0.8H, d, J = 9.0 Hz), 7.62 (0.2H, d, J = 4.5 Hz), 7.55 (0.8H, d, J = 5.2 Hz), 3.97-3.83 (0.8H, m), 3.82-3.70 (0.2H, m), 2.67-2.65 (0.6H, m), 2.64 (2.4H, d, J = 4.5 Hz), 2.18-2.06 (1.6H, m), 1.91-1.82 (0.4H, m), 1.79-1.13 (9H, m), 1.05 (2.4H, d, J = 6.7 Hz), 1.05-1.01 (0.6H, m). | 466 | 464 |

Table 1-16

| | | | | | |
|---|---|---|---|---|---|
| 121 | 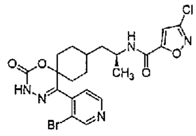 | Mixture (4:1) of two stereoisomers due to 1,4-posistions of cyclohexane ring (cis form is presumed to be major.) | 1H-NMR (DMSO-D6) δ: 11.38 (0.2H, s), 11.35 (0.8H, s), 8.91 (0.2H, s), 8.88 (0.8H, s), 8.81-8.78 (0.2H, m), 8.77 (0.8H, d, J = 9.0 Hz), 8.66 (0.2H, d, J = 5.2 Hz), 8.62 (0.8H, d, J = 5.2 Hz), 7.62 (0.2H, d, J = 4.5 Hz), 7.55 (0.8H, d, J = 5.2 Hz), 7.32 (1H, s), 4.13-3.99 (0.8H, m), 3.99-3.85 (0.2H, m), 2.22-2.04 (1.6H, m), 1.93-1.84 (0.4H, m), 1.81-1.59 (2H, m), 1.58-1.19 (7H, m), 1.11 (2.4H, d, J = 6.0 Hz), 1.10-1.07 (0.6H, m). | 510 | 508 |
| 122 | 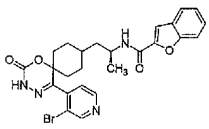 | Mixture (4:1) of two stereoisomers due to 1,4-posistions of cyclohexane ring (cis form is presumed to be major.) | 1H-NMR (DMSO-D6) δ: 11.37 (0.2H, s), 11.34 (0.8H, s), 8.91 (0.2H, s), 8.85 (0.8H, s), 8.66 (0.2H, d, J = 4.5 Hz), 8.58 (0.8H, d, J = 5.2 Hz), 8.44-8.41 (0.2H, m), 8.41 (0.8H, d, J = 8.2 Hz), 7.78-7.75 (0.2H, m), 7.74 (0.8H, d, J = 6.7 Hz), 7.66-7.60 (1.2H, m), 7.54 (0.8H, d, J = 5.2 Hz), 7.51-7.41 (2H, m), 7.36-7.29 (1H, m), 4.21-4.06 (0.8H, m), 4.06-3.93 (0.2H, m), 2,19-2,07 (1.6H, m), 1.94-1.84 (0.4H, m), 1.82-1.66 (2H, m), 1.62-1.20 (7H, m), 1.13 (2.4H, d, J = 6.7 Hz), 1.13-1.09 (0.6H, m), | 525 | 523 |

(continued)

| | | | | | |
|---|---|---|---|---|---|
| 123 | 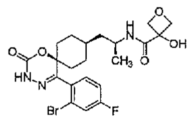 | | 1H-NMR (DMSO-D6) δ: 11.20 (1H, s), 7.73 (1H, dd, J = 8.2, 2.2 Hz), 7.53 (1H, dd, J = 8.6, 5.6 Hz), 7.42 (1H, d, J = 9.0 Hz), 7.35 (1H, td, J = 8.6, 2.2 Hz), 6.66 (1H, s), 4.69 (1H, d, J = 6.0 Hz), 4.65 (1H, d, J = 6.0 Hz), 4.39 (2H, d, J = 6.0 Hz), 3.98-3.85 (1H, m), 2.17-2.07 (2H, m), 1.68-1.57 (1H, m), 1.53-1.14 (8H, m), 1.01 (3H, d, J = 6.7 Hz). | 498 | 496 |
| 124 | 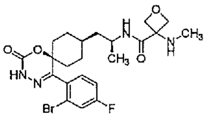 | | 1H-NMR (DMSO-D6) δ: 11.20 (1H, s), 7.74 (1H, dd, J = 8.6, 2.6 Hz), 7.52 (1H, dd, J = 9.0, 6.0 Hz), 7.45 (1H, d, J = 9.0 Hz), 7.35 (1H, td, J = 8.6, 2.6 Hz), 4.63 (1H, d, J = 6.0 Hz), 4.59 (1H, d, J = 6.0 Hz), 4.31 (1H, d, J = 6.0 Hz), 4.30 (1H, d, J = 6.0 Hz), 3.97-3.84 (1H, m), 2.99-2.81 (1H, m), 2.18-2.08 (2H, m), 2.05 (3H, s), 1.66-1.57 (1H, m), 1.54-1.16 (8H, m), 1.01 (3H, d, J = 6.0 Hz). | 511 | 509 |
| 125 | 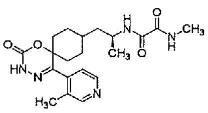 Mixture | Mixture (4:1) of two stereoisomers due to 1,4-posistions of cyclohexane ring (cis form is presumed to be major.) | 1H-NMR (DMSO-D6) δ: 11.28-11.19 (1H, m), 8.69-8.51 (2H, m), 8.51-8.37 (2H, m), 7.40-7.36 (0.2H, m), 7.36-7.31 (0.8H, m), 3.99-3.83 (0.8H, m), 3.83-3.70 (0.2H, m), 2.71-2.60 (3H, m), 2.23 (3H, s), 2.12-2.00 (1.6H, m), 1.89-1.17 (9.4H, m), 1.08-1.02 (3H, m), | 402 | 400 |
| 126 | 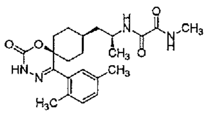 | | 1H-NMR (DMSO-D6) δ: 11.02 (1H, s), 8.63 (1H, q, J = 4.5 Hz), 8.41 (1H, d, J = 9.0 Hz), 7.17 (1H, d, J = 7.5 Hz), 7.12 (1H, d, J = 7.5 Hz), 7.04 (1H, s), 3.98-3.83 (1H, m), 2.64 (3H, d, J = 4.5 Hz), 2.28 (3H, s), 2.16 (3H, s), 2.09-1.98 (2H, m), 1.71-1.58 (1H, m), 1.58-1.39 (3H, m), 1.39-1.14 (5H, m), 1.06 (3H, d, J = 6.7 Hz). | 415 | 413 |
| 127 | 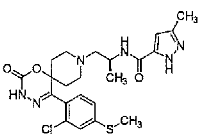 | | 1H-NMR (DMSO-D6) δ: 12.80 (1H, s), 11.21 (1H, s), 7.61-7.53 (1H, m), 7.42 (1H, d, J = 1.8 Hz), 7.36 (1H, d, J = 8.3 Hz), 7.27 (1H, dd, J = 8.4, 1.9 Hz), 6.29 (1H, s), 4.12-3.99 (1H, m), 2,76-2.64 (2H, m), 2.53 (3H, s), 2.47-2.37 (1H, m), 2.34-2.18 (6H, m), 2.09-2.00 (2H, m), 1.64-1.49 (2H, m), 1.07 (3H, d, J = 6.5 Hz). | 491 | 489 |
| 128 | 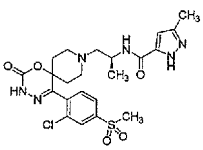 | | 1H-NMR (DMSO-D6) δ: 12.80 (1H, s), 11.38 (1H, s), 8.13 (1H, d, J = 1.8 Hz), 7.95 (1H, dd, J = 8.0, 1.8 Hz), 7.80 (1H, d, J = 8.0 Hz), 7.30-7.20 (1H, m), 6.29 (1H, s), 4.11-3.99 (1H, m), 3.34 (3H, s), 2.81-2.65 (2H, m), 2.48-2.40 (1H, m), 2.35-2.19 (6H, m), 2.13-2.05 (2H, m), 1.64-1.50 (2H, m), 1.06 (3H, d, J = 6.5 Hz). | 523 | 521 |

Table 1-17

| | | | | | |
|---|---|---|---|---|---|
| 129 | 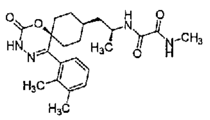 | | 1H-NMR (DMSO-D6) δ: 11.02 (1H, s), 8.65-8.57 (1H, m), 8.40 (1H, d, J = 8.8 Hz), 7.22 (1H, d, J = 6.9 Hz), 7.13 (1H, t, J = 7.6 Hz), 7.05 (1H, d, J = 7.9 Hz), 3.97-3.83 (1H, m), 2.63 (3H, d, J = 4.9 Hz), 2.26 (3H, s), 2.11 (3H, s), 2.08-1.97 (2H, m), 1.68-1.60 (1H, m), 1.57-1.10 (8H, m), 1.05 (3H, d, J = 6.7 Hz). | 415 | 413 |
| 130 | 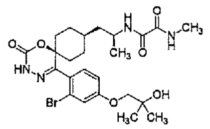 | | 1H-NMR (DMSO-D6) δ: 11.10 (1H, s), 8.63 (1H, q, J = 4.9 Hz), 8.41 (1H, d, J = 8.8 Hz), 7.32 (1H, d, J = 8.8 Hz), 7.28 (1H, d, J = 2.5 Hz), 7.01 (1H, dd, J = 8.8, 2.5 Hz), 4.64 (1H, s), 3.95-3.86 (1H, m), 3.77 (2H, s), 2.64 (3H, d, J = 4.9 Hz), 2.13-2.05 (2H, m), 1.66-1.14 (9H, m), 1.19 (6H, s), 1.05 (3H, d, J = 6.5 Hz), | 553 | 551 |
| 131 | 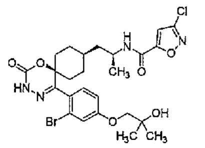 | | 1H-NMR (DMSO-D6) δ: 11.10 (1H, s), 8.77 (1H, d, J = 8.3 Hz), 7.33 (1H, s), 7.32 (1H, d, J = 8.3 Hz), 7.27 (1H, d, J = 2.5 Hz), 7.00 (1H, dd, J = 8.3, 2.5 Hz), 4.63 (1H, s), 4.10-4.02 (1H, m), 3.76 (2H, s), 2.15-2.07 (2H, m), 1.68-1.62 (1H, m), 1.53-1.22 (8H, m), 1.19 (6H, s), 1.11 (3H, d, J = 6.5 Hz). | 597 | 595 |
| 132 | 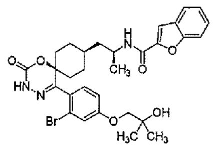 | | 1H-NMR (DMSO-D6) δ: 11.10 (1H, s), 8.40 (1H, d, J = 8.8 Hz), 7.75 (1H, d, J = 7.9 Hz), 7.63 (1H, d, J = 8.8 Hz), 7.49 (1H, s), 7.45 (1H, t, J = 8.3 Hz), 7.34-7.29 (2H, m), 7.25 (1H, d, J = 2.5 Hz), 6.97 (1H, dd, J = 8.3, 2.5 Hz), 4.61 (1H, s), 4.18-4.00 (1H, m), 3.74 (2H, s), 2.15-2.05 (2H, m), 1.74-1.66 (1H, m), 1.57-1.25 (8H, m), 1.17 (6H, s), 1.13 (3H, d, J = 6.5 Hz). | 612 | 610 |
| 133 | 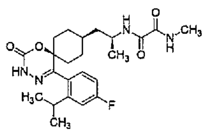 | | 1H-NMR (DMSO-D6) δ: 11.10 (1H, s), 8.62 (1H, q, J = 4.8 Hz), 8.41 (1H, d, J = 9.0 Hz), 7.30-7.25 (2H, m), 7.06 (1H, td, J = 8.5, 2.5 Hz), 3.95-3.85 (1H, m), 2.87-2.80 (1H, m), 2.64 (3H, d, J = 4.8 Hz), 2.05-1.93 (2H, m), 1.67-1.60 (1H, m), 1.52-1.18 (8H, m), 1.16 (6H, d, J = 6.5 Hz), 1.05 (3H, d, J = 6,5 Hz). | 447 | 445 |
| 134 | 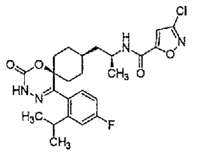 | | 1H-NMR (DMSO-D6) δ: 11.10 (1H, s), 8.77 (1H, d, J = 8.6 Hz), 7.32 (1H, s), 7.29-7.25 (2H, m), 7.05 (1H, td, J = 8.6, 2.5 Hz), 4.09-4.00 (1H, m), 2.87-2.80 (1H, m), 2.08-1.95 (2H, m), 1.68-1.61 (1H, m), 1.53-1.21 (8H, m), 1.16 (6H, d, J = 6.7 Hz), 1.11 (3H, d, J = 6.7 Hz). | 491 | 489 |
| 135 | 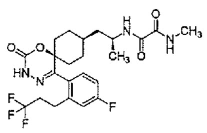 | | 1H-NMR (DMSO-D6) δ: 11.18 (1H, s), 8.63 (1H, q, J = 4.7 Hz), 8.42 (1H, d, J = 9.0 Hz), 7.41 (1H, dd, J = 8.6, 5.6 Hz), 7.36 (1H, dd, J = 10.1, 2.6 Hz), 7.15 (1H, td, J = 8.6, 2.6 Hz), 3.97-3.84 (1H, m), 2.79-2.70 (2H, m), 2.64 (3H, d, J = 5.2 Hz), 2.61-2.52 (2H, m), 2.08-1.94 (2H, m), 1.71-1.58 (1H, m), 1.58-1.17 (8H, m), 1.06 (3H, d, J = 6.7 Hz). | 501 | 499 |
| 136 | 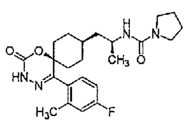 | | 1H-NMR (DMSO-D6) δ: 11.08 (1H, s), 7.33 (1H, dd, J = 8.6, 6.0 Hz), 7.18 (1H, dd, J = 10.2, 2.5 Hz), 7.07 (1H, td, J = 8.6, 2.5 Hz), 5.55 (1H, d, J = 8.3 Hz), 3.77-3.68 (1H, m), 3.18-3.11 (4H, m), 2.24 (3H, s), 2.08-2.00 (2H, m), 1.79-1.71 (4H, m), 1.65-1.58 (1H, m), 1.53-1.11 (8H, m), 0.99 (3H, d, J = 6.5 Hz). | 431 | 429 |

Table 1-18

| | | | | | |
|---|---|---|---|---|---|
| 137 | 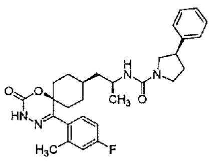 | | 1H-NMR (DMSO-D6) δ: 11.09 (1H, s), 7.36-7.17 (7H, m), 7.07 (1H, td, J = 8.6, 2.8 Hz), 5.66 (1H, d, J = 8.6 Hz), 3.80-3.71 (1H, m), 3.67-3.61 (1H, m), 3.44-3.12 (4H, m), 2.24 (3H, s), 2.22-2.14 (1H, m), 2.09-2.02 (2H, m), 1.96-1.84 (1H, m), 1.67-1.60 (1H, m), 1.33 (8H, d, J = 158.6 Hz), 1.00 (3H, d, J = 6.5 Hz). | 507 | 505 |
| 138 | 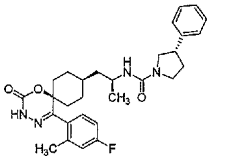 | | 1H-NMR (DMSO-D6) δ: 11.09 (1H, s), 7.36-7.16 (7H, m), 7.07 (1H, td, J = 8.5, 2.9 Hz), 5.67 (1H, d, J = 8.6 Hz), 3.80-3.71 (1H, m), 3.67 (1H, dd, J = 9.8, 7.5 Hz), 3.47-3.40 (1H, m), 3.37-3.18 (2H, m), 3.14-3.08 (1H, m), 2.24 (3H, s), 2.22-2.13 (1H, m), 2.09-2.01 (2H, m), 1.96-1.84 (1H, m), 1.67-1.60 (1H, m), 1.53-1.13 (8H, m), 1.00 (3H, d, J = 6.5 Hz). | 507 | 505 |
| 139 | 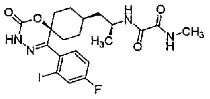 | | 1H-NMR (DMSO-D6) δ: 11.19 (1H, s), 8.67-8.60 (1H, m), 8.42 (1H, d, J = 9.5 Hz), 7.86 (1H, dd, J = 8.6, 2.5 Hz), 7.44 (1H, dd, J = 8.6, 5.8 Hz), 7.33 (1H, td, J = 8.4, 2.8 Hz), 3.97-3.83 (1H, m), 2.64 (3H, d, J = 4.9 Hz), 2.17-2.07 (2H, m), 1.70-1.59 (1H, m), 1.59-1.13 (8H, m), 1.05 (3H, d, J = 6.7 Hz). | 531 | 529 |
| 140 | 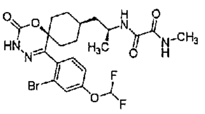 | | 1H-NMR (DMSO-D6) δ: 11.20 (1H, s), 8.63 (1H, d, J = 5.1 Hz), 8.41 (1H, d, J = 9.0 Hz), 7.60 (1H, d, J = 2.3 Hz), 7.52 (1H, d, J = 8.6 Hz), 7.36 (1H, t, J = 74.3 Hz), 7.27 (1H, dd, J = 8.4, 2.4 Hz), 3.97-3.82 (1H, m), 2.64 (3H, d, J = 4.9 Hz), 2.15-2.07 (2H, m), 1.70-1.59 (1H, m), 1.59-1.13 (8H, m), 1.05 (3H, d, J = 6.7 Hz). | 531 | 529 |
| 141 | 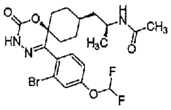 | | 1H-NMR (DMSO-D6) δ: 11.20 (1H, s), 7.61 (1H, d, J = 2.5 Hz), 7.57-7.53 (1H, m), 7.54 (1H, d, J = 8.6 Hz), 7.37 (1H, t, J = 74.0 Hz), 7.28 (1H, dd, J = 8.2, 2.4 Hz), 3.88-3.78 (1H, m), 2.19-2.06 (2H, m), 1.73 (3H, s), 1.67-1.59 (1H, m), 1.56-1.14 (8H, m), 0.97 (3H, d, J = 6.7 Hz). | 488 | 486 |
| 142 | 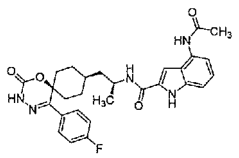 | | 1H-NMR (DMSO-D6) δ: 11.51 (1H, s), 11.11 (1H, s), 9.77 (1H, s), 8.17 (1H, d, J = 8.6 Hz), 7.53-7.46 (2H, m), 7.41 (1H, d, J = 7.4 Hz), 7.27-7.06 (5H, m), 4.25-4.13 (1H, m), 2.12 (3H, s), 2.06-1.96 (2H, m), 1.83-1.23 (9H, m), 1.15 (3H, d, J = 6.5 Hz). | 520 | 518 |
| 143 | 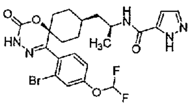 | | 1H-NMR (DMSO-D6) δ: 13.22-13.09 (1H, m), 11.20 (1H, s), 7.82-7.67 (2H, m), 7.58 (1H, d, J = 2.3 Hz), 7.51 (1H, d, J = 8.3 Hz), 7.35 (1H, t, J = 73.1 Hz), 7.24 (1H, dd, J = 8.7, 2.7 Hz), 6.63-6.53 (1H, m), 4.14-4.00 (1H, m), 2.19-2.06 (2H, m), 1.77-1.63 (1H, m), 1.63-1.17 (8H, m), 1.09 (3H, d, J = 6.5 Hz). | 540 | 538 |
| 144 | 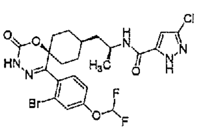 | | 1H-NMR (DMSO-D6) δ: 13.81-13.58 (1H, m), 11.20 (1H, s), 8.14 (1H, d, J = 8.6 Hz), 7.59 (1H, d, J = 2.3 Hz), 7.51 (1H, d, J = 8.3 Hz), 7.36 (1H, t, J = 72.8 Hz), 7.26 (1H, dd, J = 8.6, 2.3 Hz), 6.83 (1H, s), 4.13-3.98 (1H, m), 2.18-2.07 (2H, m), 1.73-1.63 (1H, m), 1.55-1.20 (8H, m), 1.10 (3H, d, J = 6.5 Hz), | 574 | 572 |

Table 1-19

| | | | | | |
|---|---|---|---|---|---|
| 145 | 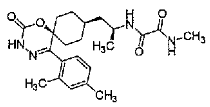 | | 1H-NMR (DMSO-D6) δ: 11.01 (1H, s), 8.65-8.60 (1H, m), 8.41 (1H, d, J = 9.2 Hz), 7.13 (1H, d, J = 7.6 Hz), 7.12-7.10 (1H, m), 7.04 (1H, d, J = 7.6 Hz), 3.95-3.83 (1H, m), 2.64 (3H, d, J = 4.9 Hz), 2.29 (3H, s), 2.18 (3H, s), 2.05-1.97 (2H, m), 1.68-1.60 (1H, m), 1.57-1.10 (8H, m), 1.05 (3H, d, J = 6.5 Hz). | 415 | 413 |
| 146 | 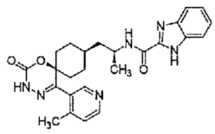 | | 1H-NMR (DMSO-D6) δ: 13.14 (1H, s), 11.20 (1H, s), 8.61 (1H, d, J = 6.7 Hz), 8.47-8.39 (2H, m), 7.68 (1H, d, J = 6.7 Hz), 7.50 (1H, d, J = 6.7 Hz), 7.34-7.20 (3H, m), 4.22-4.08 (1H, m), 2.23 (3H, s), 2.12-2.00 (2H, m), 1.80-1.69 (1H, m), 1.69-1.57 (1H, m), 1.57-1.20 (7H, m), 1.16 (3H, d, J = 6.0 Hz). | 461 | 459 |
| 147 | 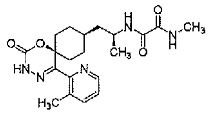 | | 1H-NMR (DMSO-D6) δ: 11.14 (1H, s), 8.66 (1H, q, J = 4.5 Hz), 8.50-8.41 (2H, m), 7.75 (1H, d, J = 7.9 Hz), 7.35 (1H, dd, J = 7.9, 4.9 Hz), 4.00-3.85 (1H, m), 2.66 (3H, d, J = 4.5 Hz), 2.31 (3H, s), 2.02-1.90 (2H, m), 1.88-1.42 (5H, m), 1.37-1.16 (4H, m), 1.07 (3H, d, J = 6.0 Hz). | 402 | 400 |
| 148 | 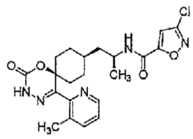 | | 1H-NMR (DMSO-D6) δ: 11.15 (1H, s), 8.80 (1H, d, J = 9.0 Hz), 8.43 (1H, d, J = 4.5 Hz), 7.75 (1H, d, J = 7.5 Hz), 7.39-7.31 (2H, m), 4.15-4.01 (1H, m), 2.30 (3H, s), 2.02-1.92 (2H, m), 1.87-1.71 (2H, m), 1.69-1.61 (1H, m), 1.58-1.46 (2H, m), 1.38-1.21 (4H, m), 1.13 (3H, d, J = 6.7 Hz). | 446 | 444 |
| 149 | 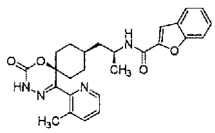 | | 1H-NMR (DMSO-D6) δ: 11.15 (1H, s), 8.44 (1H, d, J = 8.2 Hz), 8.40 (1H, d, J = 4.5 Hz), 7.76 (1H, d, J = 8.2 Hz), 7.73 (1H, d, J = 8.2 Hz), 7.65 (1H, d, J = 8.2 Hz), 7.51 (1H, s), 7.46 (1H, t, J = 7.9 Hz), 7.38-7.28 (2H, m), 4.23-4.08 (1H, m), 2.30 (3H, s), 2.04-1.92 (2H, m), 1.88-1.65 (3H, m), 1.65-1,47 (2H, m), 1.41-1.21 (4H, m), 1.15 (3H, d, J = 6.0 Hz). | 461 | 459 |
| 150 | 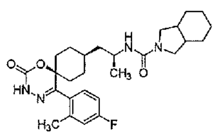 | Mixture of three stereoisomers due to octahydro-1H-isoindole moiety | 1H-NMR (DMSO-D6) δ: 11.08 (1H, s), 7.34 (1H, dd, J = 8.7, 5.9 Hz), 7.18 (1H, dd, J = 10.1, 2.7 Hz), 7.07 (1H, td, J = 8.7, 2.7 Hz), 5.50 (1H, d, J = 8.6 Hz), 3.75-3.66 (1H, m), 3.44-3.35 (2H, m), 2.70-2.61 (2H, m), 2.24 (3H, s), 2.08-2.00 (2H, m), 1.84-1.68 (4H, m), 1.64-1.57 (1H, m), 1.52-1.01 (14H, m), 0.97 (3H, d, J = 6.5 Hz). | 485 | 483 |
| 151 | 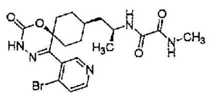 | | 1H-NMR (DMSO-D6) δ: 11.32 (1H, s), 8.65-8.59 (1H, m), 8.60 (1H, s), 8.50 (1H, d, J = 5.5 Hz), 8.41 (1H, d, J = 9.0 Hz), 7.85 (1H, d, J = 5.3 Hz), 3,97-3,84 (1H, m), 2.63 (3H, d, J = 4.9 Hz), 2.17-2.08 (2H, m), 1.71-1.60 (1H, m), 1.58-1.17 (8H, m), 1.05 (3H, d, J = 6.5 Hz). | 466 | 464 |

(continued)

| | | | | | |
|---|---|---|---|---|---|
| 152 | 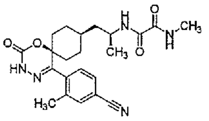 | | 1H-NMR (DMSO-D6) δ: 11.21 (1H, s), 8.62 (1H, q, J = 5.2 Hz), 8.41 (1H, d, J = 8.3 Hz), 7.83 (1H, s), 7.71 (1H, d, J = 8.3 Hz), 7.51 (1H, d, J = 8.3 Hz), 3.95-3.85 (1H, m), 2.64 (3H, d, J = 5.2 Hz), 2.27 (3H, s), 2.09-2.01 (2H, m), 1.68-1.61 (1H, m), 1.54-1.37 (3H, m), 1.35-1.16 (5H, m), 1.05 (3H, d, J = 6.7 Hz). | 426 | 424 |

Table 1-20

| | | | | | |
|---|---|---|---|---|---|
| 153 | 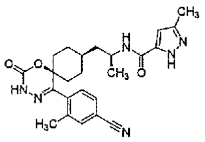 | | 1H-NMR (DMSO-D6) δ: 12.80 (1H, s), 11.20 (1H, s), 7.81 (1H, s), 7.68 (1H, d, J = 8.1 Hz), 7.58 (1H, d, J = 8.1 Hz), 7.50 (1H, d, J = 8.1 Hz), 6.30 (1H, s), 4.09-4.00 (1H, m), 2.26 (3H, s), 2.23 (3H, s), 2.10-2.01 (2H, m), 1.71-1.65 (1H, m), 1.55-1.20 (8H, m), 1.07 (3H, d, J = 6.2 Hz). | 449 | 447 |
| 154 | 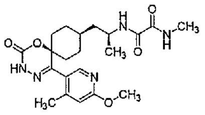 | | 1H-NMR (DMSO-D6) δ: 11.13 (1H, s), 8.63 (1H, q, J = 4.5 Hz), 8.41 (1H, d, J = 9.7 Hz), 8.07 (1H, s), 6.77 (1H, s), 3.98-3.83 (1H, m), 3.86 (3H, s), 2.64 (3H, d, J = 4.5 Hz), 2.19 (3H, s), 2.09-1.96 (2H, m), 1.72-1.59 (1H, m), 1.57-1.16 (8H, m), 1.06 (3H, d, J = 6.7 Hz). | 432 | 430 |
| 155 | 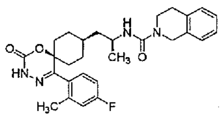 | | 1H-NMR (DMSO-D6) δ: 11.08 (1H, s), 7.31 (1H, dd, J = 8.6, 6.0 Hz), 7.21-7.04 (6H, m), 6.12 (1H, d, J = 8.3 Hz), 4.43 (2H, s), 3.84-3.73 (1H, m), 3.57-3.44 (2H, m), 2.75-2.66 (2H, m), 2.23 (3H, s), 2.05-1.97 (2H, m), 1.65-1.14 (9H, m), 1.01 (3H, d, J = 6.7 Hz). | 493 | 491 |
| 156 | 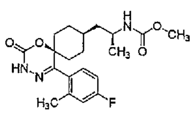 | | 1H-NMR (DMSO-D6) δ: 11.09 (1H, s), 7.34 (1H, dd, J = 8.4, 5.9 Hz), 7.18 (1H, dd, J = 10.1, 2.7 Hz), 7.07 (1H, td, J = 8.4, 2.7 Hz), 6.88 (1H, d, J = 8.6 Hz), 3.59-3.43 (1H, m), 3.47 (3H, s), 2.24 (3H, s), 2.08-2.00 (2H, m), 1.63-1.12 (9H, m), 0.99 (3H, d, J = 6.5 Hz). | 392 | 390 |
| 157 | 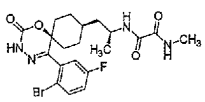 | | 1H-NMR (DMSO-D6) δ: 11.22 (1H, s), 8.65-8.59 (1H, m), 8.41 (1H, d, J = 9.0 Hz), 7.77 (1H, dd, J = 8.9, 5.2 Hz), 7.43 (1H, dd, J = 9.0, 3.0 Hz), 7.30 (1H, td, J = 8.6, 3.0 Hz), 3.97-3.83 (1H, m), 2.64 (3H, d, J = 4.9 Hz), 2.19-2.06 (2H, m), 1.70-1.16 (9H, m), 1.06 (3H, d, J = 6.7 Hz). | 483 | 481 |
| 158 | 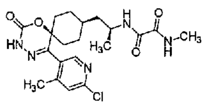 | | 1H-NMR (DMSO-D6) δ: 11.26 (1H, s), 8.65-8.59 (1H, m), 8.41 (1H, d, J = 9.2 Hz), 8.33 (1H, s), 7.54 (1H, s), 3.98-3.86 (1H, m), 2.64 (3H, d, J = 4.9 Hz), 2.26 (3H, s), 2.10-2.00 (2H, m), 1.70-1.61 (1H, m), 1.58-1.14 (8H, m), 1.06 (3H, d, J = 6.7 Hz). | 436 | 434 |
| 159 | 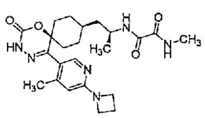 | | 1H-NMR (DMSO-D6) δ: 11.02 (1H, s), 8.66-8.60 (1H, m), 8.41 (1H, d, J = 9.0 Hz), 7.90 (1H, s), 6.24 (1H, s), 3.98-3.87 (1H, m), 3.94 (4H, t, J = 7.4 Hz), 2.65 (3H, d, J = 5.1 Hz), 2.36-2.27 (2H, m), 2.12 (3H, s), 2.03-1.94 (2H, m), 1.71-1.60 (1H, m), 1.57-1.14 (8H, m), 1.06 (3H, d, J = 6.5 Hz). | 457 | 501 (formic acid adduct) |

(continued)

| | | | | | |
|---|---|---|---|---|---|
| 160 | 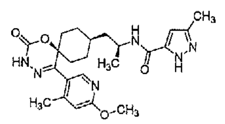 | | 1H-NMR (DMSO-D6) δ: 12.80 (1H, s), 11.13 (1H, s), 8.05 (1H, s), 7.58 (1H, d, J = 8.6 Hz), 6.76 (1H, s), 6.31 (1H, s), 4.14-3.98 (1H, m), 3.84 (3H, s), 2.23 (3H, s), 2.18 (3H, s), 2.07-1.99 (2H, m), 1.73-1.64 (1H, m), 1.57-1.16 (8H, m), 1.08 (3H, d, J = 6.5 Hz). | 455 | 453 |

Table 1-21

| | | | | | |
|---|---|---|---|---|---|
| 161 | 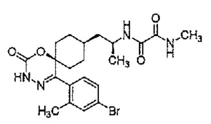 | | 1H-NMR (DMSO-D6) δ: 11.11 (1H, s), 8.62 (1H, q, J = 4.7 Hz), 8.41 (1H, d, J = 9.2 Hz), 7.56 (1H, d, J = 1.8 Hz), 7.43 (1H, dd, J = 8.1, 1.8 Hz), 7.23 (1H, d, J = 8.1 Hz), 3.95-3.86 (1H, m), 2.64 (3H, d, J = 4.7 Hz), 2.22 (3H, s), 2.05-1.98 (2H, m), 1.68-1.63 (1H, m), 1.55-1.36 (3H, m), 1.34-1.15 (5H, m), 1.05 (3H, d, J = 6.5 Hz). | 479 | 477 |
| 162 | 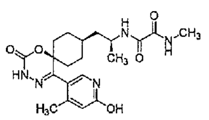 | | 1H-NMR (DMSO-D6) δ: 11.64 (1H, s), 11.07 (1H, s), 8.64 (1H, q, J = 4.5 Hz), 8.42 (1H, d, J = 9.7 Hz), 7.36 (1H, s), 6.24 (1H, s), 3.99-3.85 (1H, m), 2.65 (3H, d, J = 4.5 Hz), 2.02 (3H, s), 2.02-1.93 (2H, m), 1.71-1.62 (1H, m), 1.59-1.38 (4H, m), 1.33-1.13 (4H, m), 1.07 (3H, d, J = 6.7 Hz). | 418 | 416 |
| 163 | 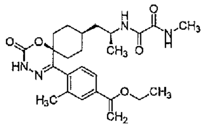 | | 1H-NMR (DMSO-D6) δ: 11.07 (1H, s), 8.62 (1H, q, J = 4.7 Hz), 8.41 (1H, d, J = 9.2 Hz), 7.53 (1H, d, J = 1.8 Hz), 7.46 (1H, dd, J = 8.0, 1.8 Hz), 7.25 (1H, d, J = 8.0 Hz), 4.79 (1H, d, J = 2.8 Hz), 4.32 (1H, d, J = 2.8 Hz), 3.95-3.87 (1H, m), 3.90 (2H, q, J = 6.9 Hz), 2.64 (3H, d, J = 4.7 Hz), 2.24 (3H, s), 2.07-1,99 (2H, m), 1.69-1.62 (1H, m), 1.53-1.39 (3H, m), 1.35 (3H, t, J = 6.9 Hz), 1.31-1.15 (5H, m), 1.06 (3H, d, J = 6.9 Hz). | 471 | 469 |
| 164 | 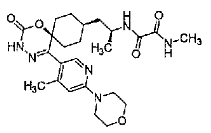 | | 1H-NMR (DMSO-D6) δ: 11.04 (1H, s), 8.66-8.59 (1H, m), 8.41 (1H, d, J = 9.0 Hz), 7.99 (1H, s), 6.75 (1H, s), 3.97-3.85 (1H, m), 3.71-3.66 (4H, m), 3.50-3.43 (4H, m), 2.64 (3H, d, J = 4.9 Hz), 2.15 (3H, s), 2.04-1.96 (2H, m), 1.69-1.61 (1H, m), 1.57-1.17 (8H, m), 1.06 (3H, d, J = 6.7 Hz). | 487 | 485 |
| 165 | 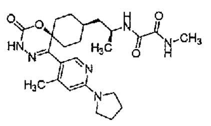 | | 1H-NMR (DMSO-D6) δ: 11.00 (1H, s), 8.65-8.60 (1H, m), 8.41 (1H, d, J = 9.2 Hz), 7.92 (1H, s), 6.33 (1H, s), 3.97-3.86 (1H, m), 3.41-3.35 (4H, m), 2.64 (3H, d, J = 4.9 Hz), 2.13 (3H, s), 2.04-1.90 (6H, m), 1.70-1.62 (1H, m), 1.58-1.15 (8H, m), 1.06 (3H, d, J = 6.5 Hz). | 471 | 469 |
| 166 | 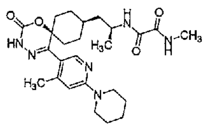 | | 1H-NMR (DMSO-D6) δ: 11.01 (1H, s), 8.66-8.61 (1H, m), 8.41 (1H, d, J = 9.0 Hz), 7.93 (1H, s), 6.71 (1H, s), 3.98-3.86 (1H, m), 3.57-3.49 (4H, m), 2.64 (3H, d, J = 4.9 Hz), 2.13 (3H, s), 2.03-1.95 (2H, m), 1.69-1.18 (15H, m), 1.06 (3H, d, J = 6.5 Hz). | 485 | 483 |
| 167 | 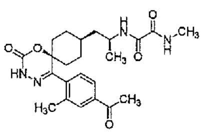 | | 1H-NMR (DMSO-D6) δ: 11.15 (1H, s), 8.62 (1H, q, J = 4.8 Hz), 8.41 (1H, d, J = 9.2 Hz), 7.89 (1H, d, J = 1.7 Hz), 7.80 (1H, dd, J = 8.1, 1.7 Hz), 7.43 (1H, d, J = 8.1 Hz), 3.95-3.85 (1H, m), 2.63 (3H, d, J = 4.8 Hz), 2.59 (3H, s), 2.30 (3H, s), 2.10-2.02 (2H, m), 1.68-1.61 (1H, m), 1.54-1.41 (3H, m), 1.34-1.17 (5H, m), 1.05 (3H, d, J = 5.8 Hz). | 443 | 441 |
| 168 | 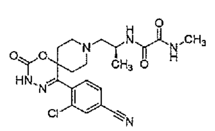 | | 1H-NMR (DMSO-D6) δ: 11.37 (1H, s), 8.64-8.58 (1H, m), 8.33 (1H, d, J = 8.5 Hz), 8.23 (1H, d, J = 1.5 Hz), 7.93 (1H, dd, J = 8.0, 1.5 Hz), 7.72 (1H, d, J = 8.0 Hz), 3.98-3.87 (1H, m), 2.73-2.59 (2H, m), 2.64 (3H, d, J = 5.0 Hz), 2.42 (1H, dd, J = 12.5, 8.3 Hz), 2.35-2.18 (3H, m), 2.12-2.01 (2H, m), 1.63-1.47 (2H, m), 1.04 (3H, d, J = 6.5 Hz). | 447 | 445 |

Table 1-22

| | | | | | |
|---|---|---|---|---|---|
| 169 | 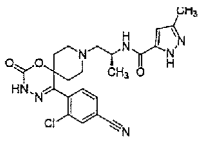 | | 1H-NMR (DMSO-D6) δ: 12,79 (1H, s), 11.36 (1H, s), 8.22 (1H, d, J = 1.3 Hz), 7.91 (1H, dd, J = 8.0, 1.5 Hz), 7.71 (1H, d, J = 8.0 Hz), 7.55 (1H, d, J = 8.0 Hz), 6.28 (1H, s), 4.10-4.00 (1H, m), 2.74-2.64 (2H, m), 2.46-2.38 (1H, m), 2.35-2.14 (6H, m), 2.07 (2H, d, J = 14.3 Hz), 1.61-1.49 (2H, m), 1.06 (3H, d, J = 6.5 Hz). | 470 | 468 |
| 170 | 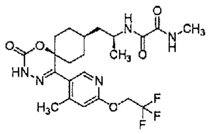 | | 1H-NMR (DMSO-D6) δ: 11.18 (1H, s), 8.62 (1H, q, J = 4.9 Hz), 8.41 (1H, d, J = 9.2 Hz), 8.12 (1H, s), 6.97 (1H, s), 5.00 (2H, q, J = 8.9 Hz), 3.99-3.84 (1H, m), 2.64 (3H, d, J = 4.9 Hz), 2.23 (3H, s), 2.10-1.97 (2H, m), 1.71-1.59 (1H, m), 1.57-1.43 (3H, m), 1.42-1.14 (5H, m), 1.06 (3H, d, J = 6.7 Hz). | 500 | 498 |
| 171 | 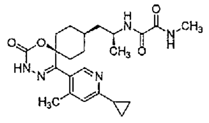 | | 1H-NMR (DMSO-D6) δ: 11.14 (1H, s), 8.63 (1H, q, J = 4.9 Hz), 8.40 (1H, d, J = 9.2 Hz), 8.25 (1H, s), 7.24 (1H, s), 3.97-3.84 (1H, m), 2.64 (3H, d, J = 4.9 Hz), 2.19 (3H, s), 2.11-1.97 (3H, m), 1.71-1.60 (1H, m), 1.56-1.41 (3H, m), 1.41-1.15 (5H, m), 1.06 (3H, d, J = 6.5 Hz), 0.99-0.89 (4H, m). | 442 | 440 |
| 172 | 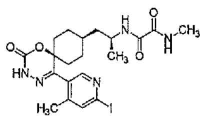 | | 1H-NMR (DMSO-D6) δ: 11.24 (1H, s), 8.62 (1H, q, J = 4.5 Hz), 8.41 (1H, d, J = 9.0 Hz), 8.25 (1H, s), 7.87 (1H, s), 3.98-3.84 (1H, m), 2.64 (3H, d, J = 4.5 Hz), 2.18 (3H, s), 2.09-1.97 (2H, m), 1.71-1.59 (1H, m), 1.57-1.15 (8H, m), 1.05 (3H, d, J = 6.7 Hz). | 528 | 526 |
| 173 | 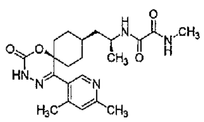 | | 1H-NMR (DMSO-D6) δ: 11.16 (1H, s), 8.66-8.59 (1H, m), 8.40 (1H, d, J = 9.0 Hz), 8.30 (1H, s), 7.21 (1H, s), 3.97-3.83 (1H, m), 2.64 (3H, d, J = 4.9 Hz), 2.44 (3H, s), 2.20 (3H, s), 2.10-1.98 (2H, m), 1.70-1,60 (1H, m), 1.57-1.17 (8H, m), 1.05 (3H, d, J = 6.5 Hz). | 416 | 414 |
| 174 | 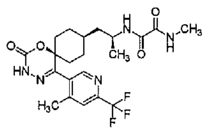 | | 1H-NMR (DMSO-D6) δ: 11.33 (1H, s), 8.69 (1H, s), 8.63 (1H, q, J = 4.5 Hz), 8.41 (1H, d, J = 8.2 Hz), 7.93 (1H, s), 3.98-3.84 (1H, m), 2.64 (3H, d, J = 4.5 Hz), 2.37 (3H, s), 2.14-2.04 (2H, m), 1.72-1.61 (1H, m), 1.58-1.15 (8H, m), 1.06 (3H, d, J = 6.0 Hz). | 470 | 468 |
| 175 | 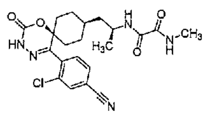 | | 1H-NMR (DMSO-D6) δ: 11.32 (1H, s), 8.67-8.59 (1H, m), 8.42 (1H, d, J = 9.2 Hz), 8.22 (1H, d, J = 1.6 Hz), 7.91 (1H, dd, J = 8.1, 1.6 Hz), 7.70 (1H, d, J = 8.1 Hz), 3.95-3.85 (1H, m), 2.64 (3H, d, J = 4.8 Hz), 2.10 (2H, d, J = 11.3 Hz), 1.69-1.62 (1H, m), 1.58-1.15 (8H, m), 1.05 (3H, d, J = 6.5 Hz). | 446 | 444 |
| 176 | 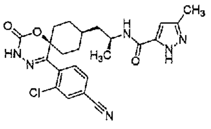 | | 1H-NMR (DMSO-D6) δ: 12.79 (1H, s), 11.30 (1H, s), 8.19 (1H, s), 7.88 (1H, d, J = 8.3 Hz), 7.69 (1H, d, J = 7.8 Hz), 7.63-7.53 (1H, m), 6.30 (1H, s), 4.12-4.00 (1H, m), 2.27-2.06 (5H, m), 1.90-1.83 (1H, m), 1.75-1.18 (8H, m), 1.07 (3H, d, J = 6.0 Hz). | 469 | 467 |

Table 1-23

| | | | | | |
|---|---|---|---|---|---|
| 177 | 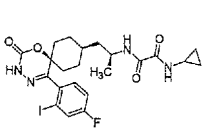 | | 1H-NMR (DMSO-D6) δ: 11.19 (1H, s), 8.68 (1H, d, J = 5.3 Hz), 8.42 (1H, d, J = 9.0 Hz), 7.86 (1H, dd, J = 8.4, 2.7 Hz), 7.44 (1H, dd, J = 8.7, 5.9 Hz), 7.33 (1H, td, J = 8.5, 2.6 Hz), 3.95-3.82 (1H, m), 2.78-2.70 (1H, m), 2.16-2.06 (2H, m), 1.69-1.60 (1H, m), 1.59-1.12 (8H, m), 1.05 (3H, d, J = 6.7 Hz), 0.65-0.56 (4H, m). | 557 | 555 |

(continued)

| | | | | | |
|---|---|---|---|---|---|
| 178 | 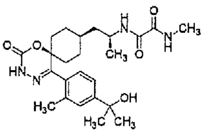 | | 1H-NMR (DMSO-D6) δ: 11.02 (1H, s), 8.63 (1H, q, J = 4.9 Hz), 8.41 (1H, d, J = 9.2 Hz), 7.38 (1H, d, J = 1.5 Hz), 7.31 (1H, dd, J = 8.1, 1.5 Hz), 7.17 (1H, d, J = 8.1 Hz), 5.01 (1H, s), 3.95-3.85 (1H, m), 2.63 (3H, d, J = 4.9 Hz), 2.21 (3H, s), 2.06-1.98 (2H, m), 1.68-1.62 (1H, m), 1.54-1.43 (3H, m), 1.42 (6H, s), 1.33-1.16 (5H, m), 1.05 (3H, d, J = 6.5 Hz). | 459 | 457 |
| 179 | 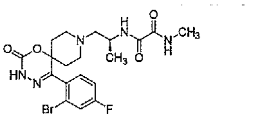 | | 1H-NMR (DMSO-D6) δ: 11.25 (1H, s), 8.63 (1H, q, J = 4.7 Hz), 8.35 (1H, d, J = 8.8 Hz), 7.75 (1H, dd, J = 8.6, 2.5 Hz), 7.54 (1H, dd, J = 8.6, 5.8 Hz), 7.36 (1H, td, J = 8.6, 2.5 Hz), 3.96-3.89 (1H, m), 2.72-2.60 (2H, m), 2.64 (3H, d, J = 4.7 Hz), 2.42 (1H, dd, J = 12.5, 8.3 Hz), 2.33-2.19 (3H, m), 2.10-2.02 (2H, m), 1.64-1.48 (2H, m), 1.04 (3H, d, J = 6.5 Hz), | 484 | 482 |
| 180 | 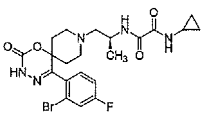 | | 1H-NMR (DMSO-D6) δ: 11.25 (1H, s), 8.68 (1H, d, J = 5.3 Hz), 8.35 (1H, d, J = 8.6 Hz), 7.75 (1H, dd, J = 8.6, 2.6 Hz), 7.54 (1H, dd, J = 8.6, 6.0 Hz), 7.36 (1H, td, J = 8.6, 2.6 Hz), 3.95-3.88 (1H, m), 2.75-2.57 (3H, m), 2.46-2.38 (1H, m), 2.33-2.19 (3H, m), 2.10-2.02 (2H, m), 1.62-1.49 (2H, m), 1.04 (3H, d, J = 6.5 Hz), 0.66-0.56 (4H, m). | 510 | 508 |
| 181 | 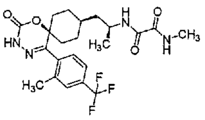 | | 1H-NMR (DMSO-D6) δ: 11.19 (1H, s), 8.66-8.59 (1H, m), 8.41 (1H, d, J = 9.2 Hz), 7.71 (1H, s), 7.59 (1H, d, J = 7.6 Hz), 7.52 (1H, d, J = 7.9 Hz), 3.95-3.85 (1H, m), 2.63 (3H, d, J = 4.9 Hz), 2.31 (3H, s), 2.10-2.02 (2H, m), 1.70-1.60 (1H, m), 1.57-1.14 (8H, m), 1.05 (3H, d, J = 6.7 Hz), | 557 | 555 |
| 182 | 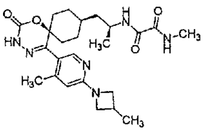 | | 1H-NMR (DMSO-D6) δ: 11.02 (1H, s), 8.66-8.60 (1H, m), 8.41 (1H, d, J = 9.2 Hz), 7.90 (1H, s), 6.23 (1H, s), 4.06 (2H, t, J = 8.4 Hz), 3.96-3.87 (1H, m), 3.51 (2H, dd, J = 7.9, 5.5 Hz), 2.83-2.72 (1H, m), 2.64 (3H, d, J = 4.9 Hz), 2.11 (3H, s), 2.04-1.94 (2H, m), 1.72-1.60 (1H, m), 1.57-1.14 (8H, m), 1.23 (3H, d, J = 6.7 Hz), 1.06 (3H, d, J = 6.7 Hz). | 557 | 555 |
| 183 | 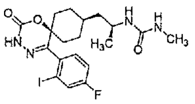 | | 1H-NMR (DMSO-D6) δ: 11.20 (1H, s), 7.87 (1H, dd, J = 8.4, 2.7 Hz), 7.46 (1H, dd, J = 8.7, 5,9 Hz), 7.34 (1H, td, J = 8.5, 2.6 Hz), 5.55 (1H, d, J = 8.6 Hz), 5.48 (1H, q, J = 4.5 Hz), 3.69-3.58 (1H, m), 2.50-2.48 (3H, m), 2.18-2.09 (2H, m), 1.67-1.58 (1H, m), 1.54-1,10 (8H, m), 0.96 (3H, d, J = 6.5 Hz). | 503 | 501 |
| 184 | 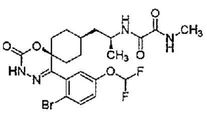 | | 1H-NMR (DMSO-D6) δ: 11.23 (1H, s), 8.62 (1H, q, J = 4.5 Hz), 8.42 (1H, d, J = 9.0 Hz), 7.78 (1H, d, J = 9.0 Hz), 7.32 (1H, t, J = 73.7 Hz), 7.28 (1H, d, J = 3.0 Hz), 7.23 (1H, dd, J = 9.0, 3.0 Hz), 3.97-3.83 (1H, m), 2.64 (3H, d, J = 4.5 Hz), 2.19-2.07 (2H, m), 1.70-1,60 (1H, m), 1.57-1.15 (8H, m), 1.06 (3H, d, J = 6.0 Hz). | 531 | 529 |

Table 1-24

| | | | | | |
|---|---|---|---|---|---|
| 185 | 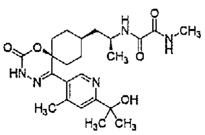 | | 1H-NMR (DMSO-D6) δ: 11.18 (1H, s), 8.63 (1H, q, J = 4.5 Hz), 8.41 (1H, d, J = 9.0 Hz), 8.36 (1H, s), 7.59 (1H, s), 5.22 (1H, s), 3.98-3.82 (1H, m), 2.63 (3H, d, J = 4.5 Hz), 2.25 (3H, s), 2.11-2.00 (2H, m), 1.72-1.61 (1H, m), 1.56-1.17 (8H, m), 1.44 (6H, s), 1.06 (3H, d, J = 6.7 Hz). | 460 | 458 |

(continued)

| | | | | | |
|---|---|---|---|---|---|
| 186 | 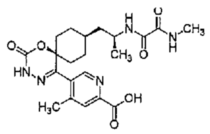 | | 1H-NMR (DMSO-D6) δ: 11.20 (1H, s), 8.62 (1H, q, J = 4.9 Hz), 8.41 (1H, d, J = 9.2 Hz), 8.25 (1H, s), 7.81 (1H, s), 3.98-3.83 (1H, m), 2.63 (3H, d, J = 4.9 Hz), 2.25 (3H, s), 2.11-2.00 (2H, m), 1.72-1.62 (1H, m), 1.56-1.38 (3H, m), 1.38-1.15 (5H, m), 1.05 (3H, d, J = 6.5 Hz). | 446 | 444 |
| 187 | 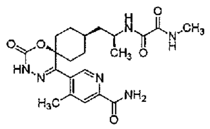 | | 1H-NMR (CD3OD) δ: 8.46 (1H, s), 8.04 (1H, s), 4.09-3.97 (1H, m), 2.78 (3H, s), 2.40 (3H, s), 2.25-2.14 (2H, m), 1.80-1.72 (1H, m), 1.63-1.24 (8H, m), 1.14 (3H, d, J = 6.5 Hz). | 445 | 443 |
| 188 | 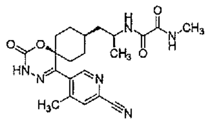 | | 1H-NMR (DMSO-D6) δ: 11.34 (1H, s), 8.67 (1H, s), 8.61 (1H, q, J = 4.9 Hz), 8.41 (1H, d, J = 9.2 Hz), 8.07 (1H, s), 3.97-3.83 (1H, m), 2.64 (3H, d, J = 4.9 Hz), 2.32 (3H, s), 2.13-2.03 (2H, m), 1.69-1.60 (1H, m), 1.58-1.14 (8H, m), 1.05 (3H, d, J = 6.7 Hz). | 427 | 425 |
| 189 | 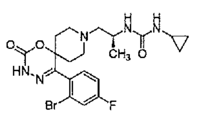 | | 1H-NMR (DMSO-D6) δ: 11.26 (1H, s), 7.76 (1H, dd, J = 8.6, 2.5 Hz), 7.56 (1H, dd, J = 8.6, 6.0 Hz), 7.38 (1H, td, J = 8.6, 2.5 Hz), 6.05 (1H, d, J = 1.8 Hz), 5.48 (1H, d, J = 7.2 Hz), 3.69-3.62 (1H, m), 2.70-2.61 (2H, m), 2.34-2.06 (7H, m), 1.69-1.50 (2H, m), 0.98 (3H, d, J = 6.5 Hz), 0.51-0.46 (2H, m), 0.26-0.21 (2H, m). | 482 | 480 |
| 190 | 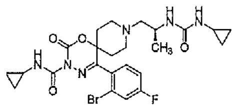 | | 1H-NMR (DMSO-D6) δ: 8.36 (1H, d, J = 3.7 Hz), 7.80 (1H, dd, J = 8.6, 2.5 Hz), 7.62 (1H, dd, J = 8.6, 6.0 Hz), 7.41 (1H, td, J = 8.6, 2.5 Hz), 6.04 (1H, s), 5.48 (1H, d, J = 6,9 Hz), 3.68-3.62 (1H, m), 2.72-2.65 (3H, m), 2.33-2.13 (7H, m), 1.70-1.56 (2H, m), 0.98 (3H, d, J = 6.5 Hz), 0.72-0.66 (2H, m), 0.56-0.46 (4H, m), 0.23-0.21 (2H, m). | 565 | 609 (formic acid adduct) |
| 191 | 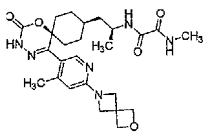 | | 1H-NMR (DMSO-D6) δ: 11.03 (1H, s), 8.66-8.60 (1H, m), 8.41 (1H, d, J = 9.0 Hz), 7.92 (1H, s), 6.28 (1H, s), 4.71 (4H, s), 4.11 (4H, s), 3.96-3.86 (1H, m), 2.64 (3H, d, J = 4.9 Hz), 2.12 (3H, s), 2.02-1.93 (2H, m), 1.72-1.62 (1H, m), 1.59-1.14 (8H, m), 1.06 (3H, d, J = 6.5 Hz). | 499 | 497 |
| 192 | 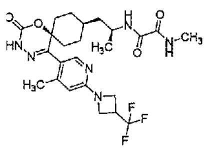 | | 1H-NMR (DMSO-D6) δ: 11.06 (1H, s), 8.65-8.60 (1H, m), 8.41 (1H, d, J = 9.0 Hz), 7.97 (1H, s), 6.39 (1H, s), 4.20 (2H, t, J = 8.9 Hz), 3.96 (2H, dd, J = 9.0, 4.5 Hz), 3.96-3.87 (1H, m), 3.80-3.65 (1H, m), 2.64 (3H, d, J = 4.9 Hz), 2.15 (3H, s), 2.04-1.95 (2H, m), 1.71-1.59 (1H, m), 1.59-1.13 (8H, m), 1.06 (3H, d, J = 6.5 Hz). | 525 | 523 |

Table 1-25

| | | | | | |
|---|---|---|---|---|---|
| 193 | 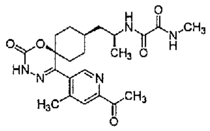 | | 1H-NMR (DMSO-D6) δ: 11.30 (1H, s), 8.67-8.58 (2H, m), 8.41 (1H, d, J = 9.2 Hz), 7.91 (1H, s), 3.99-3.82 (1H, m), 2.70-2.59 (6H, m), 2.34 (3H, s), 2.15-2.02 (2H, m), 1.71-1.60 (1H, m), 1.58-1.42 (3H, m), 1.42-1.16 (5H, m), 1.05 (3H, d, J = 6.5 Hz). | 444 | 442 |

(continued)

| | | | | | |
|---|---|---|---|---|---|
| 194 | 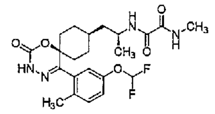 | | 1H-NMR (DMSO-D6) δ: 11.12 (1H, s), 8.62 (1H, q, J = 4.5 Hz), 8.41 (1H, d, J = 9.0 Hz), 7.35 (1H, d, J = 8.5 Hz), 7.24 (1H, t, J = 74.3 Hz), 7.15 (1H, d, J = 8.1 Hz), 7.06 (1H, s), 3.98-3.83 (1H, m), 2.63 (3H, d, J = 4.5 Hz), 2.19 (3H, s), 2.11-1.98 (2H, m), 1.71-1.59 (1H, m), 1.57-1.39 (3H, m), 1.39-1.16 (5H, m), 1.06 (3H, d, J = 6.7 Hz). | 467 | 465 |
| 195 | 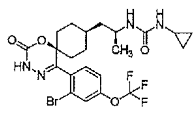 | | 1H-NMR (DMSO-D6) δ: 11.25 (1H, s), 7.86 (1H, d, J = 2.3 Hz), 7.63 (1H, d, J = 8.6 Hz), 7.50 (1H, d, J = 8.6 Hz), 5.88 (1H, d, J = 2.3 Hz), 5.45 (1H, d, J = 8.3 Hz), 3.70-3.62 (1H, m), 2.38-2.31 (1H, m), 2.18-2.10 (2H, m), 1.68-1.60 (1H, m), 1.53-1.13 (8H, m), 0.97 (3H, d, J = 6.2 Hz), 0.53-0.49 (2H, m), 0.27-0.23 (2H, m). | 547 | 545 |
| 196 | 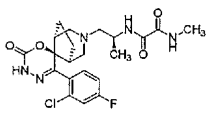 | | 1H-NMR (DMSO-D6) δ: 11.41 (1H, s), 8.64-8.57 (1H, m), 8.35 (1H, d, J = 8,3 Hz), 7.68 (1H, dd, J = 8.6, 6.1 Hz), 7.60 (1H, dd, J = 8.8, 2.8 Hz), 7.31 (1H, td, J = 8.6, 2.7 Hz), 3.98-3.86 (1H, m), 2.80-2.72 (1H, m), 2.70-2.31 (7H, m), 2.64 (3H, d, J = 5.0 Hz), 1.63-1.49 (2H, m), 1.18-1.02 (1H, m), 1.07 (3H, d, J = 6.5 Hz), 0.99-0,82 (1H, m). | 466 | 464 |
| 197 | 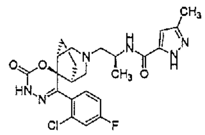 | | 1H-NMR (DMSO-D6) δ: 12.77 (1H, s), 11.40 (1H, s), 7.67 (1H, dd, J = 8.6, 6.1 Hz), 7.62-7.57 (2H, m), 7.30 (1H, td, J = 8.6, 2.5 Hz), 6.29 (1H, s), 4.11-3.98 (1H, m), 2.80-2.30 (8H, m), 2.22 (3H, s), 1.68-1.49 (2H, m), 1.18-1.02 (1H, m), 1.11 (3H, d, J = 6.5 Hz), 0.98-0.80 (1H, m). | 489 | 487 |
| 198 | 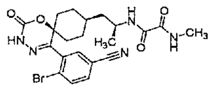 | | 1H-NMR (DMSO-D6) δ: 11.29 (1H, s), 8.65-8.59 (1H, m), 8.41 (1H, d, J = 9.0 Hz), 8.00 (1H, d, J = 1.8 Hz), 7.98 (1H, d, J = 8.3 Hz), 7.86 (1H, dd, J = 8.3, 1.8 Hz), 3.98-3.85 (1H, m), 2.64 (3H, d, J = 4.9 Hz), 2.16-2.04 (2H, m), 1.69-1.59 (1H, m), 1.59-1.13 (8H, m), 1.06 (3H, d, J = 6.5 Hz). | 490 | 488 |
| 199 | 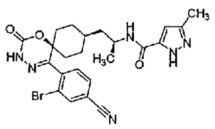 | | 1H-NMR (DMSO-D6) δ: 12.84-12.75 (1H, m), 11.30 (1H, s), 8.32 (1H, d, J = 1.6 Hz), 7.91 (1H, dd, J = 8.0, 1.6 Hz), 7.66 (1H, d, J = 8.1 Hz), 7.59 (1H, d, J = 9.2 Hz), 6.30 (1H, s), 4.11-3.96 (1H, m), 2.23 (3H, s), 2.19-2.07 (2H, m), 1.75-1.63 (1H, m), 1.61-1.15 (8H, m), 1.07 (3H, d, J = 6.5 Hz). | 513 | 511 |
| 200 | 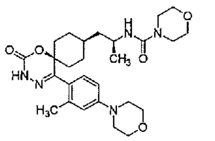 | | 1H-NMR (DMSO-D6) δ: 10.94 (1H, s), 7.09 (1H, d, J = 8.5 Hz), 6.84 (1H, d, J = 2.3 Hz), 6.78 (1H, dd, J = 8.5, 2.3 Hz), 6.08 (1H, d, J = 7.9 Hz), 3.78-3.76 (1H, m), 3.73 (4H, t, J = 4.7 Hz), 3.49 (4H, t, J = 4.7 Hz), 3.20 (4H, t, J = 4.7 Hz), 3.14 (4H, t, J = 4.7 Hz), 2.18 (3H, s), 2.05-1.97 (2H, m), 1.62-1.56 (1H, m), 1.50-1.35 (4H, m), 1.27-1.14 (4H, m), 0.99 (3H, d, J = 6.5 Hz). | 514 | 512 |

Table 1-26

| | | | | | |
|---|---|---|---|---|---|
| 201 | 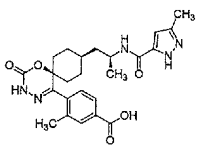 | | 1H-NMR (DMSO-D6) δ: 13.00-12.76 (2H, m), 11.14 (1H, s), 7.85 (1H, s), 7.76 (1H, d, J = 7.9 Hz), 7.68-7.56 (1H, m), 7.38 (1H, d, J = 7.9 Hz), 6.33 (1H, s), 4.10-3.98 (1H, m), 2.27 (3H, s), 2.21 (3H, s), 2.09-2.00 (2H, m), 1.71-1.65 (1H, m), 1.52-1.21 (8H, m), 1.07 (3H, d, J = 6.5 Hz). | 468 | 466 |

(continued)

| | | | | | |
|---|---|---|---|---|---|
| 202 | 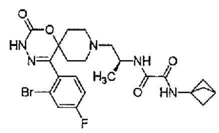 | | 1H-NMR (DMSO-D6) δ: 11.26 (1H, s), 9.15 (1H, s), 8.33 (1H, d, J = 9.0 Hz), 7.75 (1H, dd, J = 8.4, 2.5 Hz), 7.55 (1H, dd, J = 8.4, 6.1 Hz), 7.36 (1H, td, J = 8.4, 2.5 Hz), 3.94-3.87 (1H, m), 2.74-2.60 (2H, m), 2.44-2.38 (2H, m), 2.33-2.20 (3H, m), 2.09-2.03 (2H, m), 2.01 (6H, s), 1.63-1.50 (2H, m), 1.03 (3H, d, J = 6.5 Hz). | 536 | 534 |
| 203 | 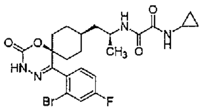 | | 1H-NMR (DMSO-D6) δ: 11.19 (1H, s), 8.68 (1H, d, J = 5.3 Hz), 8.41 (1H, d, J = 8.9 Hz), 7.73 (1H, dd, J = 8.9, 2.4 Hz), 7.52 (1H, dd, J = 8.9, 6.2 Hz), 7.34 (1H, td, J = 8.9, 2.4 Hz), 3.95-3.85 (1H, m), 2.78-2.70 (1H, m), 2.16-2.06 (2H, m), 1.68-1.61 (1H, m), 1.57-1.17 (8H, m), 1.05 (3H, d, J = 6.2 Hz), 0.65-0.58 (4H, m), | 509 | 507 |
| 204 | 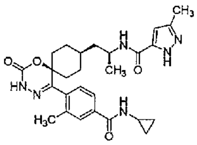 | | 1H-NMR (DMSO-D6) δ: 12.95 (0.15H, s), 12.80 (0.85H, s), 11.11 (1H, s), 8.42 (1H, d, J = 3.9 Hz), 7.93 (0.15H, s), 7.71 (1H, s), 7.62-7.57 (1.85H, m), 7.33 (1H, d, J = 7.9 Hz), 6.56 (0.15H, s), 6.30 (0.85H, s), 4.10-4.00 (1H, m), 2.86-2.80 (1H, m), 2.25 (3H, s), 2.23 (3H, s), 2.08-2.01 (2H, m), 1.71-1.65 (1H, m), 1.53-1.19 (8H, m), 1.07 (3H, d, J = 6.2 Hz), 0.71-0.66 (2H, m), 0.57-0.53 (2H, m), | 507 | 505 |
| 205 | 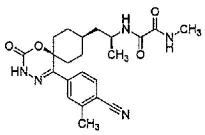 | | 1H-NMR (DMSO-D6) δ: 11.25 (1H, s), 8.65 (1H, q, J = 4.9 Hz), 8.44 (1H, d, J = 9.0 Hz), 7.81 (1H, d, J = 8.1 Hz), 7.56 (1H, s), 7.48 (1H, dd, J = 8.1, 1.4 Hz), 3.98-3.87 (1H, m), 2.66 (3H, d, J = 4.9 Hz), 2.51 (3H, s), 2.04-1.94 (2H, m), 1.77-1.46 (5H, m), 1.40-1.19 (4H, m), 1.08 (3H, d, J = 6.5 Hz). | 426 | 424 |
| 206 | 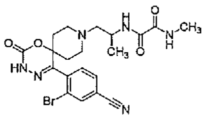 | | 1H-NMR (DMSO-D6) δ: 11.36 (1H, s), 8.65-8.57 (1H, m), 8.36 (1H, d, J = 1.5 Hz), 8.33 (1H, d, J = 8.8 Hz), 7.96 (1H, dd, J = 8.0, 1.5 Hz), 7.70 (1H, d, J = 8.0 Hz), 3.98-3.87 (1H, m), 2.75-2.58 (2H, m), 2.64 (3H, d, J = 5.0 Hz), 2.42 (1H, dd, J = 12.4, 8.4 Hz), 2.35-2.20 (3H, m), 2.13-2.04 (2H, m), 1.64-1.47 (2H, m), 1.04 (3H, d, J = 6.5 Hz). | 491 | 489 |
| 207 | 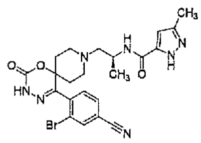 | | 1H-NMR (DMSO-D6) δ: 12.79 (1H, s), 11.35 (1H, s), 8.35 (1H, d, J = 1.5 Hz), 7.95 (1H, dd, J = 8.0, 1.5 Hz), 7.69 (1H, d, J = 7.8 Hz), 7.55 (1H, d, J = 8.5 Hz), 6.28 (1H, s), 4.10-4.00 (1H, m), 2.76-2.64 (2H, m), 2.46-2.37 (1H, m), 2.35-2.20 (6H, m), 2.09 (2H, d, J = 14.3 Hz), 1.63-1.47 (2H, m), 1.06 (3H, d, J = 6.8 Hz). | 514 | 512 |
| 208 | 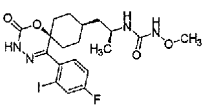 | | 1H-NMR (DMSO-D6) δ: 11.20 (1H, s), 8.88 (1H, s), 7.86 (1H, dd, J = 8.6, 2.5 Hz), 7.46 (1H, dd, J = 8.7, 5.9 Hz), 7.34 (1H, td, J = 8.6, 2.5 Hz), 6.49 (1H, d, J = 9.0 Hz), 3.84-3.70 (1H, m), 3.47 (3H, s), 2.19-2.06 (2H, m), 1.73-1.61 (1H, m), 1.57-1.14 (8H, m), 1.03 (3H, d, J = 6.7 Hz). | 519 | 517 |

Table 1-27

| | | | | | |
|---|---|---|---|---|---|
| 209 | 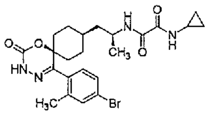 | | 1H-NMR (DMSO-D6) δ: 11.11 (1H, s), 8.68 (1H, d, J = 5.3 Hz), 8.41 (1H, d, J = 9.2 Hz), 7.56 (1H, d, J = 1.8 Hz), 7.43 (1H, dd, J = 8.2, 1.8 Hz), 7.23 (1H, d, J = 8.2 Hz), 3.93-3.84 (1H, m), 2,76-2.71 (1H, m), 2.22 (3H, s), 2.06-1.98 (2H, m), 1.68-1.60 (1H, m), 1.54-1.39 (3H, m), 1.36-1.16 (5H, m), 1.05 (3H, d, J = 6.5 Hz), 0.64-0.59 (4H, m), | 505 | 503 |

(continued)

| | | | | | |
|---|---|---|---|---|---|
| 210 | 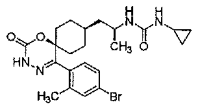 | | 1H-NMR (DMSO-D6) δ: 11.12 (1H, s), 7.57 (1H, d, J = 1.6 Hz), 7.44 (1H, dd, J = 8.0, 1.6 Hz), 7.25 (1H, d, J = 8.0 Hz), 5.88 (1H, d, J = 2.3 Hz), 5.45 (1H, d, J = 8.0 Hz), 3.69-3.63 (1H, m), 2.38-2.32 (1H, m), 2.23 (3H, s), 2.08-2.00 (2H, m), 1.66-1.59 (1H, m), 1.51-1.13 (8H, m), 0.97 (3H, d, J = 6.5 Hz), 0.54-0.50 (2H, m), 0.28-0.24 (2H, m). | 477 | 475 |
| 211 | 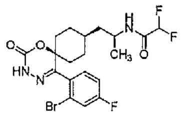 | | 1H-NMR (DMSO-D6) δ: 11.20 (1H, s), 8.52 (1H, d, J = 8.8 Hz), 7.73 (1H, dd, J = 8.8, 2.4 Hz), 7.53 (1H, dd, J = 8.8, 6.1 Hz), 7.35 (1H, td, J = 8.8, 2.4 Hz), 6.12 (1H, t, J = 53.8 Hz), 3.95-3.85 (1H, m), 2.18-2.09 (2H, m), 1.65-1.55 (1H, m), 1.51-1.18 (8H, m), 1.05 (3H, d, J = 6.5 Hz). | 476 | 474 |
| 212 | 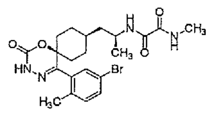 | | 1H-NMR (DMSO-D6) δ: 11.13 (1H, s), 8.63 (1H, q, J = 4.8 Hz), 8.41 (1H, d, J = 9.2 Hz), 7.53 (1H, dd, J = 8.2, 2.1 Hz), 7.46 (1H, d, J = 2.1 Hz), 7.27 (1H, d, J = 8.2 Hz), 3.94-3.87 (1H, m), 2.64 (3H, d, J = 4.8 Hz), 2.18 (3H, s), 2.09-2.01 (2H, m), 1.69-1.62 (1H, m), 1.53-1.17 (8H, m), 1.06 (3H, d, J = 6.5 Hz). | 479 | 477 |
| 213 | 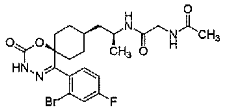 | | 1H-NMR (DMSO-D6) δ: 11.19 (1H, s), 7.96 (1H, t, J = 5.4 Hz), 7.74 (1H, dd, J = 9.4, 1.9 Hz), 7.60-7.49 (2H, m), 7.35 (1H, td, J = 8.4, 2.7 Hz), 3.91-3.78 (1H, m), 3.57 (2H, d, J = 5.4 Hz), 2.20-2.04 (2H, m), 1.79 (3H, s), 1.68-1.56 (1H, m), 1.52-1.12 (8H, m), 0.99 (3H, d, J = 7.0 Hz). | 497 | 495 |
| 214 | 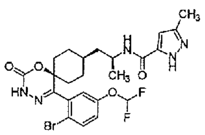 | | 1H-NMR (DMSO-D6) δ: 12.80 (1H, s), 11,22 (1H, s), 7.77 (1H, d, J = 8.6 Hz), 7.59 (1H, d, J = 9.2 Hz), 7.31 (1H, t, J = 73.5 Hz), 7.27 (1H, s), 7.21 (1H, dd, J =9.2, 1.6 Hz), 6.30 (1H, s), 4.12-3.98 (1H, m), 2.23 (3H, s), 2.19-2.07 (2H, m), 1.75-1.63 (1H, m), 1.58-1.18 (8H, m), 1.07 (3H, d, J = 6.5 Hz). | 554 | 552 |
| 215 | 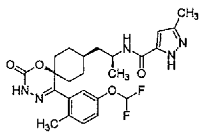 | | 1H-NMR (DMSO-D6) δ: 12.79 (1H, s), 11.12 (1H, s), 7.59 (1H, d, J = 8.1 Hz), 7.34 (1H, d, J = 8.6 Hz), 7.22 (1H, t, J = 74.1 Hz), 7.13 (1H, d, J = 8.6 Hz), 7.06 (1H, s), 6.30 (1H, s), 4.14-3.98 (1H, m), 2.22 (3H, s), 2.19 (3H, s), 2.11-1.99 (2H, m), 1.75-1.61 (1H, m), 1,59-1,16 (8H, m), 1.08 (3H, d, J = 6.5 Hz), | 490 | 488 |
| 216 | 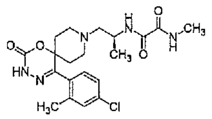 | | 1H-NMR (DMSO-D6) δ: 11.16 (1H, s), 8.65-8.58 (1H, m), 8.34 (1H, d, J = 8.8 Hz), 7.43 (1H, s), 7.35-7.28 (2H, m), 4.01-3.84 (1H, m), 2.73-2.59 (2H, m), 2.64 (3H, d, J = 4.8 Hz), 2.42 (1H, dd, J = 12.6, 8.4 Hz), 2.35-2.19 (3H, m), 2.22 (3H, s), 2.03-1.95 (2H, m), 1.64-1.45 (2H, m), 1.04 (3H, d, J = 6.5 Hz). | 436 | 434 |

Table 1-28

| | | | | | |
|---|---|---|---|---|---|
| 217 | 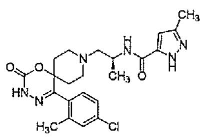 | | 1H-NMR (DMSO-D6) δ: 12.79 (1H, s), 11,16 (1H, s), 7.55 (1H, d, J = 7.5 Hz), 7.42 (1H, s), 7.33-7.25 (2H, m), 6.29 (1H, s), 4.12-3.99 (1H, m), 2.74-2.63 (2H, m), 2.41 (1H, dd, J = 12.3, 8.0 Hz), 2.35-2.14 (9H, m), 1.99 (2H, d, J = 13.5 Hz), 1.62-1.49 (2H, m), 1.07 (3H, d, J = 6.5 Hz), | 459 | 457 |
| 218 | 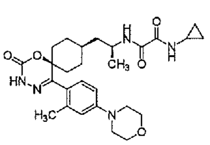 | | 1H-NMR (DMSO-D6) δ: 10.94 (1H, s), 8.69 (1H, d, J = 5.3 Hz), 8.41 (1H, d, J = 9.0 Hz), 7.08 (1H, d, J = 9.0 Hz), 6.83 (1H, d, J = 2.3 Hz), 6.78 (1H, dd, J = 9.0, 2.3 Hz), 3.94-3.85 (1H, m), 3.72 (4H, dd, J = 5.5, 4.2 Hz), 3.14 (4H, dd, J = 5.5, 4.2 Hz), 2.76-2.70 (1H, m), 2.17 (3H, s), 2.03-1.95 (2H, m), 1.68-1.61 (1H, m), 1.53-1.16 (8H, m), 1.05 (3H, d, J = 6.5 Hz), 0.63-0.58 (4H, m). | 512 | 510 |

(continued)

| | | | | | |
|---|---|---|---|---|---|
| 219 | 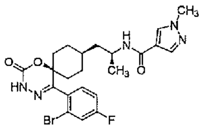 | | 1H-NMR (DMSO-D6) δ: 11.19 (1H, s), 8.06 (1H, s), 7.78 (1H, s), 7.72 (1H, dd, J = 8.6, 2.5 Hz), 7.65 (1H, d, J = 8.6 Hz), 7.51 (1H, dd, J = 8.6, 6.0 Hz), 7.32 (1H, td, J = 8.6, 2.5 Hz), 4.08-3.99 (1H, m), 3.82 (3H, s), 2.15-2.08 (2H, m), 1.70-1.63 (1H, m), 1.53-1.20 (8H, m), 1.06 (3H, d, J = 6.5 Hz), | 506 | 504 |
| 220 | 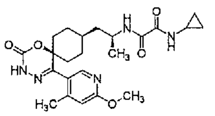 | | 1H-NMR (DMSO-D6) δ: 11.13 (1H, s), 8.68 (1H, d, J = 5.5 Hz), 8.41 (1H, d, J = 9.2 Hz), 8.07 (1H, s), 6.77 (1H, s), 3.95-3.83 (1H, m), 3.86 (3H, s), 2.78-2.69 (1H, m), 2.19 (3H, s), 2.07-1.97 (2H, m), 1.69-1.14 (9H, m), 1.06 (3H, d, J = 6.7 Hz), 0.66-0.56 (4H, m), | 458 | 456 |
| 221 | 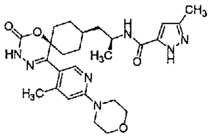 | | 1H-NMR (DMSO-D6) δ: 13.01-12.72 (1H, m), 11.04 (1H, s), 7.97 (1H, s), 7.63-7.55 (1H, m), 6.73 (1H, s), 6.31 (1H, s), 4.13-4.00 (1H, m), 3.67 (4H, t, J = 4.7 Hz), 3.64-3.56 (1H, m), 3.46 (4H, t, J = 4.9 Hz), 2.26-2.19 (2H, m), 2.14 (3H, s), 2.05-1.96 (2H, m), 1.75-1.64 (1H, m), 1.56-1.20 (8H, m), 1.08 (3H, d, J = 6.5 Hz). | 510 | 508 |
| 222 | 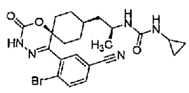 | | 1H-NMR (DMSO-D6) δ: 11.30 (1H, s), 8.03 (1H, d, J = 1.8 Hz), 7.99 (1H, d, J = 8.3 Hz), 7.87 (1H, dd, J = 8.4, 2.0 Hz), 5.89 (1H, d, J = 2.8 Hz), 5.46 (1H, d, J = 8.6 Hz), 3.72-3.61 (1H, m), 2.40-2.31 (1H, m), 2.19-2.07 (2H, m), 1.70-1.57 (1H, m), 1.56-1.06 (BH, m), 0.97 (3H, d, J = 6.5 Hz), 0.56-0.46 (2H, m), 0.31-0.22 (2H, m). | 488 | 486 |
| 223 | 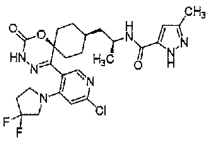 | | 1H-NMR (DMSO-D6) δ: 12.80 (1H, s), 11.30 (1H, s), 7.86 (1H, s), 7.63-7.54 (1H, m), 6.75 (1H, s), 6.31 (1H, s), 4.12-3.99 (1H, m), 3.71 (2H, t, J = 12.8 Hz), 3.49 (2H, t, J = 7.3 Hz), 2.60-2.51 (2H, m), 2.26-2.05 (1H, m), 2.23 (3H, s), 1.96-1.76 (1H, m), 1.74-1.62 (1H, m), 1.60-1.14 (8H, m), 1.08 (3H, d, J = 6.5 Hz). | 550 | 548 |
| 224 | 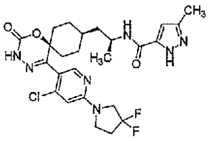 | | 1H-NMR (DMSO-D6) δ: 12.80 (1H, s), 11.15 (1H, s), 8.10 (1H, s), 7.59 (1H, d, J = 8.3 Hz), 6.75 (1H, s), 6.30 (1H, s), 4.11-4.00 (1H, m), 3.86 (2H, t, J = 13.1 Hz), 3.65 (2H, t, J = 7.4 Hz), 2.60-2.50 (2H, m), 2.23 (3H, s), 2.11-1.98 (2H, m), 1.74-1.62 (1H, m), 1.59-1.12 (8H, m), 1.08 (3H, d, J = 6.5 Hz). | 550 | 548 |

Table 1-29

| | | | | | |
|---|---|---|---|---|---|
| 225 | 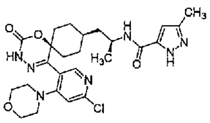 | | 1H-NMR (DMSO-D6) δ: 12.80 (1H, s), 11.25 (1H, s), 7.96 (1H, s), 7.58 (1H, d, J = 8.3 Hz), 7.08 (1H, s), 6.30 (1H, s), 4.11-3.98 (1H, m), 3.61 (4H, t, J = 4.5 Hz), 3.18-3.05 (4H, m), 2.23 (3H, s), 2.13-1.99 (2H, m), 1.72-1.63 (1H, m), 1.55-1.14 (8H, m), 1.08 (3H, d, J = 6.5 Hz). | 530 | 528 |
| 226 | 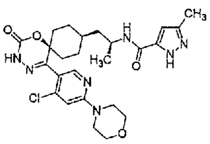 | | 1H-NMR (DMSO-D6) δ: 12.80 (1H, s), 11.15 (1H, s), 8.09 (1H, s), 7.59 (1H, d, J = 8.3 Hz), 7.00 (1H, s), 6.31 (1H, s), 4.11-3.99 (1H, m), 3.67 (4H, t, J = 4.7 Hz), 3.51 (4H, t, J = 4.9 Hz), 2.23 (3H, s), 2.10-2.00 (2H, m), 1.73-1.63 (1H, m), 1.57-1.15 (8H, m), 1.08 (3H, d, J = 6.5 Hz). | 530 | 528 |

(continued)

| | | | | | |
|---|---|---|---|---|---|
| 227 | 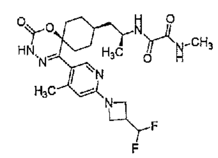 | | 1H-NMR (DMSO-D6) δ: 11.08 (1H, s), 8.66-8.60 (1H, m), 8.41 (1H, d, J = 9.2 Hz), 7.95 (1H, s), 6.46-6.37 (1H, m), 6.38 (1H, td, J = 56.4, 4.3 Hz), 4.10 (2H, t, J = 8.3 Hz), 3.97-3.86 (3H, m), 3.32-3.14 (1H, m), 2.64 (3H, d, J = 4.9 Hz), 2.15 (3H, s), 2.05-1.95 (2H, m), 1.71-1.60 (1H, m), 1.59-1.14 (8H, m), 1.06 (3H, d, J = 6.5 Hz). | 507 | 505 |
| 228 | 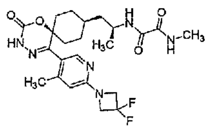 | | 1H-NMR (DMSO-D6) δ: 11.08 (1H, s), 8.66-8.60 (1H, m), 8.41 (1H, d, J = 9.2 Hz), 8.01 (1H, s), 6.50 (1H, s), 4.39 (4H, t, J = 12.5 Hz), 3.99-3.85 (1H, m), 2.64 (3H, d, J = 4.9 Hz), 2.16 (3H, s), 2.06-1.98 (2H, m), 1.70-1.60 (1H, m), 1.59-1.15 (8H, m), 1.06 (3H, d, J = 6.7 Hz). | 507 | 505 |
| 229 | 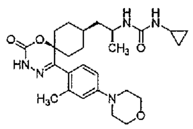 | | 1H-NMR (DMSO-D6) δ: 10.94 (1H, s), 7.09 (1H, d, J = 8.6 Hz), 6.84 (1H, d, J = 2.7 Hz), 6.78 (1H, dd, J = 8.6, 2.7 Hz), 5.88 (1H, d, J = 2.7 Hz), 5.46 (1H, d, J = 8.6 Hz), 3.73 (4H, dd, J = 5.8, 4.4 Hz), 3.69-3.64 (1H, m), 3.14 (4H, dd, J = 5.8, 4.4 Hz), 2.37-2.33 (1H, m), 2.18 (3H, s), 2.05-1.96 (2H, m), 1.65-1.58 (1H, m), 1.51-1.36 (3H, m), 1.31-1.15 (5H, m), 0.97 (3H, d, J = 6.5 Hz), 0.54-0.50 (2H, m), 0.28-0.23 (2H, m), | 484 | 482 |
| 230 | 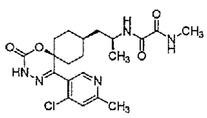 | | 1H-NMR (DMSO-D6) δ: 11.28 (1H, s), 8.62 (1H, q, J = 4.8 Hz), 8.48 (1H, s), 8.41 (1H, d, J = 9.2 Hz), 7.58 (1H, s), 3.98-3.83 (1H, m), 2.64 (3H, d, J = 4.8 Hz), 2.51 (3H, s), 2.15-2.02 (2H, m), 1.71-1.59 (1H, m), 1.57-1.15 (8H, m), 1.05 (3H, d, J = 6.5 Hz). | 436 | 434 |
| 231 | 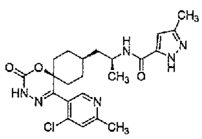 | | 1H-NMR (DMSO-D6) δ: 12.80 (1H, s), 11.28 (1H, s), 8.47 (1H, s), 7.65-7.51 (1H, m), 7.56 (1H, s), 6.31 (1H, s), 4.12-4.00 (1H, m), 2.50 (3H, s), 2.22 (3H, s), 2.14-2.02 (2H, m), 1.74-1.63 (1H, m), 1.55-1.15 (8H, m), 1.07 (3H, d, J = 6.7 Hz). | 459 | 457 |
| 232 | 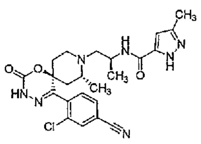 | | 1H-NMR (DMSO-D6) δ: 12.75 (1H, s), 11.34 (1H, s), 8.21 (1H, s), 7.90 (1H, d, J = 7.5 Hz), 7.68 (1H, d, J = 8.0 Hz), 7.46 (1H, d, J = 8.0 Hz), 6.22 (1H, s), 4.03-3,91 (1H, m), 2.92-2.82 (1H, m), 2.80-2.71 (1H, m), 2.41-2.30 (3H, m), 2.25 (3H, s), 2.00-1.89 (2H, m), 1.83-1.75 (1H, m), 1.68-1.56 (1H, m), 1.08 (3H, d, J = 6.8 Hz), 1.05 (3H, d, J = 6.5 Hz). | 484 | 482 |

Table 1-30

| | | | | | |
|---|---|---|---|---|---|
| 233 | 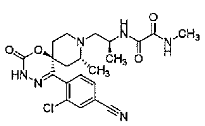 | | 1H-NMR (DMSO-D6) δ: 11.35 (1H, s), 8.60-8,53 (1H, m), 8.28-8.21 (2H, m), 7.92 (1H, dd, J = 8.0, 1.5 Hz), 7.71 (1H, d, J = 8.0 Hz), 3.90-3.76 (1H, m), 2.90-2.82 (1H, m), 2.77-2.69 (1H, m), 2.64 (3H, d, J = 5.0 Hz), 2.40-2.29 (3H, m), 2.00-1.89 (2H, m), 1.85-1.75 (1H, m), 1.63-1.52 (1H, m), 1.07 (3H, d, J = 6.8 Hz), 1.03 (3H, d, J = 6.5 Hz). | 461 | 459 |
| 234 | 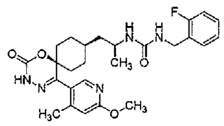 | | 1H-NMR (DMSO-D6) δ: 11.14 (1H, s), 8.08 (1H, s), 7.25-7.18 (2H, m), 7.14-7.08 (1H, m), 6.99-6.94 (1H, m), 6.81 (1H, s), 6.11 (1H, t, J = 6.1 Hz), 5.74 (1H, d, J = 8.8 Hz), 4.24 (1H, dd, J = 15.7, 6.1 Hz), 4.16 (1H, dd, J = 15.7, 6.1 Hz), 3.87 (3H, s), 3.74-3.66 (1H, m), 2.20 (3H, s), 2.10-1.99 (2H, m), 1.68-1.61 (1H, m), 1.52-1.13 (8H, m), 0.99 (3H, d, J = 6.5 Hz), | 498 | 496 |

(continued)

| | | | | | |
|---|---|---|---|---|---|
| 235 | 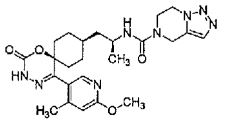 | | 1H-NMR (DMSO-D6) δ: 11.14 (1H, s), 8.07 (1H, s), 7.58 (1H, s), 6.78 (1H, s), 6.47 (1H, d, J = 8.1 Hz), 4.62 (2H, s), 4.31 (2H, t, J = 5.4 Hz), 3.85 (3H, s), 3.83-3.72 (3H, m), 2.19 (3H, s), 2.06-1.98 (2H, m), 1.63-1.15 (9H, m), 1.01 (3H, d, J = 6.5 Hz). | 497 | 495 |
| 236 | 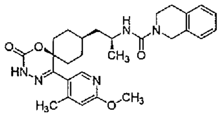 | | 1H-NMR (DMSO-D6) δ: 11.13 (1H, s), 8.06 (1H, s), 7.18-7.05 (4H, m), 6.79 (1H, s), 6.12 (1H, d, J = 8.3 Hz), 4.44 (2H, s), 3.86 (3H, s), 3.83-3.73 (1H, m), 3.61-3.38 (2H, m), 2.81-2.66 (2H, m), 2.19 (3H, s), 2.05-1.96 (2H, m), 1.63-1.56 (1H, m), 1.51-1.14 (8H, m), 1.01 (3H, d, J = 6.5 Hz), | 506 | 504 |
| 237 | 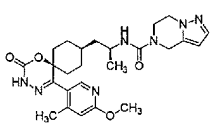 | | 1H-NMR (DMSO-D6) δ: 11.13 (1H, s), 8.07 (1H, s), 7.36 (1H, d, J = 1.8 Hz), 6.78 (1H, s), 6.40 (1H, d, J = 8.1 Hz), 6.04 (1H, d, J = 1.8 Hz), 4.54 (2H, s), 4.02 (2H, t, J = 5.4 Hz), 3.86 (3H, s), 3.81-3.72 (3H, m), 2.19 (3H, s), 2.06-1.98 (2H, m), 1.63-1.15 (9H, m), 1.01 (3H, d, J = 6.5 Hz). | 496 | 494 |
| 238 | 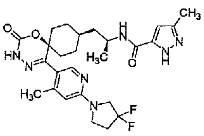 | | 1H-NMR (DMSO-D6) δ: 12.80 (1H, s), 11.04 (1H, s), 7.97 (1H, s), 7.63-7.55 (1H, m), 6.46 (1H, s), 6.31 (1H, s), 4.13-4.00 (1H, m), 3.82 (2H, t, J = 13.4 Hz), 3.61 (2H, t, J = 7.3 Hz), 2.59-2.50 (2H, m), 2.23 (3H, s), 2.15 (3H, s), 2.04-1.97 (2H, m), 1.72-1.64 (1H, m), 1.57-1.19 (8H, m), 1.08 (3H, d, J = 6.5 Hz). | 530 | 528 |
| 239 | 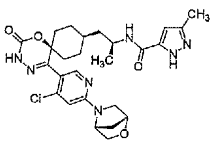 | | 1H-NMR (DMSO-D6) δ: 12.80 (1H, s), 11.13 (1H, s), 8.02 (1H, s), 7.63-7.54 (1H, m), 6.72 (1H, s), 6.31 (1H, s), 4.90 (1H, s), 4.67 (1H, s), 4.12-4.02 (1H, m), 3.76 (1H, d, J = 6.0 Hz), 3.64 (1H, d, J = 7.4 Hz), 3.44 (1H, d, J = 10.2 Hz), 3.32-3.28 (1H, m), 2.23 (3H, s), 2.11-2.00 (2H, m), 1.93-1.82 (2H, m), 1.73-1.64 (1H, m), 1.56-1.16 (8H, m), 1.08 (3H, d, J = 6.5 Hz). | 542 | 540 |
| 240 | 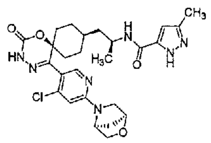 | | 1H-NMR (DMSO-D6) δ: 12.80 (1H, s), 11.13 (1H, s), 8,02 (1H, s), 7.59 (1H, d, J = 9.0 Hz), 6.80-6.65 (1H, m), 6.31 (1H, s), 4.90 (1H, s), 4.67 (1H, s), 4.13-3.98 (1H, m), 3.76 (1H, d, J = 6.0 Hz), 3.65 (1H, d, J = 7.4 Hz), 3.44 (1H, d, J = 9.0 Hz), 3.32-3.24 (1H, m), 2.23 (3H, s), 2.10-2.00 (2H, m), 1.93-1.81 (2H, m), 1.73-1.65 (1H, m), 1.55-1.15 (8H, m), 1.08 (3H, d, J = 6.5 Hz), | 542 | 540 |

Table 1-31

| | | | | | |
|---|---|---|---|---|---|
| 241 | 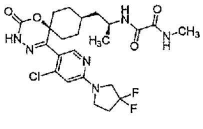 | | 1H-NMR (DMSO-D6) δ: 11.16 (1H, s), 8.66-8.60 (1H, m), 8.41 (1H, d, J = 9.2 Hz), 8.11 (1H, s), 6.76 (1H, s), 3.95-3.84 (1H, m), 3.88 (2H, t, J = 13.2 Hz), 3.66 (2H, t, J = 7.4 Hz), 2.64 (3H, d, J = 4.9 Hz), 2.61-2.52 (2H, m), 2.10-2.00 (2H, m), 1.71-1.57 (1H, m), 1.57-1.14 (8H, m), 1.06 (3H, d, J = 6.7 Hz), | 527 | 525 |
| 242 | 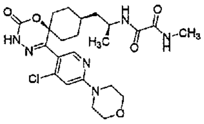 | | 1H-NMR (DMSO-D6) δ: 11.15 (1H, s), 8.66-8.60 (1H, m), 8.41 (1H, d, J = 9.2 Hz), 8.10 (1H, s), 7.02 (1H, s), 3.99-3.83 (1H, m), 3.68 (4H, t, J = 4.9 Hz), 3.53 (4H, t, J = 4.7 Hz), 2.64 (3H, d, J = 4.9 Hz), 2.08-2.00 (2H, m), 1.70-1.60 (1H, m), 1.58-1.16 (8H, m), 1.06 (3H, d, J = 6.7 Hz). | 507 | 505 |

(continued)

| | | | | | |
|---|---|---|---|---|---|
| 243 | 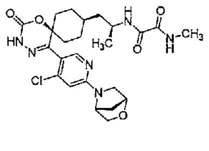 | | 1H-NMR (DMSO-D6) δ: 11.13 (1H, s), 8.67-8.60 (1H, m), 8.41 (1H, d, J = 9.0 Hz), 8.03 (1H, s), 6.79-6.69 (1H, m), 4.91 (1H, s), 4.68 (1H, s), 3.97-3.85 (1H, m), 3.77 (1H, d, J = 7.4 Hz), 3.65 (1H, d, J = 7.4 Hz), 3.48-3.43 (1H, m), 3.33-3.27 (1H, m), 2.64 (3H, d, J = 4.9 Hz), 2.09-1.99 (2H, m), 1.94-1.83 (2H, m), 1.71-1.62 (1H, m), 1.58-1.14 (8H, m), 1.06 (3H, d, J = 6.5 Hz). | 519 | 563 (formic acid adduct) |
| 244 | 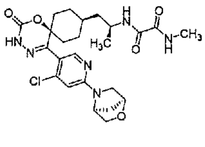 | | 1H-NMR (DMSO-D6) δ: 11.13 (1H, s), 8.67-8.60 (1H, m), 8.41 (1H, d, J = 9.5 Hz), 8.03 (1H, s), 6.78-6.69 (1H, m), 4.91 (1H, s), 4.68 (1H, s), 3.97-3.85 (1H, m), 3.77 (1H, d, J = 7.4 Hz), 3.65 (1H, d, J = 7.4 Hz), 3.48-3.42 (1H, m), 3.32-3.28 (1H, m), 2.64 (3H, d, J = 4.9 Hz), 2.08-2.00 (2H, m), 1.92-1.83 (2H, m), 1.70-1.61 (1H, m), 1.58-1.16 (8H, m), 1.06 (3H, d, J = 6.5 Hz), | 519 | 563 (formic acid adduct) |
| 245 | 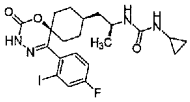 | | 1H-NMR (DMSO-D6) δ: 11.20 (1H, s), 7.87 (1H, dd, J = 8.4, 2.7 Hz), 7.46 (1H, dd, J = 8.6, 5.8 Hz), 7.34 (1H, td, J = 8.5, 2.6 Hz), 5.89 (1H, d, J = 2.8 Hz), 5.46 (1H, d, J = 8.3 Hz), 3.72-3.61 (1H, m), 2.40-2.31 (1H, m), 2.19-2.08 (2H, m), 1.69-1.57 (1H, m), 1.55-1.08 (8H, m), 0.97 (3H, d, J = 6.5 Hz), 0.55-0.48 (2H, m), 0.29-0.23 (2H, m). | 529 | 527 |
| 246 | 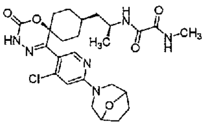 | | 1H-NMR (DMSO-D6) δ: 11.14 (1H, s), 8.66-8.61 (1H, m), 8.41 (1H, d, J = 9.2 Hz), 8,07 (1H, s), 6.90 (1H, s), 4.45-4.40 (2H, m), 3.97-3.81 (1H, m), 3.86 (2H, d, J = 11.6 Hz), 3.03-2.96 (1H, m), 2.64 (3H, d, J = 4.9 Hz), 2.09-1.99 (2H, m), 1.87-1.80 (2H, m), 1.75-1.17 (12H, m), 1.06 (3H, d, J = 6.5 Hz), | 533 | 577 (formic acid adduct) |
| 247 | 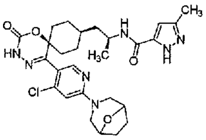 | | 1H-NMR (DMSO-D6) δ: 12.80 (1H, s), 11.14 (1H, s), 8.06 (1H, s), 7.63-7.55 (1H, m), 6.88 (1H, s), 6.31 (1H, s), 4.44-4,39 (2H, m), 4.11-4.00 (1H, m), 3.85 (2H, d, J = 12.5 Hz), 3.02-2.95 (2H, m), 2.23 (3H, s), 2.09-2.01 (2H, m), 1.87-1.64 (5H, m), 1.56-1.17 (8H, m), 1.08 (3H, d, J = 6.5 Hz), | 556 | 554 |
| 248 | 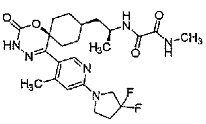 | | 1H-NMR (DMSO-D6) δ: 11.04 (1H, s), 8.66-8.59 (1H, m), 8.41 (1H, d, J = 9.2 Hz), 7.98 (1H, s), 6.47 (1H, s), 3.96-3.84 (1H, m), 3.84 (2H, t, J = 13.4 Hz), 3.63 (2H, t, J = 7.3 Hz), 2.64 (3H, d, J = 4.9 Hz), 2.60-2,52 (2H, m), 2.16 (3H, s), 2.04-1.96 (2H, m), 1.69-1.60 (1H, m), 1.58-1.14 (8H, m), 1.06 (3H, d, J = 6.7 Hz). | 507 | 505 |

Table 1-32

| | | | | | |
|---|---|---|---|---|---|
| 249 | 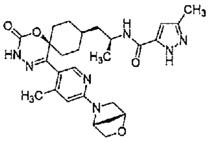 | | 1H-NMR (DMSO-D6) δ: 13.02-12.64 (1H, m), 11.04 (1H, s), 7.91 (1H, s), 7.79-7.49 (1H, m), 6.58-6.24 (2H, m), 4.86 (1H, s), 4.66 (1H, s), 4.13-4.00 (1H, m), 3.76 (1H, d, J = 7.2 Hz), 3.64 (1H, d, J = 7.2 Hz), 3.44 (1H, d, J = 10.2 Hz), 3.24 (1H, d, J = 10.2 Hz), 2.22 (3H, s), 2.13 (3H, s), 2.05-1.96 (2H, m), 1.93-1.80 (2H, m), 1.73-1.63 (1H, m), 1.56-1.16 (8H, m), 1.08 (3H, d, J = 6.5 Hz). | 522 | 520 |

(continued)

| | | | | | |
|---|---|---|---|---|---|
| 250 | 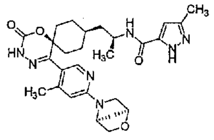 | | 1H-NMR (DMSO-D6) δ: 12.80 (1H, s), 11.02 (1H, s), 7.91 (1H, s), 7.63-7.54 (1H, m), 6.42 (1H, s), 6.31 (1H, s), 4.85 (1H, s), 4.65 (1H, s), 4.11-4.00 (1H, m), 3.76 (1H, d, J = 7.2 Hz), 3.63 (1H, d, J = 7.2 Hz), 3.43 (1H, d, J = 10.2 Hz), 3.22 (1H, d, J = 10.2 Hz), 2.23 (3H, s), 2.12 (3H, s), 2.05-1.95 (2H, m), 1.91-1.80 (2H, m), 1.72-1.63 (1H, m), 1.57-1.21 (8H, m), 1.08 (3H, d, J = 6.7 Hz). | 522 | 520 |
| 251 | 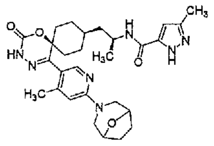 | | 1H-NMR (DMSO-D6) δ: 12.80 (1H, s), 11.03 (1H, s), 7.95 (1H, s), 7.59 (1H, d, J = 8.1 Hz), 6.62 (1H, s), 6.31 (1H, s), 4.42 (2H, s), 4.12-3.98 (1H, m), 3.81 (2H, d, J = 12.3 Hz), 2.93 (2H, d, J = 12.3 Hz), 2.23 (3H, s), 2.13 (3H, s), 2.04-1.95 (2H, m), 1.88-1.63 (5H, m), 1.57-1.18 (8H, m), 1.08 (3H, d, J = 6.5 Hz). | 536 | 534 |
| 252 | 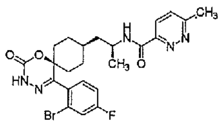 | | 1H-NMR (DMSO-D6) δ: 11.19 (1H, s), 8.84 (1H, d, J = 9.0 Hz), 8.05 (1H, d, J = 8.8 Hz), 7.73 (1H, d, J = 8.8 Hz), 7.70 (1H, dd, J = 8.8, 2.7 Hz), 7.50 (1H, dd, J = 8.8, 6.0 Hz), 7.30 (1H, td, J = 8.8, 2.7 Hz), 4.22-4.13 (1H, m), 2.69 (3H, s), 2.16-2.08 (2H, m), 1.75-1.60 (2H, m), 1.58-1.49 (1H, m), 1.42-1.23 (6H, m), 1.16 (3H, d, J = 6.5 Hz). | 518 | 516 |
| 253 | 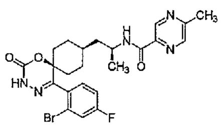 | | 1H-NMR (DMSO-D6) δ: 11.19 (1H, s), 9.01 (1H, d, J = 1.0 Hz), 8.58 (1H, d, J = 1.0 Hz), 8.50 (1H, d, J = 8.6 Hz), 7.71 (1H, dd, J = 8.6, 2.5 Hz), 7.50 (1H, dd, J = 8.6, 6.0 Hz), 7.31 (1H, td, J = 8.6, 2.5 Hz), 4.18-4.11 (1H, m), 2.58 (3H, s), 2.18-2.06 (2H, m), 1.73-1.68 (1H, m), 1.65-1.45 (2H, m), 1.43-1.22 (6H, m), 1.13 (3H, d, J = 6.7 Hz). | 518 | 516 |
| 254 | 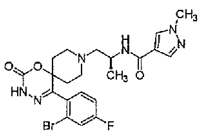 | | 1H-NMR (DMSO-D6) δ: 11.25 (1H, s), 8.05 (1H, s), 7.76 (1H, s), 7.74 (1H, dd, J = 8.6, 2.6 Hz), 7.64 (1H, d, J = 7.4 Hz), 7.54 (1H, dd, J = 8.6, 6.0 Hz), 7.35 (1H, td, J = 8.6, 2.6 Hz), 4.09-4.02 (1H, m), 3.82 (3H, s), 2.73-2.66 (2H, m), 2.40-2.21 (4H, m), 2.11-2.04 (2H, m), 1.63-1.51 (2H, m), 1.05 (3H, d, J = 6.7 Hz). | 507 | 505 |
| 255 | 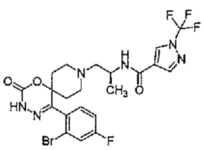 | | 1H-NMR (DMSO-D6) δ: 11.25 (1H, s), 8.85 (1H, s), 8.24 (1H, s), 8.03 (1H, d, J = 8.1 Hz), 7.74 (1H, dd, J = 8.6, 2.5 Hz), 7.53 (1H, dd, J = 8.6, 5.9 Hz), 7.34 (1H, td, J = 8.6, 2.5 Hz), 4.13-4.05 (1H, m), 2.75-2.65 (2H, m), 2.41-2.24 (4H, m), 2.12-2.03 (2H, m), 1.63-1.51 (2H, m), 1.08 (3H, d, J = 6.7 Hz), | 561 | 559 |
| 256 | 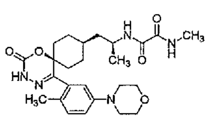 | | 1H-NMR (DMSO-D6) δ: 10.99 (1H, s), 8.63 (1H, q, J = 4.9 Hz), 8.41 (1H, d, J = 9.0 Hz), 7.14 (1H, d, J = 8.4 Hz), 6.91 (1H, dd, J = 8.4, 2.3 Hz), 6.70 (1H, d, J = 2.3 Hz), 3.95-3.85 (1H, m), 3.72 (4H, dd, J = 5.6, 4.6 Hz), 3.06 (4H, dd, J = 5.6, 4.6 Hz), 2.64 (3H, d, J = 4.9 Hz), 2.10 (3H, s), 2.09-2.00 (2H, m), 1.69-1.61 (1H, m), 1.52-1.39 (3H, m), 1.32-1.16 (5H, m), 1.06 (3H, d, J = 6.7 Hz), | 486 | 484 |

Table 1-33

| | | | | | |
|---|---|---|---|---|---|
| 257 | 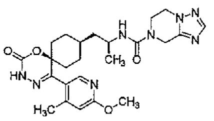 | | 1H-NMR (DMSO-D6) δ: 11.13 (1H, s), 8.07 (1H, s), 7.90 (1H, s), 6.78 (1H, s), 6.50 (1H, d, J = 8.1 Hz), 4.61 (2H, s), 4.09 (2H, t, J = 5.3 Hz), 3.88-3.71 (3H, m), 3.86 (3H, s), 2.19 (3H, s), 2.06-1.97 (2H, m), 1.63-1.14 (9H, m), 1.02 (3H, d, J = 6.5 Hz). | 497 | 495 |

(continued)

| | | | | | |
|---|---|---|---|---|---|
| 258 | 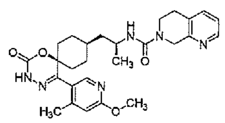 | | 1H-NMR (DMSO-D6) δ: 11.13 (1H, s), 8.33 (1H, dd, J = 4.7, 1.5 Hz), 8.06 (1H, s), 7.50 (1H, dd, J = 7.6, 1.5 Hz), 7.15 (1H, dd, J = 7.6, 4.7 Hz), 6.79 (1H, s), 6.27 (1H, d, J = 8.3 Hz), 4.49 (2H, s), 3.86 (3H, s), 3.83-3.74 (1H, m), 3.61-3.49 (2H, m), 2.76-2.70 (2H, m), 2.19 (3H, s), 2.05-1.97 (2H, m), 1.63-1.56 (1H, m), 1.52-1.15 (8H, m), 1.01 (3H, d, J = 6.5 Hz), | 507 | 505 |
| 259 | 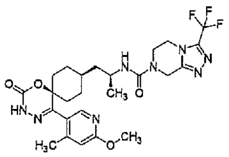 | | 1H-NMR (DMSO-D6) δ: 11.14 (1H, s), 8.06 (1H, s), 6.78 (1H, s), 6.55 (1H, d, J = 8.1 Hz), 4.75 (2H, s), 4.13-4.07 (2H, m), 3.85 (3H, s), 3.83-3.70 (3H, m), 2.19 (3H, s), 2.07-1.98 (2H, m), 1.62-1,16 (9H, m), 1.02 (3H, d, J = 6.7 Hz), | 565 | 563 |
| 260 | 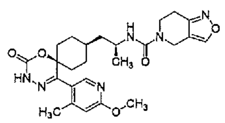 | | 1H-NMR (DMSO-D6) δ: 11.13 (1H, s), 8.65 (1H, s), 8.06 (1H, s), 6.78 (1H, s), 6.32 (1H, d, J = 8.1 Hz), 4.41 (1H, d, J = 16.0 Hz), 4.36 (1H, d, J = 16.0 Hz), 3.86 (3H, s), 3.80-3.71 (1H, m), 3.63-3.51 (2H, m), 2.70 (2H, t, J = 5.9 Hz), 2.19 (3H, s), 2.06-1.97 (2H, m), 1.62-1.55 (1H, m), 1.52-1.14 (8H, m), 1.00 (3H, d, J = 6.7 Hz). | 497 | 495 |
| 261 | 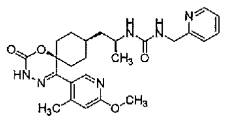 | | 1H-NMR (DMSO-D6) δ: 11,14 (1H, s), 8.46-8.41 (1H, m), 8.08 (1H, s), 7.59 (1H, td, J = 7.7, 1.8 Hz), 7.22-7.15 (2H, m), 6.81 (1H, s), 6.25 (1H, t, J = 5.9 Hz), 5.88 (1H, d, J = 8.3 Hz), 4.28 (1H, dd, J = 16.4, 5.9 Hz), 4.22 (1H, dd, J = 16.4, 5.9 Hz), 3.87 (3H, s), 3.74-3.64 (1H, m), 2.20 (3H, s), 2.10-1.98 (2H, m), 1.69-1.60 (1H, m), 1.54-1.13 (8H, m), 0.99 (3H, d, J = 6.5 Hz). | 481 | 479 |
| 262 | 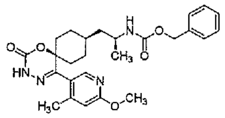 | | 1H-NMR (DMSO-D6) δ: 11.14 (1H, s), 8.08 (1H, s), 7.33-7.21 (5H, m), 7.05 (1H, d, J = 8.6 Hz), 6.81 (1H, s), 5.02 (1H, d, J = 12.7 Hz), 4.93 (1H, d, J = 12.7 Hz), 3.87 (3H, s), 3.65-3.56 (1H, m), 2.20 (3H, s), 2.09-1.98 (2H, m), 1.67-1.58 (1H, m), 1.54-1.13 (8H, m), 1.01 (3H, d, J = 6.5 Hz). | 481 | 479 |
| 263 | 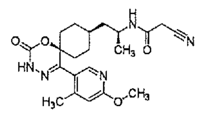 | | 1H-NMR (DMSO-D6) δ: 11.15 (1H, s), 8.07 (1H, s), 8.00 (1H, d, J = 8.1 Hz), 6.79 (1H, s), 3.88-3.76 (1H, m), 3.86 (3H, s), 3.57 (1H, d, J = 18.0 Hz), 3.50 (1H, d, J = 18.0 Hz), 2.20 (3H, s), 2.09-2.00 (2H, m), 1.63-1.57 (1H, m), 1.53-1.15 (8H, m), 1.01 (3H, d, J = 6.5 Hz). | 414 | 412 |
| 264 | 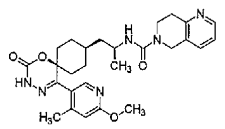 | | 1H-NMR (DMSO-D6) δ: 11.13 (1H, s), 8.31 (1H, dd, J = 4.9, 1.4 Hz), 8.06 (1H, s), 7.50 (1H, dd, J = 7.6, 1.4 Hz), 7.17 (1H, dd, J = 7.6, 4.9 Hz), 6.78 (1H, s), 6.24 (1H, d, J = 8.3 Hz), 4.47 (2H, s), 3.86 (3H, s), 3.82-3.73 (1H, m), 3.68-3.56 (2H, m), 2.81 (2H, t, J = 5.9 Hz), 2.19 (3H, s), 2.06-1.97 (2H, m), 1.64-1.56 (1H, m), 1.53-1.15 (8H, m), 1.01 (3H, d, J = 6.5 Hz). | 507 | 505 |

Table 1-34

| | | | | | |
|---|---|---|---|---|---|
| 265 | 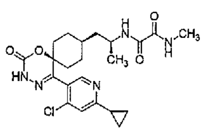 | | 1H-NMR (DMSO-D6) δ: 11.27 (1H, s), 8.63 (1H, q, J = 4.8 Hz), 8.413 (1H, d, J = 9.2 Hz), 8.412 (1H, s), 7.62 (1H, s), 3.99-3.83 (1H, m), 2.64 (3H, d, J = 4.8 Hz), 2.23-2.13 (1H, m), 2.13-2.01 (2H, m), 1.72-1.59 (1H, m), 1.57-1.12 (8H, m), 1.05 (3H, d, J = 6.5 Hz), 1.05-0.94 (4H, m). | 462 | 460 |
| 266 | 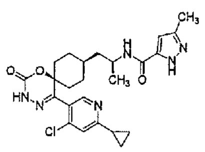 | | 1H-NMR (DMSO-D6) δ: 12.80 (1H, s), 11.27 (1H, s), 8.41 (1H, s), 7.67-7.55 (1H, m), 7.61 (1H, s), 6.31 (1H, s), 4.14-3.98 (1H, m), 2.23 (3H, s), 2.21-2.01 (3H, m), 1.76-1.62 (1H, m), 1.58-1.15 (8H, m), 1.07 (3H, d, J = 6.5 Hz), 1.05-0.92 (4H, m). | 485 | 483 |

(continued)

| | | | | | |
|---|---|---|---|---|---|
| 267 | 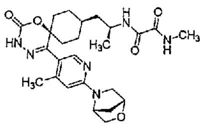 | | 1H-NMR (DMSO-D6) δ: 11.02 (1H, s), 8.66-8.61 (1H, m), 8.41 (1H, d, J = 9.5 Hz), 7.92 (1H, s), 6.43 (1H, s), 4.86 (1H, s), 4.66 (1H, s), 3.97-3.87 (1H, m), 3.77 (1H, d, J = 7.4 Hz), 3.64 (1H, d, J = 7.4 Hz), 3.44 (1H, d, J = 10.4 Hz), 3.23 (1H, d, J = 10.4 Hz), 2.64 (3H, d, J = 4.9 Hz), 2.13 (3H, s), 2.04-1.95 (2H, m), 1.93-1.81 (2H, m), 1.73-1.61 (1H, m), 1.58-1.16 (8H, m), 1.06 (3H, d, J = 6.5 Hz). | 499 | 543 (formic acid adduct) |
| 268 | 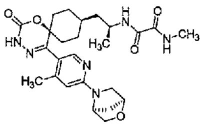 | | 1H-NMR (DMSO-D6) δ: 11.02 (1H, s), 8.66-8.60 (1H, m), 8.41 (1H, d, J = 9.2 Hz), 7.92 (1H, s), 6.44 (1H, s), 4.86 (1H, s), 4.66 (1H, s), 3.98-3.84 (1H, m), 3.77 (1H, d, J = 7.2 Hz), 3.63 (1H, d, J = 7.2 Hz), 3.44 (1H, d, J = 9.9 Hz), 3.23 (1H, d, J = 9.9 Hz), 2.64 (3H, d, J = 4.9 Hz), 2.13 (3H, s), 2.04-1.95 (2H, m), 1.92-1.81 (2H, m), 1.70-1.61 (1H, m), 1.59-1.17 (8H, m), 1.06 (3H, d, J = 6.5 Hz). | 499 | N.D. |
| 269 | 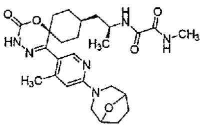 | | 1H-NMR (DMSO-D6) δ: 11.03 (1H, s), 8.66-8.60 (1H, m), 8.41 (1H, d, J = 9.0 Hz), 7.96 (1H, s), 6.63 (1H, s), 4.43 (2H, s), 3.98-3.84 (1H, m), 3.82 (2H, d, J = 12.0 Hz), 2.94 (2H, dd, J = 12.5, 2.3 Hz), 2.64 (3H, d, J = 4.9 Hz), 2.14 (3H, s), 2.03-1.95 (2H, m), 1.87-1.61 (5H, m), 1.60-1.15 (8H, m), 1.06 (3H, d, J = 6.5 Hz). | 513 | N.D. |
| 270 | 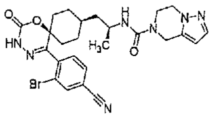 | | 1H-NMR (DMSO-D6) δ: 11.30 (1H, s), 8.36 (1H, d, J = 1.6 Hz), 7.96 (1H, dd, J = 7.9, 1.6 Hz), 7.67 (1H, d, J = 7.9 Hz), 7.36 (1H, d, J = 1.8 Hz), 6.39 (1H, d, J = 8.3 Hz), 6.04 (1H, d, J = 1.8 Hz), 4.53 (2H, s), 4.01 (2H, t, J = 5.4 Hz), 3.83-3.69 (3H, m), 2.16-2.06 (2H, m), 1.63-1.55 (1H, m), 1.54-1.12 (8H, m), 1.00 (3H, d, J = 6.5 Hz). | 554 | 552 |
| 271 | 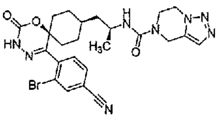 | | 1H-NMR (DMSO-D6) δ: 11.31 (1H, s), 8.35 (1H, d, J = 1.6 Hz), 7.96 (1H, dd, J = 8.0, 1.5 Hz), 7.67 (1H, d, J = 8.1 Hz), 7.58 (1H, s), 6.47 (1H, d, J = 8.1 Hz), 4.67-4,56 (2H, m), 4.30 (2H, t, J = 5.4 Hz), 3.82-3.72 (3H, m), 2.16-2.08 (2H, m), 1.63-1.56 (1H, m), 1.51-1.13 (8H, m), 1.00 (3H, d, J = 6.5 Hz). | 555 | 553 |
| 272 | 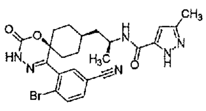 | | 1H-NMR (DMSO-D6) δ: 12.80 (1H, s), 11.29 (1H, s), 8.00 (1H, d, J = 2.0 Hz), 7.96 (1H, d, J = 8.4 Hz), 7.84 (1H, dd, J = 8.4, 2.0 Hz), 7.63-7.55 (1H, m), 6.31 (1H, s), 4.13-3.98 (1H, m), 2.23 (3H, s), 2,20-2,03 (2H, m), 1.74-1.62 (1H, m), 1.59-1.15 (8H, m), 1.08 (3H, d, J = 6.9 Hz), | 513 | 511 |

Table 1-35

| | | | | | |
|---|---|---|---|---|---|
| 273 | 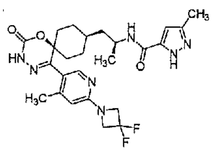 | | 1H-NMR (DMSO-D6) δ: 12.80 (1H, s), 11.08 (1H, s), 7.99 (1H, s), 7.63-7.55 (1H, m), 6.48 (1H, s), 6.31 (1H, s), 4.38 (4H, t, J = 12.5 Hz), 4.11-4.01 (1H, m), 2.23 (3H, s), 2.16 (3H, s), 2.07-1.98 (2H, m), 1.73-1.65 (1H, m), 1.59-1.18 (8H, m), 1.08 (3H, d, J = 6.5 Hz). | 516 | 514 |

(continued)

| | | | | | |
|---|---|---|---|---|---|
| 274 | 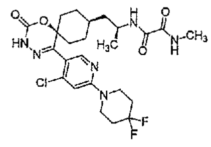 | | 1H-NMR (DMSO-D6) δ: 11.16 (1H, s), 8.66-8.60 (1H, m), 8.41 (1H, d, J = 9.0 Hz), 8.10 (1H, s), 7.16 (1H, s), 3.97-3.86 (1H, m), 3.75 (4H, t, J = 5.7 Hz), 2.64 (3H, d, J = 4.9 Hz), 2.09-1.94 (6H, m), 1.70-1.62 (1H, m), 1.57-1.15 (8H, m), 1.06 (3H, d, J = 6.7 Hz), | 541 | 539 |
| 275 | 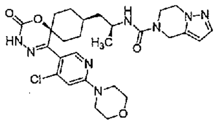 | | 1H-NMR (DMSO-D6) δ: 11.15 (1H, s), 8.10 (1H, s), 7.36 (1H, d, J = 1.8 Hz), 7.03 (1H, s), 6.40 (1H, d, J = 8.3 Hz), 6.04 (1H, s), 4.54 (2H, s), 4.02 (2H, t, J = 5.3 Hz), 3.80-3.73 (3H, m), 3.71-3.65 (4H, m), 3.57-3.49 (4H, m), 2.09-1.99 (2H, m), 1.64-1.55 (1H, m), 1.53-1.14 (8H, m), 1.01 (3H, d, J = 6.7 Hz). | 571 | 569 |
| 276 | 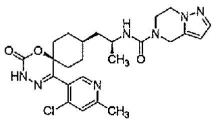 | | 1H-NMR (DMSO-D6) δ: 11.28 (1H, s), 8.48 (1H, s), 7.59 (1H, s), 7.35 (1H, d, J = 1.3 Hz), 6.39 (1H, d, J = 8.1 Hz), 6.03 (1H, d, J = 1.3 Hz), 4.53 (2H, s), 4.01 (2H, t, J = 5.4 Hz), 3.85-3.68 (3H, m), 2.51 (3H, s), 2.16-2.02 (2H, m), 1.66-1.54 (1H, m), 1.54-1.14 (8H, m), 1.01 (3H, d, J = 6.7 Hz). | 500 | 498 |
| 277 | 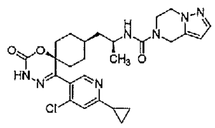 | | 1H-NMR (DMSO-D6) δ: 11.26 (1H, s), 8.42 (1H, s), 7.64 (1H, s), 7.34 (1H, d, J = 0.9 Hz), 6.39 (1H, d, J = 8.1 Hz), 6.03 (1H, d, J = 0.9 Hz), 4.53 (2H, s), 4.01 (2H, t, J = 5.4 Hz), 3.86-3.68 (3H, m), 2.24-2.14 (1H, m), 2.14-2.01 (2H, m), 1.64-1.54 (1H, m), 1.54-1.12 (8H, m), 1.08-0.95 (4H, m), 1.01 (3H, d, J = 6.3 Hz). | 526 | 524 |
| 278 | 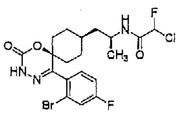 | Mixture of two diastereomers due to chlorofluoromethyl moiety | 1H-NMR (DMSO-D6) δ: 11.20 (1H, s), 8.42 (1H, d, J = 8.6 Hz), 7.74 (1H, dd, J = 8.6, 2.4 Hz), 7.53 (1H, dd, J = 8.6, 6.2 Hz), 7.35 (1H, td, J = 8.4, 2.4 Hz), 6.63 (1H, d, J = 49.5 Hz), 3.93-3.84 (1H, m), 2.18-2.10 (2H, m), 1.65-1.56 (1H, m), 1.51-1.19 (8H, m), 1.05 (3H, dd, J = 6.2, 4.5 Hz), | 492 | 490 |
| 279 | 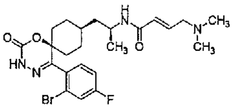 | | 1H-NMR (DMSO-D6) δ: 11.20 (1H, s), 7.73 (2H, dd, J = 8.6, 2.5 Hz), 7.53 (1H, dd, J = 8.6, 6.0 Hz), 7.34 (1H, td, J = 8.6, 2.5 Hz), 6.49 (1H, dt, J = 15.5, 6.1 Hz), 5.96 (1H, d, J = 15.5 Hz), 3.95-3.85 (1H, m), 2.96 (2H, d, J = 6.1 Hz), 2.13 (6H, s), 2.11-2.09 (2H, m), 1.65-1.59 (1H, m), 1.52-1.18 (8H, m), 1.01 (3H, d, J = 6.5 Hz). | 509 | 507 |

(continued)

| | | | | | |
|---|---|---|---|---|---|
| 280 | 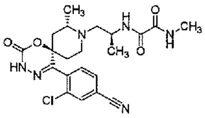 | | 1H-NMR (DMSO-D6) δ: 11.35 (1H, s), 8.63-8.56 (1H, m), 8.30 (1H, d, J = 8.8 Hz), 8.23 (1H, d, J = 1.5 Hz), 7.93 (1H, dd, J = 8.0, 1.5 Hz), 7.73 (1H, d, J = 8.0 Hz), 3.84-3.74 (1H, m), 2.90-2.72 (2H, m), 2.65 (3H, d, J = 4.8 Hz), 2.42 (1H, dd, J = 12.6, 7.6 Hz), 2.35-2.25 (2H, m), 2.00-1.89 (2H, m), 1.87-1.79 (1H, m), 1.69-1.59 (1H, m), 1.05 (3H, d, J = 6.8 Hz), 1.02 (3H, d, J = 6.5 Hz). | 461 | 459 |

Table 1-36

| | | | | | |
|---|---|---|---|---|---|
| 281 | 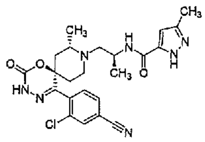 | | 1H-NMR (DMSO-D6) δ: 12.78 (1H, s), 11.35 (1H, s), 8.21 (1H, d, J = 1.5 Hz), 7.91 (1H, d, J = 7.8 Hz), 7.71 (1H, d, J = 7.8 Hz), 7.55-7.47 (1H, m), 6.28 (1H, s), 3.99-3.87 (1H, m), 2.94-2.75 (2H, m), 2.44 (1H, dd, J = 12.6, 7.1 Hz), 2.38-2.13 (7H, m), 2.01-1.90 (2H, m), 1.87-1.79 (1H, m), 1.68-1.58 (1H, m), 1.07 (3H, d, J = 6.8 Hz), 1.05 (3H, d, J = 6.5 Hz), | 484 | 482 |
| 282 | 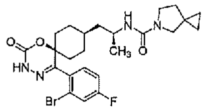 | | 1H-NMR (DMSO-D6) δ: 11.19 (1H, s), 7.74 (1H, dd, J = 8.6, 2.5 Hz), 7.54 (1H, dd, J = 8.6, 6.1 Hz), 7.35 (1H, td, J = 8.6, 2.5 Hz), 5.56 (1H, d, J = 8.6 Hz), 3.76-3.69 (1H, m), 3.35-3.31 (2H, m), 3.11-3.02 (2H, m), 2.15-2.10 (2H, m), 1.70 (2H, t, J = 6.8 Hz), 1.65-1.59 (1H, m), 1.52-1.13 (8H, m), 0.99 (3H, d, J = 6.5 Hz), 0.51 (4H, s). | 521 | 519 |
| 283 | 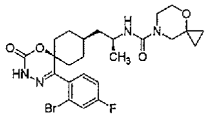 | | 1H-NMR (DMSO-D6) δ: 11.20 (1H, s), 7.74 (1H, dd, J = 8.6, 2.5 Hz), 7.54 (1H, dd, J = 8.6, 6.1 Hz), 7.35 (1H, td, J = 8.6, 2.5 Hz), 6.02 (1H, d, J = 8.6 Hz), 3.80-3.73 (1H, m), 3.53 (2H, t, J = 4.7 Hz), 3.29-3.16 (4H, m), 2.17-2.09 (2H, m), 1.66-1.60 (1H, m), 1.51-1.13 (8H, m), 0.98 (3H, d, J = 6.5 Hz), 0.60-0.57 (2H, m), 0.49-0.46 (2H, m). | 537 | 535 |
| 284 | 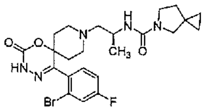 | | 1H-NMR (DMSO-D6) δ: 11.26 (1H, s), 7.76 (1H, dd, J = 8.7, 2.5 Hz), 7.56 (1H, dd, J = 8.7, 5.9 Hz), 7.37 (1H, td, J = 8.7, 2.5 Hz), 5.52 (1H, d, J = 7.9 Hz), 3.79-3.70 (1H, m), 3.28 (2H, t, J = 6.8 Hz), 3.09-2.98 (2H, m), 2.70-2.62 (2H, m), 2.32-2.17 (4H, m), 2.14-2.02 (2H, m), 1.67 (2H, q, J = 6.2 Hz), 1.61-1.54 (2H, m), 0.99 (3H, d, J = 6.2 Hz), 0.53-0.46 (4H, m). | 522 | 520 |
| 285 | 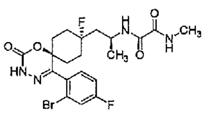 | | 1H-NMR (DMSO-D6) δ: 11.30 (1H, s), 8.64 (1H, q, J = 4.8 Hz), 8.60 (1H, d, J = 9.2 Hz), 7.75 (1H, dd, J = 8.6, 2.7 Hz), 7.56 (1H, dd, J = 8.6, 5.9 Hz), 7.37 (1H, ddd, J = 8.6, 8.1, 2.7 Hz), 4.16-3.99 (1H, m), 2.65 (3H, d, J = 4.8 Hz), 2.16-1.97 (3H, m), 1.86-1.52 (7H, m), 1.08 (3H, d, J = 6.5 Hz), | 501 | 499 |
| 286 | 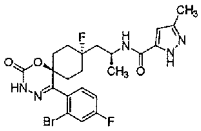 | | 1H-NMR (DMSO-D6) δ: 12.83 (1H, s), 11.29 (1H, s), 7.81 (1H, d, J = 8.6 Hz), 7.74 (1H, dd, J = 8.6, 2.7 Hz), 7.55 (1H, dd, J = 8.6, 5.9 Hz), 7.36 (1H, ddd, J = 8.6, 8.1, 2.7 Hz), 6.32 (1H, s), 4.29-4.16 (1H, m), 2.24 (3H, s), 2.14-1.95 (3H, m), 1.88-1.53 (7H, m), 1.11 (3H, d, J = 6.5 Hz). | 524 | 522 |

(continued)

| | | | | | |
|---|---|---|---|---|---|
| 287 | 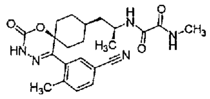 | | 1H-NMR (DMSO-D6) δ: 11.19 (1H, s), 8.64 (1H, q, J = 4.8 Hz), 8.42 (1H, d, J = 9.2 Hz), 7.81 (1H, s), 7.80 (1H, d, J = 8.1 Hz), 7.53 (1H, d, J = 8.1 Hz), 3.95-3.86 (1H, m), 2.64 (3H, d, J = 4.8 Hz), 2.30 (3H, s), 2.08-2.00 (2H, m), 1.68-1.61 (1H, m), 1.55-1.44 (3H, m), 1.39-1.18 (5H, m), 1.06 (3H, d, J = 6.5 Hz). | 426 | 424 |
| 288 | 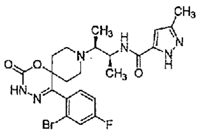 | | 1H-NMR (DMSO-D6) δ: 12.67 (1H, s), 11.21 (1H, s), 7.71 (1H, d, J = 8.6 Hz), 7.50 (1H, d, J = 7.4 Hz), 7.38-7.31 (2H, m), 6.18 (1H, s), 3.78-3.70 (1H, m), 2.72-2.65 (2H, m), 2.58-2.53 (2H, m), 2.36-2.29 (1H, m), 2.26 (3H, s), 2.09-1.92 (2H, m), 1.58-1.48 (1H, m), 1.45-1.35 (1H, m), 1.09 (3H, d, J = 6.5 Hz), 0.92 (3H, d, J = 6.5 Hz). | 521 | 519 |

Table 1-37

| | | | | | |
|---|---|---|---|---|---|
| 289 | 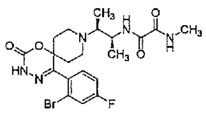 | | 1H-NMR (DMSO-D6) δ: 11.23 (1H, s), 8.56 (1H, d, J = 5.0 Hz), 8.27 (1H, d, J = 7.9 Hz), 7.74 (1H, dd, J = 8.6, 2.5 Hz), 7.47 (1H, dd, J = 8.6, 6.0 Hz), 7.33 (1H, td, J = 8.6, 2.5 Hz), 3.65-3.58 (1H, m), 2.72-2.65 (2H, m), 2.62 (3H, d, J = 5.0 Hz), 2.47-2.41 (2H, m), 2.36-2.28 (1H, m), 2.10-1.95 (2H, m), 1.60-1.40 (2H, m), 1.06 (3H, d, J = 6.5 Hz), 0.90 (3H, d, J = 6.5 Hz). | 498 | N.D. |
| 290 | 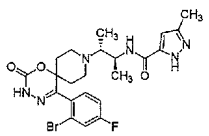 | | 1H-NMR (DMSO-D6) δ: 12.83 (1H, s), 11.26 (1H, s), 7.77 (1H, dd, J = 8.6, 2.6 Hz), 7.65 (1H, d, J = 8.1 Hz), 7.57 (1H, dd, J = 8.6, 6.0 Hz), 7.39 (1H, td, J = 8.6, 2.6 Hz), 6.32 (1H, s), 3.98-3.91 (1H, m), 2.79-2.70 (1H,m), 2.60-2.54 (2H, m), 2.46-2.40 (2H, m), 2.24 (3H, s), 2.15-2.05 (2H, m), 1.70-1.50 (2H, m), 1.06 (3H, d, J = 6,3 Hz), 0.86 (3H, d, J = 6.3 Hz). | 521 | 519 |
| 291 | 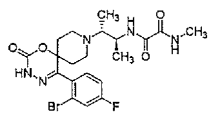 | | 1H-NMR (DMSO-D6) δ: 11.26 (1H, s), 8.65 (1H, q, J = 4.9 Hz), 8.46 (1H, d, J = 9.5 Hz), 7.76 (1H, dd, J = 8.6, 2.4 Hz), 7.56 (1H, dd, J = 8.6, 6.0 Hz), 7.38 (1H, td, J = 8.6, 2.4 Hz), 3.82-3.74 (1H, m), 2.77-2.68 (1H, m), 2.65 (3H, d, J = 4.9 Hz), 2.61-2.38 (4H, m), 2.14-2.06 (2H, m), 1.68-1.48 (2H, m), 1.04 (3H, d, J = 6.6 Hz), 0.81 (3H, d, J = 6.6 Hz). | 498 | N.D. |
| 292 | 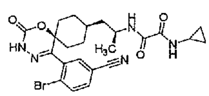 | | 1H-NMR (DMSO-D6) δ: 11.30 (1H, s), 8.69 (1H, d, J = 5.1 Hz), 8.43 (1H, d, J = 9.2 Hz), 8.00 (1H, d, J = 1.8 Hz), 7.98 (1H, d, J = 8.5 Hz), 7.86 (1H, dd, J = 8.5, 1.8 Hz), 3.95-3.85 (1H, m), 2.76-2.70 (1H, m), 2.15-2.05 (2H, m), 1.68-1.61 (1H, m), 1.56-1.35 (4H, m), 1.33-1.14 (4H, m), 1.06 (3H, d, J = 6.7 Hz), 0.64-0.58 (4H, m). | 516 | 514 |
| 293 | 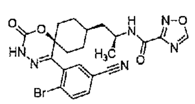 | | 1H-NMR (DMSO-D6) δ: 11.31 (1H, s), 9.77 (1H, s), 8.79 (1H, d, J = 8.6 Hz), 8.01 (1H, d, J = 1.6 Hz), 7.97 (1H, d, J = 8.6 Hz), 7.85 (1H, dd, J = 8.4, 1,9 Hz), 4.16-4,05 (1H, m), 2.19-2.06 (2H, m), 1.72-1.64 (1H, m), 1.63-1.18 (8H, m), 1.12 (3H, d, J = 6.5 Hz). | 501 | 499 |
| 294 | 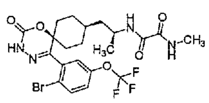 | | 1H-NMR (DMSO-D6) δ: 11.27 (1H, s), 8.67-8.60 (1H, m), 8.43 (1H, d, J = 9.2 Hz), 7.88 (1H, d, J = 8.6 Hz), 7.56 (1H, d, J = 2.7 Hz), 7.48-7.42 (1H, m), 3.96-3.85 (1H, m), 2.63 (3H, d, J = 4.8 Hz), 2.19-2.08 (2H, m), 1.69-1.62 (1H, m), 1.59-1.12 (8H, m), 1.05 (3H, d, J = 6.5 Hz). | 549 | 547 |

(continued)

| | | | | | |
|---|---|---|---|---|---|
| 295 | 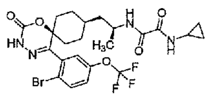 | | 1H-NMR (DMSO-D6) δ: 11.27 (1H, s), 8.69 (1H, d, J = 5.4 Hz), 8.43 (1H, d, J = 9.2 Hz), 7.88 (1H, d, J = 8.6 Hz), 7.56 (1H, d, J = 2.7 Hz), 7.48-7.42 (1H, m), 3.95-3.83 (1H, m), 2.76-2.70 (1H, m), 2.18-2.07 (2H, m), 1.69-1.60 (1H, m), 1.60-1.12 (8H, m), 1.05 (3H, d, J = 6.5 Hz), 0.66-0.53 (4H, m). | 575 | 573 |
| 296 | 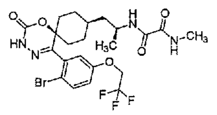 | | 1H-NMR (DMSO-D6) δ: 11.18 (1H, s), 8.67-8.59 (1H, m), 8.43 (1H, d, J = 9.2 Hz), 7.66 (1H, d, J = 9.2 Hz), 7.13-7.08 (2H, m), 4.90-4.77 (2H, m), 3.97-3.85 (1H, m), 2.63 (3H, d, J = 4.8 Hz), 2.19-2.08 (2H, m), 1.69-1.60 (1H, m), 1.59-1.14 (8H, m), 1.05 (3H, d, J = 6.5 Hz). | 563 | 561 |

Table 1-38

| | | | | | |
|---|---|---|---|---|---|
| 297 | 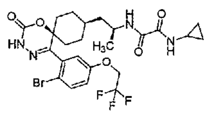 | | 1H-NMR (DMSO-D6) δ: 11.18 (1H, s), 8.69 (1H, d, J = 5.4 Hz), 8.43 (1H, d, J = 9.2 Hz), 7.69-7.64 (1H, m), 7.13-7.07 (2H, m), 4.91-4.77 (2H, m), 3.97-3.83 (1H, m), 2.78-2.68 (1H, m), 2.18-2.07 (2H, m), 1.69-1.61 (1H, m), 1.59-1.14 (8H, m), 1.05 (3H, d, J = 6.5 Hz), 0.66-0.53 (4H, m). | 589 | 587 |
| 298 | 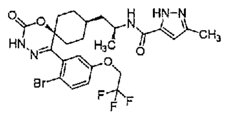 | | 1H-NMR (DMSO-D6) δ: 12.80 (1H, s), 11.18 (1H, s), 7.65 (1H, d, J = 8.1 Hz), 7.60 (1H, d, J = 8.1 Hz), 7.12-7.06 (2H, m), 6.30 (1H, s), 4.88-4.76 (2H, m), 4.13-4.00 (1H, m), 2.23 (3H, s), 2.19-2.08 (2H, m), 1.73-1.63 (1H, m), 1.57-1.17 (8H, m), 1.07 (3H, d, J = 6.5 Hz), | 586 | 584 |
| 299 | 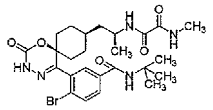 | | 1H-NMR (DMSO-D6) δ: 11.21 (1H, s), 8.64-8.58 (1H, m), 8.39 (1H, d, J = 9.0 Hz), 7.91 (1H, s), 7.80 (2H, s), 7.75 (1H, s), 3.85-3.86 (1H, m), 2.63 (3H, d, J = 4.8 Hz), 2.17 (2H, d, J = 13.0 Hz), 1.71-1.64 (1H, m), 1.55-1.15 (8H, m), 1.38 (9H, s), 1.06 (3H, d, J = 6.5 Hz), | 564 | 562 |
| 300 | 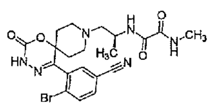 | | 1H-NMR (DMSO-D6) δ: 11.35 (1H, s), 8.64-8.58 (1H, m), 8.35 (1H, d, J = 8.5 Hz), 8.07 (1H, d, J = 2.0 Hz), 8.00 (1H, d, J = 8.5 Hz), 7.88 (1H, dd, J = 8.3, 2.0 Hz), 3.99-3.87 (1H, m), 2.75-2.60 (2H, m), 2.64 (3H, d, J = 5.0 Hz), 2.43 (1H, dd, J = 12.6, 8.1 Hz), 2.35-2.20 (3H, m), 2.14-2.03 (2H, m), 1.75-1.48 (2H, m), 1.05 (3H, d, J = 6.8 Hz). | 491 | 489 |
| 301 | 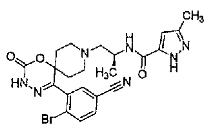 | | 1H-NMR (DMSO-D6) δ: 12.79 (1H, s), 11.34 (1H, s), 8.06 (1H, d, J = 1.8 Hz), 7.99 (1H, d, J = 8.3 Hz), 7.87 (1H, dd, J = 8.3, 2.0 Hz), 7.60-7.50 (1H, m), 6.28 (1H, s), 4.15-3.99 (1H, m), 2.70 (2H, t, J = 11.6 Hz), 2.43 (1H, dd, J = 12.3, 7.8 Hz), 2.35-2.15 (6H, m), 2.09 (2H, d, J = 12.8 Hz), 1.69-1.54 (2H, m), 1.07 (3H, d, J = 6.5 Hz). | 514 | 512 |
| 302 | 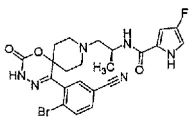 | | 1H-NMR (DMSO-D6) δ: 11.46-11.09 (2H, m), 8.06 (1H, d, J = 1.8 Hz), 7.99 (1H, d, J = 8.3 Hz), 7.87 (1H, dd, J = 8.4, 1.9 Hz), 7.64 (1H, d, J = 8.3 Hz), 6.74-6.71 (1H, m), 6.57 (1H, s), 4.12-4.02 (1H, m), 2.77-2.63 (2H, m), 2.39 (1H, dd, J = 12.4, 7.1 Hz), 2.35-2.21 (3H, m), 2.14-2.04 (2H, m), 1.72-1.56 (2H, m), 1.07 (3H, d, J = 6.5 Hz). | 517 | 515 |

(continued)

| | | | | | |
|---|---|---|---|---|---|
| 303 | 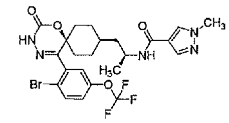 | | 1H-NMR (DMSO-D6) δ: 11.27 (1H, s), 8.05 (1H, s), 7.87 (1H, m), 7.77 (1H, s), 7.65 (1H, m), 7.56 (1H, m), 7.44 (1H, m), 4.04 (1H, s), 3.81 (3H, s), 2.14 (2H, d, J = 12.4 Hz), 1.67 (1H, d, J = 8.6 Hz), 1.52-1.24 (8H, m), 1.06 (3H, d, J = 6.5 Hz). | 572 | 570 |
| 304 | 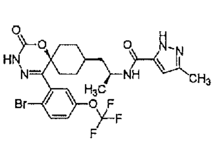 | | 1H-NMR (DMSO-D6) δ: 12.80 (1H, s), 11.27 (1H, s), 7.87 (1H, m), 7.60 (1H, d, J = 9.2 Hz), 7.55 (1H, m), 7.43 (1H, d, J = 9.2 Hz), 6.29 (1H, s), 4.05-4.03 (1H, m), 2.23 (3H, s), 2.14 (2H, d, J = 12.4 Hz), 1.70 (1H, s), 1.51-1.25 (8H, m), 1.07 (3H, d, J = 5.9 Hz). | 572 | 570 |

Table 1-39

| | | | | | |
|---|---|---|---|---|---|
| 305 | 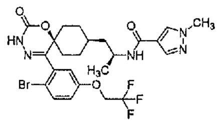 | | 1H-NMR (DMSO-D6) δ: 11.18 (1H, s), 8.05 (1H, s), 7.78 (1H, s), 7.67 (1H, s), 7.64 (1H, s), 7.11-7.08 (2H, m), 4.82 (2H, q, J = 8.8 Hz), 4.09-3.96 (1H, m), 3.81 (3H, s), 2.14 (2H, d, J = 11.9 Hz), 1.67 (1H, d, J = 9.7 Hz), 1.51-1.24 (8H, m), 1.06 (3H, d, J = 6.5 Hz). | 586 | 584 |
| 306 | 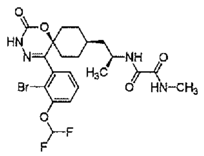 | | 1H-NMR (DMSO-D6) δ: 11.23 (1H, s), 8.64 (1H, q, J = 4.7 Hz), 8.43 (1H, d, J = 9.2 Hz), 7.51 (1H, t, J = 7.8 Hz), 7.40 (1H, d, J = 8.1 Hz), 7.33 (1H, t, J = 73.8 Hz), 7.32 (1H, d, J = 6.5 Hz), 3.91-3.88 (1H, m), 2.63 (3H, d, J = 4.8 Hz), 2.13 (2H, d, J = 11.9 Hz), 1.64 (1H, s), 1.55-1.17 (8H, m), 1.05 (3H, d, J = 6.5 Hz). | 531 | 529 |
| 307 | 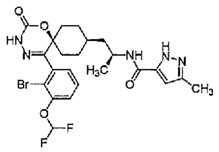 | | 1H-NMR (DMSO-D6) δ: 12.81 (1H, s), 11.22 (1H, s), 7.61 (1H, d, J = 8.6 Hz), 7.51-7.48 (1H, m), 7.38 (1H, d, J = 8.1 Hz), 7.32 (1H, t, J = 73.3 Hz), 7.31 (1H, d, J = 7.3 Hz), 6.30 (1H, s), 4.05 (1H, m), 2.23 (3H, s), 2.13 (2H, d, J = 15.6 Hz), 1.69 (1H, s), 1.49-1.24 (8H, m), 1.07 (3H, d, J = 6.5 Hz). | 554 | 552 |
| 308 | 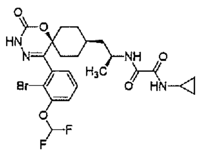 | | 1H-NMR (DMSO-D6) δ: 11.23 (1H, s), 8.69 (1H, d, J = 5.4 Hz), 8.42 (1H, d, J = 9.2 Hz), 7.52 (1H, t, J = 8.1 Hz), 7.41 (1H, d, J = 8.1 Hz), 7.33 (1H, t, J = 73.3 Hz), 7.32 (1H, d, J = 7.5 Hz), 3.89 (1H, s), 2.74-2.72 (1H, m), 2.13 (2H, d, J = 10.8 Hz), 1.65 (1H, d, J = 7.0 Hz), 1.54-1.20 (8H, m), 1.05 (3H, d, J = 6.5 Hz), 0.62-0.60 (4H, m), | 557 | 555 |
| 309 | 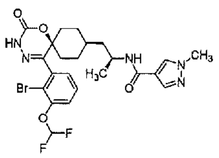 | | 1H-NMR (DMSO-D6) δ: 11.23 (1H, s), 8.05 (1H, s), 7.78 (1H, s), 7.65 (1H, d, J = 8.6 Hz), 7.52-7.48 (1H, m), 7.39 (1H, d, J = 8.6 Hz), 7.32 (1H, t, J = 73.3 Hz), 7.31 (1H, d, J = 3.2 Hz), 4.05-4.01 (1H, m), 3.81 (3H, s), 2.13 (2H, d, J = 9.7 Hz), 1.68 (1H, s), 1.52-1.25 (8H, m), 1.05 (3H, d, J = 6.5 Hz). | 554 | 552 |
| 310 | 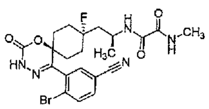 | | 1H-NMR (DMSO-D6) δ: 11.40 (1H, s), 8.64 (1H, q, J = 4.8 Hz), 8.61 (1H, d, J = 8.6 Hz), 8.10 (1H, s), 8.01 (1H, d, J = 8.6 Hz), 7.88 (1H, d, J = 8.6 Hz), 4.16-4.01 (1H, m), 2.65 (3H, d, J = 4.8 Hz), 2.16-1.98 (3H, m), 1.86-1.56 (7H, m), 1.08 (3H, d, J = 6.5 Hz). | 508 | 506 |

(continued)

| | | | | | |
|---|---|---|---|---|---|
| 311 | 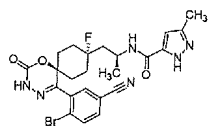 | | 1H-NMR (DMSO-D6) δ: 12.82 (1H, s), 11.39 (1H, s), 8.09 (1H, s), 8.00 (1H, d, J = 8.6 Hz), 7.87 (1H, d, J = 8.6 Hz), 7.81 (1H, d, J = 8.6 Hz), 6.32 (1H, s), 4.30-4.15 (1H, m), 2.24 (3H, s), 2.15-1.97 (3H, m), 1.89-1.56 (7H, m), 1.11 (3H, d, J = 6.5 Hz). | 531 | 529 |
| 312 | 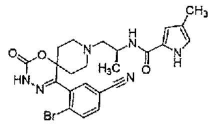 | | 1H-NMR (DMSO-D6) δ: 11.35 (1H, br s), 10.95 (1H, s), 8.06 (1H, d, J = 1.8 Hz), 7.99 (1H, d, J = 8.3 Hz), 7.87 (1H, dd, J = 8.3, 2.0 Hz), 7.46 (1H, d, J = 8.3 Hz), 6.58 (1H, s), 6.52 (1H, s), 4.11-4.02 (1H, m), 2.78-2.68 (2H, m), 2.40 (1H, dd, J = 12.3, 7.3 Hz), 2.32-2.22 (3H, m), 2.14-2.04 (2H, m), 1.99 (3H, s), 1.73-1.53 (2H, m), 1.06 (3H, d, J = 6.5 Hz), | 513 | 511 |

Table 1-40

| | | | | | |
|---|---|---|---|---|---|
| 313 | 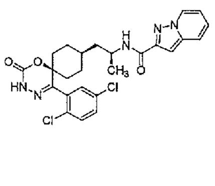 | | 1H-NMR (DMSO-D6) δ: 11.24 (1H, s), 8.64 (1H, d, J = 7.2 Hz), 8.07 (1H, d, J = 9.0 Hz), 7.75 (1H, d, J = 9.0 Hz), 7.61-7.57 (2H, m), 7.53 (1H, dd, J = 9.0, 2.4 Hz), 7.27 (1H, dd, J = 9,0, 6.8 Hz), 7.01 (1H, t, J = 6.8 Hz), 6.94 (1H, s), 4.19-4.10 (1H, m), 2.17-2.07 (2H, m), 1.77-1.68 (1H, m), 1.63-1.21 (8H, m), 1.13 (3H, d, J = 6.7 Hz). | 514 | 512 |
| 314 | 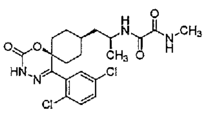 | | 1H-NMR (DMSO-D6) δ: 11.24 (1H, s), 8.64 (1H, q, J = 4.9 Hz), 8.42 (1H, d, J = 9.2 Hz), 7.65-7.55 (3H, m), 3.95-3.86 (1H, m), 2.64 (3H, d, J = 4.9 Hz), 2.16-2.06 (2H, m), 1.71-1.61 (1H, m), 1.57-1.13 (8H, m), 1.06 (3H, d, J = 6.5 Hz). | 455 | 453 |
| 315 | 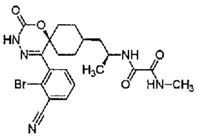 | | 1H-NMR (DMSO-D6) δ: 11.32 (1H, s), 8.64 (1H, q, J = 4.8 Hz), 8.43 (1H, d, J = 8.6 Hz), 8.03 (1H, dd, J = 7.8, 1.3 Hz), 7.79 (1H, dd, J = 7.8, 1.3 Hz), 7.65 (1H, t, J = 7.8 Hz), 3.90-3.90 (1H, m), 2.63 (3H, d, J = 4,8 Hz), 2.12 (2H, d, J = 11.9 Hz), 1.64 (1H, s), 1.53-1.21 (8H, m), 1.05 (3H, d, J = 6.5 Hz). | 490 | 488 |
| 316 | 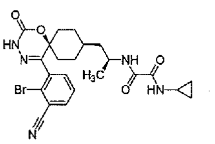 | | 1H-NMR (DMSO-D6) δ: 11.32 (1H, s), 8.69 (1H, d, J = 5.4 Hz), 8.43 (1H, d, J = 9.2 Hz), 8.04 (1H, dd, J = 7.8, 1.3 Hz), 7.79 (1H, dd, J = 7.8, 1.3 Hz), 7.66 (1H, t, J = 7.8 Hz), 3.90-3.89 (1H, m), 2.75-2.72 (1H, m), 2.11 (2H, d, J = 9.2 Hz), 1.64 (1H, s), 1.55-1.21 (8H, m), 1.05 (3H, d, J = 6.5 Hz), 0.63-0.60 (4H, m). | 516 | 514 |
| 317 | 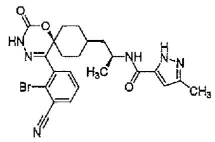 | | 1H-NMR (DMSO-D6) δ: 12.81 (1H, s), 11.31 (1H, s), 8.01 (1H, dd, J = 7.8, 1.3 Hz), 7.78 (1H, dd, J = 7.8, 1.3 Hz), 7.63 (1H, br s), 7.61 (1H, s), 6.31 (1H, br s), 4.07-4.05 (1H, m), 2.22 (3H, s), 2.12 (2H, s), 1.69 (1H, d, J = 8.1 Hz), 1.49-1.26 (8H, m), 1.07 (3H, d, J = 6.5 Hz). | 513 | 511 |
| 318 | 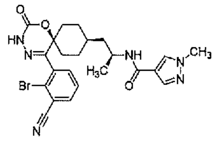 | | 1H-NMR (DMSO-D6) δ: 11.32 (1H, s), 8.05 (1H, s), 8.02 (1H, m), 7,80-7.78 (2H, m), 7.67-7.61 (2H, m), 4.04-4.02 (1H, m), 3.81 (3H, s), 2.13 (2H, d, J = 11.9 Hz), 1.67 (1H, d, J = 9.2 Hz), 1.52-1.23 (8H, m), 1.05 (3H, d, J = 6.5 Hz). | 513 | 511 |

(continued)

| | | | | | |
|---|---|---|---|---|---|
| 319 | 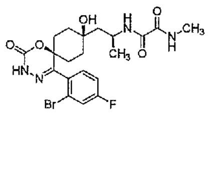 | | 1H-NMR (DMSO-D6) δ: 11.25 (1H, s), 8.62 (1H, q, J = 4.8 Hz), 8.55 (1H, d, J = 8.1 Hz), 7.74 (1H, dd, J = 8.6, 2.7 Hz), 7.53 (1H, dd, J = 8.6, 5.9 Hz), 7.35 (1H, td, J = 8.6, 2.7 Hz), 4.57 (1H, s), 3.96-3.81 (1H, m), 2.66 (3H, d, J = 4.8 Hz), 2.11-1.94 (2H, m), 1.76-1.29 (8H, m), 1.08 (3H, d, J = 6.5 Hz), | 499 | 497 |
| 320 | 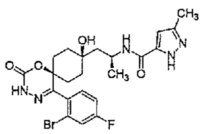 | | 1H-NMR (DMSO-D6) δ: 12.81 (1H, s), 11.24 (1H, s), 7.90-7.79 (1H, m), 7.74 (1H, d, J = 8.1 Hz), 7.59-7.48 (1H, m), 7.39-7.26 (1H, m), 6.29 (1H, s), 4.59 (1H, s), 4.07-3.93 (1H, m), 2.25 (3H, s), 2.11-1.94 (2H, m), 1.78-1.28 (8H, m), 1.12 (3H, d, J = 5.9 Hz). | 522 | 520 |

Table 1-41

| | | | | | |
|---|---|---|---|---|---|
| 321 | 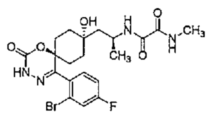 | | 1H-NMR (DMSO-D6) δ: 11.20 (1H, s), 8.63 (1H, q, J = 4.8 Hz), 8.56 (1H, d, J = 7.5 Hz), 7.75 (1H, dd, J = 8.6, 2.7 Hz), 7.52 (1H, dd, J = 8.6, 5.9 Hz), 7.37 (1H, ddd, J = 8.6, 8.1, 2.7 Hz), 4.17 (1H, s), 4.10-3.94 (1H, m), 2.65 (3H, d, J = 4.8 Hz), 1.97-1.85 (2H, m), 1.85-1.66 (3H, m), 1.63-1.33 (5H, m), 1.08 (3H, d, J = 5.9 Hz). | 499 | 497 |
| 322 | 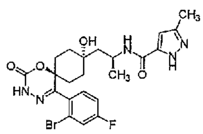 | | 1H-NMR (DMSO-D6) δ: 12.80 (1H, s), 11.18 (1H, s), 7.85 (1H, d, J = 7.5 Hz), 7.74 (1H, dd, J = 8.6, 2.7 Hz), 7.51 (1H, dd, J = 8.6, 5.9 Hz), 7.37 (1H, ddd, J = 8.6, 8.1, 2.7 Hz), 6.31 (1H, s), 4.25-4.08 (1H, m), 4.21 (1H, s), 2.24 (3H, s), 1.97-1.82 (2H, m), 1.82-1.66 (3H, m), 1.66-1.34 (5H, m), 1.12 (3H, d, J = 6.5 Hz). | 522 | 520 |
| 323 | 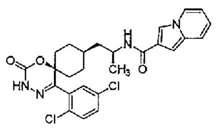 | | 1H-NMR (DMSO-D6) δ: 11.24 (1H, s), 8.22 (1H, dd, J = 7.0, 1.0 Hz), 7.91 (1H, d, J = 1.4 Hz), 7.81 (1H, d, J = 8.4 Hz), 7.61-7.57 (2H, m), 7.53 (1H, dd, J = 8.4, 2.4 Hz), 7.40 (1H, d, J = 9.2 Hz), 6.76 (1H, s), 6.70 (1H, ddd, J = 9.2, 6.5, 1.0 Hz), 6.57 (1H, td, J = 6.5, 1.4 Hz), 4.15-4.05 (1H, m), 2.17-2.08 (2H, m), 1.74-1.66 (1H, m), 1.58-1.21 (8H, m), 1.10 (3H, d, J = 6,5 Hz), | 513 | 511 |
| 324 | 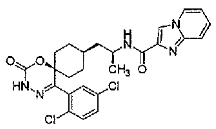 | | 1H-NMR (DMSO-D6) δ: 11.24 (1H, s), 8.55 (1H, dt, J = 6.9, 1.1 Hz), 8.31 (1H, d, J = 0.6 Hz), 7.97 (1H, d, J = 9.0 Hz), 7.61-7.51 (4H, m), 7.32 (1H, ddd, J = 9.0, 6.9, 1.1 Hz), 6.96 (1H, td, J = 6.9, 1.1 Hz), 4.18-4.08 (1H, m), 2.17-2.07 (2H, m), 1.76-1.68 (1H, m), 1.63-1.20 (8H, m), 1.13 (3H, d, J = 6.7 Hz). | 514 | 512 |
| 325 | 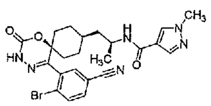 | | 1H-NMR (DMSO-D6) δ: 11.30 (1H, s), 8.06 (1H, s), 8.00 (1H, d, J = 1.8 Hz), 7.97 (1H, d, J = 8.3 Hz), 7.85 (1H, dd, J = 8.3, 2.1 Hz), 7.78 (1H, d, J = 0.7 Hz), 7.66 (1H, d, J = 8.3 Hz), 4.10-3.96 (1H, m), 3.82 (3H, s), 2.18-2.05 (2H, m), 1.72-1.61 (1H, m), 1.59-1,16 (8H, m), 1.06 (3H, d, J = 6.5 Hz). | 513 | 511 |
| 326 | 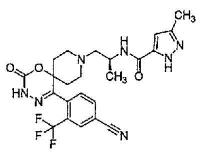 | | 1H-NMR (DMSO-D6) δ: 12.80 (1H, s), 11.43 (1H, s), 8.47 (1H, s), 8.23 (1H, d, J = 7.8 Hz), 7.87 (1H, d, J = 7.8 Hz), 7.62-7.54 (1H, m), 6.30 (1H, s), 4.11-4.01 (1H, m), 2.77-2.66 (2H, m), 2.53-2.37 (1H, m), 2,36-2.16 (6H, m), 2.04-1.88 (2H, m), 1.71-1,53 (2H, m), 1.08 (3H, d, J = 6.5 Hz), | 504 | 502 |

(continued)

| | | | | | |
|---|---|---|---|---|---|
| 327 | 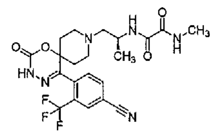 | | 1H-NMR (DMSO-D6) δ: 11.43 (1H, s), 8.66-8.59 (1H, m), 8.48 (1H, s), 8.35 (1H, d, J = 8.5 Hz), 8.25 (1H, d, J = 8,0 Hz), 7.89 (1H, d, J = 8.0 Hz), 3.99-3.87 (1H, m), 2.76-2.61 (2H, m), 2.65 (3H, d, J = 4.8 Hz), 2.44 (1H, dd, J = 12.5, 8.0 Hz), 2.36-2.20 (3H, m), 2.03-1.90 (2H, m), 1.72-1.52 (2H, m), 1.05 (3H, d, J = 6.5 Hz), | 481 | 479 |
| 328 | 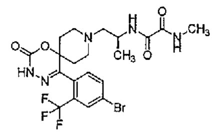 | | 1H-NMR (DMSO-D6) δ: 11.34 (1H, s), 8.67-8.59 (1H, m), 8.35 (1H, d, J = 8.3 Hz), 8.09 (1H, s), 7.99 (1H, d, J = 8.3 Hz), 7.60 (1H, d, J = 8.3 Hz), 3.99-3.85 (1H, m), 2.74-2.60 (2H, m), 2.65 (3H, d, J = 4.8 Hz), 2.43 (1H, dd, J = 12.6, 7.9 Hz), 2.35-2.20 (3H, m), 2.01-1.84 (2H, m), 1.70-1,53 (2H, m), 1.05 (3H, d, J = 6.5 Hz). | 534 | 532 |

Table 1-42

| | | | | | |
|---|---|---|---|---|---|
| 329 | 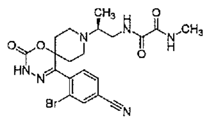 | | 1H-NMR (DMSO-D6) δ: 11.35 (1H, s), 8.66-8.59 (1H, m), 8.41-8.35 (2H, m), 7.97 (1H, dd, J = 7.9, 1.4 Hz), 7.70 (1H, d, J = 8.0 Hz), 3.17-3.00 (2H, m), 2.73 (1H, q, J = 7.5 Hz), 2.68-2.58 (3H, m), 2.65 (3H, d, J = 4.8 Hz), 2.41 (1H, t, J = 11.5 Hz), 2.16-2.06 (2H, m), 1.64-1.47 (2H, m), 0.87 (3H, d, J = 6.8 Hz), | 491 | 489 |
| 330 | 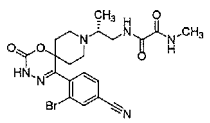 | | 1H-NMR (DMSO-D6) δ: 11.35 (1H, s), 8.66-8.59 (1H, m), 8.41-8.35 (2H, m), 7.97 (1H, dd, J = 7.9, 1.4 Hz), 7.70 (1H, d, J = 8.0 Hz), 3.17-3.00 (2H, m), 2.73 (1H, q, J = 7.5 Hz), 2.68-2.58 (3H, m), 2.65 (3H, d, J = 4.8 Hz), 2.41 (1H, t, J = 11.5 Hz), 2.16-2.06 (2H, m), 1.64-1.47 (2H, m), 0.87 (3H, d, J = 6.8 Hz). | 491 | 489 |
| 331 | 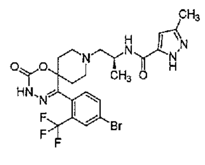 | | 1H-NMR (DMSO-D6) δ: 12.82 (1H, br s), 11.29 (1H, br s), 8.08 (1H, d, J = 1.8 Hz), 7.97 (1H, dd, J = 8.5, 1.5 Hz), 7,66-7.55 (1H, m), 7.58 (1H, d, J = 8.5 Hz), 6.34 (1H, s), 4.11-4.01 (1H, m), 2.75-2.65 (2H, m), 2.43 (1H, dd, J = 12.9, 7.6 Hz), 2.35-2.24 (3H, m), 2.24 (3H, s), 2.02-1.86 (2H, m), 1.70-1.54 (2H, m), 1.08 (3H, d, J = 6.5 Hz), | 557 | 555 |
| 332 | 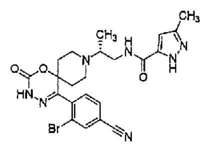 | | 1H-NMR (DMSO-D6) δ: 12.77 (1H, s), 11.35 (1H, s), 8.37 (1H, s), 7.97 (1H, d, J = 7.8 Hz), 7.68 (2H, d, J = 8.0 Hz), 6.28 (1H, s), 2.80-2.38 (7H, m), 2.25 (3H, s), 2.11 (2H, d, J = 13.3 Hz), 1.65-1.48 (2H, m), 0.90 (3H, d, J = 6.5 Hz). | 514 | 512 |
| 333 | 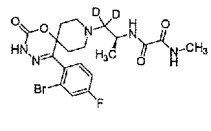 | | 1H-NMR (CDCl3) δ: 8.17 (1H, s), 7.47-7.37 (3H, m), 7.24-7.20 (1H, m), 7.13 (1H, td, J = 8.2, 2.5 Hz), 4.00-3.93 (1H, m), 2.89 (3H, d, J = 5.5 Hz), 2.75-2.68 (1H, m), 2.67-2.61 (2H, m), 2.47 (1H, td, J = 12.1, 2.9 Hz), 2.25-2.16 (2H, m), 1.82-1.65 (2H, m), 1.18 (3H, d, J = 6.1 Hz). | 486 | 484 |
| 334 | 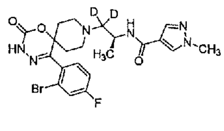 | | 1H-NMR (DMSO-D6) δ: 11.26 (1H, s), 8.05 (1H, s), 7.77-7.72 (2H, m), 7.65 (1H, d, J = 8.3 Hz), 7.54 (1H, dd, J = 8.6, 6.0 Hz), 7.35 (1H, td, J = 8.5, 2.6 Hz), 4.08-4.00 (1H, m), 3.82 (3H, s), 2.74-2.65 (2H, m), 2.35-2.23 (2H, m), 2.12-2.02 (2H, m), 1.64-1.51 (2H, m), 1.05 (3H, d, J = 6.7 Hz). | 509 | 507 |
| 335 | 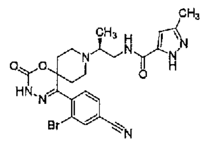 | | 1H-NMR (DMSO-D6) δ: 12.77 (1H, s), 11.35 (1H, s), 8.37 (1H, s), 7.97 (1H, d, J = 7.8 Hz), 7.68 (2H, d, J = 8.0 Hz), 6.28 (1H, s), 2.80-2.38 (7H, m), 2.25 (3H, s), 2.11 (2H, d, J = 13.3 Hz), 1.65-1.48 (2H, m), 0.90 (3H, d, J = 6.5 Hz). | 514 | 512 |

(continued)

| | | | | | |
|---|---|---|---|---|---|
| 336 | 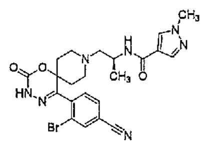 | | 1H-NMR (DMSO-D6) δ: 11.36 (1H, s), 8.36 (1H, s), 8.04 (1H, s), 7.95 (1H, d, J = 8.0 Hz), 7.76 (1H, s), 7.69 (1H, d, J = 7.8 Hz), 7.63 (1H, d, J = 8.0 Hz), 4.13-4.00 (1H, m), 3.83 (3H, s), 2.75-2.65 (2H, m), 2.42-2.20 (4H, m), 2.14-2.05 (2H, m), 1.63-1.51 (2H, m), 1.05 (3H, d, J = 6.8 Hz). | 514 | 512 |

Table 1-43

| | | | | | |
|---|---|---|---|---|---|
| 337 | 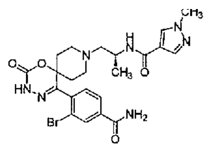 | | 1H-NMR (DMSO-D6) δ: 11.27 (1H, s), 8.19 (1H, s), 8.15 (1H, s), 8.03 (1H, s), 7.92 (1H, dd, J = 8.0, 1.3 Hz), 7.76 (1H, s), 7.66-7.53 (3H, m), 4.10-3.98 (1H, m), 3.82 (3H, s), 2.74-2.65 (2H, m), 2.41-2.20 (4H, m), 2.14-2.05 (2H, m), 1.65-1.53 (2H, m), 1.06-1.02 (3H, m). | 532 | 530 |
| 338 | 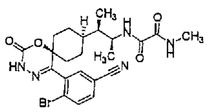 | | 1H-NMR (DMSO-D6) δ: 11.30 (1H, s), 8.67-8.61 (1H, m), 8.43 (1H, d, J = 9.2 Hz), 8.04-7.97 (2H, m), 7.89-7.85 (1H, m), 3.85-3.75 (1H, m), 2.65 (3H, d, J = 4.3 Hz), 2.24-2.06 (2H, m), 1.59-1.21 (8H, m), 1.02 (3H, d, J = 6.5 Hz), 0.77 (3H, d, J = 7.0 Hz). | 504 | 502 |
| 339 | 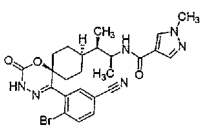 | | 1H-NMR (DMSO-D6) δ: 11.30 (1H, s), 8.07 (1H, s), 8.03-7.96 (2H, m), 7.88-7.83 (1H, m), 7.80 (1H, s), 7.68 (1H, d, J = 8.1 Hz), 4.06-3.97 (1H, m), 3.82 (3H, s), 2.23-2.09 (2H, m), 1.61-1.21 (8H, m), 1.00 (3H, d, J = 7.0 Hz), 0.80 (3H, d, J = 7.0 Hz). | 527 | 525 |

Table 1-44

| | | | | | |
|---|---|---|---|---|---|
| 2-001 | 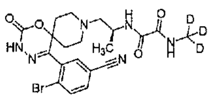 | | 1H-NMR (DMSO-D6) δ: 11.35 (1H, s), 8.58 (1H, s), 8.35 (1H, d, J = 8.8 Hz), 8.07 (1H, d, J = 2.0 Hz), 8.00 (1H, d, J = 8.5 Hz), 7.88 (1H, dd, J = 8.4, 1.9 Hz), 3.99-3.86 (1H, m), 2.75-2.60 (2H, m), 2.43 (1H, dd, J = 12.4, 8.1 Hz), 2.34-2.20 (3H, m), 2.13-2.02 (2H, m), 1.72-1.48 (2H, m), 1.05 (3H, d, J = 6.5 Hz), | 494 | 492 |
| 2-002 | 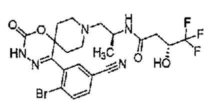 | | 1H-NMR (DMSO-D6) δ: 11.35 (2H, s), 8.07 (1H, d, J = 1.8 Hz), 8.00 (1H, d, J = 8.3 Hz), 7.88 (1H, dd, J = 8.4, 1.9 Hz), 7.81 (1H, d, J = 7.8 Hz), 6.31 (1H, s), 4.34-4.22 (1H, m), 3.96-3.83 (1H, m), 2.73-2.63 (2H, m), 2.36-2.22 (4H, m), 2.18 (1H, dd, J = 12.4, 7.1 Hz), 2.14-2.04 (2H, m), 1.72-1.58 (2H, m), 0.99 (3H, d, J = 6.8 Hz). | 546 | 544 |
| 2-003 | 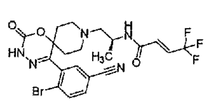 | | 1H-NMR (DMSO-D6) δ: 11.35 (1H, s), 8.29 (1H, d, J = 7.8 Hz), 8.08 (1H, d, J = 1.8 Hz), 8.00 (1H, d, J = 8.3 Hz), 7.88 (1H, dd, J = 8.4, 1.9 Hz), 6.72-6.69 (2H, m), 4.02-3.90 (1H, m), 2.74-2.63 (2H, m), 2.37-2.20 (4H, m), 2.14-2.05 (2H, m), 1.69-1.55 (2H, m), 1.04 (3H, d, J = 6.5 Hz), | 528 | 526 |

(continued)

| | | | | | |
|---|---|---|---|---|---|
| 2-004 | 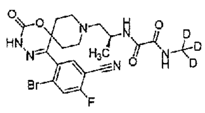 | | 1H-NMR (DMSO-D6) δ: 11.36 (1H, s), 8.59 (1H, s), 8.35 (1H, d, J = 8.3 Hz), 8.19 (1H, d, J = 6.8 Hz), 8.15 (1H, d, J = 9.3 Hz), 4.00-3.89 (1H, m), 2.75-2.60 (3H, m), 2.49-2.40 (1H, m), 2.34-2.20 (2H, m), 2.13-2.00 (2H, m), 1.81-1.59 (2H, m), 1.05 (3H, d, J = 6.5 Hz). | 512 | 510 |
| 2-005 | 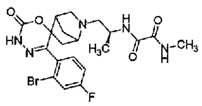 | mixture of two stereoisomers | 1H-NMR (DMSO-D6) δ: 11.33 (1H, s), 8.67 (1H, d, J = 4.9 Hz), 8.37 (1H, d, J = 7.9 Hz), 7.75 (1H, dd, J = 8.4, 2.4 Hz), 7.60 (1H, dd, J = 8.6, 5.8 Hz), 7.39 (1H, td, J = 8.4, 2.6 Hz), 3.81-3.74 (1H, m), 3.09 (1H, brs), 2.92-2.80 (2H, br m), 2.66 (3H, d, J = 5.1 Hz), 2.59-2.47 (4H, m), 1.99 (1H, br s), 1.80 (1H, brs), 1.64-1.62 (1H, br m), 1.09-1.07 (3H, m). | 496 | 494 |
| 2-006 | 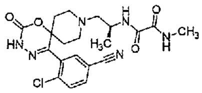 | | 1H-NMR (DMSO-D6) δ: 11.35 (1H, s), 8.64-8.58 (1H, m), 8.35 (1H, d, J = 8.5 Hz), 8.11 (1H, d, J = 2.0 Hz), 7.99 (1H, dd, J = 8.4, 1.9 Hz), 7.84 (1H, d, J = 8.3 Hz), 3.98-3.86 (1H, m), 2.75-2.60 (2H, m), 2.64 (3H, d, J = 6.5 Hz), 2.43 (1H, dd, J = 12.4, 8.1 Hz), 2.36-2.18 (3H, m), 2.15-1.98 (2H, m), 1.68-1.49 (2H, m), 1.05 (3H, d, J = 6.5 Hz). | 447 | 445 |
| 2-007 | 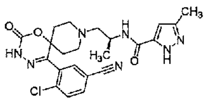 | | 1H-NMR (DMSO-D6) δ: 12.78 (1H, s), 11.35 (1H, s), 8.10 (1H, d, J = 2.0 Hz), 7.98 (1H, dd, J = 8.3, 2.0 Hz), 7.83 (1H, d, J = 8.5 Hz), 7.55 (1H, d, J = 8.3 Hz), 6.28 (1H, s), 4.13-4.01 (1H, m), 2.76-2.64 (2H, m), 2.42 (1H, dd, J = 12.3, 7.5 Hz), 2.34-2.26 (3H, m), 2.23 (3H, s), 2.12-2.02 (2H, m), 1.68-1.49 (2H, m), 1.07 (3H, d, J = 6.5 Hz). | 470 | 468 |
| 2-008 | 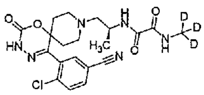 | | 1H-NMR (DMSO) δ: 11.35 (1H, s, ), 8.58 (1H, s, ), 8.34 (1H, d, J = 8.8 Hz, ), 8.11 (1H, d, J = 1.8 Hz, ), 7.99 (1H, dd, J = 8.5, 2.0 Hz, ), 7.84 (1H, d, J = 8.5 Hz, ), 3.96-3.88 (1H, m, ), 2.75-2.59 (2H, m, ), 2.43 (1H, dd, J = 12.6, 8.1 Hz, ), 2.34-2.19 (3H, m, ), 2.12-2.01 (2H, m, ), 1.69-1.48 (2H, m, ), 1.05 (3H, d, J = 6.5 Hz, ). | 450 | 448 |
| 2-009 | 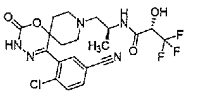 | | 1H-NMR (DMSO-D6) δ: 11.33 (1H, s), 8.05 (1H, d, J = 1.8 Hz), 7.99 (1H, dd, J = 8,3, 2.0 Hz), 7.84 (1H, d, J = 8.3 Hz), 7.77 (1H, d, J = 8.8 Hz), 7.05 (1H, d, J = 6.8 Hz), 4.44-4.34 (1H, m), 4.03-3.92 (1H, m), 2.75-2.66 (1H, m), 2.63-2.55 (1H, m), 2.38-1.98 (6H, m), 1.67-1.42 (2H, m), 1.01 (3H, d, J = 6.8 Hz). | 488 | 486 |

(continued)

| | | | | | |
|---|---|---|---|---|---|
| 2-010 | 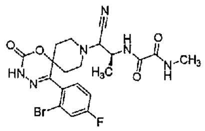 | mixture of two diastereomers | 1H-NMR (DMSO-D6) δ: 11.34 (0.5H, s), 11.31 (0.5H, s), 8.93 (0.5H, d, J = 9.7 Hz), 8.77 (0.5H, q, J = 4.9 Hz), 8.65 (0.5H, q, J = 4.9 Hz), 8.52 (0.5H, d, J = 9.5 Hz), 7.78-7.74 (1H, m), 7,58 (0.5H, dd, J = 8.6, 6.0 Hz), 7.49 (0.5H, dd, J = 8.6, 6.0 Hz), 7.41-7.31 (1H, m), 4.21-4.09 (1H, m), 3.84 (1H, t, J = 10.6 Hz), 2.80-2.54 (6H, m), 2.41-2.30 (1H, m), 2.18-2.07 (2H, m), 1.75-1.60 (1H, m), 1.58-1.40 (1H, m), 1.24 (1.5H, d, J = 6.7 Hz), 1.07 (1.5H, d, J = 6.7 Hz). | 509 | 507 |

Table 1-45

| | | | | | |
|---|---|---|---|---|---|
| 2-011 | 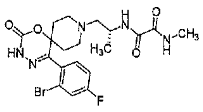 | | 1H-NMR (DMSO-D6) δ: 11.26 (1H, s), 8.63 (1H, d, J = 4.9 Hz), 8.36 (1H, d, J = 8.8 Hz), 7.75 (1H, dd, J = 8.6, 2.5 Hz), 7.54 (1H, dd, J = 8.6, 6.0 Hz), 7.36 (1H, td, J = 8.5, 2.6 Hz), 3.96-3.89 (1H, m), 2.72-2.59 (6H, m), 2.44-2.20 (3H, m), 2.10-2.03 (2H, m), 1.59-1.52 (2H, m), 1.04 (3H, d, J = 6.7 Hz), | 484 | 482 |
| 2-012 | 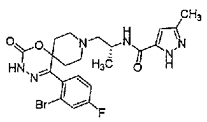 | | 1H-NMR (DMSO-D6) δ: 12.80 (1H, s), 11.25 (1H, s), 7.74 (1H, dd, J = 8.6, 2.5 Hz), 7.59-7.51 (2H, m), 7.35 (1H, td, J = 8.4, 2.8 Hz), 6.29 (1H, s), 4.11-4.02 (1H, m), 2.75-2.65 (3H, m), 2.46-2.39 (1H, m), 2.33-2.22 (5H, m), 2.11-2.04 (2H, br m), 1.61-1.51 (2H, br m), 1.07 (3H, d, J = 6.5 Hz), | 507 | 505 |
| 2-013 | 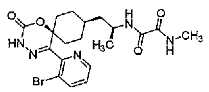 | | 1H-NMR (DMSO-D6) δ: 11.27 (1H, s), 8.69-8.62 (2H, m), 8.48-8.41 (1H, m), 8.26-8.21 (1H, m), 7.47-7.42 (1H, m), 3.97-3.85 (1H, m), 2.65 (3H, d, J = 4.8 Hz), 2.08-2.00 (2H, m), 1.69-1.44 (5H, m), 1.34-1.14 (4H, m), 1.06 (3H, d, J = 6.5 Hz). | 466 | 464 |
| 2-014 | 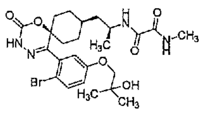 | | 1H-NMR (DMSO-D6) δ: 11.18 (1H, s), 8.69-8.63 (1H, m), 8.45 (1H, d, J = 9.2 Hz), 7.62 (1H, d, J = 9.2 Hz), 7.04-6.96 (2H, m), 4.65 (1H, s), 3.99-3.89 (1H, m), 3.78 (2H, s), 2.67 (3H, d, J = 4.9 Hz), 2.22-2.14 (2H, m), 1.73-1.17 (15H, m), 1.09 (3H, d, J = 6.7 Hz), | 553 | 551 |
| 2-015 | 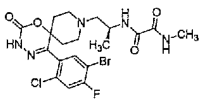 | | 1H-NMR (DMSO-D6) δ: 11.30 (1H, s), 8.65-8.58 (1H, m), 8.35 (1H, d, J = 8.8 Hz), 7.95 (1H, d, J = 7.3 Hz), 7.81 (1H, d, J = 8.5 Hz), 3.98-3.88 (1H, m), 2.76-2.62 (2H, m), 2.64 (3H, d, J = 5.0 Hz), 2.43 (1H, dd, J = 12.5, 8.0 Hz), 2.35-2.18 (3H, m), 2.12-1.98 (2H, m), 1.68-1.49 (2H, m), 1.05 (3H, d, J = 6.5 Hz), | 518 | 516 |
| 2-016 | 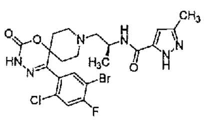 | | 1H-NMR (DMSO-D6) δ: 12.79 (1H, s), 11.30 (1H, s), 7.95 (1H, d, J = 7.3 Hz), 7.80 (1H, d, J = 8.8 Hz), 7.61-7.50 (1H, m), 6.28 (1H, s), 4.12-4.01 (1H, m), 2.76-2.65 (2H, m), 2.45-2.38 (1H, m), 2.34-2.18 (6H, m), 2.10-2.02 (2H, m), 1.65-1.50 (2H, m), 1.07 (3H, d, J = 6.5 Hz). | 541 | 539 |
| 2-017 | 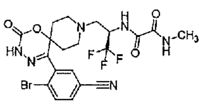 | | 1H-NMR (DMSO-D6) δ: 11.38 (1H, s), 9.23 (1H, d, J = 9.0 Hz), 8.78 (1H, q, J = 4.9 Hz), 8.07 (1H, d, J = 1.8 Hz), 8.00 (1H, d, J = 8.3 Hz), 7.88 (1H, dd, J = 8.4, 2.0 Hz), 4.65-4.55 (1H, m), 2.95 (1H, dd, J = 13.1, 9.8 Hz), 2.74-2.58 (7H, m), 2.33-2.26 (1H, m), 2.14-2.05 (2H, br m), 1.68-1,47 (2H, br m). | 545 | 543 |

(continued)

| | | | | | |
|---|---|---|---|---|---|
| 2-018 | 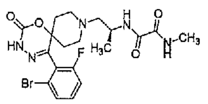 | | 1H-NMR (DMSO-D6) δ: 11.44 (1H, s), 8.67-8.60 (1H, m), 8.37 (1H, d, J = 8.1 Hz), 7.67-7.62 (1H, m), 7.54-7.41 (2H, m), 3.97-3.87 (1H, m), 2.76-2.60 (2H, m), 2.64 (3H, d, J = 4.8 Hz), 2.46-2.02 (6H, m), 1.72-1.46 (2H, m), 1.05 (3H, d, J = 6.5 Hz), | 484 | N.D. |
| 2-019 | 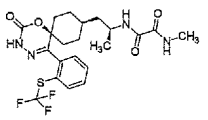 | | 1H-NMR (DMSO-D6) δ: 11.30 (1H, s), 8.66-8.60 (1H, m), 8.42 (1H, d, J = 9.2 Hz), 7.87-7.82 (1H, m), 7.66-7.61 (3H, m), 3.96-3.86 (1H, m), 2.64 (3H, d, J = 4.8 Hz), 2.08-1.99 (2H, m), 1.72-1.11 (9H, m), 1.06 (3H, d, J = 6.5 Hz). | 487 | 485 |
| 2-020 | 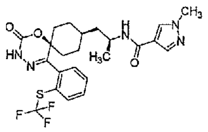 | | 1H-NMR (DMSO-D6) δ: 11.30 (1H, s), 8.06 (1H, s), 7.87-7.81 (1H, m), 7.78 (1H, s), 7.68-7.59 (4H, m), 4.09-3.98 (1H, m), 3.82 (3H, s), 2.09-2.01 (2H, m), 1.73-1.21 (9H, m), 1.06 (3H, d, J = 6.5 Hz). | 510 | 508 |

Table 1-46

| | | | | | |
|---|---|---|---|---|---|
| 2-021 | 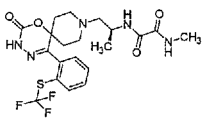 | | 1H-NMR (DMSO-D6) δ: 11.36 (1H, s), 8.71-8.59 (1H, m), 8.42-8.33 (1H, m), 7.93-7.83 (1H, m), 7.73-7.61 (3H, m), 4.02-3.87 (1H, m), 2.78-2.60 (2H, m), 2.64 (3H, d, J = 4.8 Hz), 2.43-2.19 (4H, m), 2.10-1.95 (2H, m), 1.75-1.54 (2H, m), 1.15-1.01 (3H, m). | 488 | 486 |
| 2-022 | 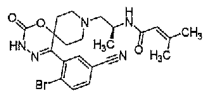 | | 1H-NMR (DMSO-D6) δ: 11.34 (1H, s), 8.07 (1H, d, J = 2.0 Hz), 8.01 (1H, d, J = 8.5 Hz), 7.89 (1H, dd, J = 8.3, 2.0 Hz), 7.41 (1H, d, J = 8.3 Hz), 5.56 (1H, s, ), 3.96-3.86 (1H, m), 2.76-2.63 (2H, m), 2.34-2.21 (3H, m), 2.19-2.03 (3H, m), 1.98 (3H, s), 1.74 (3H, s), 1.69-1.51 (2H, m), 0.98 (3H, d, J = 6.5 Hz). | 488 | 486 |
| 2-023 | 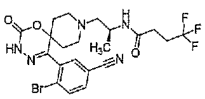 | | 1H-NMR (DMSO-D6) δ: 11.34 (1H, s), 8.07 (1H, d, J = 2.0 Hz), 8.01 (1H, d, J = 8.5 Hz), 7.89 (1H, dd, J = 8.3, 2.0 Hz), 7.41 (1H, d, J = 8.3 Hz), 5.56 (1H, s), 3.96-3.86 (1H, m), 2.76-2.63 (2H, m), 2.34-2.21 (3H, m), 2.19-2.03 (3H, m), 1.98 (3H, s), 1.74 (3H, s), 1.69-1.51 (2H, m), 0.98 (3H, d, J = 6.5 Hz). | 530 | 528 |
| 2-024 | 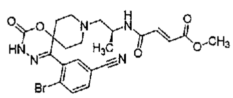 | | 1H-NMR (DMSO-D6) &: 11.35 (1H, s), 8.36 (1H, d, J = 1.5 Hz), 7.95 (1H, dd, J = 8.0, 1.5 Hz), 7.82 (1H, d, J = 8.5 Hz), 7.70-7.66 (2H, m), 7.47 (1H, d, J = 1.0 Hz), 4.13-4.00 (1H, m), 3.73 (3H, s), 3.48-3.40 (1H, m), 2.75-2.65 (2H, m), 2.40-2.22 (3H, m), 2.14-2.03 (2H, m), 1.64-1.48 (2H, m), 1.08-1.03 (3H, m). | 518 | 516 |
| 2-025 | 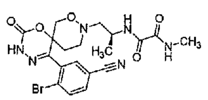 | | 1H-NMR (DMSO-D6) δ: 11.47 (1H, s), 8.68-8.60 (1H, m), 8.53-8.48 (0.5H, m), 8.40-8.35 (0.5H, m), 8.13-8.09 (1H, m), 8.04-7.99 (1H, m), 7.93-7.87 (1H, m), 4.24-4.11 (1H, m), 4.10-3.98 (1H, m), 3.90-3.73 (1H, m), 2.92-2.58 (7H, m), 2.27-2.18 (1H, m), 1.97-1.84 (1H, m), 1.11-1.03 (3H, m). | 493 | 491 |
| 2-026 | 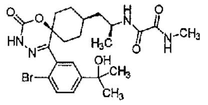 | | 1H-NMR (DMSO-D6) δ: 11.15 (1H, s), 8.65-8.59 (1H, m), 8.41 (1H, d, J = 9.2 Hz), 7.63 (1H, d, J = 9.2 Hz), 7.47-7.40 (2H, m), 5.19 (1H, s), 3.96-3.82 (1H, m), 2.63 (3H, d, J = 5.4 Hz), 2.20-2.09 (2H, m), 1.71-1.11 (9H, m), 1.41 (6H, s), 1.06 (3H, d, J = 6.5 Hz). | 523 | 521 |

(continued)

| | | | | | |
|---|---|---|---|---|---|
| 2-027 | 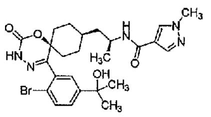 | | 1H-NMR (DMSO-D6) δ: 11.15 (1H, s), 8.04 (1H, s), 7.76 (1H, s), 7.67-7.60 (2H, m), 7.48-7.37 (2H, m), 5.18 (1H, s), 4.09-3.96 (1H, m), 3.81 (3H, s), 2.21-2.09 (2H, m), 1.73-1.15 (9H, m), 1.39 (6H, s), 1.06 (3H, d, J = 4.3 Hz). | 546 | 544 |
| 2-028 | 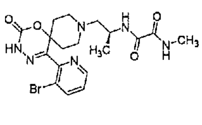 | | 1H-NMR (DMSO-D6) δ: 11.36 (1H, s), 8.71-8.65 (2H, m), 8.43-8.39 (1H, m), 8.30-8.27 (1H, m), 7.52-7.47 (1H, m), 4.02-3.92 (1H, m), 2.78-2.61 (2H, m), 2.69 (3H, d, J = 4.9 Hz), 2.55-2.24 (4H, m), 2.06-1.77 (4H, m), 1.09 (3H, d, J = 6.5 Hz). | 467 | N.D. |
| 2-029 | 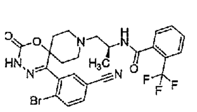 | | 1H-NMR (DMSO-D6) δ; 11.35 (1H, s), 8.17 (1H, d, J = 8.5 Hz), 8.08 (1H, d, J = 2.0 Hz), 8.01 (1H, d, J = 8.5 Hz), 7.89 (1H, dd, J = 8.5, 2.0 Hz), 7.73 (1H, d, J = 7.5 Hz), 7.70-7.58 (2H, m), 7.41 (1H, d, J = 7.3 Hz), 4.14-4.03 (1H, m), 2.81-2.74 (1H, m), 2.71-2.64 (1H, m), 2.42-2.21 (4H, m), 2.17-2.07 (2H, m), 1.74-1.53 (2H, m), 1.06 (3H, d, J = 6.5 Hz). | 578 | 576 |
| 2-030 | 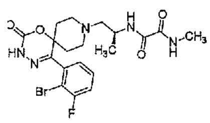 | | 1H-NMR (DMSO-D6) δ: 11.30 (1H, s), 8.62 (1H, d, J = 5.1 Hz), 8.35 (1H, d, J = 8.8 Hz), 7.56-7.47 (2H, m), 7.32 (1H, dd, J = 7.3, 1.7 Hz), 3.96-3.88 (1H, m), 2.73-2.60 (5H, m), 2.42 (1H, dd, J = 12.5, 8.1 Hz), 2.33-2.21 (3H, m), 2.11-2.03 (2H, br m), 1.63-1.50 (2H, m), 1.04 (3H, d, J = 6.7 Hz). | 484 | 482 |

Table 1-47

| | | | | | |
|---|---|---|---|---|---|
| 2-031 | 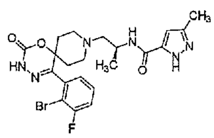 | | 1H-NMR (DMSO-D6) δ: 12.79 (1H, s), 11.30 (1H, s), 7.58-7.46 (3H, m), 7.31 (1H, dd, J = 6.8, 2.0 Hz), 6.28 (1H, s), 4.09-4.02 (1H, m), 2.74-2.65 (2H, br m), 2.42 (1H, dd, J = 12.5, 7.4 Hz), 2.33-2.24 (6H, m), 2.11-2.04 (2H, br m), 1.63-1.53 (2H, br m), 1.06 (3H, d, J = 6.7 Hz). | 507 | 505 |
| 2-032 | 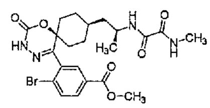 | | 1H-NMR (DMSO-D6) δ: 11.25 (1H, s), 8,65-8,59 (1H, m), 8.43-8.38 (1H, m), 7.94-7.90 (2H, m), 7.86-7.83 (1H, m), 3.96-3.83 (1H, m), 3.87 (3H, s), 2.63 (3H, d, J = 4.8 Hz), 2.21-2.12 (2H, m), 1.71-1.15 (9H, m), 1.05 (3H, d, J = 6.5 Hz). | 523 | 521 |
| 2-033 | 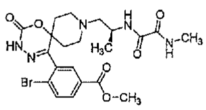 | | 1H-NMR (DMSO-D6) δ: 11.32 (1H, s), 8.65-8,58 (1H, m), 8.39-8.33 (1H, m), 7.95-7.92 (2H, m), 7.89-7.86 (1H, m), 3.97-3.85 (1H, m), 3.87 (3H, s), 2.75-2.60 (2H, m), 2.62 (3H, d, J = 4.8 Hz), 2.46-2.07 (6H, m), 1.63-1.46 (2H, m), 1.04 (3H, d, J = 7.0 Hz). | 524 | 522 |
| 2-034 | 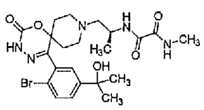 | | 1H-NMR (DMSO-D6) δ: 11.20 (1H, s), 8.64-8.58 (1H, m), 8.38-8.33 (1H, m), 7.67-7.63 (1H, m), 7.50-7.45 (1H, m), 7.43-7.40 (1H, m), 5.21 (1H, s), 3.97-3.87 (1H, m), 2.74-2.60 (2H, m), 2.63 (3H, d, J = 4.8 Hz), 2.46-2.02 (6H, m), 1.65-1.48 (2H, m), 1.41 (6H, s), 1.04 (3H, d, J = 6.5 Hz). | 524 | 522 |
| 2-035 | 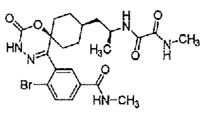 | | 1H-NMR (DMSO-D6) δ: 11.23 (1H, s), 8.65-8.57 (2H, m), 8.43-8.38 (1H, m), 7.87-7.80 (2H, m), 7.78-7.76 (1H, m), 3.96-3.84 (1H, m), 2.78 (3H, d, J = 4.3 Hz), 2.63 (3H, d, J = 4.8 Hz), 2.20-2.10 (2H, m), 1.71-1.17 (9H, m), 1.05 (3H, d, J = 6.5 Hz). | 522 | 520 |

(continued)

| | | | | | |
|---|---|---|---|---|---|
| 2-036 | 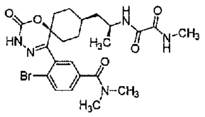 | | 1H-NMR (DMSO-D6) δ: 11.21 (1H, s), 8.66-8.60 (1H, m), 8.44-8.38 (1H, m), 7.82-7.78 (1H, m), 7.48-7.45 (1H, m), 7.43-7.39 (1H, m), 3.96-3.85 (1H, m), 2.97 (3H, s), 2.87 (3H, s), 2,64 (3H, d, J = 4.8 Hz), 2.19-2.09 (2H, m), 1.71-1.13 (9H, m), 1.05 (3H, d, J = 6.5 Hz). | 536 | 534 |
| 2-037 | 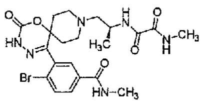 | | 1H-NMR (DMSO-D6) δ: 11.29 (1H, s), 8.65-8.57 (2H, m), 8.38-8.34 (1H, m), 7.88-7.83 (2H, m), 7.81-7.78 (1H, m), 3.98-3.89 (1H, m), 2.79 (3H, d, J = 4.3 Hz), 2.75-2.62 (2H, m), 2.63 (3H, d, J = 4.8 Hz), 2.47-2.06 (6H, m), 1.71-1.51 (2H, m), 1.04 (3H, d, J = 6.5 Hz). | 523 | 521 |
| 2-038 | 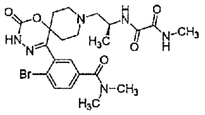 | | 1H-NMR (DMSO-D6) δ: 11.27 (1H, s), 8.67-8.58 (1H, m), 8.37-8.31 (1H, m), 7.85-7.80 (1H, m), 7.51-7.48 (1H, m), 7.45-7.40 (1H, m), 3.98-3.87 (1H, m), 2.98 (3H, s), 2.87 (3H, s), 2.74-2.60 (2H, m), 2.64 (3H, d, J = 4.8 Hz), 2.47-2.03 (6H, m), 1.67-1.49 (2H, m), 1.04 (3H, d, J = 6.5 Hz). | 537 | 535 |
| 2-039 | 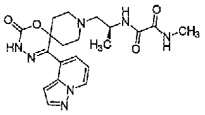 | | 1H-NMR (DMSO-D6) δ: 11.30 (1H, s), 8.77 (1H, d, J = 6.9 Hz), 8.61 (1H, t, J = 4.9 Hz), 8.36 (1H, d, J = 8.6 Hz), 8.04 (1H, d, J = 2.3 Hz), 7.28 (1H, dd, J = 6.9, 0.9 Hz), 6.95 (1H, t, J = 7.1 Hz), 6.60 (1H, dd, J = 2.3, 0.9 Hz), 3.96-3.89 (1H, m), 2.73-2.60 (5H, m), 2.42 (1H, dd, J = 12.5, 8.1 Hz), 2.35-2.24 (3H, m), 2.08-2.04 (2H, br m), 1.79-1.66 (2H, m), 1.04 (3H, d, J = 6.7 Hz). | 428 | 426 |
| 2-040 | 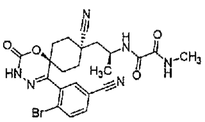 | | 1H-NMR (DMSO-D6) δ: 11.44 (1H, s), 8.72 (1H, d, J = 9.0 Hz), 8.59 (1H, d, J = 4.9 Hz), 8.14 (1H, d, J = 1.8 Hz), 8.03 (1H, d, J = 8.6 Hz), 7.90 (1H, dd, J = 8.3, 2.1 Hz), 4.11 (1H, d, J = 9.7 Hz), 2.64 (3H, d, J = 4.9 Hz), 2.23 (2H, t, J = 11.8 Hz), 2.10-2.04 (1H, m), 2.01-1.97 (1H, m), 1.79-1.56 (6H, m), 1.12 (3H, d, J = 6.7 Hz). | 515 | 513 |

Table 1-48

| | | | | | |
|---|---|---|---|---|---|
| 2-041 | 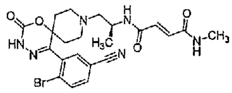 | | 1H-NMR (DMSO-D6) δ: 11.35 (1H, s), 8.31-8.24 (1H, m), 8.15 (1H, d, J = 8.0 Hz), 8.08 (1H, d, J = 2.0 Hz), 8.00 (1H, d, J = 8.3 Hz), 7.87 (1H, dd, J = 8.3, 2.0 Hz), 6.77 (1H, d, J = 15.3 Hz), 6.72 (1H, d, J = 15.3 Hz), 4.02-3.89 (1H, m), 2.71-2.65 (1H, m), 2.67 (3H, d, J = 5.0 Hz), 2.36-2.16 (5H, m), 2.15-2.03 (2H, m), 1.71-1.53 (2H, m), 1.02 (3H, d, J = 6.5 Hz). | 517 | 515 |
| 2-042 | 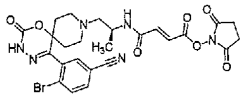 | | 1H-NMR (DMSO-D6) δ: 11.35 (1H, s), 8.53-8.45 (1H, m), 8.08 (1H, d, J = 1.8 Hz), 8.00 (1H, d, J = 8.5 Hz), 7.87 (1H, dd, J = 8.4, 2.1 Hz), 7.27 (1H, d, J = 15.5 Hz), 6.77 (1H, d, J = 15.5 Hz), 4.06-3.94 (1H, m), 2.77 (4H, s), 2.73-2.64 (1H, m), 2.41-2.22 (5H, m), 2.18-2.03 (2H, m), 1.71-1.57 (2H, m), 1.06 (3H, d, J = 6.5 Hz). | 601 | 599 |

(continued)

| | | | | | |
|---|---|---|---|---|---|
| 2-043 | 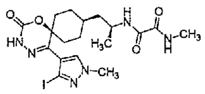 | | 1H-NMR (DMSO-D6) δ: 11.11 (1H, s), 8.68-8.62 (1H, m), 8.47-8.41 (1H, m), 8.00 (1H, s), 3.99-3.82 (1H, m), 3.85 (3H, s), 2.65 (3H, d, J = 4.8 Hz), 1.97-1.87 (2H, m), 1.81-1.43 (5H, m), 1.35-1.16 (4H, m), 1.07 (3H, d, J = 6.5 Hz), | 517 | 515 |
| 2-044 | 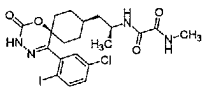 | | 1H-NMR (DMSO-D6) δ: 11.27 (1H, s), 8.70-8.63 (1H, m), 8.48-8.42 (1H, m), 7.99 (1H, d, J = 8.4 Hz), 7.53 (1H, d, J = 2.4 Hz), 7.32 (1H, dd, J = 8.4, 2.4 Hz), 4.00-3.89 (1H, m), 2.68 (3H, d, J = 4.9 Hz), 2.22-2.13 (2H, m), 1,74-1.17 (9H, m), 1.10 (3H, d, J = 6.5 Hz). | 547 | 545 |
| 2-045 | 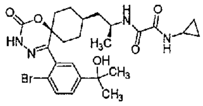 | | 1H-NMR (DMSO-D6) δ: 11.14 (1H, s), 8.70-8.65 (1H, m), 8.44-8.38 (1H, m), 7.66-7.61 (1H, m), 7.48-7.43 (1H, m), 7.42-7.39 (1H, m), 5.19 (1H, s), 3.94-3.84 (1H, m), 2.76-2.67 (1H, m), 2.20-2.09 (2H, m), 1.71-1.11 (9H, m), 1.41 (6H, s), 1.05 (3H, d, J = 6.5 Hz), 0.66-0.55 (4H, m). | 549 | 547 |
| 2-046 | 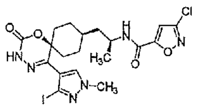 | | 1H-NMR (DMSO-D6) δ: 11.11 (1H, s), 8.80 (1H, d, J = 8.1 Hz), 7.99 (1H, s), 7.34 (1H, s), 4.14-4.01 (1H, m), 3.84 (3H, s), 1.98-1.20 (11H, m), 1.13 (3H, d, J = 6.5 Hz). | 561 | 559 |
| 2-047 | 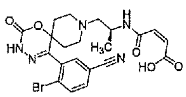 | | 1H-NMR (DMSO-D6) δ: 11.35 (1H, s), 8.07 (1H, d, J = 2.0 Hz), 8.00 (1H, d, J = 8.5 Hz), 7.87 (1H, dd, J = 8.5, 2.0 Hz), 6.13 (1H, d, J = 12.8 Hz), 6.07-5.98 (1H, m), 3.97-3.89 (1H, m), 2,74-2.67 (2H, m), 2.40-2,20 (4H, m), 2.14-2.04 (2H, m), 1.73-1.58 (2H, m), 1.04 (3H, d, J = 6.5 Hz). | 504 | 502 |
| 2-048 | 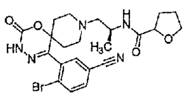 | Mixture of two diastereomers due to 2-position of furan ring | 1H-NMR (DMSO-D6) δ: 11.34-11.34 (1H, m), 8.09-8.07 (1H, m), 8.04-7.99 (1H, m), 7.92-7.87 (1H, m), 7.37 (0.5H, d, J = 8.8 Hz), 7.23 (0.5H, d, J = 8.8 Hz), 4.16-4.05 (1H, m), 3.97-3.61 (3H, m), 2.76-2.54 (2H, m), 2.41-1.87 (7H, m), 1.81-1.35 (5H, m), 1.00 (3H, d, J = 6.5 Hz), | 504 | 502 |
| 2-049 | 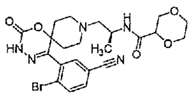 | Mixture of two diastereomers due to 2-position of dioxane ring | 1H-NMR (DMSO-D6) δ: 11.36-11.32 (1H, m), 8.09-8.06 (1H, m), 8.03-7.98 (1H, m), 7.91-7.85 (1H, m), 7.44 (0.5H, d, J = 8.6 Hz), 7.36 (0.5H, d, J = 8.6 Hz), 4,00-3,19 (8H, m), 2.73-2.58 (2H, m), 2.40-2.01 (6H, m), 1.72-1.41 (2H, m), 1.00 (3H, d, J = 6.5 Hz). | 520 | 518 |
| 2-050 | 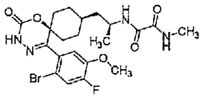 | | 1H-NMR (DMSO-D6) δ: 11.18 (1H, s), 8.66-8.59 (1H, m), 8.44-8.38 (1H, m), 7.44-7.36 (1H, m), 7.25-7.19 (1H, m), 3.95-3.84 (4H, m), 2.64 (3H, d, J = 4.8 Hz), 2.16-2.06 (2H, m), 1.69-1.12 (9H, m), 1.05 (3H, d, J = 6.5 Hz), | 513 | 511 |

Table 1-49

| | | | | | |
|---|---|---|---|---|---|
| 2-051 | 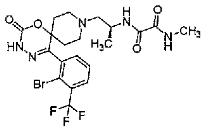 | | 1H-NMR (DMSO-D6) δ: 11.30 (1H, s), 8.77 (1H, d, J = 6.9 Hz), 8.61 (1H, t, J = 4.9 Hz), 8.36 (1H, d, J = 8.6 Hz), 8.04 (1H, d, J = 2.3 Hz), 7.28 (1H, dd, J = 6.9, 0.9 Hz), 6.95 (1H, t, J = 7.1 Hz), 6.60 (1H, dd, J = 2.3, 0.9 Hz), 3.96-3.89 (1H, m), 2.73-2.60 (5H, m), 2.42 (1H, dd, J = 12.5, 8.1 Hz), 2.35-2.24 (3H, m), 2.08-2.04 (2H, br m), 1.79-1.66 (2H, m), 1.04 (3H, d, J = 6.7 Hz), | 428 | 426 |
| 2-052 | 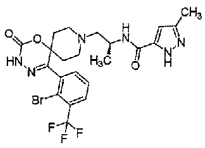 | | 1H-NMR (DMSO-D6) δ: 11.44 (1H, s), 8.72 (1H, d, J = 9.0 Hz), 8.59 (1H, d, J = 4.9 Hz), 8.14 (1H, d, J = 1.8 Hz), 8.03 (1H, d, J = 8.6 Hz), 7.90 (1H, dd, J = 8.3, 2.1 Hz), 4.11 (1H, d, J = 9.7 Hz), 2.64 (3H, d, J = 4.9 Hz), 2.23 (2H, t, J = 11.8 Hz), 2.10-2.04 (1H, m), 2.01-1.97 (1H, m), 1.79-1.56 (6H, m), 1.12 (3H, d, J = 6.7 Hz). | 515 | 513 |
| 2-053 | 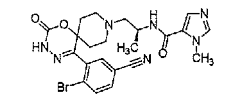 | | 1H-NMR (DMSO-D6) δ: 11.34 (1H, s), 8.06 (1H, d, J = 2.0 Hz), 8.00 (1H, d, J = 8.3 Hz), 7.87 (1H, dd, J = 8.3, 2.0 Hz), 7.83 (1H, d, J = 8.3 Hz), 7.67 (1H, s), 7.48 (1H, d, J = 1.0 Hz), 4.13-4.04 (1H, m), 3.73 (3H, s), 2.77-2.65 (2H, m), 2.41-2.23 (4H, m), 2.14-2.05 (2H, m), 1.68-1.52 (2H, m), 1.07 (3H, d, J = 6.5 Hz). | 514 | 512 |
| 2-054 | 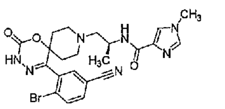 | | 1H-NMR (DMSO-D6) δ: 11.34 (1H, s), 8.06 (1H, d, J = 2.2 Hz), 8.00 (1H, d, J = 8.5 Hz), 7.90-7.85 (1H, m), 7.58 (1H, s), 7.54 (1H, d, J = 1.0 Hz), 7.48 (1H, d, J = 8.3 Hz), 4.08-4,00 (1H, m), 3.67 (3H, s), 2.74-2.64 (2H, m), 2.43 (1H, dd, J = 12.3, 7.8 Hz), 2.34-2.23 (3H, m), 2.13-2.03 (2H, m), 1.70-1.53 (2H, m), 1.08 (3H, d, J = 6.5 Hz), | 514 | N.D. |
| 2-055 | 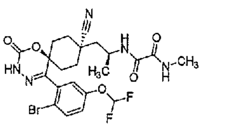 | | 1H-NMR (DMSO-D6) δ: 11.37 (1H, s), 8.75-8.70 (1H, m), 8.63-8.57 (1H, m), 7.85-7.80 (1H, m), 7.50-7.30 (2H, m), 7.29-7.24 (1H, m), 4.17-4.07 (1H, m), 2.64 (3H, d, J = 4.8 Hz), 2.28-1.96 (4H, m), 1.81-1.53 (6H, m), 1.12 (3H, d, J = 6.5 Hz), | 556 | 554 |
| 2-056 | 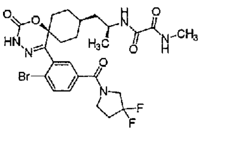 | | 1H-NMR (DMSO-D6) δ: 11.22 (1H, s), 8.67-8.57 (1H, m), 8.46-8.37 (1H, m), 7.86-7.79 (1H, m), 7.63-7.51 (2H, m), 3.97-3.82 (3H, m), 3.74-3.61 (2H, m), 2.63 (3H, d, J = 4.8 Hz), 2.55-2.36 (2H, m), 2.20-2.09 (2H, m), 1.71-1.14 (9H, m), 1.05 (3H, d, J = 6.5 Hz), | 598 | 596 |
| 2-057 | 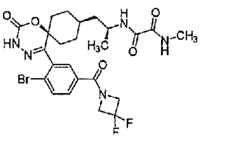 | | 1H-NMR (CDCl3) δ: 8.27 (1H, s), 7.77-7.72 (1H, m), 7.53-7.49 (2H, m), 7.46-7.40 (1H, m), 7.20-7.14 (1H, m), 4.60-4.50 (4H, m), 4,09-3.98 (1H, m), 2.88 (3H, d, J = 5.4 Hz), 2.37-2.27 (2H, m), 1.80-1.33 (9H, m), 1.16 (3H, d, J = 6.5 Hz), | 584 | 582 |
| 2-058 | 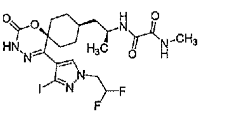 | | 1H-NMR (DMSO-D6) δ: 11.17 (1H, s), 8.69-8.62 (1H, m), 8.49-8.41 (1H, m), 8.09 (1H, s), 6.55-6.23 (1H, m), 4.70-4.58 (2H, m), 3.99-3.87 (1H, m), 2.65 (3H, d, J = 4.8 Hz), 1.98-1.88 (2H, m), 1.84-1.41 (5H, m), 1.35-1,17 (4H, m), 1.07 (3H, d, J = 6.5 Hz), | 567 | 565 |
| 2-059 | 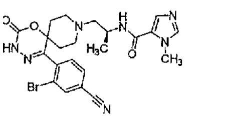 | | 1H-NMR (DMSO-D6) δ: 11.35 (1H, s), 8.36 (1H, d, J = 1.5 Hz), 7.95 (1H, dd, J = 8.0, 1.5 Hz), 7.82 (1H, d, J = 8.5 Hz), 7.70-7.66 (2H, m), 7.47 (1H, d, J = 1.2 Hz), 4.10-4.01 (1H, m), 3.73 (3H, s), 2.76-2.64 (2H, m), 2.42-2.22 (4H, m), 2.15-2.05 (2H, m), 1.63-1.47 (2H, m), 1,09-1.00 (3H, m). | 514 | 512 |

(continued)

| | | | | | |
|---|---|---|---|---|---|
| 2-060 | 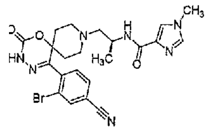 | | 1H-NMR (DMSO-D6) δ: 11.36 (1H, s), 8.35 (1H, d, J = 1.5 Hz), 7.96 (1H, dd, J = 8.0, 1.5 Hz), 7.69 (1H, d, J = 8.0 Hz), 7.59 (1H, s), 7.54 (1H, d, J = 1.0 Hz), 7.48 (1H, d, J = 8.3 Hz), 4.09-3.97 (1H, m), 3.67 (3H, s), 2.73-2.64 (2H, m), 2.43 (1H, dd, J = 12.4, 7.9 Hz), 2.35-2.22 (3H, m), 2.12-2.03 (2H, m), 1.64-1.48 (2H, m), 1.07 (3H, d, J = 6.5 Hz). | 514 | 512 |

Table 1-50

| | | | | | |
|---|---|---|---|---|---|
| 2-061 | 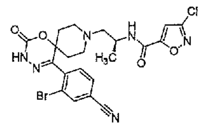 | | 1H-NMR (DMSO-D6) δ: 11.36 (1H, s), 8.71 (1H, d, J = 8.3 Hz), 8.35 (1H, d, J = 1.5 Hz), 7.94 (1H, dd, J = 7.9, 1.6 Hz), 7.68 (1H, d, J = 8.0 Hz), 7.28 (1H, s), 4.14-4.04 (1H, m), 2.76-2.61 (2H, m), 2.46-2.23 (4H, m), 2.12-2.07 (2H, m), 1.58-1.51 (2H, m), 1.09 (3H, d, J = 6.5 Hz). | 535 | 533 |
| 2-062 | 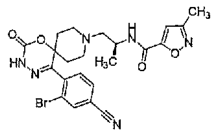 | | 1H-NMR (DMSO-D6) δ: 11.36 (1H, s), 8.51 (1H, d, J= 8.5 Hz), 8.35 (1H, d, J = 1.5 Hz), 7.95 (1H, dd, J = 8.0, 1.5 Hz), 7.68 (1H, d, J = 8.0 Hz), 6.82 (1H, s), 4.13-4.01 (1H, m), 2.76-2.61 (2H, m), 2.46-2.38 (1H, m), 2.36-2.24 (3H, m), 2.28 (3H, s), 2.12-2.05 (2H, m), 1.61-1.46 (2H, m), 1.08 (3H, d, J = 6.5 Hz). | 515 | 513 |
| 2-063 | 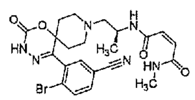 | | 1H-NMR (DMSO-D6) δ: 11.35 (1H, s), 8.32-7.83 (6H, m), 6.75 (1H, d, J = 5.0 Hz), 4.00-3.84 (1H, m), 2.73-2.64 (1H, m), 2.67 (3H, d, J = 4.8 Hz), 2.35-2.02 (7H, m), 1.70-1.56 (2H, m), 1.02 (3H, d, J = 6.5 Hz), | 517 | 515 |
| 2-064 | 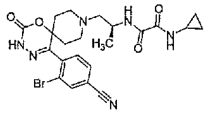 | | 1H-NMR (DMSO-D6) δ: 11.36 (1H, s), 8.67 (1H, d, J = 5.3 Hz), 8.38-8.31 (2H, m), 7.96 (1H, dd, J = 8.0, 1.5 Hz), 7.70 (1H, d, J = 8.0 Hz), 3.97-3.86 (1H, m), 2.76-2.67 (2H, m), 2.65-2.59 (1H, m), 2.42 (1H, dd, J = 12.5, 8.3 Hz), 2.36-2.19 (3H, m), 2.16-2.03 (2H, m), 1.64-1.48 (2H, m), 1.04 (3H, d, J = 6.5 Hz), 0.68-0.53 (4H, m). | 517 | 515 |
| 2-065 | 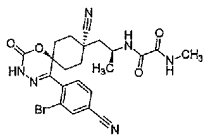 | | 1H-NMR (DMSO-D6) δ: 11.46 (1H, s), 8.73 (1H, d, J = 9.2 Hz), 8.60 (1H, d, J = 4.8 Hz), 8.39 (1H, s), 8.01 (1H, d, J = 8.1 Hz), 7.75 (1H, d, J = 8.1 Hz), 4.12 (1H, brs), 2.64 (3H, d, J = 4.8 Hz), 2.24 (2H, t, J = 10.5 Hz), 2.07 (1H, dd, J = 13.7, 11.0 Hz), 1.99 (1H, br s), 1.79-1.53 (6H, m), 1.12 (3H, d, J = 6.5 Hz). | 515 | 513 |
| 2-066 | 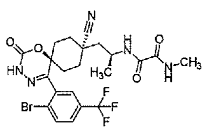 | | 1H-NMR (DMSO-D6) δ: 11.43 (1H, s), 8.75-8.69 (1H, m), 8.62-8.57 (1H, m), 8.06-8.01 (1H, m), 7.94 (1H, s), 7.83-7.78 (1H, m), 4.16-4.05 (1H, m), 2.65-2.62 (3H, m), 2.33-2.19 (2H, m), 2.11-1.96 (2H, m), 1.81-1.52 (6H, m), 1.12 (3H, d, J = 6.5 Hz), | 558 | 556 |
| 2-067 | 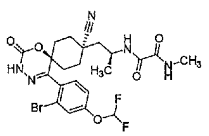 | | 1H-NMR (DMSO-D6) δ: 11.34 (1H, s), 8.75-8.69 (1H, m), 8.63-8.57 (1H, m), 7.65-7.62 (1H, m), 7.60-7.55 (1H, m), 7.41-7.20 (2H, m), 4.18-4.06 (1H, m), 2.66-2.62 (3H, m), 2.27-1.97 (4H, m), 1.82-1.54 (6H, m), 1.12 (3H, d, J = 6.5 Hz), | 556 | 554 |
| 2-068 | 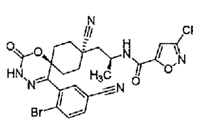 | | 1H-NMR (DMSO-D6) δ: 11.44 (1H, s), 9.01 (1H, d, J = 8.6 Hz), 8.16-8.13 (1H, m), 8.04-8.00 (1H, m), 7.91-7.88 (1H, m), 7.30-7.28 (1H, m), 4.35-4.24 (1H, m), 2.32-2.17 (2H, m), 2.07-1.97 (2H, m), 1.87-1.54 (6H, m), 1,20-1.16 (3H, m). | 559 | 557 |

(continued)

| | | | | | |
|---|---|---|---|---|---|
| 2-069 | 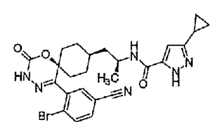 | | 1H-NMR (DMSO-D6) δ: 12.91 (0.25H, s), 12.85 (0.75H, s), 11.29 (1H, s), 8.01-7.93 (2.5H, m), 7.86-7.81 (1H, m), 7.61-7.54 (0.5H, m), 6.49 (0.25H, s), 6.22 (0.75H, s), 4.10-4.00 (1H, m), 2,19-1.79 (3H, m), 1.74-1.14 (9H, m), 1.07 (3H, d, J = 6.5 Hz), 0.98-0,55 (4H, m). | 539 | 537 |
| 2-070 | 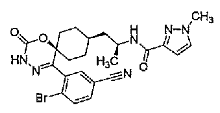 | | 1H-NMR (DMSO-D6) δ: 11.29 (1H, s), 8.01-7.94 (2H, m), 7.86-7.81 (1H, m), 7.74-7.71 (1H, m), 7.68-7.63 (1H, m), 6.57-6.54 (1H, m), 4.12-4.02 (1H, m), 3.88-3.86 (3H, m), 2.17-2.06 (2H, m), 1.73-1.18 (9H, m), 1.08 (3H, d, J = 6.5 Hz). | 513 | 511 |

Table 1-51

| | | | | | |
|---|---|---|---|---|---|
| 2-071 | 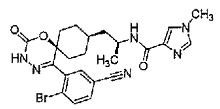 | | 1H-NMR (DMSO-D6) δ: 11.28 (1H, s), 8.01-7.94 (2H, m), 7.86-7.81 (1H, m), 7.61-7.59 (1H, m), 7.55-7.53 (1H, m), 7.47-7.41 (1H, m), 4.09-3.99 (1H, m), 3.66 (3H, s), 2.16-2.06 (2H, m), 1.74-1.18 (9H, m), 1.07 (3H, d, J = 7.0 Hz), | 513 | 511 |
| 2-072 | 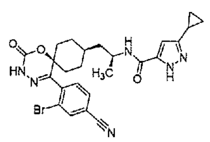 | | 1H-NMR (DMSO-D6) δ: 12.95-12.82 (1H, m), 11.30 (1H, s), 8.34-8.31 (1H, m), 7.94-7.89 (1H, m), 7.69-7.64 (1H, m), 7.61-7.54 (1H, m), 6.54-6.18 (1H, m), 4.12-3.98 (1H, m), 2.17-2.07 (2H, m), 1.95-1.64 (2H, m), 1.57-1.12 (8H, m), 1.07 (3H, d, J = 5.9 Hz), 0.98-0.56 (4H, m). | 539 | 537 |
| 2-073 | 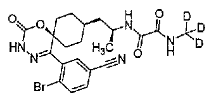 | | 1H-NMR (DMSO-D6) δ: 11.28 (1H, s), 8.59 (1H, s), 8.40 (1H, d, J = 9.0 Hz), 8.00 (1H, d, J = 1.8 Hz), 7.98 (1H, d, J = 8.3 Hz), 7.85 (1H, dd, J = 8.3, 1.8 Hz), 3.98-3.85 (1H, m), 2.16-2.04 (2H, m), 1.69-1.59 (1H, m), 1.59-1.13 (8H, m), 1.06 (3H, d, J = 6.5 Hz). | 493 | 491 |
| 2-074 | 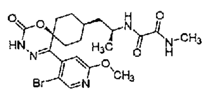 | | 1H-NMR (DMSO-D6) δ: 11.30 (1H, s), 8.65-8.60 (1H, m), 8.47-8.38 (2H, m), 7,00 (1H, s), 3.96-3.84 (1H, m), 3.88 (3H, s), 2.64 (3H, d, J = 4.8 Hz), 2.15-2.06 (2H, m), 1.70-1.15 (9H, m), 1.05 (3H, d, J = 6.5 Hz). | 496 | 494 |
| 2-075 | 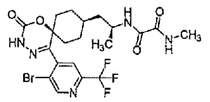 | | 1H-NMR (DMSO-D6) δ: 11.43 (1H, s), 9.09 (1H, s), 8.65-8.58 (1H, m), 8.43-8.38 (1H, m), 8.08 (1H, s), 3.96-3,84 (1H, m), 2.63 (3H, d, J = 4.8 Hz), 2.20-2.11 (2H, m), 1.69-1.16 (9H, m), 1.05 (3H, d, J = 6.5 Hz). | 534 | 532 |
| 2-076 | 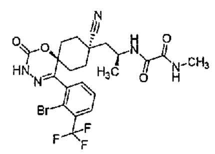 | | 1H-NMR (DMSO-D6) δ: 11.44 (1H, s), 8.72 (1H, d, J = 9.2 Hz), 8.63-8.55 (1H, m), 8.00-7.95 (1H, m), 7.85-7.80 (1H, m), 7.76-7.69 (1H, m), 4.19-4.04 (1H, m), 2.64 (3H, d, J = 4.8 Hz), 2.37-1.92 (4H, m), 1.84-1.35 (6H, m), 1.12 (3H, d, J = 6.5 Hz). | 558 | 556 |
| 2-077 | 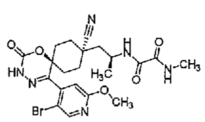 | | 1H-NMR (DMSO-D6) δ: 11.46 (1H, s), 8,75-8.70 (1H, m), 8,63-8.57 (1H, m), 8.49 (1H, s), 7.11 (1H, s), 4.17-4.06 (1H, m), 3.90-3.87 (3H, m), 2.64 (3H, d, J = 3.8 Hz), 2.27-1.97 (4H, m), 1.82-1.53 (6H, m), 1.12 (3H, d, J = 6.5 Hz). | 521 | 519 |
| 2-078 | 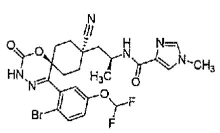 | | 1H-NMR (DMSO-D6) δ: 11.36 (1H, s), 7.86-7.79 (2H, m), 7.61 (1H, s), 7.56 (1H, s), 7.49-7.10 (3H, m), 4.32-4.21 (1H, m), 3.66 (3H, s), 2.29-1.96 (4H, m), 1.88-1.54 (6H, m), 1.14 (3H, d, J = 6.5 Hz). | 579 | 577 |

(continued)

| | | | | | |
|---|---|---|---|---|---|
| 2-079 | 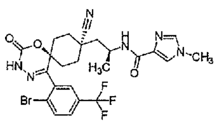 | | 1H-NMR (DMSO-D6) δ: 11.42 (1H, s), 8.06-8.01 (1H, m), 7.96-7.91 (1H, m), 7.85-7.76 (2H, m), 7.61 (1H, s), 7.55 (1H, s), 4.32-4.20 (1H, m), 3.66 (3H, s), 2.33-1.95 (4H, m), 1.88-1.50 (6H, m), 1.14 (3H, d, J = 7.0 Hz). | 581 | 579 |
| 2-080 | 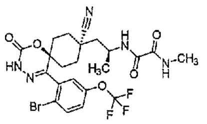 | | 1H-NMR (DMSO-D6) δ: 11.41 (1H, s), 8.75-8.70 (1H, m), 8.63-8.57 (1H, m), 7.95-7.90 (1H, m), 7.67-7.64 (1H, m), 7.51-7.46 (1H, m), 4.17-4.05 (1H, m), 2.64 (3H, d, J = 4.8 Hz), 2.30-1.94 (4H, m), 1.81-1.52 (6H, m), 1.12 (3H, d, J = 7.0 Hz), | 574 | 572 |

Table 1-52

| | | | | | |
|---|---|---|---|---|---|
| 2-081 | 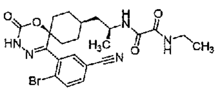 | | 1H-NMR (DMSO-D6) δ: 11.29 (1H, s), 8.71-8.64 (1H, m), 8.44-8.37 (1H, m), 8.01-7.95 (2H, m), 7.89-7.82 (1H, m), 3.96-3.85 (1H, m), 3.18-3.06 (2H, m), 2.16-2.05 (2H, m), 1.69-1.16 (9H, m), 1.09-0.99 (6H, m). | 504 | 502 |
| 2-082 | 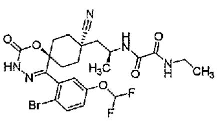 | | 1H-NMR (DMSO-D6) δ: 11.37 (1H, s), 8.75-8.69 (1H, m), 8.68-8.62 (1H, m), 7.85-7.80 (1H, m), 7.50-7,10 (3H, m), 4.17-4.05 (1H, m), 3.21-3.04 (2H, m), 2.29-1.96 (4H, m), 1.82-1.56 (6H, m), 1.12 (3H, d, J = 6.5 Hz), 1.02 (3H, t, J = 7.3 Hz), | 570 | 568 |
| 2-083 | 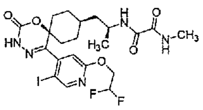 | | 1H-NMR (DMSO-D6) δ: 11.32 (1H, s), 8,66-8,58 (2H, m), 8.45-8.38 (1H, m), 7.10 (1H, s), 6.56-6.24 (1H, m), 4.63-4.51 (2H, m), 3.97-3.84 (1H, m), 2.64 (3H, d, J = 3,8 Hz), 2.16-2.07 (2H, m), 1.71-1.13 (9H, m), 1.06 (3H, d, J = 6.5 Hz). | 594 | 592 |
| 2-084 | 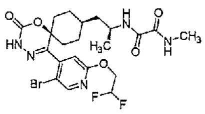 | | 1H-NMR (DMSO-D6) δ: 11.32 (1H, s), 8.66-8.60 (1H, m), 8.47 (1H, s), 8.44-8.38 (1H, m), 7.14 (1H, s), 6,56-6,24 (1H, m), 4.64-4.52 (2H, m), 3.96-3.85 (1H, m), 2.64 (3H, d, J = 4.8 Hz), 2.15-2.07 (2H, m), 1.70-1.16 (9H, m), 1.05 (3H, d, J = 6.5 Hz). | 546 | 544 |
| 2-085 | 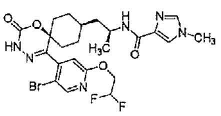 | | 1H-NMR (DMSO-D6) δ: 11.32 (1H, s), 8.46 (1H, s), 7.60 (1H, s), 7.54 (1H, s), 7.45 (1H, d, J = 9.2 Hz), 7.14 (1H, s), 6.57-6.21 (1H, m), 4.63-4.50 (2H, m), 4.11-3.96 (1H, m), 3.66 (3H, s), 2.17-2.07 (2H, m), 1.76-1.15 (9H, m), 1.07 (3H, d, J = 6.5 Hz), | 569 | 567 |
| 2-086 | 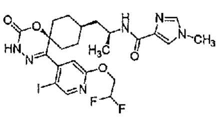 | | 1H-NMR (DMSO-D6) δ: 11.31 (1H, s), 8.59 (1H, s), 7.61 (1H, s), 7.54 (1H, s), 7.45 (1H, d, J = 8.6 Hz), 7.10 (1H, s), 6.55-6.21 (1H, m), 4.61-4.50 (2H, m), 4.09-3.98 (1H, m), 3.66 (3H, s), 2.17-2.06 (2H, m), 1.75-1.14 (9H, m), 1.07 (3H, d, J = 6.5 Hz). | 617 | 615 |
| 2-087 | 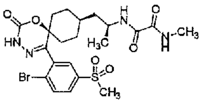 | | 1H-NMR (DMSO-D6) δ: 11.31 (1H, s), 8.64-8.58 (1H, m), 8.43-8.38 (1H, m), 8.07-8.02 (1H, m), 7.92-7.88 (2H, m), 3.96-3.84 (1H, m), 3.32 (3H, s), 2.63 (3H, d, J = 4.8 Hz), 2.21-2.12 (2H, m), 1.71-1.13 (9H, m), 1.05 (3H, d, J = 6.5 Hz), | 543 | 541 |
| 2-088 | 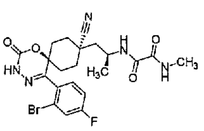 | | 1H-NMR (DMSO-D6) δ: 11.34 (1H, s), 8.75-8.69 (1H, m), 8.63-8.57 (1H, m), 7.80-7,75 (1H, m), 7.61-7.55 (1H, m), 7.43-7.36 (1H, m), 4.18-4.06 (1H, m), 2.64 (3H, d, J = 4.8 Hz), 2.26-1.95 (4H, m), 1.81-1.53 (6H, m), 1.12 (3H, d, J = 6.5 Hz), | 508 | 506 |

(continued)

| | | | | | |
|---|---|---|---|---|---|
| 2-089 | 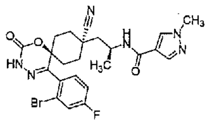 | | 1H-NMR (DMSO-D6) δ: 11.33 (1H, s), 8.03 (1H, s), 7.86 (1H, d, J = 9.2 Hz), 7,78-7,73 (2H, m), 7.60-7.54 (1H, m), 7.41-7.34 (1H, m), 4.32-4.20 (1H, m), 3.82 (3H, s), 2.27-2.15 (2H, m), 2.03-1.51 (8H, m), 1.12 (3H, d, J = 6.5 Hz). | 531 | 529 |
| 2-090 | 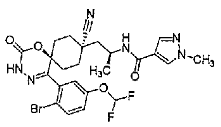 | | 1H-NMR (DMSO-D6) δ: 11.37 (1H, s), 8.03 (1H, s), 7.90-7.78 (2H, m), 7.76 (1H, s), 7.50-7.08 (3H, m), 4.32-4.20 (1H, m), 3.82 (3H, s), 2.29-2.16 (2H, m), 2.04-1.54 (8H, m), 1.12 (3H, d, J = 6.5 Hz). | 579 | 577 |

Table 1-53

| | | | | | |
|---|---|---|---|---|---|
| 2-091 | 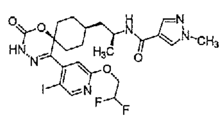 | | 1H-NMR (DMSO-D6) δ: 11.31 (1H, s), 8.59 (1H, s), 8.05 (1H, s), 7.78 (1H, s), 7.64 (1H, d, J = 8.1 Hz), 7.10 (1H, s), 6.56-6.21 (1H, m), 4.63-4.49 (2H, m), 4.09-3.94 (1H, m), 3.81 (3H, s), 2.17-2.07 (2H, m), 1.73-1.14 (9H, m), 1.06 (3H, d, J = 5.9 Hz). | 617 | 615 |
| 2-092 | 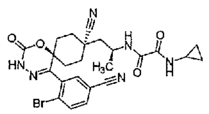 | | 1H-NMR (DMSO-D6) δ; 11.45 (1H, s), 8.75-8.70 (1H, m), 8.67-8.63 (1H, m), 8.15-8.13 (1H, m), 8.05-8.01 (1H, m), 7.93-7.88 (1H, m), 4.16-4.04 (1H, m), 2.78-2.69 (1H, m), 2.29-1.94 (4H, m), 1.82-1.53 (6H, m), 1.12 (3H, d, J = 6.5 Hz), 0.64-0.56 (4H, m). | 541 | 539 |
| 2-093 | 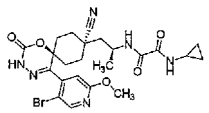 | | 1H-NMR (DMSO-D6) δ: 11.46 (1H, s), 8.75-8.69 (1H, m), 8.67-8.63 (1H, m), 8.49 (1H, s), 7.11 (1H, s), 4.15-4.02 (1H, m), 3.89 (3H, s), 2.77-2.68 (1H, m), 2.27-1.96 (4H, m), 1.82-1.53 (6H, m), 1.12 (3H, d, J = 6.5 Hz), 0.66-0.54 (4H, m). | 547 | 545 |
| 2-094 | 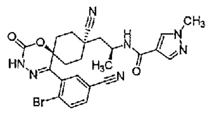 | | 1H-NMR (DMSO-D6) δ: 11.44 (1H, s), 8.14 (1H, s), 8.04-8.00 (2H, m), 7.91-7.83 (2H, m), 7.76 (1H, s), 4.35-4.19 (1H, m), 3.82 (3H, s), 2.31-2.16 (2H, m), 2.04-1.54 (8H, m), 1.13 (3H, d, J = 6.5 Hz). | 538 | 536 |
| 2-095 | 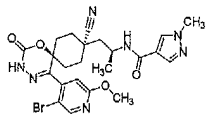 | | 1H-NMR (CDCl3) δ: 8.49 (1H, s), 8.34 (1H, s), 7.81 (1H, s), 7.75 (1H, s), 6.62 (1H, s), 5.68 (1H, d, J = 9.7 Hz), 4.59-4.47 (1H, m), 3.92 (3H, s), 3.89 (3H, s), 2.37-2.27 (2H, m), 2.18-1.69 (8H, m), 1.27 (3H, d, J = 6.5 Hz). | 544 | 542 |
| 2-096 | 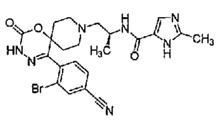 | | 1H-NMR (DMSO-D6) δ: 8.35 (1H, d, J = 1.5 Hz), 7.95 (1H, dd, J = 8.0, 1.5 Hz), 7.70 (1H, d, J = 8.0 Hz), 7.42-7.35 (2H, m), 4.10-3.98 (1H, m), 2.74-2.65 (2H, m), 2.46-2.38 (1H, m), 2.34-2.21 (4H, m), 2.24 (3H, s), 2.15-2.04 (2H, m), 1.65-1.51 (2H, m), 1.06 (3H, d, J = 6.5 Hz), | 514 | 512 |
| 2-097 | 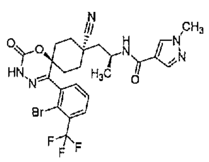 | | 1H-NMR (DMSO-D6) δ: 11.43 (1H, s), 8.02 (1H, s), 7.98-7.94 (1H, m), 7.88-7.79 (2H, m), 7.76 (1H, s), 7.73-7.68 (1H, m), 4.33-4.20 (1H, m), 3.83-3.80 (3H, m), 2.36-2.13 (2H, m), 2.04-1.60 (8H, m), 1.12 (3H, d, J = 6.5 Hz). | 581 | 579 |
| 2-098 | 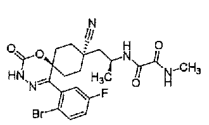 | | 1H-NMR (DMSO-D6) δ: 11.38 (1H, s), 8.75-8.70 (1H, m), 8.63-8.57 (1H, m), 7.84-7.79 (1H, m), 7.56-7.51 (1H, m), 7.38-7.30 (1H, m), 4.17-4.06 (1H, m), 2.64 (3H, d, J = 4.8 Hz), 2.27-1.95 (4H, m), 1.81-1.53 (6H, m), 1.12 (3H, d, J = 6.5 Hz). | 508 | 506 |

(continued)

| | | | | | |
|---|---|---|---|---|---|
| 2-099 | 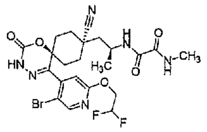 | | 1H-NMR (DMSO-D6) δ: 11.49 (1H, s), 8.77-8.68 (1H, m), 8.63-8.56 (1H, m), 8.52 (1H, s), 7.25 (1H, s), 6.58-6.24 (1H, m), 4.67-4.54 (2H, m), 4.07-3.97 (1H, m), 2.66 (3H, d, J = 3.8 Hz), 2.08-1.55 (10H, m), 1.11 (3H, d, J = 6.5 Hz). | 571 | 569 |
| 2-100 | 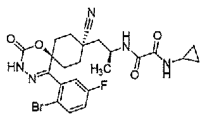 | | 1H-NMR (DMSO-D6) δ: 11.38 (1H, s), 8.72 (1H, d, J = 9.2 Hz), 8.65 (1H, d, J = 5.4 Hz), 7.84-7.79 (1H, m), 7.56-7.51 (1H, m), 7.38-7.31 (1H, m), 4.16-4.04 (1H, m), 2.78-2.69 (1H, m), 2.27-1.94 (4H, m), 1.82-1.54 (6H, m), 1.12 (3H, d, J = 6.5 Hz), 0.65-0.55 (4H, m). | 534 | 532 |

Table 1-54

| | | | | | |
|---|---|---|---|---|---|
| 2-101 | 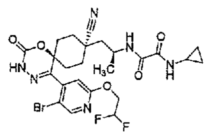 | | 1H-NMR (DMSO-D6) δ: 11.49 (1H, s), 8.75-8.68 (1H, m), 8.67-8.60 (1H, m), 8.51 (1H, s), 7.25 (1H, s), 6.58-6.24 (1H, m), 4.67-4.55 (2H, m), 4.07-3.94 (1H, m), 2.80-2.69 (1H, m), 2.08-1.54 (10H, m), 1.10 (3H, d, J = 6.5 Hz), 0.69-0.55 (4H, m). | 597 | 595 |
| 2-102 | 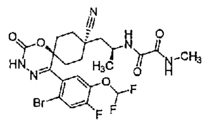 | | 1H-NMR (DMSO-D6) δ: 11.40 (1H, s), 8.77-8.67 (1H, m), 8.66-8.56 (1H, m), 7.67-7.50 (2H, m), 7.27 (1H, t, J = 71.9 Hz), 4.19-4.05 (1H, m), 2.66-2.62 (3H, m), 2.31-1.93 (4H, m), 1.82-1,50 (6H, m), 1.12 (3H, d, J = 6,5 Hz). | 574 | 572 |
| 2-103 | 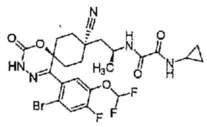 | | 1H-NMR (DMSO-D6) δ: 11.40 (1H, s), 8.72 (1H, d, J = 9.7 Hz), 8.65 (1H, d, J = 4.8 Hz), 7.66-7.59 (1H, m), 7.57-7.51 (1H, m), 7.27 (1H, t, J = 72.2 Hz), 4.17-4.04 (1H, m), 2.77-2.69 (1H, m), 2.27-1.93 (4H, m), 1.82-1.50 (6H, m), 1.12 (3H, d, J = 6.5 Hz), 0.68-0,53 (4H, m). | 600 | 598 |
| 2-104 | 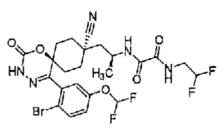 | | 1H-NMR (DMSO-D6) δ: 11.38 (1H, s), 8.97-8.90 (1H, m), 8.88-8.82 (1H, m), 7.85-7.80 (1H, m), 7.50-7.10 (3H, m), 6.20-5.87 (1H, m), 4.20-4.07 (1H, m), 3.65-3.40 (2H, m), 2.30-1.95 (4H, m), 1.83-1.54 (6H, m), 1.13 (3H, d, J = 6.5 Hz). | 606 | 604 |
| 2-105 | 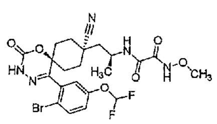 | | 1H-NMR (CDCl3) δ: 9.73 (1H, s), 8.34 (1H, s), 7.66 (1H, d, J = 9.2 Hz), 7.39-7.33 (1H, m), 7.15-7.10 (1H, m), 7.02 (1H, d, J = 2.7 Hz), 6.51 (1H, t, J = 72.7 Hz), 4.35-4.22 (1H, m), 3.82 (3H, s), 2.41-2.29 (2H, m), 2.10-1.70 (8H, m), 1.30 (3H, d, J = 6.5 Hz). | 572 | 570 |
| 2-106 | 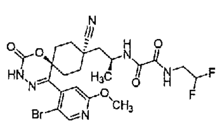 | | 1H-NMR (DMSO-D6) δ: 11.46 (1H, s), 8.96-8.91 (1H, m), 8.88-8.82 (1H, m), 8.49 (1H, s), 7.11 (1H, s), 6.21-5.86 (1H, m), 4.18-4.07 (1H, m), 3.88 (3H, s), 3.66-3.39 (2H, m), 2.29-1.95 (4H, m), 1.81-1.55 (6H, m), 1.13 (3H, d, J = 6.5 Hz). | 571 | 569 |
| 2-107 | 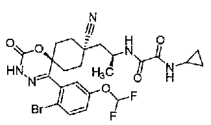 | | 1H-NMR (DMSO-D6) δ: 11.38 (1H, s), 8.75-8.69 (1H, m), 8.67-8.63 (1H, m), 7.85-7.81 (1H, m), 7.52-7.11 (3H, m), 4.16-4.01 (1H, m), 2.78-2.67 (1H, m), 2.29-1.93 (4H, m), 1.86-1.52 (6H, m), 1.12 (3H, d, J = 6.5 Hz), 0.67-0.54 (4H, m). | 582 | 580 |
| 2-108 | 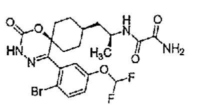 | | 1H-NMR (DMSO-D6) δ: 11.22 (1H, s), 8.38 (1H, d, J = 8.6 Hz), 7.98 (1H, s), 7.81-7.76 (1H, m), 7.71 (1H, s), 7.53-7.12 (3H, m), 3.95-3.80 (1H, m), 2.21-2.08 (2H, m), 1.70-1.14 (9H, m), 1.05 (3H, d, J = 6.5 Hz). | 517 | 515 |

(continued)

| | | | | | |
|---|---|---|---|---|---|
| 2-109 | 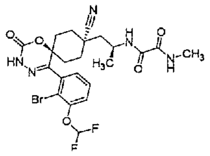 | | 1H-NMR (DMSO-D6) δ: 11.36 (1H, s), 8.75-8.69 (1H, m), 8.63-8.57 (1H, m), 7.59-7.52 (1H, m), 7.46-7.42 (1H, m), 7.39-7.34 (2H, m), 4.16-4.05 (1H, m), 2.66-2.61 (3H, m), 2.29-1.94 (4H, m), 1.81-1.57 (6H, m), 1.11 (3H, d, J = 4.8 Hz). | 556 | 554 |
| 2-110 | 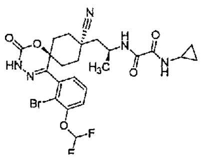 | | 1H-NMR (DMSO-D6) δ: 11.37 (1H, s), 8.74-8.69 (1H, m), 8,67-8.63 (1H, m), 7.59-7.53 (1H, m), 7.47-7.42 (1H, m), 7.39-7.35 (2H, m), 4.16-4.03 (1H, m), 2.78-2.68 (1H, m), 2.29-1.93 (4H, m), 1.82-1.55 (6H, m), 1.12 (3H, d, J = 6.5 Hz), 0.67-0.56 (4H, m). | 582 | 580 |

Table 1-55

| | | | | | |
|---|---|---|---|---|---|
| 2-111 | 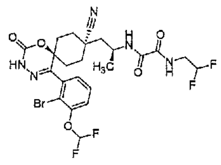 | | 1H-NMR (DMSO-D6) δ: 11.37 (1H, s), 8.97-8.90 (1H, m), 8.88-8.82 (1H, m), 7.59-7.52 (1H, m), 7.47-7.42 (1H, m), 7.39-7.35 (2H, m), 6.20-5.87 (1H, m), 4,20-4,07 (1H, m), 3.64-3.38 (2H, m), 2.30-1.94 (4H, m), 1.83-1.53 (6H, m), 1.13 (3H, d, J = 6.5 Hz). | 606 | 604 |
| 2-112 | 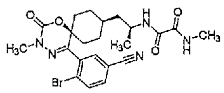 | | 1H-NMR (DMSO-D6) δ: 8.66-8.59 (1H, m), 8.46-8.38 (1H, m), 8.04-7.96 (2H, m), 7.90-7.84 (1H, m), 3.96-3.85 (1H, m), 3.32 (3H, s), 2.64 (3H, d, J = 4.8 Hz), 2.16-2.05 (2H, m), 1.70-1.38 (4H, m), 1.31-1.14 (5H, m), 1.05 (3H, d, J = 7.0 Hz). | 504 | N.D. |
| 2-113 | 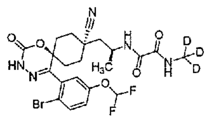 | | 1H-NMR (DMSO-D6) δ: 11.38 (1H, s), 8.72 (1H, d, J = 9.2 Hz), 8.57 (1H, s), 7.85-7.81 (1H, m), 7.50-7.11 (3H, m), 4.16-4.05 (1H, m), 2.28-1.95 (4H, m), 1.81-1.57 (6H, m), 1.12 (3H, d, J = 7.0 Hz). | 559 | 557 |
| 2-114 | 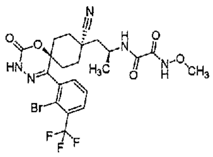 | | 1H-NMR (CDCl3) δ: 9.77 (1H, s), 8.49 (1H, s), 7.83-7.79 (1H, m), 7.57-7.51 (1H, m), 7.43-7.38 (2H, m), 4.36-4.23 (1H, m), 3.81 (3H, s), 2.45-2.35 (2H, m), 2.09-1.70 (8H, m), 1.29 (3H, d, J = 7.0 Hz). | 574 | 572 |
| 2-115 | 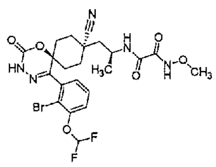 | | 1H-NMR (CDCl3) δ: 9.84 (1H, s), 8.53 (1H, s), 7.48-7.39 (2H, m), 7.35-7.30 (1H, m), 7.14-7.09 (1H, m), 6.58 (1H, t, J = 72.7 Hz), 4.35-4.22 (1H, m), 3.81 (3H, s), 2.43-2,30 (2H, m), 2.09-1.68 (8H, m), 1.29 (3H, d, J = 7.0 Hz), | 572 | 570 |
| 2-116 | 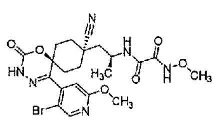 | | 1H-NMR (CDCl3) δ: 9.96 (1H, s), 8.77 (1H, s), 8.35 (1H, s), 7.56 (1H, d, J = 9.7 Hz), 6.64 (1H, s), 4.36-4.24 (1H, m), 3.93 (3H, s), 3.82 (3H, s), 2.36-2.29 (2H, m), 2.08-1.70 (8H, m), 1.29 (3H, d, J = 6.5 Hz). | 537 | 535 |
| 2-117 | 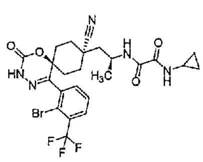 | | 1H-NMR (DMSO-D6) δ: 11.44 (1H, s), 8.74-8.69 (1H, m), 8,67-8,63 (1H, m), 8,00-7.95 (1H, m), 7.85-7.80 (1H, m), 7.76-7.70 (1H, m), 4.16-4.04 (1H, m), 2.78-2.69 (1H, m), 2.34-1.93 (4H, m), 1.84-1.60 (6H, m), 1.12 (3H, d, J = 6.5 Hz), 0.66-0.53 (4H, m). | 584 | 582 |

(continued)

| | | | | | |
|---|---|---|---|---|---|
| 2-118 | 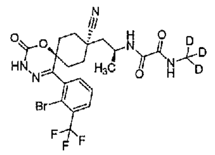 | | 1H-NMR (DMSO-D6) δ: 11.44 (1H, s), 8.74-8.69 (1H, m), 8.56 (1H, s), 7.99-7.95 (1H, m), 7.84-7.80 (1H, m), 7.75-7.69 (1H, m), 4.15-4,04 (1H, m), 2.33-1.95 (4H, m), 1.81-1.59 (6H, m), 1.11 (3H, d, J = 6.5 Hz), | 561 | 559 |
| 2-119 | 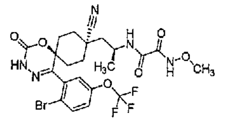 | | 1H-NMR (DMSO-D6) δ: 12.01 (1H, s), 11.42 (1H, s), 8.86-8.80 (1H, m), 7.96-7.90 (1H, m), 7.67-7.64 (1H, m), 7.51-7.47 (1H, m), 4.17-4.04 (1H, m), 3.59 (3H, s), 2.31-1.95 (4H, m), 1.83-1.52 (6H, m), 1.12 (3H, d, J = 6.6 Hz), | 590 | 588 |
| 2-120 | 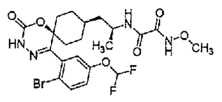 | | 1H-NMR (DMSO-D6) δ: 12.02 (1H, s), 11.23 (1H, s), 8.53 (1H, d, J = 8.1 Hz), 7.79 (1H, d, J = 9.2 Hz), 7.53-7.12 (3H, m), 3.98-3.85 (1H, m), 3.60 (3H, s), 2.20-2.08 (2H, m), 1,70-1.14 (9H, m), 1.05 (3H, d, J = 6.5 Hz). | 547 | 545 |

Table 1-56

| | | | | | |
|---|---|---|---|---|---|
| 2-121 | 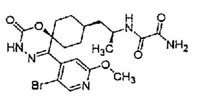 | | 1H-NMR (DMSO-D6) δ: 11.30 (1H, s), 8.44 (1H, s), 8.42-8.34 (1H, m), 7.99 (1H, s), 7.72 (1H, s), 7.01 (1H, s), 3.94-3.82 (1H, m), 3.88 (3H, s), 2.17-2.06 (2H, m), 1.69-1.15 (9H, m), 1.05 (3H, d, J = 6.5 Hz). | 482 | 480 |
| 2-122 | 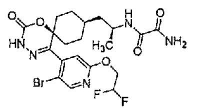 | | 1H-NMR (DMSO-D6) δ: 11.32 (1H, s), 8.47 (1H, s), 8.41-8.34 (1H, m), 7.99 (1H, s), 7.72 (1H, s), 7.15 (1H, s), 6.57-6.25 (1H, m), 4.66-4.52 (2H, m), 3.96-3.82 (1H, m), 2.19-2.07 (2H, m), 1.71-1.15 (9H, m), 1.05 (3H, d, J = 6.5 Hz). | 532 | 530 |
| 2-123 | 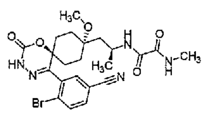 | | 1H-NMR (DMSO-D6) δ: 11.32 (1H, s), 8.65-8.54 (2H, m), 8.09-8.05 (1H, m), 8.03-7.98 (1H, m), 7.91-7.86 (1H, m), 4.01-3.92 (1H, m), 2.92 (3H, s), 2.67-2.63 (3H, m), 1.99-1.42 (10H, m), 1.08 (3H, d, J = 6.5 Hz), | 520 | 518 |
| 2-124 | 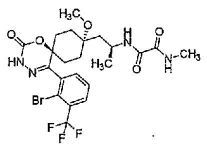 | | 1H-NMR (DMSO-D6) δ: 11.31 (1H, s), 8.65-8,51 (2H, m), 8.00-7.92 (1H, m), 7.80-7.66 (2H, m), 4.04-3.91 (1H, m), 2.90 (3H, s), 2.65 (3H, d, J = 4.8 Hz), 2.01-1.40 (10H, m), 1.08 (3H, d, J = 6.5 Hz). | 563 | 561 |
| 2-125 | 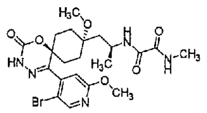 | | 1H-NMR (DMSO-D6) δ: 11.33 (1H, s), 8.66-8.53 (2H, m), 8.46 (1H, s), 7.03 (1H, s), 4.03-3.91 (1H, m), 3.89 (3H, s), 2.93 (3H, s), 2.65 (3H, d, J = 4.8 Hz), 1.97-1.68 (4H, m), 1.65-1.41 (6H, m), 1.08 (3H, d, J = 6.5 Hz). | 526 | 524 |
| 2-126 | 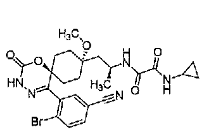 | | 1H-NMR (DMSO-D6) δ: 11.33 (1H, s), 8.68-8.64 (1H, m), 8.59-8.54 (1H, m), 8.06 (1H, s), 8.03-7.98 (1H, m), 7.90-7.86 (1H, m), 4.02-3.92 (1H, m), 2.92 (3H, s), 2.78-2.69 (1H, m), 2.00-1.69 (4H, m), 1.64-1.43 (6H, m), 1.08 (3H, d, J = 6.5 Hz), 0.68-0.57 (4H, m), | 546 | 544 |
| 2-127 | 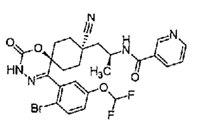 | | 1H-NMR (DMSO-D6) δ: 11.38 (1H, s), 8.97-8.95 (1H, m), 8.69-8.66 (1H, m), 8.56-8,52 (1H, m), 8.16-8.12 (1H, m), 7.81 (1H, d, J = 8.8 Hz), 7.50-7.46 (1H, m), 7.40-7.38 (1H, m), 7.28 (1H, t, J = 73.6 Hz), 7.27-7.23 (1H, m), 4.41-4.32 (1H, m), 2.31-2.19 (2H, m), 2.08-1.99 (2H, m), 1.88-1.57 (6H, m), 1.20-1.16 (3H, m). | 576 | 574 |

(continued)

| | | | | | |
|---|---|---|---|---|---|
| 2-128 | 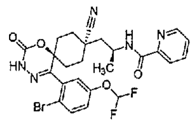 | | 1H-NMR (DMSO-D6) δ: 11.36 (1H, s), 8.78-8.73 (1H, m), 8.64-8.60 (1H, m), 8,03-7.93 (2H, m), 7.82-7.78 (1H, m), 7.60-7.55 (1H, m), 7.39-7.36 (1H, m), 7.28 (1H, t, J = 73.5 Hz), 7.27-7.21 (1H, m), 4.40-4.30 (1H, m), 2.29-1.99 (4H, m), 1.85-1.54 (6H, m), 1.20 (3H, d, J = 6.5 Hz), | 576 | 574 |
| 2-129 | 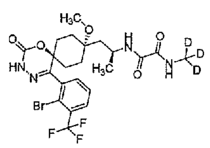 | | 1H-NMR (DMSO-D6) δ: 11.31 (1H, s), 8.59-8.53 (2H, m), 7.97-7.93 (1H, m), 7.78-7.66 (2H, m), 4.03-3.90 (1H, m), 2.90 (3H, s), 2.01-1.41 (10H, m), 1.07 (3H, d, J = 6.5 Hz). | 566 | 564 |
| 2-130 | 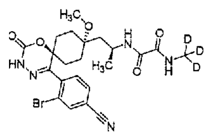 | | 1H-NMR (DMSO-D6) δ: 11.33 (1H, s), 8.60-8.54 (2H, m), 8.38-8.35 (1H, m), 8.00-7.95 (1H, m), 7.70-7.66 (1H, m), 4.03-3.90 (1H, m), 2.91 (3H, s), 1.99-1.68 (4H, m), 1.62-1.44 (6H, m), 1.08 (3H, d, J = 6.5 Hz). | 523 | 521 |

Table 1-57

| | | | | | |
|---|---|---|---|---|---|
| 2-131 | 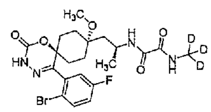 | | 1H-NMR (DMSO-D6) δ: 11.25 (1H, s), 8.60-8.52 (2H, m), 7.83-7.76 (1H, m), 7.49-7.43 (1H, m), 7.35-7.28 (1H, m), 4.02-3.91 (1H, m), 2.92 (3H, s), 1.99-1.67 (4H, m), 1.64-1.41 (6H, m), 1.08 (3H, d, J = 6.5 Hz). | 516 | 514 |
| 2-132 | 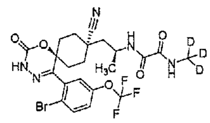 | | 1H-NMR (DMSO-D6) δ: 11.41 (1H, s), 8.73 (1H, d, J = 9.2 Hz), 8.58 (1H, s), 7.92 (1H, d, J = 8.8 Hz), 7.67-7.64 (1H, m), 7.51-7.46 (1H, m), 4.16-4.03 (1H, m), 2.30-1.94 (4H, m), 1.84-1.51 (6H, m), 1.12 (3H, d, J = 6.6 Hz). | 577 | 575 |
| 2-133 | 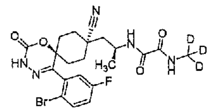 | | 1H-NMR (DMSO-D6) δ: 11.38 (1H, s), 8.73 (1H, d, J = 9.7 Hz), 8.58 (1H, s), 7.85-7.79 (1H, m), 7.58-7.50 (1H, m), 7.40-7.30 (1H, m), 4.19-4.06 (1H, m), 2.29-1.95 (4H, m), 1.83-1.53 (6H, m), 1.12 (3H, d, J = 6.6 Hz). | 511 | 509 |
| 2-134 | 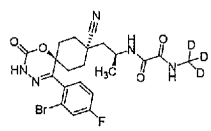 | | 1H-NMR (DMSO-D6) δ: 11.34 (1H, s), 8.72 (1H, d, J = 9.2 Hz), 8.57 (1H, s), 7.82-7.74 (1H, m), 7.62-7.54 (1H, m), 7.44-7.36 (1H, m), 4,17-4,05 (1H, m), 2.27-2.14 (2H, m), 2.13-1.96 (2H, m), 1.81-1.53 (6H, m), 1.12 (3H, d, J = 6.5 Hz). | 511 | 509 |
| 2-135 | 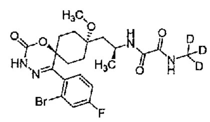 | | 1H-NMR (DMSO-D6) δ: 11.22 (1H, s), 8.62-8.52 (2H, m), 7.78-7.70 (1H, m), 7.56-7.47 (1H, m), 7.41-7.33 (1H, m), 4.03-3.91 (1H, m), 2.93-2.89 (3H, m), 1.96-1.79 (3H, m), 1.75-1.66 (1H, m), 1.63-1.42 (6H, m), 1.08 (3H, d, J = 6.5 Hz). | 516 | 514 |
| 2-136 | 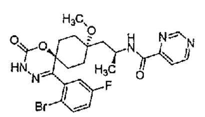 | | 1H-NMR (DMSO-D6) δ: 11.23 (1H, s), 9.31 (1H, s), 9.04 (1H, d, J = 4.8 Hz), 8.87 (1H, d, J = 8.6 Hz), 8.00 (1H, d, J = 5.4 Hz), 7.81-7.75 (1H, m), 7.48-7.42 (1H, m), 7.35-7.27 (1H, m), 4.28-4.14 (1H, m), 2.95 (3H, s), 2.02-1.86 (3H, m), 1.80-1.73 (1H, m), 1.69-1.46 (6H, m), 1.17 (3H, d, J = 6.5 Hz). | 534 | 532 |
| 2-137 | 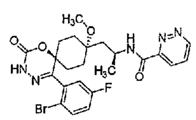 | | 1H-NMR (DMSO-D6) δ: 11.23 (1H, s), 9.40-9.37 (1H, m), 9.10 (1H, d, J = 8.6 Hz), 8.20-8,16 (1H, m), 7.91-7.86 (1H, m), 7.81-7.75 (1H, m), 7.47-7.42 (1H, m), 7.34-7.27 (1H, m), 4.34-4.22 (1H, m), 2.96 (3H, s), 2.04-1.48 (10H, m), 1.20 (3H, d, J = 6.5 Hz), | 534 | 532 |

(continued)

| | | | | | |
|---|---|---|---|---|---|
| 2-138 | 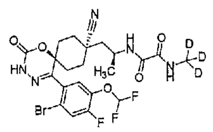 | | 1H-NMR (DMSO-D6) δ: 11.40 (1H, s), 8.73 (1H, d, J = 9.2 Hz), 8.57 (1H, s), 7.67-7.59 (1H, m), 7.57-7.51 (1H, m), 7.27 (1H, t, J = 72.2 Hz), 4.18-4.05 (1H, m), 2.29-1.93 (4H, m), 1.83-1,50 (6H, m), 1.12 (3H, d, J = 6.5 Hz), | 577 | 575 |
| 2-139 | 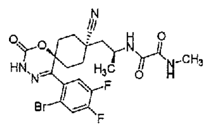 | | 1H-NMR (DMSO-D6) δ: 11.40 (1H, s), 8.73 (1H, d, J = 9.7 Hz), 8.63-8.57 (1H, m), 8.08-8.01 (1H, m), 7.88-7.81 (1H, m), 4.17-4.06 (1H, m), 2.64 (3H, d, J = 4.8 Hz), 2.27-2.15 (2H, m), 2.13-1.96 (2H, m), 1.81-1.55 (6H, m), 1.12 (3H, d, J = 6.5 Hz), | 526 | 524 |
| 2-140 | 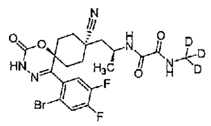 | | 1H-NMR (DMSO-D6) δ: 11.40 (1H, s), 8.73 (1H, d, J = 9.7 Hz), 8.57 (1H, s), 8.08-8.01 (1H, m), 7.88-7.81 (1H, m), 4.18-4.06 (1H, m), 2.28-1.93 (4H, m), 1.84-1.55 (6H, m), 1.12 (3H, d, J = 6.5 Hz). | 529 | 527 |

Table 1-58

| | | | | | |
|---|---|---|---|---|---|
| 2-141 | 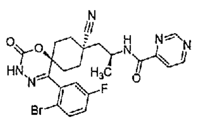 | | 1H-NMR (DMSO-D6) δ: 11.36 (1H, s), 9.30 (1H, s), 9.06-9.00 (2H, m), 8.01-7.97 (1H, m), 7.82-7.76 (1H, m), 7.55-7.49 (1H, m), 7.35-7.28 (1H, m), 4.44-4.28 (1H, m), 2.31-2.01 (4H, m), 1,86-1,52 (6H, m), 1.21 (3H, d, J = 6.5 Hz). | 529 | 527 |
| 2-142 | 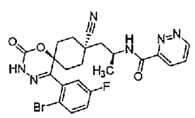 | | 1H-NMR (DMSO-D6) δ: 11.36 (1H, s), 9.38 (1H, d, J = 5.4 Hz), 9.27 (1H, d, J = 9.7 Hz), 8.20-8.15 (1H, m), 7.90-7.85 (1H, m), 7.82-7.77 (1H, m), 7.55-7.49 (1H, m), 7.35-7.28 (1H, m), 4,50-4.36 (1H, m), 2.31-2.03 (4H, m), 1.87-1.54 (6H, m), 1.24 (3H, d, J = 6.5 Hz). | 529 | 527 |
| 2-143 | 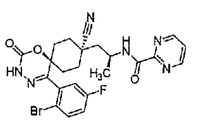 | | 1H-NMR (DMSO-D6) δ: 11.36 (1H, s), 8.94 (2H, d, J = 4.8 Hz), 8.89 (1H, d, J = 9.2 Hz), 7.83-7.77 (1H, m), 7.67-7.63 (1H, m), 7.55-7.50 (1H, m), 7.35-7.28 (1H, m), 4.41-4.30 (1H, m), 2.31-2,01 (4H, m), 1.87-1.55 (6H, m), 1.20 (3H, d, J = 7.0 Hz). | 529 | 527 |
| 2-144 | 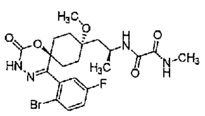 | | 1H-NMR (DMSO-D6) δ: 11.25 (1H, s), 8.65-8.53 (2H, m), 7.84-7.76 (1H, m), 7.49-7.42 (1H, m), 7.36-7.28 (1H, m), 4.02-3.91 (1H, m), 2.92 (3H, d, J = 1.1 Hz), 2.65 (3H, dd, J = 4.8, 1.1 Hz), 1.98-1.66 (4H, m), 1.63-1.40 (6H, m), 1.08 (3H, d, J = 6.5 Hz). | 513 | 511 |
| 2-145 | 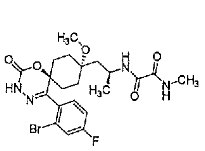 | | 1H-NMR (DMSO-D6) δ: 11.21 (1H, s), 8.65-8.53 (2H, m), 7.76-7.71 (1H, m), 7.55-7.48 (1H, m), 7.40-7.33 (1H, m), 4.03-3.91 (1H, m), 2.91 (3H, d, J = 1.1 Hz), 2.65 (3H, d, J = 4.3 Hz), 1.96-1.67 (4H, m), 1.64-1.41 (6H, m), 1.08 (3H, d, J = 6.5 Hz). | 513 | 511 |
| 2-146 | 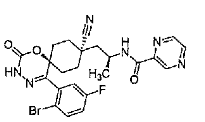 | | 1H-NMR (DMSO-D6) δ: 11.36 (1H, s), 9.16 (1H, s), 8.92 (1H, d, J = 9.7 Hz), 8.86-8.83 (1H, m), 8.72-8.69 (1H, m), 7.83-7.77 (1H, m), 7.55-7.49 (1H, m), 7.35-7.29 (1H, m), 4.45-4.32 (1H, m), 2,35-2.03 (4H, m), 1.85-1.55 (6H, m), 1.21 (3H, d, J = 6.5 Hz). | 529 | 527 |

(continued)

| | | | | | |
|---|---|---|---|---|---|
| 2-147 | 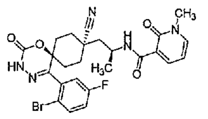 | | 1H-NMR (DMSO-D6) δ: 11.37 (1H, s), 9.89-9.80 (1H, m), 8.30-8.24 (1H, m), 8.04-7.99 (1H, m), 7.83-7.76 (1H, m), 7.56-7.50 (1H, m), 7.36-7.29 (1H, m), 6.50-6.43 (1H, m), 4.38-4.24 (1H, m), 3.54 (3H, s), 2.29-2.16 (2H, m), 2.09-1.54 (8H, m), 1.21-1.15 (3H, m), | 558 | 556 |
| 2-148 | 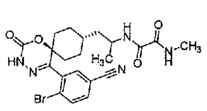 | racemate | 1H-NMR (DMSO-D6) δ: 11.32 (1H, s), 8.64-8.58 (1H, m), 8.46 (1H, d, J = 9.2 Hz), 8.10 (1H, s), 8.03-7.99 (1H, m), 7.91-7.86 (1H, m), 3.83-3.73 (1H, m), 2.66 (3H, d, J = 4.8 Hz), 1.94-1.55 (7H, m), 1.47-1.15 (4H, m), 1.04 (3H, d, J = 6.6 Hz). | 490 | 488 |
| 2-149 | 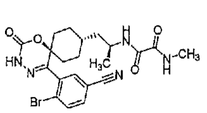 | | 1H-NMR (DMSO-D6) δ: 11.32 (1H, s), 8.64-8.58 (1H, m), 8.46 (1H, d, J = 9.2 Hz), 8.10 (1H, s), 8.03-7.99 (1H, m), 7.91-7.86 (1H, m), 3,83-3.73 (1H, m), 2.66 (3H, d, J = 4.8 Hz), 1.94-1.55 (7H, m), 1.47-1.15 (4H, m), 1.04 (3H, d, J = 6.6 Hz). | 490 | 488 |
| 2-150 | 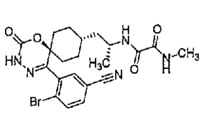 | | 1H-NMR (DMSO-D6) δ: 11.32 (1H, s), 8.64-8.58 (1H, m), 8.46 (1H, d, J = 9.2 Hz), 8.10 (1H, s), 8.03-7.99 (1H, m), 7.91-7.86 (1H, m), 3.83-3.73 (1H, m), 2.66 (3H, d, J = 4.8 Hz), 1.94-1.55 (7H, m), 1.47-1.15 (4H, m), 1.04 (3H, d, J = 6.6 Hz), | 490 | 488 |

Table 1-59

| | | | | | |
|---|---|---|---|---|---|
| 2-151 | 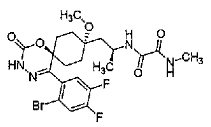 | | 1H-NMR (DMSO-D6) δ: 11.27 (1H, s), 8.65-8.52 (2H, m), 8.05-7.97 (1H, m), 7.80-7.72 (1H, m), 4.02-3.91 (1H, m), 2.93 (3H, s), 2.65 (3H, d, J = 4.8 Hz), 1.96-1.69 (4H, m), 1.64-1.41 (6H, m), 1.08 (3H, d, J = 6.5 Hz). | 531 | 529 |
| 2-152 | 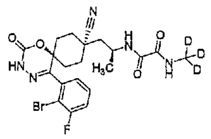 | | 1H-NMR (DMSO-D6) δ: 11.39 (1H, s), 8.72 (1H, d, J = 9.2 Hz), 8.57 (1H, s), 7.59-7.48 (2H, m), 7.39-7.34 (1H, m), 4.17-4.05 (1H, m), 2.28-2.15 (2H, m), 2.13-1.95 (2H, m), 1.82-1.52 (6H, m), 1.12 (3H, d, J = 6.5 Hz). | 511 | 509 |
| 2-153 | 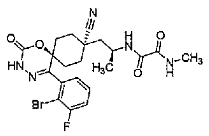 | | 1H-NMR (DMSO-D6) δ: 11.39 (1H, s), 8.72 (1H, d, J = 9.2 Hz), 8.63-8.57 (1H, m), 7.58-7.47 (2H, m), 7.38-7.34 (1H, m), 4.17-4.04 (1H, m), 2.64 (3H, d, J = 4.8 Hz), 2.27-1.95 (4H, m), 1.81-1.53 (6H, m), 1.12 (3H, d, J = 6.5 Hz). | 508 | 506 |
| 2-154 | 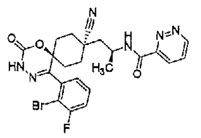 | | 1H-NMR (DMSO-D6) δ: 11.38 (1H, s), 9.40-9.36 (1H, m), 9.27 (1H, d, J = 9.2 Hz), 8.19-8.15 (1H, m), 7.91-7.85 (1H, m), 7.55-7.45 (2H, m), 7.36-7.31 (1H, m), 4.48-4.35 (1H, m), 2.30-2.03 (4H, m), 1.88-1.52 (6H, m), 1.23 (3H, d, J = 7.0 Hz). | 529 | 527 |
| 2-155 | 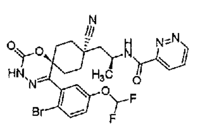 | | 1H-NMR (DMSO-D6) δ: 11.36 (1H, s), 9.39-9.36 (1H, m), 9.27 (1H, d, J = 9.2 Hz), 8.19-8.16 (1H, m), 7.88 (1H, dd, J = 8.6, 4.8 Hz), 7.80 (1H, d, J = 8.6 Hz), 7.39-7.36 (1H, m), 7.28 (1H, t, J = 73.5 Hz), 7.27-7.22 (1H, m), 4.48-4.36 (1H, m), 2,31-2,02 (4H, m), 1.87-1.55 (6H, m), 1.24 (3H, d, J = 6.5 Hz). | 577 | 575 |

(continued)

| | | | | | |
|---|---|---|---|---|---|
| 2-156 | 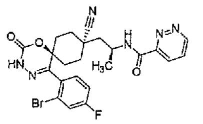 | | 1H-NMR (DMSO-D6) δ: 11.33 (1H, s), 9.39-9.36 (1H, m), 9.27 (1H, d, J = 9.2 Hz), 8.20-8.15 (1H, m), 7.88 (1H, dd, J = 8.6, 4.8 Hz), 7.75 (1H, dd, J = 8.6, 2.7 Hz), 7,59-7.53 (1H, m), 7.40-7,33 (1H, m), 4.48-4.36 (1H, m), 2.29-2.02 (4H, m), 1.86-1.52 (6H, m), 1.23 (3H, d, J = 6.5 Hz). | 529 | 527 |
| 2-157 | 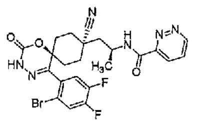 | | 1H-NMR (DMSO-D6) δ: 11.39 (1H, s), 9.40-9.36 (1H, m), 9.27 (1H, d, J = 9.7 Hz), 8.19-8.15 (1H, m), 8.05-7.99 (1H, m), 7.90-7.79 (2H, m), 4.47-4.38 (1H, m), 2.29-2.04 (4H, m), 1.86-1.56 (6H, m), 1.24 (3H, d, J = 6.5 Hz). | 547 | 545 |
| 2-158 | 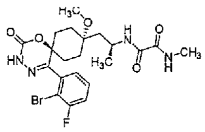 | | 1H-NMR (DMSO-D6) δ: 11.27 (1H, s), 8.64-8.53 (2H, m), 7.57-7.46 (2H, m), 7.30 (1H, d, J = 7.0 Hz), 4.04-3.91 (1H, m), 2.91 (3H, s), 2.65 (3H, d, J = 4.8 Hz), 1.96-1.67 (4H, m), 1.65-1.41 (6H, m), 1.08 (3H, d, J = 6.5 Hz), | 513 | 511 |
| 2-159 | 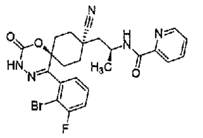 | | 1H-NMR (DMSO-D6) δ: 11.38 (1H, s), 8.76 (1H, d, J = 9.2 Hz), 8.62 (1H, d, J = 4.3 Hz), 8.03-7.93 (2H, m), 7.59-7.45 (3H, m), 7.36-7.32 (1H, m), 4.41-4.29 (1H, m), 2.29-2.01 (4H, m), 1.87-1.53 (6H, m), 1.20 (3H, d, J = 7.0 Hz). | 528 | 526 |
| 2-160 | 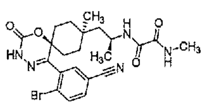 | | 1H-NMR (DMSO-D6) δ: 11.30 (1H, s), 8.64-8.56 (2H, m), 8.08 (1H, d, J = 1.6 Hz), 8,01-7,97 (1H, m), 7.89-7.85 (1H, m), 4.06-3.96 (1H, m), 2.65 (3H, d, J = 4.8 Hz), 2.03-1.89 (2H, m), 1.76-1.43 (4H, m), 1.27-1.17 (4H, m), 1.06 (3H, d, J = 6.5 Hz), 0.76 (3H, | 504 | 502 |

Table 1-60

| | | | | | |
|---|---|---|---|---|---|
| 2-161 | 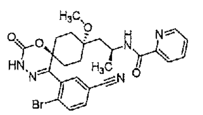 | | 1H-NMR (DMSO-D6) δ: 11.31 (1H, s), 8.64-8.57 (2H, m), 8.07-7.94 (4H, m), 7.89-7.85 (1H, m), 7.60-7.55 (1H, m), 4.24-4.14 (1H, m), 2.96 (3H, s), 1.98-1.47 (10H, m), 1.17 (3H, d, J = 6.5 Hz). | 540 | 538 |
| 2-162 | 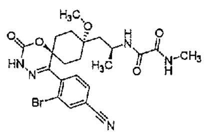 | | 1H-NMR (DMSO-D6) δ: 11.33 (1H, s), 8.64-8.53 (2H, m), 8.36 (1H, d, J = 1.6 Hz), 8.00-7.96 (1H, m), 7.68 (1H, d, J = 8.1 Hz), 4.02-3.91 (1H, m), 2.91 (3H, s), 2.65 (3H, d, J = 4.8 Hz), 1.98-1.79 (3H, m), 1.77-1.68 (1H, m), 1.62-1.42 (6H, m), 1.08 (3H, d, J = 7.0 Hz). | 520 | 518 |
| 2-163 | 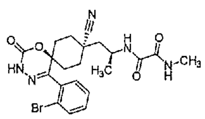 | | 1H-NMR (DMSO-D6) δ: 11.31 (1H, s), 8.71 (1H, d, J = 9.7 Hz), 8.63-8.56 (1H, m), 7.77 (1H, d, J = 7.5 Hz), 7.51-7.47 (2H, m), 7.45-7.40 (1H, m), 4,17-4,04 (1H, m), 2.64 (3H, d, J = 4.8 Hz), 2.27-2.16 (2H, m), 2.11-1.95 (2H, m), 1.81-1.52 (6H, m), 1.12 (3H, d, J = 6.5 Hz), | 490 | 488 |
| 2-164 | 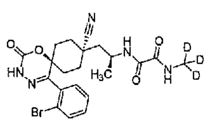 | | 1H-NMR (DMSO-D6) δ: 11.31 (1H, s), 8.71 (1H, d, J = 9.2 Hz), 8.57 (1H, s), 7.77 (1H, d, J = 8.1 Hz), 7.54-7.38 (3H, m), 4.19-4.03 (1H, m), 2.27-2.16 (2H, m), 2.12-1.93 (2H, m), 1.82-1.52 (6H, m), 1.12 (3H, d, J = 6.5 Hz). | 493 | 491 |
| 2-165 | 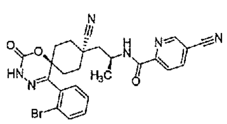 | | 1H-NMR (DMSO-D6) δ: 11.30 (1H, s), 9.09 (1H, d, J = 2.2 Hz), 8.99 (1H, d, J = 9.2 Hz), 8.50-8.46 (1H, m), 8.14 (1H, d, J = 8.1 Hz), 7.75 (1H, d, J = 7.5 Hz), 7.48-7.44 (2H, m), 7.43-7.37 (1H, m), 4.41-4.31 (1H, m), 2.29-2.01 (4H, m), 1.84-1.53 (6H, m), 1.20 (3H, d, J = 6.5 Hz). | 535 | 533 |

(continued)

| | | | | | |
|---|---|---|---|---|---|
| 2-166 | 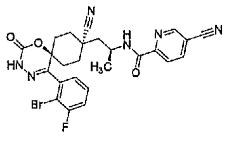 | | 1H-NMR (DMSO-D6) δ: 11.38 (1H, s), 9.10-9.08 (1H, m), 8.99 (1H, d, J = 9.7 Hz), 8.50-8.46 (1H, m), 8.14 (1H, d, J = 8.1 Hz), 7.55-7.46 (2H, m), 7.36-7.32 (1H, m), 4.41-4.31 (1H, m), 2.28-2.01 (4H, m), 1.83-1,52 (6H, m), 1.20 (3H, d, J = 6.5 Hz). | 553 | 551 |
| 2-167 | 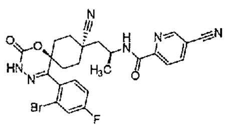 | | 1H-NMR (DMSO-D6) δ: 11.33 (1H, s), 9.09 (1H, d, J = 2.2 Hz), 8.99 (1H, d, J = 9.7 Hz), 8,50-8,46 (1H, m), 8.16-8.13 (1H, m), 7.75 (1H, dd, J = 8.6, 2.2 Hz), 7.58-7.53 (1H, m), 7.40-7.33 (1H, m), 4.42-4.31 (1H, m), 2.28-2.01 (4H, m), 1.83-1.51 (6H, m), 1.20 (3H, d, J = 7.0 Hz). | 553 | 551 |
| 2-168 | 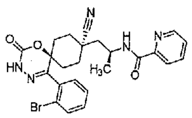 | | 1H-NMR (DMSO-D6) δ: 11.29 (1H, s), 8.75 (1H, d, J = 9.7 Hz), 8.61 (1H, d, J = 3.8 Hz), 8.03-7.93 (2H, m), 7.75 (1H, d, J = 8.1 Hz), 7.59-7.54 (1H, m), 7.48-7.44 (2H, m), 7.43-7.36 (1H, m), 4.40-4.29 (1H, m), 2.32-2,01 (4H, m), 1.87-1.52 (6H, m), 1.20 (3H, d, J = 6.5 Hz). | 510 | 508 |
| 2-169 | 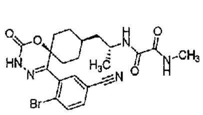 | | 1H-NMR (DMSO-D6) δ: 11.29 (1H, s), 8.65-8.59 (1H, m), 8.41 (1H, d, J = 9.0 Hz), 8.00 (1H, d, J = 1.8 Hz), 7.98 (1H, d, J = 8,3 Hz), 7.86 (1H, dd, J = 8.3, 1.8 Hz), 3.98-3.85 (1H, m), 2.64 (3H, d, J = 4.9 Hz), 2.16-2.04 (2H, m), 1.69-1.59 (1H, m), 1.59-1.13 (8H, m), 1.06 (3H, d, J = 6.5 Hz). | 490 | 488 |
| 2-170 | 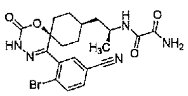 | | 1H-NMR (DMSO-D6) δ: 11.30 (1H, s), 8.39 (1H, d, J = 8.6 Hz), 8.02-7.95 (3H, m), 7.88-7.83 (1H, m), 7.72 (1H, brs), 3.94-3.82 (1H, m), 2.16-2.06 (2H, m), 1.68-1.60 (1H, m), 1.56-1.12 (8H, m), 1.05 (3H, d, J = 6.5 Hz), | 476 | 474 |

Table 1-61

| | | | | | |
|---|---|---|---|---|---|
| 2-171 | 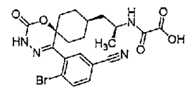 | | 1H-NMR (DMSO-D6) δ: 11.30 (1H, s), 8.07-7,80 (4H, m), 3.84-3.74 (1H, m), 2.21-2.01 (2H, m), 1.70-1.10 (9H, m), 1.01 (3H, d, J = 6.6 Hz). | 477 | 475 |
| 2-172 | 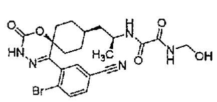 | | 1H-NMR (DMSO-D6) δ: 11.30 (1H, s), 9.11 (1H, t, J = 6.7 Hz), 8.52 (1H, d, J = 9.2 Hz), 8.00 (1H, d, J = 2.2 Hz), 7.98 (1H, d, J = 8.6 Hz), 7.86 (1H, dd, J = 8.6, 2.2 Hz), 5.65 (1H, t, J = 6.7 Hz), 4.54 (2H, t, J = 6.7 Hz), 3.98-3.86 (1H, m), 2.16-2.06 (2H, m), 1.68-1,16 (9H, m), 1.06 (3H, d, J = 6.5 Hz). | 488 (M-17) | 504 |

Experimental Example 1: Evaluation of human PLD1 enzyme inhibitory activity

A method for measuring human PLD1 enzyme inhibitory activity using an enzyme in which a FLAG-tag is added to the N-terminus of full-length human PLD1 (1-1036 amino acids) is shown below.

1. Production of full-length human PLD1-expression plasmid

DNA fragment in which a FLAG-tag sequence is added to the 5'-end of full-length human PLD1 was amplified by PCR method, using commercially available human PLD1 gene (Promega KK) as a template. The amplified DNA fragment was fused with vector pVL1393 for baculovirus production (Pharmingen) digested with BamHI and EcoRI, using In-Fusion HD Cloning Kit (Takara Bio). The full-length human PLD1-expression plasmid DNA for baculovirus was isolated from Escherichia coli DH5α (TOYOBO) transformed with the obtained In-Fusion reaction product. Next, DNA fragment in which a FLAG-tag sequence is added to the 5'-end of full-length human PLD1 was amplified by PCR method, using the full-length human PLD1-expression plasmid DNA for baculovirus as a template. The amplified DNA fragment was fused with a DNA fragment in which a linker sequence is introduced into pcDNA3.4-TOPO (Life Technologies), using In-Fusion HD Cloning Kit (Takara Bio). The full-length human PLD1-expression plasmid DNA for animal cells was isolated from Escherichia coli

DH5α transformed with the obtained In-Fusion reaction product. The base sequence of the full-length human PLD1 cloned into vector was determined by a Dye Terminator method using BigDye Terminator v3.1 Cycle Sequencing Kit (Applied Biosystems). The determined sequence was a sequence in which the FLAG-tag sequence was inserted at the 5'-end of the full-length human PLD1 (GenBank Accession
Number:NM_001130081).

2. Production of full-length human PLD1 protein in Expi293F cell expression system

Expi293F cells (Life Technologies) were cultured with shaking using Expi293 Expression Medium (Life Technologies) as a medium at 37°C in the presence of 8% $CO_2$. The gene introduction reagent PEI was prepared by dissolving Polyethylenimine Max (nominally MW 40,000) (Polysciences) in MilliQ water, adjusting the pH of the solution to 7.0 with NaOH to obtain a 1 mg/mL solution, and then filtering the solution through 0.22 μm filter. The full-length human PLD1-expression plasmid DNA for animal cells was transfected into Expi293F cells using PEI, and after 48 hours of shaking culture, the cells were collected and stored at -80°C.

3. Purification of full-length human PLD1 protein

To the full-length human PLDl-expressed cells was added a Homogenate buffer (20 mmol/L Na-phosphate pH 7.5, 250 mmol/L NaCl, 1 mmol/L $MgCl_2$, 1%β-OG, 0.05 mmol/L DTT+complete EDTA-free (Roche Diagnostics K.K.)), and the cells were lysed by Microfluidizer Processor M-110EH (Mizuho Kogyo Co., Ltd.). The lysate was centrifuged at 10,100×g at 4°C for 15 minutes, and the supernatant was collected and filtered through 0.45 μm filter. To the centrifuged supernatant was added ANTI-FLAG M2 Affinity Gel (SIGMA-Aldrich) resin equilibrated with a Homogenate buffer, and the mixed solution was stirred at 4°C for 1 hr or more. The mixed solution was loaded onto Poly-Prep column, and the resin was washed with a Wash buffer (20 mmol/L Na-phosphate pH 7.5, 150 mmol/L NaCl, 1 mmol/L $MgCl_2$, 0.02% Triton, 0.05 mmol/L DTT). A Wash buffer containing 400 μg/mL DYKDDDDK Peptide (Scrum Inc.) was added to the column to elute the protein bound to the resin. The eluted fractions were subjected to SDS-PAGE followed by CBB staining to identify the fractions containing the full-length human PLD1. The eluted fractions of the full-length human PLD1 were collected and concentrated using Amicon Ultra-15 100k (Merck Millipore). The concentrated fractions were collected, flash-frozen in liquid nitrogen and stored at -80°C.

4. Evaluation human PLD1 inhibitory activity

A DMSO or test substance solution (the DMSO final concentration: 5%) prepared by diluting DMSO or test substance with an Assay buffer (50 mmol/L HEPES pH 7.5, 80 mmol/L KCl, 3 mmol/L EGTA, 3.6 mmol/L $MgCl_2$, 0.01% NP-40, 0.1% BSA), and the solution was added to a 384 well Assay Plate (Black, Polystyrene, Non-Treated, Cat No.3573, Corning) by 5 μL/well. A full-length human PLD1 enzyme solution (6 nmol/L) diluted with an Assay buffer was added thereto by 5 μL/well (the Assay buffer was added to blank wells). A 1,2-diheptanoyl-sn-glycero-3-phosphocholine (Avanti Polar Lipids) substrate solution (6 mmol/L) diluted with an Assay buffer was added thereto by 5 μL/well, and enzymatic reaction was carried out at room temperature for 60 minutes. Immediately before stopping the reaction, a detection solution (200 μmol/L AmplexRed (Thermofisher), 0.1 U/mL Choline Oxidase (Sigma-Aldrich), 4 U/mL Horseradish peroxidase (Thermofisher)) containing 4 μmol/L of 5-fluoro-2-indolyl deschlorohalopemide (R&D Systems) as a reaction terminator was prepared with an Assay buffer, the detection solution was added thereto at 5 μL/well to terminate the enzymatic reaction. Immediately after stopping the reaction and 30 minutes after incubation at room temperature, the fluorescence values at Ex: 531 nm/Em: 590 nm were measured by ARVO X5 (PerkinElmer). The inhibition rate was calculated from the change in the fluorescence values immediately after stopping the reaction and after 30 minutes. Data were obtained by subtracting the average fluorescence value of the blank wells from the average fluorescence value of the treated wells. The inhibition rate at each concentration of the test substance was calculated from the following formula:

$$100-(B/A)\times 100$$

A: data of solvent control
B: data of test substance-treatment

The $IC_{50}$ value (50% inhibitory concentration) of the test substance was calculated by fitting the inhibition rate at each concentration of the test substance to a logistic curve. The results of the compounds of each example are shown in Tables 2-1 to 2-14 below. For Examples 34, 186, 2-047, 2-099, 2-101, 2-150 and 2-171, the inhibition rate of PLD1 at 10 μM

compound is shown, and Example 34 gave 36% inhibition, Example 186 gave 33% inhibition, Example 2-047 gave 32% inhibition, Example 2-099 gave 21% inhibition, Example 2-101 gave 13% inhibition, Examples 2-150 gave 25% inhibition, and Example 2-171 gave 12% inhibition.

Experimental Example 2: Evaluation of human PLD2 enzyme inhibitory activity

A method for measuring human PLD2 enzyme inhibitory activity using an enzyme in which a FLAG-tag is added to the N-terminus of full-length human PLD2 (1-933 amino acids) is shown below.

1. Production of full-length human PLD2-expression plasmid

DNA fragment in which a FLAG-tag sequence is added to the 5'-end of full-length human PLD2 was amplified by PCR method, using commercially available human PLD2 gene (Promega KK) as a template. The amplified DNA fragment was fused with vector pVL1393 for baculovirus production (Pharmingen) digested with BamHI and EcoRI, using In-Fusion HD Cloning Kit (Takara Bio). The full-length human PLD2-expression plasmid DNA for baculovirus was isolated from Escherichia coli DH5α (TOYOBO) transformed with the obtained In-Fusion reaction product. Next, DNA fragment in which a FLAG-tag sequence is added to the 5'-end of full-length human PLD2 was amplified by PCR method, using the full-length human PLD2-expression plasmid DNA for baculovirus as a template. The amplified DNA fragment was fused with a DNA fragment in which a linker sequence is introduced into pcDNA3.4-TOPO (Life Technologies), using In-Fusion HD Cloning Kit (Takara Bio). The full-length human PLD2-expression plasmid DNA for animal cells was isolated from Escherichia coli DH5α transformed with the obtained In-Fusion reaction product. The base sequence of the full-length human PLD2 cloned into vector was determined by a Dye Terminator method using BigDye Terminator v3.1 Cycle Sequencing Kit (Applied Biosystems). The determined sequence was a sequence in which the FLAG-tag sequence was inserted at the 5'-end of the full-length human PLD2 (GenBank Accession Number:NM_002663) .

2. Production of full-length human PLD2 protein in Expi293F cell expression system

Expi293F cells (Life Technologies) were cultured with shaking using Expi293 Expression Medium (Life Technologies) as a medium at 37°C in the presence of 8% $CO_2$. The gene introduction reagent PEI was prepared by dissolving Polyethylenimine Max (nominally MW 40,000) (Polysciences) in MilliQ water, adjusting the pH of the solution to 7.0 with NaOH to obtain a 1 mg/mL solution, and then filtering the solution through 0.22 μm filter. The full-length human PLD2-expression plasmid DNA for animal cells was transfected into Expi293F cells using PEI, and after 48 hours of shaking culture, the cells were collected and stored at -80°C.

3. Purification of full-length human PLD2 protein

To the full-length human PLD2-expressed cells was added a Homogenate buffer (20 mmol/L Na-phosphate pH 7.5, 250 mmol/L NaCl, 1 mmol/L $MgCl_2$, 1%β-OG, 0.05 mmol/L DTT+complete EDTA-free (Roche Diagnostics K.K.)), and the cells were lysed by Microfluidizer Processor M-110EH (Mizuho Kogyo Co., Ltd.). The lysate was centrifuged at 10,100×g at 4°C for 15 minutes, and the supernatant was collected and filtered through 0.45 μm filter. To the centrifuged supernatant was added ANTI-FLAG M2 Affinity Gel (SIGMA-Aldrich) resin equilibrated with a Homogenate buffer, and the mixed solution was stirred at 4°C for 1 hr or more. The mixed solution was loaded onto Poly-Prep column, and the resin was washed with a Wash buffer (20 mmol/L Na-phosphate pH 7.5, 150 mmol/L NaCl, 1 mmol/L $MgCl_2$, 0.02% Triton, 0.05 mmol/L DTT). A Wash buffer containing 400 μg/mL DYKDDDDK Peptide (Scrum Inc.) was added to the column to elute the protein bound to the resin. The eluted fractions were subjected to SDS-PAGE followed by CBB staining to identify the fractions containing the full-length human PLD2. The eluted fractions of the full-length human PLD2 were collected and concentrated using Amicon Ultra-15 100k (Merck Millipore). The concentrated fractions were collected, flash-frozen in liquid nitrogen and stored at -80°C.

4. Evaluation human PLD2 inhibitory activity

A DMSO or test substance solution (the DMSO final concentration: 5%) prepared by diluting DMSO or test substance with an Assay buffer (50 mmol/L HEPES pH 7.5, 80 mmol/L KCl, 3 mmol/L EGTA, 3.6 mmol/L $MgCl_2$, 0.01% NP-40, 0.1% BSA), and the solution was added to a 384 well Assay Plate (Black, Polystyrene, Non-Treated, Cat No.3573, Corning) by 5 μL/well. A full-length human PLD2 enzyme solution (6 nmol/L) diluted with an Assay buffer was added thereto by 5 μL/well (the Assay buffer was added to blank wells). A 1,2-diheptanoyl-sn-glycero-3-phosphocholine (Avanti Polar Lipids) substrate solution (6 mmol/L) diluted with an Assay buffer was added thereto by 5 μL/well, and enzymatic

reaction was carried out at room temperature for 60 minutes. Immediately before stopping the reaction, a detection solution (200 μmol/L AmplexRed (Thermofisher), 0.1 U/mL Choline Oxidase (Sigma-Aldrich), 4 U/mL Horseradish peroxidase (Thermofisher)) containing 4 μmol/L of 5-fluoro-2-indolyl deschlorohalopemide (R&D Systems) as a reaction terminator was prepared with an Assay buffer, the detection solution was added thereto at 5 μL/well to terminate the enzymatic reaction. Immediately after stopping the reaction and 30 minutes after incubation at room temperature, the fluorescence values at Ex: 531 nm/Em: 590 nm were measured by ARVO X5 (PerkinElmer). The inhibition rate was calculated from the change in the fluorescence values immediately after stopping the reaction and after 30 minutes. Data were obtained by subtracting the average fluorescence value of the blank wells from the average fluorescence value of the treated wells. The inhibition rate at each concentration of the test substance was calculated from the following formula:

$$100-(B/A)\times 100$$

A: data of solvent control
B: data of test substance-treatment

The $IC_{50}$ value (50% inhibitory concentration) of the test substance was calculated by fitting the inhibition rate at each concentration of the test substance to a logistic curve. The results of the compounds of each example are shown in Tables 2-1 to 2-14 below. For Examples 34, 186, 2-047, 2-101, 2-112, 2-150 and 2-171, the inhibition rate of PLD2 at 10 μM compound is shown, and Example 34 gave 17% inhibition, Example 186 gave 18% inhibition, Example 2-047 gave Example 34% inhibition, Example 2-101 gave 28% inhibition, Example 2-112 gave 39% inhibition, Example 2-150 gave 30% inhibition, and Example 2-171 gave 23% inhibition.

Table 2-1

| Example No. | PLD1 $IC_{50}$ (μM) | PLD2 $IC_{50}$ (μM) |
|---|---|---|
| 1 | 0.033 | < 0.010 |
| 2 | 0.020 | < 0.010 |
| 3 | 0.027 | < 0.010 |
| 4 | 0.036 | 0.011 |
| 5 | 0.164 | 0.171 |
| 6 | 0.069 | 0.120 |
| 7 | 0.030 | 0.044 |
| 8 | 0.030 | 0.010 |
| 9 | 0.083 | 0.056 |
| 10 | 0.092 | 0.106 |
| 11 | 0.228 | 0.394 |
| 12 | < 0.010 | < 0.010 |
| 13 | 0.036 | < 0.010 |
| 14 | 0.010 | < 0.010 |
| 15 | 0.025 | < 0.010 |
| 16 | 0.926 | 0.968 |
| 17 | 0.816 | 1.044 |
| 18 | 0.852 | 0.157 |
| 19 | 0.017 | 0.010 |
| 20 | 1.646 | 3.769 |
| 21 | 0.307 | 0.346 |
| 22 | 0.066 | 0.049 |
| 23 | 0.039 | 0.051 |

(continued)

| Example No. | PLD1 $IC_{50}$ (μM) | PLD2 $IC_{50}$ (μM) |
|---|---|---|
| 24 | 0.041 | 0.031 |
| 25 | 0.108 | 0.101 |
| 26 | < 0.010 | < 0.010 |
| 27 | 0.014 | 0.013 |
| 28 | 0.341 | 0.303 |
| 29 | 0.999 | 1.715 |
| 30 | 0.123 | 0.153 |
| 31 | 0.024 | < 0.010 |
| 32 | 0.032 | < 0.010 |
| 33 | 0.025 | < 0.010 |
| 34 | 36% inhibition at 10μM | 17% inhibition at 10μM |
| 35 | 0.257 | 0.367 |
| 36 | 0.221 | 0.137 |
| 37 | < 0.010 | < 0.010 |
| 38 | 0.035 | 0.088 |
| 39 | 0.093 | 0.024 |
| 40 | 0.013 | < 0.010 |

Table 2-2

| | | |
|---|---|---|
| 41 | 0.229 | 0.500 |
| 42 | 0.247 | 0.261 |
| 43 | 0.221 | 0.184 |
| 44 | 0.018 | < 0.010 |
| 45 | 0.027 | 0.023 |
| 46 | 0.012 | < 0.010 |
| 47 | 0.119 | < 0.010 |
| 48 | 0.014 | < 0.010 |
| 49 | 0.031 | 0.011 |
| 50 | 0.033 | 0.051 |
| 51 | 0.033 | 0.016 |
| 52 | 0.021 | < 0.010 |
| 53 | 0.019 | < 0.010 |
| 54 | 0.020 | < 0.010 |
| 55 | 0.015 | < 0.010 |
| 56 | 0.026 | 0.017 |
| 57 | 0.016 | < 0.010 |
| 58 | 0.014 | < 0.010 |
| 59 | < 0.010 | < 0.010 |
| 60 | < 0.010 | < 0.010 |

(continued)

| | | |
|---|---|---|
| 61 | 0.014 | 0.011 |
| 62 | 0.140 | 0.223 |
| 63 | < 0.010 | < 0.010 |
| 64 | 0.018 | < 0.010 |
| 65 | < 0.010 | < 0.010 |
| 66 | 0.016 | < 0.010 |
| 67 | < 0.010 | < 0.010 |
| 68 | 2.028 | 4.659 |
| 69 | < 0.010 | < 0.010 |
| 70 | 0.028 | 0.020 |
| 71 | < 0.010 | < 0.010 |
| 72 | 0.034 | 0.024 |
| 73 | 0.023 | 0.015 |
| 74 | < 0.010 | < 0.010 |
| 75 | 1.628 | 1.992 |
| 76 | 0.017 | 0.017 |
| 77 | 0.026 | < 0.010 |
| 78 | 0.350 | 0.252 |
| 79 | < 0.010 | < 0.010 |
| 80 | < 0.010 | < 0.010 |

Table 2-3

| | | |
|---|---|---|
| 81 | 0.012 | < 0.010 |
| 82 | 0.421 | 0.358 |
| 83 | 0.259 | 0.221 |
| 84 | 0.070 | 0.053 |
| 85 | 0.012 | < 0,010 |
| 86 | 0.013 | < 0.010 |
| 87 | 0.039 | < 0.010 |
| 88 | 0.056 | 0.065 |
| 89 | 0.038 | 0.037 |
| 90 | 0.045 | 0.018 |
| 91 | 0.023 | 0.015 |
| 92 | 0.026 | 0.013 |
| 93 | 0.032 | < 0.010 |
| 94 | 0.068 | 0.060 |
| 95 | 8.056 | 6.165 |
| 96 | 0.018 | < 0.010 |
| 97 | < 0.010 | < 0.010 |
| 98 | 0.057 | 0.078 |

(continued)

| | | |
|---|---|---|
| 99 | 0.241 | 0.373 |
| 100 | 0.041 | 0.033 |
| 101 | < 0.010 | < 0.010 |
| 102 | 0.288 | 0.666 |
| 103 | 0.170 | 0.379 |
| 104 | 0.075 | 0.217 |
| 105 | 0.027 | 0.041 |
| 106 | 0.026 | 0.048 |
| 107 | 0.085 | 0.139 |
| 108 | 0.025 | 0.038 |
| 109 | 0.012 | < 0.010 |
| 110 | 0.083 | 0.099 |
| 111 | 0.017 | 0.021 |
| 112 | 0.031 | 0.020 |
| 113 | 0.017 | 0.013 |
| 114 | 0.013 | < 0.010 |
| 115 | 0.015 | < 0.010 |
| 116 | 0.043 | 0.037 |
| 117 | 0.021 | 0.018 |
| 118 | 0.050 | 0.073 |
| 119 | 0.011 | < 0.010 |
| 120 | 0.012 | 0.011 |

Table 2-4

| | | |
|---|---|---|
| 121 | < 0.010 | < 0.010 |
| 122 | < 0.010 | < 0.010 |
| 123 | 0.202 | 0.502 |
| 124 | 0.638 | 2.736 |
| 125 | 0.040 | 0.057 |
| 126 | 0.013 | < 0.010 |
| 127 | 0.025 | < 0.010 |
| 128 | 0.186 | 0.060 |
| 129 | 0.018 | < 0.010 |
| 130 | 0.018 | < 0.010 |
| 131 | 0.010 | < 0.010 |
| 132 | 0.014 | < 0.010 |
| 133 | 0.029 | 0.031 |
| 134 | 0.021 | 0.030 |
| 135 | 0.086 | 0.033 |
| 136 | 0,155 | 0.170 |

(continued)

| | | |
|---|---|---|
| 137 | 0.505 | 1.586 |
| 138 | 0.774 | 1.396 |
| 139 | < 0.010 | < 0.010 |
| 140 | < 0.010 | < 0.010 |
| 141 | 0.141 | 0.164 |
| 142 | 0.022 | < 0.010 |
| 143 | 0.027 | 0.025 |
| 144 | 0.011 | < 0.010 |
| 145 | 0.017 | < 0.010 |
| 146 | 0.020 | < 0.010 |
| 147 | 0.032 | 0.032 |
| 148 | 0.020 | 0.018 |
| 149 | 0.012 | < 0.010 |
| 150 | 0.880 | 0.640 |
| 151 | 0.018 | < 0.010 |
| 152 | 0.023 | 0.013 |
| 153 | 0.027 | 0.028 |
| 154 | 0.027 | 0.014 |
| 155 | 0.014 | < 0.010 |
| 156 | 0.236 | 0.412 |
| 157 | < 0.010 | < 0.010 |
| 158 | 0.027 | 0.015 |
| 159 | 0.028 | < 0.010 |
| 160 | 0.026 | 0.017 |

Table 2-5

| | | |
|---|---|---|
| 161 | 0.020 | 0.013 |
| 162 | 0.322 | 0.536 |
| 163 | < 0.010 | < 0.010 |
| 164 | 0.038 | < 0.010 |
| 165 | 0.019 | < 0.010 |
| 166 | 0.019 | < 0.010 |
| 167 | 0.020 | < 0.010 |
| 168 | 0.056 | 0.035 |
| 169 | 0.034 | 0.020 |
| 170 | 0.019 | < 0.010 |
| 171 | 0.013 | < 0.010 |
| 172 | 0.023 | 0.012 |
| 173 | 0.029 | 0.010 |
| 174 | 0.042 | 0.017 |

(continued)

| | | |
|---|---|---|
| 175 | 0.021 | 0.015 |
| 176 | 0.029 | 0.029 |
| 177 | 0.010 | < 0.010 |
| 178 | 0.014 | 0.017 |
| 179 | 0.025 | < 0.010 |
| 180 | 0,028 | 0.012 |
| 181 | 0.013 | < 0.010 |
| 182 | 0.019 | < 0.010 |
| 183 | 0.042 | 0.107 |
| 184 | < 0.010 | < 0.010 |
| 185 | 0.068 | 0.064 |
| 186 | 33% inhibition at 10μM | 18% inhibition at 10uM |
| 187 | 0.222 | 0.455 |
| 188 | 0.044 | 0.033 |
| 189 | 0.130 | 0.140 |
| 190 | 0.642 | 0.776 |
| 191 | 0.038 | < 0.010 |
| 192 | 0.021 | < 0.010 |
| 193 | 0.055 | 0.046 |
| 194 | < 0.010 | < 0.010 |
| 195 | 0.039 | 0.067 |
| 196 | 0.027 | < 0.010 |
| 197 | 0.026 | < 0.010 |
| 198 | 0.011 | < 0.010 |
| 199 | 0.010 | < 0.010 |
| 200 | 0.103 | 0.082 |

Table 2-6

| | | |
|---|---|---|
| 201 | 0.328 | 0.371 |
| 202 | 0.832 | 1.247 |
| 203 | 0.012 | < 0.010 |
| 204 | 0.123 | 0.146 |
| 205 | 0.035 | 0.102 |
| 206 | 0.036 | 0.013 |
| 207 | 0.025 | < 0.010 |
| 208 | 0.045 | 0.224 |
| 209 | 0.018 | 0.018 |
| 210 | 0.296 | 1.087 |
| 211 | 0.102 | 0.429 |
| 212 | < 0.010 | < 0.010 |

(continued)

| | | |
|---|---|---|
| 213 | 0.086 | 0.115 |
| 214 | < 0.010 | < 0.010 |
| 215 | < 0.010 | < 0.010 |
| 216 | 0.029 | 0.019 |
| 217 | 0.017 | < 0.010 |
| 218 | 0.023 | 0.013 |
| 219 | < 0.010 | < 0.010 |
| 220 | 0.042 | 0.033 |
| 221 | 0.016 | < 0.010 |
| 222 | 0.059 | 0.171 |
| 223 | 0.396 | 0.246 |
| 224 | 0.013 | < 0.010 |
| 225 | 0.513 | 0.203 |
| 226 | 0.018 | < 0.010 |
| 227 | 0.019 | < 0.010 |
| 228 | 0.028 | < 0.010 |
| 229 | 0.197 | 0.310 |
| 230 | 0.021 | < 0.010 |
| 231 | 0.024 | 0.012 |
| 232 | 2.580 | 2.132 |
| 233 | 1.861 | 1. 796 |
| 234 | 0.247 | 0.474 |
| 235 | 0.035 | 0.013 |
| 236 | 0.015 | < 0.010 |
| 237 | 0.034 | < 0.010 |
| 238 | 0.012 | < 0.010 |
| 239 | 0.018 | < 0.010 |
| 240 | 0.013 | < 0.010 |

Table 2-7

| | | |
|---|---|---|
| 241 | 0.017 | < 0.010 |
| 242 | 0.016 | < 0.010 |
| 243 | 0.024 | < 0.010 |
| 244 | 0.019 | < 0.010 |
| 245 | 0.040 | 0.107 |
| 246 | 0.015 | < 0.010 |
| 247 | 0.014 | < 0.010 |
| 248 | 0.017 | < 0.010 |
| 249 | 0.025 | < 0.010 |
| 250 | 0.022 | < 0.010 |

(continued)

| | | |
|---|---|---|
| 251 | 0.017 | < 0.010 |
| 252 | 0.174 | 0.335 |
| 253 | 0.252 | 0.324 |
| 254 | 0.029 | < 0.010 |
| 255 | 0.032 | 0.014 |
| 256 | 0.027 | < 0.010 |
| 257 | 0.262 | 0.215 |
| 258 | 0.186 | 0.249 |
| 259 | 1.450 | 2.499 |
| 260 | 0.031 | < 0.010 |
| 261 | 0.168 | 0.313 |
| 262 | 0.015 | < 0.010 |
| 263 | 2.777 | 4.499 |
| 264 | 0.036 | < 0.010 |
| 265 | 0.013 | < 0.010 |
| 266 | 0.015 | < 0.010 |
| 267 | 0.026 | < 0.010 |
| 268 | 0.030 | < 0.010 |
| 269 | 0.018 | < 0.010 |
| 270 | 0.017 | < 0.010 |
| 271 | 0.018 | < 0.010 |
| 272 | 0.015 | < 0.010 |
| 273 | 0.019 | < 0.010 |
| 274 | 0.017 | < 0.010 |
| 275 | 0.025 | < 0.010 |
| 276 | 0.021 | < 0.010 |
| 277 | 0.017 | < 0.010 |
| 278 | 0.246 | 1.176 |
| 279 | 0.086 | 0.044 |
| 280 | 0.147 | 0.316 |

Table 2-8

| | | |
|---|---|---|
| 281 | 0.169 | 0.232 |
| 282 | 0.097 | 0.146 |
| 283 | 0.043 | 0.048 |
| 284 | 0.061 | 0.040 |
| 285 | < 0.010 | < 0.010 |
| 286 | 0.014 | < 0.010 |
| 287 | 0.013 | < 0.010 |
| 288 | 0.026 | < 0.010 |

(continued)

| | | |
|---|---|---|
| 289 | 0.018 | < 0.010 |
| 290 | 0.122 | 0.046 |
| 291 | 0.150 | 0.234 |
| 292 | 0.023 | < 0.010 |
| 293 | 0.127 | 0.325 |
| 294 | 0.011 | < 0.010 |
| 295 | < 0.010 | < 0.010 |
| 296 | 0.013 | < 0.010 |
| 297 | 0.013 | < 0.010 |
| 298 | 0.015 | < 0.010 |
| 299 | 0.025 | < 0.010 |
| 300 | 0.038 | < 0.010 |
| 301 | 0.030 | < 0.010 |
| 302 | 0.017 | < 0.010 |
| 303 | 0.011 | < 0.010 |
| 304 | < 0.010 | < 0.010 |
| 305 | 0.015 | < 0.010 |
| 306 | < 0.010 | < 0.010 |
| 307 | 0.013 | < 0.010 |
| 306 | 0.015 | < 0.010 |
| 309 | 0.015 | < 0.010 |
| 310 | 0.014 | < 0.010 |
| 311 | 0.015 | < 0.010 |
| 312 | 0.029 | < 0.010 |
| 313 | 0.023 | 0.025 |
| 314 | < 0.010 | < 0.010 |
| 315 | 0.025 | 0.024 |
| 316 | 0.046 | 0.061 |
| 317 | 0.071 | 0.065 |
| 318 | 0.037 | 0.041 |
| 319 | 2.092 | 0.744 |
| 320 | 3.881 | 1.577 |

Table 2-9

| | | |
|---|---|---|
| 321 | 0.042 | < 0.010 |
| 322 | 0.030 | < 0.010 |
| 323 | 0.010 | < 0.010 |
| 324 | 0.020 | 0.022 |
| 325 | 0.015 | < 0.010 |
| 326 | 0.054 | 0.038 |

(continued)

| | | |
|---|---|---|
| 327 | 0.110 | 0.051 |
| 328 | 0.048 | 0.034 |
| 329 | 0.051 | < 0.010 |
| 330 | 3.151 | 0.026 |
| 331 | 0.032 | 0.022 |
| 332 | 2.595 | 0.015 |
| 333 | 0.025 | < 0.010 |
| 334 | 0.026 | < 0.010 |
| 335 | 0.126 | < 0.010 |
| 336 | 0.033 | < 0.010 |
| 337 | 0.074 | 0.028 |
| 338 | 0.024 | < 0.010 |
| 339 | 0.039 | 0.010 |

Table 2-10

| | | |
|---|---|---|
| 2-001 | 0.041 | < 0.010 |
| 2-002 | 1.254 | 1.865 |
| 2-003 | 0.038 | 0.012 |
| 2-004 | 0.068 | < 0.010 |
| 2-005 | 0.433 | 0.035 |
| 2-006 | 0.056 | 0.011 |
| 2-007 | 0.028 | < 0.010 |
| 2-008 | 0.062 | 0.011 |
| 2-009 | 2.154 | 1.037 |
| 2-010 | 1.015 | 1.258 |
| 2-011 | 2.110 | 2.943 |
| 2-012 | 2.044 | 0.245 |
| 2-013 | 0.012 | < 0.010 |
| 2-014 | 0.022 | < 0.010 |
| 2-015 | 0.026 | 0.012 |
| 2-016 | 0.019 | < 0.010 |
| 2-017 | 0.099 | 0.045 |
| 2-018 | 0.022 | < 0.010 |
| 2-019 | 0.012 | < 0.010 |
| 2-020 | 0.021 | 0.016 |
| 2-021 | 0.027 | 0.015 |
| 2-022 | 0.738 | 0.388 |
| 2-023 | 0.102 | 0.064 |
| 2-024 | 0.046 | 0.028 |
| 2-025 | 0.219 | 0.205 |

(continued)

| | | |
|---|---|---|
| 2-026 | 0.012 | < 0.010 |
| 2-027 | 0.013 | < 0.010 |
| 2-028 | 0.045 | 0.032 |
| 2-029 | 1.507 | 8.407 |
| 2-030 | 0.031 | 0.018 |
| 2-031 | 0.019 | < 0.010 |
| 2-032 | 0.011 | < 0.010 |
| 2-033 | 0.035 | < 0.010 |
| 2-034 | 0.031 | 0.010 |
| 2-035 | 0.021 | < 0.010 |
| 2-036 | 0.017 | < 0.010 |
| 2-037 | 0.043 | 0.013 |
| 2-038 | 0.060 | 0.011 |
| 2-039 | 0.153 | 0.264 |
| 2-040 | 0.021 | < 0.010 |

Table 2-11

| | | |
|---|---|---|
| 2-041 | 0.148 | 0.103 |
| 2-042 | 3.018 | 0.383 |
| 2-043 | 0.033 | 0.152 |
| 2-044 | < 0.010 | < 0.010 |
| 2-045 | 0.018 | < 0.010 |
| 2-046 | 0.024 | 0.118 |
| 2-047 | 32% inhibition at 10μM | 34% inhibition at 10uM |
| 2-048 | 0.644 | 1.014 |
| 2-049 | 1.743 | 1. 443 |
| 2-050 | 0.012 | < 0.010 |
| 2-051 | 0.025 | < 0.010 |
| 2-052 | 0.021 | < 0.010 |
| 2-053 | 0.676 | 0.245 |
| 2-054 | 0.037 | < 0.010 |
| 2-055 | 0.011 | < 0.010 |
| 2-056 | 0.017 | < 0.010 |
| 2-057 | 0.018 | < 0.010 |
| 2-058 | 0.026 | 0.066 |
| 2-059 | 0.731 | 0.505 |
| 2-060 | 0.027 | 0.011 |
| 2-061 | 0.014 | < 0.010 |
| 2-062 | 0.025 | 0.025 |
| 2-063 | 0.173 | 0.106 |

(continued)

| | | |
|---|---|---|
| 2-064 | 0.027 | 0.011 |
| 2-065 | 0.021 | < 0.010 |
| 2-066 | 0.015 | < 0.010 |
| 2-067 | 0.015 | < 0.010 |
| 2-068 | < 0.010 | < 0.010 |
| 2-069 | < 0.010 | < 0.010 |
| 2-070 | 0.068 | 0.038 |
| 2-071 | 0.013 | < 0.010 |
| 2-072 | 0.014 | 0.024 |
| 2-073 | 0.014 | 0.002 |
| 2-074 | 0.011 | < 0.010 |
| 2-075 | 0.016 | < 0.010 |
| 2-076 | 0.011 | < 0.010 |
| 2-077 | 0.015 | < 0.010 |
| 2-078 | < 0.010 | < 0.010 |
| 2-079 | 0.011 | 0.010 |
| 2-080 | < 0.010 | < 0.010 |

Table 2-12

| | | |
|---|---|---|
| 2-081 | < 0.010 | < 0.010 |
| 2-082 | < 0.010 | < 0.010 |
| 2-083 | < 0.010 | < 0.010 |
| 2-084 | 0.013 | < 0.010 |
| 2-085 | 0.022 | < 0.010 |
| 2-086 | 0.015 | < 0.010 |
| 2-087 | 0.012 | < 0.010 |
| 2-088 | 0.019 | < 0.010 |
| 2-089 | 0.018 | < 0.010 |
| 2-090 | 0.013 | < 0.010 |
| 2-091 | < 0.010 | < 0.010 |
| 2-092 | 0.026 | < 0.010 |
| 2-093 | 0.038 | 0.013 |
| 2-094 | 0.026 | < 0.010 |
| 2-095 | 0.035 | 0.012 |
| 2-096 | 0.055 | 0.031 |
| 2-097 | 0.020 | < 0.010 |
| 2-098 | 0.013 | < 0.010 |
| 2-099 | 21% inhibition at 10uM | 6.889 |
| 2-100 | 0.015 | < 0.010 |
| 2-101 | 13% inhibition at 10μM | 28% Inhibition at 10uM |

(continued)

| | | |
|---|---|---|
| 2-102 | 0.014 | < 0.010 |
| 2-103 | 0.023 | < 0.010 |
| 2-104 | 0.021 | 0.011 |
| 2-105 | 0.017 | < 0.010 |
| 2-106 | 0.073 | 0.127 |
| 2-107 | 0.011 | < 0.010 |
| 2-108 | < 0.010 | < 0.010 |
| 2-109 | 0.011 | < 0.010 |
| 2-110 | 0.021 | < 0.010 |
| 2-111 | 0.025 | 0.029 |
| 2-112 | 3.590 | 39% inhibition at 10μM |
| 2-113 | 0.014 | < 0.010 |
| 2-114 | 0.052 | < 0.010 |
| 2-115 | 0.038 | < 0.010 |
| 2-116 | 0.081 | 0.017 |
| 2-117 | 0.030 | < 0.010 |
| 2-118 | 0.019 | < 0.010 |
| 2-119 | 0.034 | < 0.010 |
| 2-120 | 0.021 | < 0.010 |

Table 2-13

| | | |
|---|---|---|
| 2-121 | 0.026 | 0.021 |
| 2-122 | 0.014 | < 0.010 |
| 2-123 | 0.026 | < 0.010 |
| 2-124 | 0.022 | < 0.010 |
| 2-125 | 0.043 | 0.016 |
| 2-126 | 0.035 | 0.014 |
| 2-127 | < 0.010 | < 0.010 |
| 2-128 | < 0.010 | < 0.010 |
| 2-129 | 0.025 | < 0.010 |
| 2-130 | 0.032 | < 0.010 |
| 2-131 | 0.015 | < 0.010 |
| 2-132 | 0.011 | < 0.010 |
| 2-133 | 0.014 | < 0,010 |
| 2-134 | 0.018 | < 0.010 |
| 2-135 | 0.020 | < 0.010 |
| 2-136 | 0.017 | 0.015 |
| 2-137 | 0.029 | 0.033 |
| 2-138 | 0.014 | < 0.010 |
| 2-139 | 0.014 | < 0.010 |

(continued)

| | | |
|---|---|---|
| 2-140 | 0.017 | < 0.010 |
| 2-141 | < 0.010 | < 0.010 |
| 2-142 | 0.012 | < 0.010 |
| 2-143 | 0.262 | 0.738 |
| 2-144 | 0.012 | < 0.010 |
| 2-145 | 0.014 | < 0.010 |
| 2-146 | 0.019 | 0.016 |
| 2-147 | 0.136 | 0.152 |
| 2-148 | 0.140 | 0.046 |
| 2-149 | 0.045 | 0.023 |
| 2-150 | 25% inhibition at 10μM | 30% inhibition at 10μM |
| 2-151 | 0.024 | < 0.010 |
| 2-152 | 0.016 | < 0.010 |
| 2-153 | 0.016 | < 0.010 |
| 2-154 | 0.021 | 0.017 |
| 2-155 | < 0.010 | < 0.010 |
| 2-156 | 0.013 | < 0.010 |
| 2-157 | 0.017 | < 0.010 |
| 2-158 | 0.016 | < 0.010 |
| 2-159 | 0.015 | < 0.010 |
| 2-160 | 0.011 | < 0.010 |

Table 2-14

| | | |
|---|---|---|
| 2-161 | 0.014 | < 0.010 |
| 2-162 | 0.021 | < 0.010 |
| 2-163 | < 0.010 | < 0.010 |
| 2-164 | < 0.010 | < 0.010 |
| 2-165 | < 0.010 | < 0.010 |
| 2-166 | 0.011 | < 0.010 |
| 2-167 | 0.011 | < 0.010 |
| 2-168 | < 0.010 | < 0.010 |
| 2-169 | 0.045 | 0.100 |
| 2-170 | 0.009 | 0.002 |
| 2-171 | 12% inhibition at 10μM | 23% inhibition at 10μM |
| 2-172 | 0.020 | 0.003 |

The formulation examples of the present invention include the following formulations. However, the present invention is not limited by such formulation examples.

Formulation Example 1 (Production of capsule)

| | |
|---|---|
| 1) Compound of Example 1 | 30 mg |
| 2) Microcrystalline cellulose | 10 mg |
| 3) Lactose | 19 mg |
| 4) Magnesium stearate | 1 mg |

1), 2), 3) and 4) are mixed and filled in a gelatin capsule.

Formulation Example 2 (Production of tablet)

| | |
|---|---|
| 1) Compound of Example 1 | 10 g |
| 2) Lactose | 50 g |
| 3) Corn starch | 15 g |
| 4) Carmellose calcium | 44 g |
| 5) Magnesium stearate | 1 g |

The total amount of 1), 2), 3) and 30 g of 4) are kneaded with water, vacuum dried and then granulated. The granulated powder is mixed with 14 g of 4) and 1 g of 5), and the mixture is tableted by a tableting machine. In this way, 1000 tablets containing 10 mg of the compound of Example 1 per tablet are obtained.

## Industrial Applicability

Since Compound [I] or Compound [Ia] or a pharmaceutically acceptable salt thereof of the present invention has a PLD inhibitory activity, it may be useful for the treatment or prophylaxis of thrombosis and cancer.

## Claims

1. A compound represented by Formula [Ia] or a pharmaceutically acceptable salt thereof:

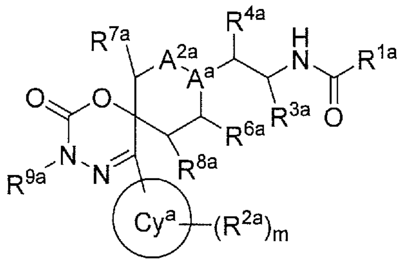

[ Ia ]

wherein

$A^a$ is $CR^{10a}$ or N;
$A^{2a}$ is $CR^{5a}$ or 0;
$Cy^a$ is

(1) $C_{6-10}$ aryl,
(2) 5 to 10-membered heteroaryl containing one or two nitrogen atoms as a ring constituting atom, besides carbon atom, or
(3) a 9- or 10-membered partially unsaturated fused cyclic group containing one or two oxygen atoms as a ring constituting atom, besides carbon atom;

$R^{1a}$ is

(1) $C_{1-6}$ alkyl wherein the alkyl is optionally substituted by

(a) hydroxy,
(b) cyano,
(c) $SO_2R^{11}$ wherein $R^{11}$ is $C_{1-4}$ alkyl, or
(d) $NHCOR^{12}$ wherein $R^{12}$ is $C_{1-4}$ alkyl,

(2) $C_{2-4}$ alkenyl wherein the alkenyl is optionally substituted by 1 to 3 of

(a) $NR^{13}R^{14}$ wherein $R^{13}$ and $R^{14}$ are each independently hydrogen or $C_{1-4}$ alkyl,
(b) halogen,
(c) $COR^{35a}$ wherein $R^{35a}$ is hydroxy or $C_{1-4}$ alkoxy,
(d) $CONR^{36a}R^{37a}$ wherein $R^{36a}$ and $R^{37a}$ are each independently hydrogen or $C_{1-4}$ alkyl, or
(e) a partial structural formula:

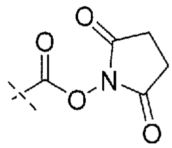
,

(3) $C_{1-4}$ haloalkyl wherein the haloalkyl is optionally substituted by hydroxy,
(4) $C_{1-6}$ alkoxy wherein the alkoxy is optionally substituted by phenyl,
(5) $NR^{15}R^{16}$ wherein $R^{15}$ and $R^{16}$ are each independently

(a) hydrogen,
(b) $C_{1-4}$ alkyl wherein the alkyl is optionally substituted by

(i) phenyl wherein the phenyl is optionally substituted by halogen, or
(ii) pyridyl,

(c) $C_{1-4}$ alkoxy, or
(d) $C_{3-4}$ cycloalkyl,

(6) $COR^{17a}$ wherein $R^{17a}$ is $C_{1-4}$ alkyl or hydroxy,
(7) $CONR^{18a}R^{19a}$ wherein $R^{18a}$ and $R^{19a}$ are each independently

(a) hydrogen,
(b) $C_{1-4}$ alkyl wherein the alkyl is optionally substituted by hydroxy,
(c) $C_{3-4}$ cycloalkyl,
(d) $C_{5-8}$ bridged cycloalkyl,
(e) $C_{1-4}$ haloalkyl, or
(f) $C_{1-4}$ alkoxy,

(8) $C_{3-4}$ cycloalkyl wherein the cycloalkyl is optionally substituted by

(a) hydroxy,
(b) halogen, or
(c) phenyl,

(9) 4 to 7-membered heterocycloalkyl containing one or two heteroatoms selected from the group consisting of nitrogen and oxygen atoms as a ring constituting atom, besides carbon atom, wherein the heterocycloalkyl is optionally substituted by

(a) hydroxy,
(b) oxo,
(c) $NR^{20}R^{21}$ wherein $R^{20}$ and $R^{21}$ are each independently hydrogen or $C_{1-4}$ alkyl, or
(d) phenyl,

(10) 6 to 11-membered spiro heterocycloalkyl containing one or two heteroatoms selected from the group consisting of nitrogen and oxygen atoms as a ring constituting atom, besides carbon atom,
(11) phenyl wherein the phenyl is optionally substituted by one or two of

(a) halogen, or
(b) $C_{1-4}$ haloalkyl,

(12) 5 to 10-membered heteroaryl containing 1 to 3 heteroatoms selected from the group consisting of nitrogen and oxygen atoms as a ring constituting atom, besides carbon atom, wherein the heteroaryl is optionally substituted by one or two $R^{22a}$,
(13) a 8 to 10-membered saturated or partially unsaturated fused cyclic group containing 1 to 4 heteroatoms selected from the group consisting of nitrogen and oxygen atoms as a ring constituting atom, besides carbon atom, wherein the fused cyclic group is optionally substituted by $C_{1-4}$ haloalkyl, or
(14) a 5 to 7-membered partially unsaturated cyclic group containing one or two nitrogen atoms as a ring constituting atom, besides carbon atom, wherein the partially unsaturated cyclic group is optionally substituted by an oxo group and $C_{1-4}$ alkyl;

$R^{2a}$ in the number of m are each independently

(1) hydroxy,
(2) cyano,
(3) halogen,
(4) $C_{1-6}$ alkyl wherein the alkyl is optionally substituted by

(a) hydroxy, or
(b) $C_{3-4}$ cycloalkyl,

(5) $C_{2-4}$ alkenyl wherein the alkenyl is optionally substituted by $C_{1-4}$ alkoxy,
(6) $C_{1-4}$ haloalkyl,
(7) $C_{1-4}$ alkoxy wherein the alkoxy is optionally substituted by 1 to 3 substituents selected from the group consisting of

(a) hydroxy, and
(b) halogen,

(8) $SR^{23a}$ wherein $R^{23a}$ is $C_{1-4}$ alkyl or $C_{1-4}$ haloalkyl,
(9) $COR^{24a}$ wherein $R^{24a}$ is

(a) hydroxy,
(b) $C_{1-4}$ alkyl, or
(c) $C_{1-4}$ alkoxy,

(10) $CONR^{25a}R^{26a}$ wherein $R^{25a}$ and $R^{26a}$ are each independently

(a) hydrogen,
(b) $C_{1-6}$ alkyl, or
(c) $C_{3-4}$ cycloalkyl, or
$R^{25a}$ and $R^{26a}$ are bonded to each other to form 4 to 7-membered heterocycloalkyl together with the nitrogen atom to which they are bonded, wherein the heterocycloalkyl contains one or two heteroatoms selected from the group consisting of nitrogen and oxygen atoms as a ring constituting atom, besides carbon atom, and is optionally substituted by one or two halogens,

(11) $SO_2R^{27}$ wherein $R^{27}$ is $C_{1-6}$ alkyl,
(12) $C_{3-4}$ cycloalkyl,
(13) 4 to 7-membered heterocycloalkyl containing one or two heteroatoms selected from the group consisting of nitrogen and oxygen atoms as a ring constituting atom, besides carbon atom, wherein the heterocycloalkyl is optionally substituted by one or two substituents selected from the group consisting of

(a) halogen,
(b) $C_{1-4}$ alkyl, and
(c) $C_{1-4}$ haloalkyl,

(14) 5 to 9-membered bridged heterocycloalkyl containing one or two heteroatoms selected from the group consisting of nitrogen and oxygen atoms as a ring constituting atom, besides carbon atom,
(15) 6 to 11-membered spiro heterocycloalkyl containing one or two heteroatoms selected from the group consisting of nitrogen and oxygen atoms as a ring constituting atom, besides carbon atom, or
(16) phenyl;

$R^{3a}$ is

(1) hydrogen,
(2) $C_{1-4}$ alkyl, or
(3) $C_{1-4}$ haloalkyl;

$R^{4a}$ is

(1) hydrogen,
(2) $C_{1-4}$ alkyl, or
(3) cyano;

$R^{5a}$ is hydrogen or $C_{1-4}$ alkyl;
the combination of $R^{6a}$, $R^{7a}$ and $R^{8a}$ is

(1) a combination where $R^{6a}$ is hydrogen or $C_{1-4}$ alkyl, and $R^{7a}$ and $R^{8a}$ are both hydrogens,
(2) a combination where $R^{6a}$ is hydrogen or $C_{1-4}$ alkyl, and $R^{7a}$ and $R^{8a}$ are bonded to each other to form a cyclopentane ring together with the spiro carbon atom and the carbon atoms to which they are bonded, or
(3) a combination where $R^{6a}$ and $R^{7a}$ are bonded to each other to form a cyclopentane ring together with the spiro carbon atom and the carbon atoms to which they are bonded, and $R^{82}$ is hydrogen;

$R^{9a}$ is

(1) hydrogen,
(2) $CONHR^{28}$ wherein $R^{28}$ is $C_{3-4}$ cycloalkyl, or
(3) $C_{1-4}$ alkyl;

$R^{10a}$ is

(1) hydrogen,
(2) hydroxy,
(3) halogen,
(4) $C_{1-4}$ alkyl,
(5) cyano, or
(6) $C_{1-4}$ alkoxy;

one or two $R^{22a}$ are each independently

(1) halogen,
(2) $C_{1-4}$ alkyl,
(3) $C_{1-4}$ haloalkyl,
(4) $C_{1-4}$ alkoxy,
(5) $NHCOR^{29}$ wherein $R^{29}$ is $C_{1-4}$ alkyl,
(6) $SO_2R^{30}$ wherein $R^{30}$ is $C_{1-4}$ alkyl,
(7) cyano, or
(8) $C_{3-4}$ cycloalkyl; and

m is 0, 1, 2 or 3.

**2.** The compound according to Claim 1 or a pharmaceutically acceptable salt thereof, which is represented by Formula [IIa]:

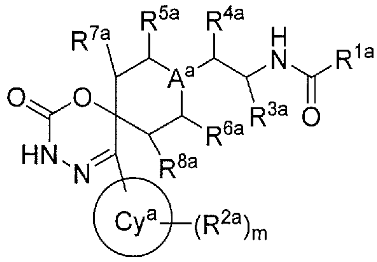

[ IIa ]

wherein
$A^a$, $Cy^a$, $R^{1a}$, $R^{2a}$, $R^{3a}$, $R^{4a}$, $R^{5a}$, $R^{6a}$, $R^{7a}$, $R^{8a}$ and m are as defined in Claim 1.

**3.** The compound according to Claim 1 or a pharmaceutically acceptable salt thereof, which is represented by Formula [IIIa]:

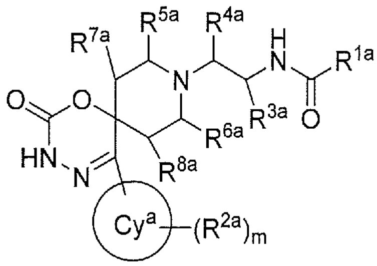

[ IIIa ]

wherein
$Cy^a$, $R^{1a}$, $R^{2a}$, $R^{3a}$, $R^{4a}$, R5a, $R^{6a}$, $R^{7a}$, $R^{8a}$ and m are as defined in Claim 1.

**4.** The compound according to Claim 1 or a pharmaceutically acceptable salt thereof, which is represented by Formula [IVa]:

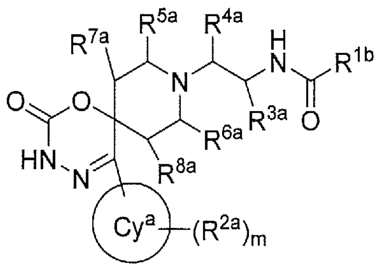

[ IVa ]

wherein

$R^{1b}$ is

(1) $C_{1-6}$ alkyl wherein the alkyl is optionally substituted by

(a) hydroxy,

(b) cyano,
(c) $SO_2R^{11}$ wherein $R^{11}$ is $C_{1-4}$ alkyl, or
(d) $NHCOR^{12}$ wherein $R^{12}$ is $C_{1-4}$ alkyl,

(2) $C_{2-4}$ alkenyl wherein the alkenyl is optionally substituted by 1 to 3 of

(a) $NR^{13}R^{14}$ wherein $R^{13}$ and $R^{14}$ are each independently hydrogen or $C_{1-4}$ alkyl,
(b) halogen,
(c) $COR^{35a}$ wherein $R^{35a}$ is hydroxy or $C_{1-4}$ alkoxy,
(d) $CONR^{36a}R^{37a}$ wherein $R^{36a}$ and $R^{37a}$ are each independently hydrogen or $C_{1-4}$ alkyl, or
(e) a partial structural formula:

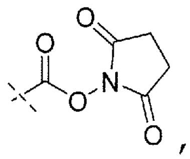

(3) $C_{1-6}$ alkoxy wherein the alkoxy is optionally substituted by phenyl,
(4) $NR^{15}R^{16}$ wherein $R^{15}$ and $R^{16}$ are each independently

(a) hydrogen,
(b) $C_{1-4}$ alkyl wherein the alkyl is optionally substituted by

(i) phenyl wherein the phenyl is optionally substituted by halogen, or
(ii) pyridyl,

(c) $C_{1-4}$ alkoxy, or
(d) $C_{3-4}$ cycloalkyl,

(5) $CONR^{18a}R^{19a}$ wherein $R^{18a}$ and $R^{19a}$ are each independently

(a) hydrogen,
(b) $C_{1-4}$ alkyl wherein the alkyl is optionally substituted by hydroxy,
(c) $C_{3-4}$ cycloalkyl,
(d) $C_{5-8}$ bridged cycloalkyl,
(e) $C_{1-4}$ haloalkyl, or
(f) $C_{1-4}$ alkoxy,

(6) 6 to 11-membered spiro heterocycloalkyl containing one or two heteroatoms selected from the group consisting of nitrogen and oxygen atoms as a ring constituting atom, besides carbon atom,
(7) 5 to 10-membered heteroaryl containing 1 to 3 heteroatoms selected from the group consisting of nitrogen and oxygen atoms as a ring constituting atom, besides carbon atom, wherein the heteroaryl is optionally substituted by one or two $R^{22a}$,
(8) a 8 to 10-membered saturated or partially unsaturated fused cyclic group containing 1 to 4 heteroatoms selected from the group consisting of nitrogen and oxygen atoms as a ring constituting atom, besides carbon atom, wherein the fused cyclic group is optionally substituted by $C_{1-4}$ haloalkyl, or
(9) a 5 to 7-membered partially unsaturated cyclic group containing one or two nitrogen atoms as a ring constituting atom, besides carbon atom, wherein the partially unsaturated cyclic group is optionally substituted by an oxo group and $C_{1-4}$ alkyl; and

$Cy^a$, $R^{2a}$, $R^{3a}$, $R^{4a}$, $R^{5a}$, $R^{6a}$, $R^{7a}$, $R^{8a}$, $R^{22a}$ and m are as defined in Claim 1.

**5.** The compound according to Claim 4 or a pharmaceutically acceptable salt thereof, which is represented by Formula [Va]:

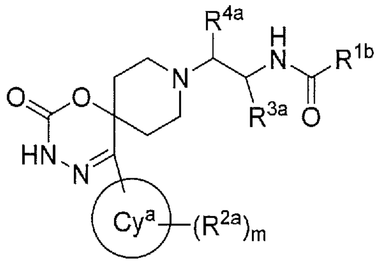

[ Va ]

wherein

$Cy^a$, $R^{2a}$, $R^{3a}$, $R^{4a}$ and m are as defined in Claim 1; and
$R^{1b}$ is as defined in Claim 4.

**6.** The compound according to Claim 4 or a pharmaceutically acceptable salt thereof, which is represented by Formula [VIa]:

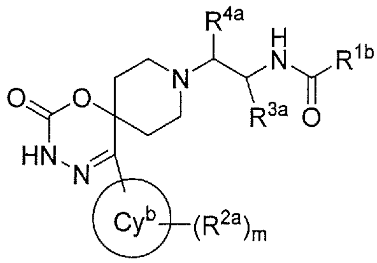

[ VIa ]

wherein

$Cy^b$ is

(1) $C_{6-10}$ aryl, or
(2) 5- or 6-membered heteroaryl containing one or two nitrogen atoms as a ring constituting atom, besides carbon atom;

$R^{1b}$ is as defined in Claim 4; and
$R^{2a}$, $R^{3a}$, $R^{4a}$ and m are as defined in Claim 1.

**7.** The compound according to Claim 4 or a pharmaceutically acceptable salt thereof, which is represented by Formula [VIIa]:

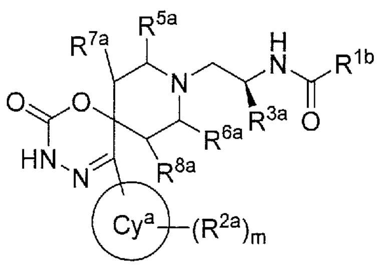

[ VIIa ]

wherein

$Cy^a$, $R^{2a}$, $R^{3a}$, $R^{5a}$, $R^{6a}$, $R^{7a}$, $R^{82}$ and m are as defined in Claim 1; and
$R^{1b}$ is as defined in Claim 4.

**8.** The compound according to Claim 4 or a pharmaceutically acceptable salt thereof, which is represented by Formula [VIIIa]:

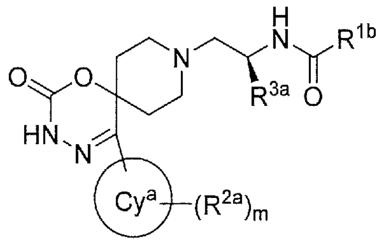

[ VIIIa ]

wherein

$Cy^a$, $R^{2a}$, $R^{3a}$ and m are as defined in Claim 1; and
$R^{1b}$ is as defined in Claim 4.

**9.** The compound according to Claim 6 or a pharmaceutically acceptable salt thereof, which is represented by Formula [IXa]:

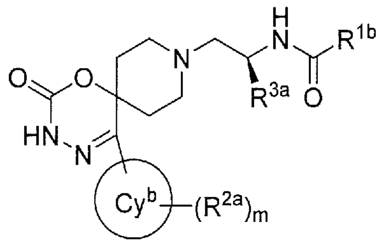

[ IXa ]

wherein

$Cy^b$ is as defined in Claim 6;
$R^{1b}$ is as defined in Claim 4; and
$R^{2a}$, $R^{3a}$ and m are as defined in Claim 1.

**10.** The compound according to Claim 1 or a pharmaceutically acceptable salt thereof, which is represented by Formula [Xa]:

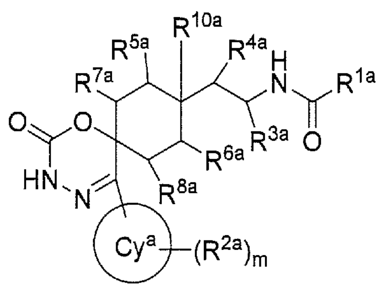

[ Xa ]

wherein

$Cy^a$, $R^{1a}$, $R^{2a}$, $R^{3a}$, $R^{4a}$, $R^{5a}$, $R^{6a}$, $R^{7a}$, $R^{8a}$, $R^{10a}$ and m are as defined in Claim 1.

**11.** The compound according to Claim 1 or a pharmaceutically acceptable salt thereof, which is represented by Formula [XIa]:

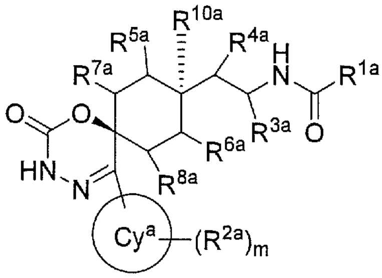

[ XIa ]

wherein
$Cy^a$, $R^{1a}$, $R^{2a}$, R3a, R4a, R5a, $R^{6a}$, $R^{7a}$, $R^{8a}$, $R^{10a}$ and m are as defined in Claim 1.

**12.** The compound according to Claim 1 or a pharmaceutically acceptable salt thereof, which is represented by Formula [XIIa]:

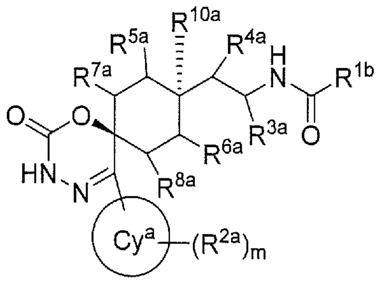

[ XIIa ]

wherein

$R^{1b}$ is

(1) $C_{1-6}$ alkyl wherein the alkyl is optionally substituted by

(a) hydroxy,
(b) cyano,
(c) $SO_2R^{11}$ wherein $R^{11}$ is $C_{1-4}$ alkyl, or
(d) $NHCOR^{12}$ wherein $R^{12}$ is $C_{1-4}$ alkyl,

(2) $C_{2-4}$ alkenyl wherein the alkenyl is optionally substituted by 1 to 3 of

(a) $NR^{13}R^{14}$ wherein $R^{13}$ and $R^{14}$ are each independently hydrogen or $C_{1-4}$ alkyl,
(b) halogen,
(c) $COR^{35a}$ wherein $R^{35a}$ is hydroxy or $C_{1-4}$ alkoxy,
(d) $CONR^{36a}R^{37a}$ wherein $R^{36a}$ and $R^{37a}$ are each independently hydrogen or $C_{1-4}$ alkyl, or
(e) a partial structural formula:

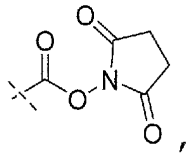

(3) $C_{1-6}$ alkoxy wherein the alkoxy is optionally substituted by phenyl,
(4) $NR^{15}R^{16}$ wherein $R^{15}$ and $R^{16}$ are each independently

(a) hydrogen,
(b) $C_{1-4}$ alkyl wherein the alkyl is optionally substituted by

(i) phenyl wherein the phenyl is optionally substituted by halogen, or
(ii) pyridyl,

(c) $C_{1-4}$ alkoxy, or
(d) $C_{3-4}$ cycloalkyl,

(5) $CONR^{18a}R^{19a}$ wherein $R^{18a}$ and $R^{19a}$ are each independently

(a) hydrogen,
(b) $C_{1-4}$ alkyl wherein the alkyl is optionally substituted by hydroxy,
(c) $C_{3-4}$ cycloalkyl,
(d) $C_{5-8}$ bridged cycloalkyl,
(e) $C_{1-4}$ haloalkyl, or
(f) $C_{1-4}$ alkoxy,

(6) 6 to 11-membered spiro heterocycloalkyl containing one or two heteroatoms selected from the group consisting of nitrogen and oxygen atoms as a ring constituting atom, besides carbon atom,
(7) 5 to 10-membered heteroaryl containing 1 to 3 heteroatoms selected from the group consisting of nitrogen and oxygen atoms as a ring constituting atom, besides carbon atom, wherein the heteroaryl is optionally substituted by one or two $R^{22a}$,
(8) a 8 to 10-membered saturated or partially unsaturated fused cyclic group containing 1 to 4 heteroatoms selected from the group consisting of nitrogen and oxygen atoms as a ring constituting atom, besides carbon atom, wherein the fused cyclic group is optionally substituted by $C_{1-4}$ haloalkyl, or
(9) a 5 to 7-membered partially unsaturated cyclic group containing one or two nitrogen atoms as a ring constituting atom, besides carbon atom, wherein the partially unsaturated cyclic group is optionally substituted by an oxo group and $C_{1-4}$ alkyl; and

$Cy^a$, $R^{2a}$, $R^{3a}$, $R^{4a}$, $R^{5a}$, $R^{6a}$, $R^{7a}$, $R^{8a}$, $R^{10a}$, $R^{22a}$ and m are as defined in Claim 1.

**13.** The compound according to Claim 12 or a pharmaceutically acceptable salt thereof, which is represented by Formula [XIIIa]:

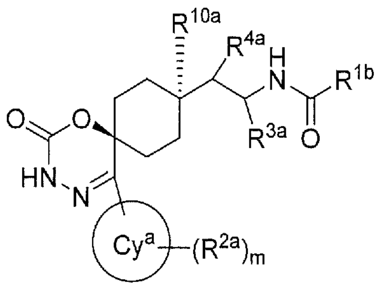

[ XIIIa ]

wherein

$Cy^a$, $R^{2a}$, $R^{3a}$, $R^{4a}$, $R^{10a}$ and m are as defined in Claim 1; and $R^{1b}$ is as defined in Claim 12.

**14.** The compound according to Claim 12 or a pharmaceutically acceptable salt thereof, which is represented by Formula [XIVa]:

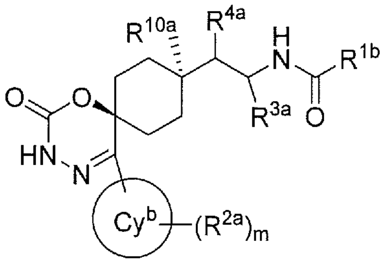

[ XIVa ]

wherein

$Cy^b$ is

(1) $C_{6-10}$ aryl, or
(2) 5- or 6-membered heteroaryl containing one or two nitrogen atoms as a ring constituting atom, besides carbon atom;

$R^{1b}$ is as defined in Claim 12; and
$R^{2a}$, $R^{3a}$, $R^{4a}$, $R^{10a}$ and m are as defined in Claim 1.

**15.** The compound according to Claim 12 or a pharmaceutically acceptable salt thereof, which is represented by Formula [XVa]:

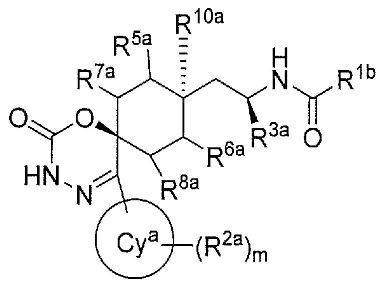

[ XVa ]

wherein

$Cy^a$, $R^{2a}$, $R^{3a}$, $R^{5a}$, $R^{6a}$, $R^{7a}$, $R^{8a}$, $R^{10a}$ and m are as defined in Claim 1; and
$R^{1b}$ is as defined in Claim 12.

**16.** The compound according to Claim 12 or a pharmaceutically acceptable salt thereof, which is represented by Formula [XVIa]:

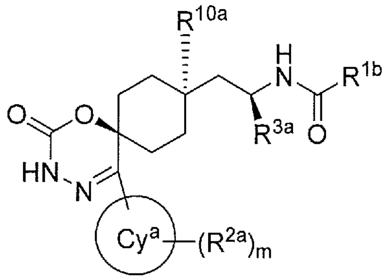

[ XVIa ]

wherein

$Cy^a$, $R^{2a}$, $R^{3a}$, $R^{10a}$ and m are as defined in Claim 1; and
$R^{1b}$ is as defined in Claim 12.

**17.** The compound according to Claim 14 or a pharmaceutically acceptable salt thereof, which is represented by Formula [XVIIa]:

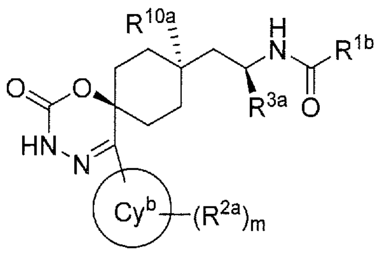

[ XVIIa ]

wherein

$Cy^b$ is as defined in Claim 14;
$R^{1b}$ is as defined in Claim 12; and
$R^{2a}$, $R^{3a}$, $R^{10a}$ and m are as defined in Claim 1.

**18.** A compound selected from the group consisting of compounds represented by the following formulae:

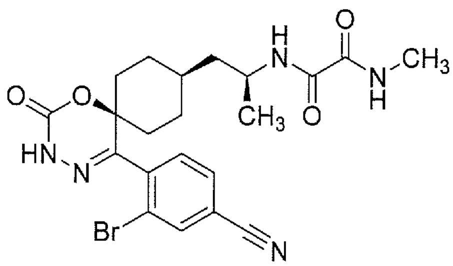

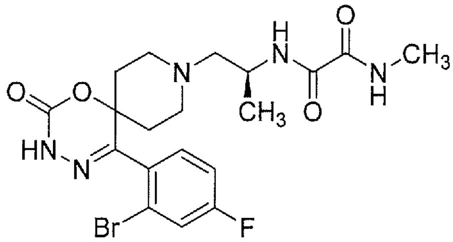

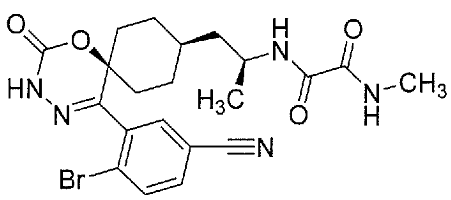

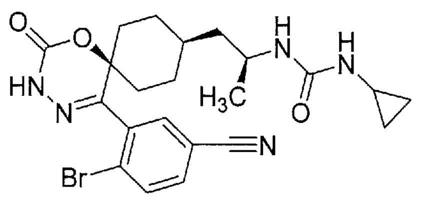

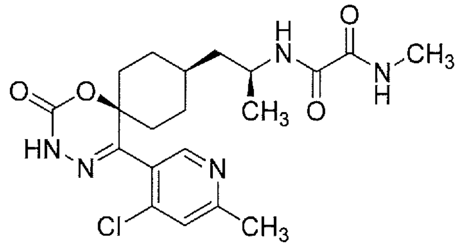

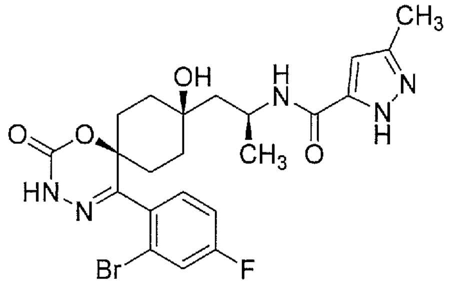

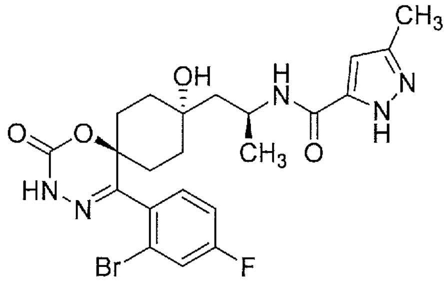

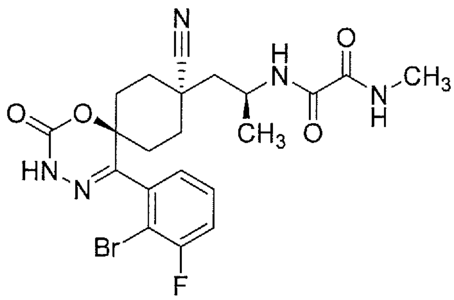

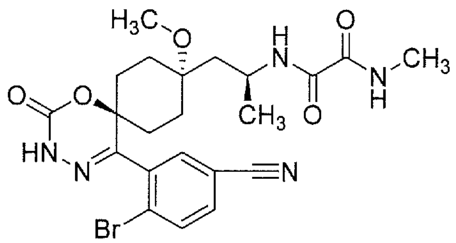

and

or a pharmaceutically acceptable salt thereof.

**19.** A pharmaceutical composition comprising a compound as defined in any one of Claims 1 to 18, or a pharmaceutically acceptable salt thereof, and a pharmaceutically acceptable carrier.

**20.** A pharmaceutical composition comprising a compound as defined in any one of Claims 1 to 18, or a pharmaceutically acceptable salt thereof, in combination with one or more other drugs, and a pharmaceutically acceptable carrier.

**21.** A PLD inhibitor comprising a compound as defined in any one of Claims 1 to 18, or a pharmaceutically acceptable salt thereof.

**22.** A PLD1 inhibitor comprising a compound as defined in any one of Claims 1 to 18, or a pharmaceutically acceptable salt thereof.

**23.** A PLD1/2 inhibitor comprising a compound as defined in any one of Claims 1 to 18, or a pharmaceutically acceptable salt thereof.

**24.** An agent for the treatment or prophylaxis of a disease selected from the group consisting of thrombosis and cancer, comprising a compound as defined in any one of Claims 1 to 18, or a pharmaceutically acceptable salt thereof.

**25.** An agent for the treatment or prophylaxis of a disease selected from the group consisting of thrombosis and cancer, comprising a compound as defined in any one of Claims 1 to 18, or a pharmaceutically acceptable salt thereof, in combination with one or more other drugs.

**26.** A method for inhibiting PLD in a mammal, comprising administering a therapeutically effective amount of a compound as defined in any one of Claims 1 to 18, or a pharmaceutically acceptable salt thereof to the mammal.

**27.** A method for inhibiting PLD1 in a mammal, comprising administering a therapeutically effective amount of a

compound as defined in any one of Claims 1 to 18, or a pharmaceutically acceptable salt thereof to the mammal.

**28.** A method for inhibiting PLD1/2 in a mammal, comprising administering a therapeutically effective amount of a compound as defined in any one of Claims 1 to 18, or a pharmaceutically acceptable salt thereof to the mammal.

**29.** A method for treating or preventing a disease selected from the group consisting of thrombosis and cancer in a mammal, comprising administering a therapeutically effective amount of a compound as defined in any one of Claims 1 to 18, or a pharmaceutically acceptable salt thereof to the mammal.

**30.** A method for treating or preventing a disease selected from the group consisting of thrombosis and cancer in a mammal, comprising administering a therapeutically effective amount of a compound as defined in any one of Claims 1 to 18, or a pharmaceutically acceptable salt thereof, in combination with one or more other drugs, to the mammal.

**31.** Use of a compound as defined in any one of Claims 1 to 18, or a pharmaceutically acceptable salt thereof, for the manufacture of a PLD inhibitor.

**32.** Use of a compound as defined in any one of Claims 1 to 18, or a pharmaceutically acceptable salt thereof, for the manufacture of a PLD1 inhibitor.

**33.** Use of a compound as defined in any one of Claims 1 to 18, or a pharmaceutically acceptable salt thereof, for the manufacture of a PLD1/2 inhibitor.

**34.** Use of a compound as defined in any one of Claims 1 to 18, or a pharmaceutically acceptable salt thereof, for the manufacture of an agent for the treatment or prophylaxis of a disease selected from the group consisting of thrombosis and cancer.

**35.** Use of a compound as defined in any one of Claims 1 to 18, or a pharmaceutically acceptable salt thereof, in combination with one or more other drugs, for the manufacture of an agent for the treatment or prophylaxis of a disease selected from the group consisting of thrombosis and cancer.

**36.** A compound as defined in any one of Claims 1 to 18, or a pharmaceutically acceptable salt thereof, for use in the inhibition of PLD.

**37.** A compound as defined in any one of Claims 1 to 18, or a pharmaceutically acceptable salt thereof, for use in the inhibition of PLD1.

**38.** A compound as defined in any one of Claims 1 to 18, or a pharmaceutically acceptable salt thereof, for use in the inhibition of PLD1/2.

**39.** A compound as defined in any one of Claims 1 to 18, or a pharmaceutically acceptable salt thereof, for use in the treatment or prophylaxis of a disease selected from the group consisting of thrombosis and cancer.

**40.** A compound as defined in any one of Claims 1 to 18, or a pharmaceutically acceptable salt thereof, in combination with one or more other drugs, for use in the treatment or prophylaxis of a disease selected from the group consisting of thrombosis and cancer.

## INTERNATIONAL SEARCH REPORT

International application No.
**PCT/JP2023/022253**

**A. CLASSIFICATION OF SUBJECT MATTER**

***C07D 273/04***(2006.01)i; ***A61K 31/5395***(2006.01)i; ***A61K 31/55***(2006.01)i; ***A61P 7/02***(2006.01)i; ***A61P 35/00***(2006.01)i; ***A61P 43/00***(2006.01)i; ***C07D 413/04***(2006.01)i; ***C07D 413/12***(2006.01)i; ***C07D 413/14***(2006.01)i; ***C07D 471/04***(2006.01)i; ***C07D 487/04***(2006.01)i; ***C07D 491/08***(2006.01)i; ***C07D 491/107***(2006.01)i; ***C07D 498/04***(2006.01)i; ***C07D 498/10***(2006.01)i

FI: C07D273/04 CSP; A61P43/00 111; A61P35/00; A61P7/02; A61K31/5395; C07D498/10; C07D413/04; C07D413/12; C07D487/04 145; C07D471/04 112; C07D498/04 101; C07D471/04 106A; C07D471/04 104A; C07D471/04 108A; C07D487/04 141; C07D413/14; C07D491/08; A61K31/55; C07D491/107

According to International Patent Classification (IPC) or to both national classification and IPC

**B. FIELDS SEARCHED**

Minimum documentation searched (classification system followed by classification symbols)

C07D273/04; A61K31/5395; A61K31/55; A61P7/02; A61P35/00; A61P43/00; C07D413/04; C07D413/12; C07D413/14; C07D471/04; C07D487/04; C07D491/08; C07D491/107; C07D498/04; C07D498/10

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| A | METRICK, Claire M. et al., Human PLD structures enable drug design and characterization of isoenzyme selectivity, Nature Chemical Biology, 2020, 16(4), 391-399, DOI: 10.1038/s41589-019-0458-4<br>in particular, p. 395 | 1-40 |
| A | JP 2013-500260 A (VANDERBILT UNIVERSITY) 07 January 2013 (2013-01-07) | 1-40 |
| A | JP 2012-528201 A (THE TRUSTEES OF COLUMBIA UNIVERSITY IN THE CITY OF NEW YORK) 12 November 2012 (2012-11-12) | 1-40 |
| A | LAVIERI, Robert et al., Design and synthesis of isoform-selective phospholipase D (PLD) inhibitors. Part II. Identification of the 1,3,8-triazaspiro[4,5]decan-4-one privileged structure that engenders PLD2 selectivity, Bioorganic & Medicinal Chemistry Letters, 2009, 19(8), 2240-2243, DOI: 10.1016/j.bmcl.2009.02.125<br>entire text | 1-40 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **27 July 2023** | **08 August 2023** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

**INTERNATIONAL SEARCH REPORT**
**Information on patent family members**

International application No.
**PCT/JP2023/022253**

| Patent document cited in search report | Publication date (day/month/year) | Patent family member(s) | Publication date (day/month/year) |
|---|---|---|---|
| JP 2013-500260 A | 07 January 2013 | US 2012/0214832 A1 | |
| | | WO 2011/011680 A1 | |
| | | EP 2456307 A1 | |
| | | CA 2768940 A | |
| | | AU 2010275526 A | |
| | | SG 178102 A | |
| | | IL 217720 A | |
| | | CN 102573474 A | |
| | | KR 10-2012-0090034 A | |
| | | MX 2012001064 A | |
| | | RU 2012106657 A | |
| | | BR 112012001586 A | |
| JP 2012-528201 A | 12 November 2012 | US 2012/0178719 A1 | |
| | | WO 2010/138869 A1 | |
| | | EP 2435563 A1 | |
| | | CA 2764038 A | |
| | | CN 102459584 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- *Cell*, 26 January 1996, vol. 84 (2), 289-297
- *J Thromb Haemost.*, 2014, vol. 12 (3), 418-420
- *Nat Med.*, June 2009, vol. 15 (6), 665-673
- *The Journal of Japanese College of Angiology*, 2011, vol. 51 (3), 275-281
- *Thromb Haemost.*, June 2010, vol. 103 (6), 1128-1135
- *Chem Rev.*, 12 October 2011, vol. 111 (10), 6064-119
- *Sci Signal.*, 05 January 2010, vol. 3 (103), ra1
- *J Thromb Haemost.*, November 2012, vol. 10 (11), 2361-72
- *J Biol Chem.*, 15 August 2014, vol. 289 (33), 22567-22574
- *J Biol Chem.*, 15 August 2014, vol. 289 (33), 22557-22566
- *J Biol Chem.*, 2014, vol. 289 (33), 22575-22582
- *Pharmacol Rev*, October 2014, vol. 66 (4), 1033-1079
- *Nat Rev Drug Discov*, May 2017, vol. 16 (5), 351-367
- *Adv Biol Regul.*, January 2018, vol. 67, 134-140
- **BERGE et al.** *J. Pharm. Sci.*, 1977, vol. 66, 1-19
- **STAHL et al.** Handbook of Pharmaceutical Salt: Properties, Selection, and Use. Wiley-VCH, Weinheim, 2002
- **PAULEKUHN et al.** *J. Med. Chem*, 2007, vol. 50, 6665-6672